(19) Europäisches Patentamt
European Patent Office
Office européen des brevets

(11) EP 3 666 770 A1

(12) EUROPEAN PATENT APPLICATION
published in accordance with Art. 153(4) EPC

(43) Date of publication:
**17.06.2020 Bulletin 2020/25**

(21) Application number: **18843992.1**

(22) Date of filing: **30.07.2018**

(51) Int Cl.:
*C07D 401/14* (2006.01)
*C07D 403/04* (2006.01)
*C07D 471/04* (2006.01)
*C07D 403/12* (2006.01)
*A61K 31/4184* (2006.01)
*A61K 31/437* (2006.01)
*A61K 31/4709* (2006.01)
*A61K 31/4375* (2006.01)
*A61P 35/00* (2006.01)

(86) International application number:
**PCT/RU2018/050089**

(87) International publication number:
**WO 2019/031990 (14.02.2019 Gazette 2019/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.08.2017 RU 2017128123**
**06.03.2018 RU 2018108178**

(71) Applicant: **Joint Stock Company "Biocad"**
**St.Petersburg 198515 (RU)**

(72) Inventors:
- **MINDICH, Aleksei Leonidovich**
  **St.Petersburg 195426 (RU)**
- **GORBUNOVA, Svetlana Leonidovna**
  **St.Petersburg 197198 (RU)**
- **POPKOVA, Aleksandra Vladimirovna**
  **Lomonosov District**
  **Leningradskaya obl. 188516 (RU)**
- **SHEKHAUTSOU, Artsiom Evgenievich**
  **g. Byhov 213320 (BY)**
- **ALAFINOV, Andrei Ivanovich**
  **Leningradskaja obl.**
  **g. Vsevolozhsk 188641 (RU)**
- **ALESHUNIN, Pavel Aleksandrovich**
  **St.Petersburg 197136 (RU)**
- **EVDOKIMOV, Anton Aleksandrovich**
  **St.Petersburg 191024 (RU)**
- **ZAVIALOV, Kirill Vadimovich**
  **St.Petersburg 198261 (RU)**
- **KASATKINA, Mariia Andreevna**
  **g. Krasnodar 350040 (RU)**
- **KOZHEMYAKINA, Natalia Vladimirovna**
  **St.Petersburg 192238 (RU)**
- **KUSHAKOVA, Anna Sergeevna**
  **Kirishi**
  **Leningradskaya obl. 187110 (RU)**
- **MAKSIMENKO, Elena Aleksandrovna**
  **St.Petersburg 192283 (RU)**
- **MISHINA, Mariia Sergeevna**
  **St.Petersburg 195279 (RU)**
- **REKHARSKY, Mikhail Vladimirovich**
  **Moscow 117534 (RU)**
- **CHESTNOVA, Anna Jur'evna**
  **Leningradskaja obl.**
  **g. Gatchina 188307 (RU)**
- **IAKOVLEV, Pavel Andreevich**
  **St.Petersburg 196135 (RU)**
- **MOROZOV, Dmitry Valentinovich**
  **St. Petersburg, 190000 (RU)**

(74) Representative: **Held, Stephan**
**Meissner Bolte Patentanwälte**
**Rechtsanwälte Partnerschaft mbB**
**Widenmayerstraße 47**
**80538 München (DE)**

# (54) NOVEL HETEROCYCLIC COMPOUNDS AS CDK8/19 INHIBITORS

(57) The present invention relates to novel compounds of formula (I):

EP 3 666 770 A1

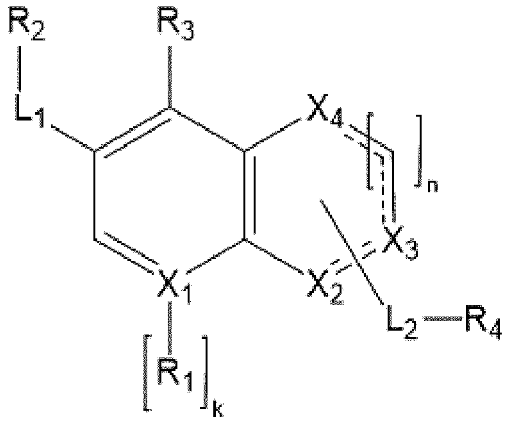

I,

or a pharmaceutically acceptable salt or stereoisomer thereof, which exhibit the properties of a CDK 8/19 inhibitor. The invention also relates to a pharmaceutical composition containing said compounds and to the use thereof as pharmaceutical drugs for treating diseases or disorders.

## Description

### Field of invention

The present invention relates to novel CDK8/19 inhibitors, methods for their preparations, pharmaceutical compositions comprising the present compounds and methods of using said compounds or said compositions in the treatments of diseases and disorders.

### Background of the invention

CDK8, along with its closely related isoform, in terms of structure and function, CDK19, is an oncogenic transcription regulating kinase (Xu, W. & JI, J. Y. (2011) Dysregulation of CDK8 and Cyclin C in tumorigenesis, J. Genet. Genomics 38, 439-452; Galbraith, M. D., et al. (2010) CDK8: a positive regulator of transcription, Transcription. 1, 4-12; Firestein, R. & Hahn, W. C. (2009) Revving the Throttle on an oncogene: CDK8 takes the driver seat, Cancer Res 69, 7899-7901). In contrast to better-known members of the CDK family (such as CDK1, CDK2, and CDK4/6), CDK8 plays no role in cell cycle regulation, but CDK8 knockout in embryonic stem cells prevents embryonic development (Adler, A. S., et al. (2012) CDK8 maintains tumor de-differentiation and embryonic stem cell pluripotency, Cancer Res. 72, 2129-2139) due to its essential role in the formation of the pluripotent stem cell phenotype (Firestein, R., et al. (2008) CDK8 is a colorectal cancer oncogene that regulates beta-catenin activity, Nature 455, 547-551). It should be noted that the inhibition of CDK8 does not suppress the growth of normal cells (Adler, A. S., et al. (2012) CDK8 maintains tumor de-differentiation and embryonic stem cell pluripotency, Cancer Res. 72, 2129-2139, Kapoor, A., et al. (2010) The histone variant macroH2A suppresses melanoma progression through regulation of CDK8, Nature 468, 1105-1109). The role of CDK8 in carcinogenesis is due to its unique function as a regulator of several transcriptional programs (Xu, W. & JI, J. Y. (2011) Dysregulation of CDK8 and Cyclin C in tumorigenesis, J. Genet. Genomics 38, 439-452). High expression of CDK8 has been identified in colon cancer (Firestein, R., et al. (2010) CDK8 expression in 470 colorectal cancers in relation to beta-catenin activation, other molecular alterations and patient survival, Int. J. Cancer 126, 2863-2873), melanoma (Kapoor, A., et al. (2010) The histone variant macroH2A suppresses melanoma progression through regulation of CDK8, Nature 468, 1105-1109), at the same time, the higher expression of CDK8 being observed in these cancer types in ~50% of cases; a similar situation is observed in relation to breast cancer as well (Broude E., et al. (2015) Expression of CDK8 and CDK8-interacting genes as potential biomarkers in breast cancer, Curr. Cancer Drug Targets, 15(8), 739-749). Higher expression of CDK8 is associated with worse prognosis in colon cancer (Gyorffy, B., et al. (2010) An online survival analysis tool to rapidly assess the effect of 22,277 genes on breast cancer prognosis using microarray data of 1,809 patients, Breast Cancer Res. Treat. 123, 725-731).

The known cancer-relevant mechanisms of CDK8 include positive regulation of Wnt/β-catenin pathway (Kapoor, A., et al. (2010) The histone variant macroH2A suppresses melanoma progression through regulation of CDK8, Nature 468, 1105-1109; Alarcon, C, et al. (2009) Nuclear CDKs drive Smad transcriptional activation and turnover in BMP and TGF-beta pathways, Cell 139, 757- 769), transcription induced by growth factor NF-kB (DiDonato, J. A., et al. (2012) NF-kappaB and the link between inflammation and cancer, Immunol. Rev. 246, 379-400) and TGFβ signaling pathway (Acharyya, S., et al. (2012) A CXCL1 paracrine network links cancer chemoresistance and metastasis, Cell 150, 165-178). It was also demonstrated that CDK8 can maintain the pluripotent phenotype of embryonic stem cells and can be associated with the cancer stem cell phenotype (Firestein, R., et al. (2008) CDK8 is a colorectal cancer oncogene that regulates beta-catenin activity, Nature 455, 547-551). Chemotherapy drugs that cause DNA damage induce TNF-a, which is an activator of the transcription factor NFKB (Fabian et al. (2005) A small molecule-kinase interaction map for clinical kinase inhibitors, Nat. Biotechnol. 23, 329-336), in endothelial cells and in other stromal elements of the tumor microenvironment. Stroma-derived TNFa acts on tumor cells, where it induces NFkB-mediated production of related tumor-promoting cytokines CXCL1 and CXCL2, stimulating survival and growth of tumor cells. CXCL 1/2 attract myeloid cells to the tumor, by binding to CXCR2 receptor on the myeloid cell surface. Myeloid cells then secrete small calcium-binding proteins S 100A8 and A9 that are associated with chronic inflammation and cancer. S 100A8/9 act on tumor cells, promoting both their metastasis and survival of chemotherapy. (Huang, et al. (2012) MED12 Controls the response to multiple cancer drugs through regulation of TGF-β receptor signaling, Cell 151, 937-950).

Currently, the search for new compounds inhibiting cyclin-dependent protein kinases CDK8/19 is relevant.

### Description of the invention

The terms used in the description of this invention appear below.

"Alkyl" means an aliphatic straight chain or branched chain hydrocarbon group having from 1 to 12 carbon atoms, more preferably from 1 to 6 carbon atoms. Branched chain means alkyl chain having one or more "lower alkyl" substituents. Examples of alkyl groups include, but are not limited to, methyl, ethyl, *n*-propyl, *iso*-propyl, *n*-butyl, *iso*-butyl,

*sec*-butyl, *tert*-butyl, *n*-pentyl, 2-pentyl, 3-pentyl, *neo*- pentyl, *n*-hexyl. Alkyl may have substituents which may be same or different structure.

"Cycloalkyl" means a saturated carbocyclic ring that contains from 3 to 10 carbon ring atoms. Examples of cycloalkyl groups include, but are not limited to, monocyclic groups, such as cyclopropyl, cyclobutyl, cyclopentyl, cyclohexyl, cycloheptyl, cyclooctyl, cyclononyl or cyclodecyl, bicyclic groups, such as bicycloheptyl or bicyclooctyl. Cycloalkyl may have substituents which may be same or different structure.

"Aryl" means an aromatic monocyclic or polycyclic system having from 6 to 14 carbon atoms, more preferably from 6 to 10 carbon atoms. Examples of aryl groups include, but are not limited to, phenyl, naphthyl, anthranil and the like. Aryl may have cyclic system substituents which may be same or different structure. Aryl can be annelated with a nonaromatic cyclic system or heterocycle.

"Alkyloxy" or "Alkoxy" means an alkyl-O- group, wherein alkyl is defined in this section. Examples of alkoxy groups include, but are not limited to, methoxy, ethoxy, *n*-propoxy, *iso*-propoxy and *n*-butoxy.

"Amino group" means R'R"N-group substituted or unsubstituted by optionally identical substituents R' and R".

"Alkyl sulfonyl" ($-S(O)_2-C_1-C_6$alkyl) means "alkyl" as defined above attached to an appropriate molecule fragment through a sulfonyl group $-SO_2-$. Examples of alkyl sulfonyls include, but are not limited to, methylsulfonyl, ethylsulfonyl, etc.

"Lower alkyl" means a straight chain or branched chain alkyl having from 1 to 4 carbon atoms.

"Halo" or "Halogen" (Hal) means fluoro, chloro, bromo and iodo.

"Heterocycle", "heterocyclyl", "heterocyclic ring" means a monocyclic or polycyclic system having from 3 to 11 carbon atoms, of which one or more carbon atoms are substituted by one or more heteroatoms, such as nitrogen, oxygen, sulfur. Heterocycle can be condensed with aryl or heteroaryl. Heterocycle may have one or more substituents which may be same or different structure. Nitrogen and sulfur atoms of heterocycle could be oxidized to N-oxide, S-oxide or S-dioxide. Heterocycle may be fully saturated, partially saturated and unsaturated. Examples of heterocycle include, but are not limited to, azetidine, pyrrolidine, piperidine, 2,8-diazaspiro [4.5]decane, piperazine, morpholine, and others.

"Heteroaryl" means an aromatic monocyclic or polycyclic system having from 5 to 11 carbon atoms, preferably from 5 to 10, of which one or more carbon atoms are substituted by one or more heteroatoms, such as nitrogen, sulfur or oxygen. Nitrogen atom of heteroaryl could be oxidized to N-oxide. Heteroaryl may have one or more substituents which may be same or different structure. Examples of heteroaryl are pyrrolyl, furanyl, thienyl, pyridyl, pyrazinyl, pyrimidinyl, pyridazinyl, isoxazolyl, isothiazolyl, tetrazolyl, oxazolyl, thiazolyl, pyrazolyl, furazanyl, triazolyl, 1,2,4-thiadiazolyl, quinoxalinyl, phthalazinyl, imidazo[1,2-a]pyridinyl, imidazo[2,1-b]thiazolyl, benzofurazanyl, indolyl, azaindolyl, benzimidazolyl, benzothiazenyl, quinolinyl, imidazolyl, pyrazolyl, thienopyridyl, quinazolinyl, naphthyridinyl, thienopyrimidinyl, pyrrolopyridinyl, imidazopyridyl, isoquinolinyl, benzoazaindolyl, 1,2,4-triazinyl, thienopyrrolyl, furopyrrolyl, and the like.

"Partially saturated" means a ring system including at least one double or triple bond. The term "partly saturated" relates to rings having many sites for saturation and does not include aryl and heteroaryl systems as they defined above.

The term "oxo" used in this document relates to the radical =O.

"Substituent" means a chemical radical attached to a scaffold (fragment).

"Solvate" is a molecular aggregate that consists of the compound of the present invention, or its pharmaceutically acceptable salt, with one or more solvent molecules. The solvent molecules are molecules of common pharmaceutical solvents, known to be safe for recipients, e.g. water, ethanol, ethylene glycol, etc. Other solvents, such as methanol, methyl-tert-butyl ether, ethyl acetate, methyl acetate, (8)-propylene glycol or (R)-propylene glycol, 1,4-butanediol, and the like, can be used to form intermediate solvates for obtaining preferable solvates.

The term "hydrate" refers to a complex in which the solvent molecule is water

Solvates and/or hydrates preferably exist in crystalline form.

Terms "bond", "chemical bond", or "single bond" refer to a chemical bonding of two atoms or two moieties (i.e., groups, fragments) when two atoms joined by the bond are considered as a part of larger substructure.

The term "chiral" refers to molecules that have the property of being incompatible with their mirror image, whereas the term "achiral" refers to molecules that have the property of being compatible with their mirror image.

The term "stereoisomers" refers to compounds that have identical chemical composition and the same structure, but differ in the spatial arrangement of atoms or their groups. Stereoisomers may include geometric isomers, enantiomers, diastereomers.

The term "diastereomer" refers to a stereoisomer with two or more centers of chirality, and such molecules are not mirror images of each other. Diastereomers have different physical properties, for example, melting points, boiling points, spectral properties and reactivity. Mixtures of diastereomers could be separated using high-resolution analytical techniques, such as electrophoresis and chromatography.

The term "enantiomers" refers to two stereoisomers of a compound being mirror images of one another and not compatible in space.

The terms "racemic mixture" and "racemate" refer to an equimolar mixture of two enantiomers that are not optical active. Enantiomers can be isolated from the racemic mixture separately by chiral resolution, such as, for example,

supercritical fluid chromatography (SFC).

The compounds of the invention may contain asymmetric or chiral centers and, therefore, exist in different stereoisomeric forms. It is contemplated that all stereoisomeric forms of the compounds of the invention, including but not limited to diastereomers, enantiomers and atropisomers, as well as mixtures thereof, such as racemic mixtures, are part of the present invention. Many organic compounds exist in optically active forms, i. e. they have the ability to rotate the plane of linearly polarized light. When describing an optically active compound, the prefixes R and S are used to designate the absolute configuration of the molecule with respect to its chiral center(s). A particular stereoisomer can also be defined as an enantiomer, and a mixture of such isomers is often referred to as an enantiomeric mixture.

The term "atropisomers" refers to compounds having spatial isomerism caused by the absence of rotation around a simple bond, for example, in diphenyls, dinaphthyls and others.

The term "protecting group" refers to groups that are used to block the reactivity of functional groups, such as an amino group, carboxyl group or hydroxy group. Examples of protecting groups include, but are not limited to, tert-butyloxycarbonyl (Boc), benzyloxycarbonyl (Cbz), 2-(trimethylsilyl) ethoxy) methyl acetal (SEM), trialkylsilyl, alkyl(diaryl)silyl or alkyl.

The term "excipient" is used herein to describe any ingredient other than the compound(s) of the invention.

"Pharmaceutical composition" means a composition, comprising a compound of the invention and one or more pharmaceutically acceptable excipients. The excipient can be selected from a group consisting of pharmaceutically acceptable and pharmacologically compatible fillers, solvents, diluents, carriers, auxiliary, distributing and sensing agents, delivery agents, such as preservatives, stabilizers, filler, disintegrators, moisteners, emulsifiers, suspending agents, thickeners, sweeteners, flavouring agents, aromatizing agents, antibacterial agents, fungicides, lubricants, and prolonged delivery controllers, the

choice and suitable proportions of which depend on the type and way of administration and dosage. Examples of suitable suspending agents are ethoxylated isostearyl alcohol, polyoxyethene, sorbitol and sorbitol ether, microcrystalline cellulose, aluminum metahydroxide, bentonite, agar-agar and tragacant and their mixtures as well. Protection against action of microorganisms can be provided by various antibacterial and antifungal agents, such as, for example, parabens, chlorobutanole, sorbic acid, and similar compounds. Composition may also contain isotonic agents, such as, for example, sugars, sodium chloride, and similar compounds. Prolonged action of composition may be achieved by agents slowing down absorption of active ingredient, for example, aluminum monostearate and gelatine. Examples of suitable carriers, solvents, diluents and delivery agents include water, ethanol, polyalcohols and their mixtures, natural oils (such as olive oil) and organic esters (such as ethyl oleate) for injections. Examples of fillers are lactose, milk-sugar, sodium citrate, calcium carbonate, calcium phosphate and the like. Examples of disintegrators and distributors are starch, alginic acid and its salts, silicates and the like. Examples of suitable lubricants are magnesium stearate, sodium lauryl sulfate, talc and polyethylene glycol of high molecular weight. A pharmaceutical composition for peroral, sublingual, transdermal, intramuscular, intravenous, subcutaneous, local or rectal administration of active ingredient, alone or in combination with another active compound may be administered to human and animals in a standard administration form, in a mixture with traditional pharmaceutical carriers. Suitable standard administration forms include peroral forms such as tablets, gelatin capsules, pills, powders, granules, chewing-gums and peroral solutions or suspensions; sublingual and transbuccal administration forms; aerosols; implants; local, transdermal, subcutaneous, intramuscular, intravenous, intranasal or intraocular forms and rectal administration forms.

"Pharmaceutically acceptable salt" means relatively nontoxic both organic and inorganic salts of acids and bases disclosed in this invention. Salts of compounds provided herein can be obtained from inorganic or organic acids and bases. Examples of salts prepared in this manner include hydrochlorides, hydrobromides, sulfates, bisulfates, phosphates, nitrates, acetates, oxalates, valeriates, oleates, palmitates, stearates, laurates, borates, benzoates, lactates, p-toluenesulfonates, citrates, maleates, fumarates, succinates, tartrates, methane sulphonates, malonates, salicylates, propionates, ethane sulphonates, benzene sulfonates, sulfamates and the like; sodium, potassium, ammonium, calcium, magnesium, iron, zinc, copper, manganese and aluminum salts, primary, secondary and tertiary amine salts, substituted amine salts, including naturally-occurring substituted amine salts, cyclic amine salts, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, ethylenediamine, glucosamine, methylglucamine, theobromine, purines, piperazine, piperidine, N-ethylpiperidine (Detailed description of such salts properties is given in: Berge S.M., et al., "Pharmaceutical Salts" J. Pharm. Sci. 1977, 66: 1 - 19). Aminoacids may be selected from aminoacids-lysine, ornithine and arginine.

"Medicament" is a compound (or a mixture of compounds as a pharmaceutical composition) in the form of tablets, capsules, injections, ointments and other ready forms intended for restoration, improvement or modification of physiological functions in humans and animals, and for treatment and prophylaxis of diseases, for diagnostics, anesthesia, contraception, cosmetology and others.

"Treat", "treating" and "treatment" refer to a method of alleviating or abrogating a biological disorder and/or at least one of its attendant symptoms. As used herein, to "alleviate" a disease, disorder or condition means reducing the

severity and/or occurrence frequency of the symptoms of the disease, disorder, or condition. Further, references herein to "treatment" include references to curative, palliative and prophylactic treatment.

In one aspect, the subject of treatment, or patient, is a mammal, preferably a human subject. Said subject may be either male or female, of any age.

"Disorder" means any condition that would benefit from treatment with the compound of the present invention. This means chronic and acute disorders or diseases including those pathological conditions that predispose the mammal to the disorder in question. Non-limiting examples of disorders to be treated herein include oncological diseases, in particular breast cancer, triple-negative breast cancer (TNBC), ovarian cancer, metastatic ovarian cancer, stomach cancer, metastatic stomach cancer, endometrial, salivary gland, lung, kidney or colon cancer; colorectal cancer, melanoma, metastatic melanoma, thyroid, pancreas, prostate or bladder cancer; haemato-oncological diseases, leucoses, acute myeloid leukemia and lymphoid malignancies, neuronal, glial, astrocytal, hypothalamic and other glandular, macrophagal, epithelial, stromal and blastocoelic disorders; inflammatory, angiogenic and immunologic disorders.

"Therapeutically effective amount" refers to that amount of the therapeutic agent being administered which will relieve to some extent one or more of the symptoms of the disease/ disorder being treated.

In this description and in the subsequent claims, unless the context otherwise provides, the words "comprise," "have," "include," or variations such as "comprises," "comprising," "has," "having," "includes" or "including", and all grammatical variations thereof will be understood to imply the inclusion of a stated integer or group of integers but not the exclusion of any other integer or group of integers.

## Detailed description of the invention

In one embodiment, the present invention relates to the compound of formula I:

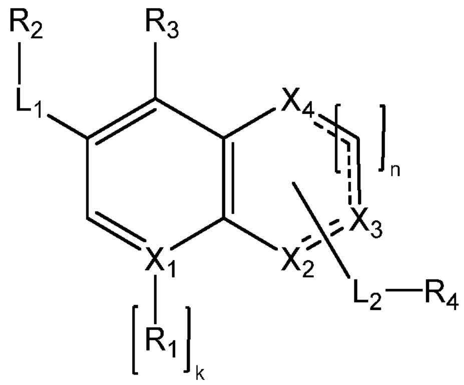

**I**,

or pharmaceutically acceptable salt or stereoisomer thereof,
wherein $X_1$ is N, C, CH;

each $X_2$, $X_3$, $X_4$ independently is $C(H)_m$, NH, N, $CR_{13}$, $CHR_{13}$;
each $L_1$, $L_2$ independently is a chemical bond, $-C(R_{6b})_2-$, -O-, -C(O)-, -C(O)-O-, -NH-, $-C(=NR_{19})-$;
each n, k independently is selected from 0, 1;
m is 0, 1, 2;
each $R_1$, $R_3$, $R_{13}$ independently is H, Hal, cyano, $C_1$-$C_6$ alkyl, $NH_2$;
each $R_2$, $R_4$ independently is selected from group, consisting of:

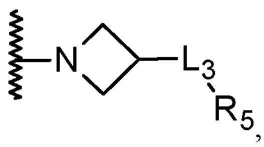,
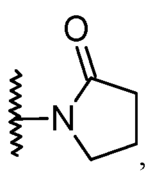,
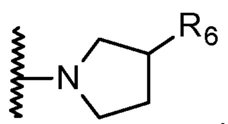,
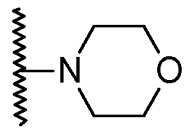,

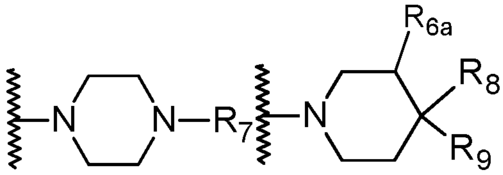, 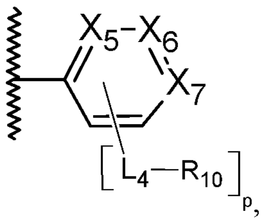,

$-NR_{11}R_{12}$; $C_1$-$C_6$ alkyl, unsubstituted or substituted by one or several substituents $R_{14}$;
each $X_5$, $X_6$, $X_7$ independently is C, CH, N;
each $L_3$, $L_4$ independently is a chemical bond, -C(O)-, -O-, $-CH_2-$, -NH-, $-C(O)-NR_{7a}-$, -C(=NH)-;
p = 0, 1, 2, 3, 4;
$R_5$ is H; Hal; cyano; $C_1$-$C_6$ alkyl; $C_1$-$C_6$ alkyloxy; $C_1$-$C_6$ alkyloxy $C_1$-$C_6$ alkyl; $NR_{15}R_{16}$; aryl, unsubstituted or substituted by one or several substituents, selected from group, consisting of Hal, $C_1$-$C_6$ alkyl, $C_1$-$C_6$ alkyloxy, $NR_{15}R_{16}$; 5-6 membered heterocyclyl with 1-2 heteroatoms, selected from N and/or O, unsubstituted or substituted by one or several substituents, selected from group, consisting of Hal, $C_1$-$C_6$ alkyl, $C_1$-$C_6$ alkyloxy, $NR_{15}R_{16}$ ;
each $R_6$, $R_{6a}$, $R_{6b}$ independently is H, Hal, hydroxy, $C_1$-$C_6$ alkyl, $C_1$-$C_6$ alkyloxy;
each $R_7$, $R_{7a}$ independently is H, $C_1$-$C_6$ alkyl;
each $R_8$, $R_9$ independently is H, $C_1$-$C_6$ alkyl, $-C(O)-NR_{21}R_{22}$, -CN, $-C(O)-OR_{20}$; or
$R_8$ and $R_9$ together with the carbon atom they are attached to, form 5-6-membered heterocyclic ring with 1-2 heteroatoms, selected from nitrogen and/or oxygen, wherein heterocyclic ring, formed by $R_8$ and $R_9$, could be unsubstituted or substituted by 1 or 2 substituents, selected from oxo group, $C_1$-$C_6$ alkyl;
each $R_{10}$ independently is selected from group, consisting of H, Hal, $C_1$-$C_6$ alkyl, hydroxy, cyano, $C_1$-$C_6$ alkyloxy, $C_1$-$C_6$ alkyloxy $C_1$-$C_6$ alkyl, $-NR_{23}R_{24}$; 5-6 membered heterocyclyl with 1-2 heteroatoms, selected from N, O and/or S, unsubstituted or substituted by one or several $C_1$-$C_6$ alkyl; 5-6 membered heteroaryl with 1-2 heteroatoms, selected from N, O and/or S, unsubstituted or substituted by one or several $C_1$-$C_6$ alkyl; $-S(O)_2$-$C_1$-$C_6$ alkyl;
each $R_{11}$, $R_{12}$ independently is H; $C_1$-$C_6$ alkyl unsubstituted or substituted by hydroxy, $C_3$-$C_6$ cycloalkyl, $-NR_{23a}R_{24a}$; $C_1$-$C_6$ alkoxy $C_1$-$C_6$ alkyl; $C_3$-$C_6$ cycloalkyl;
each $R_{14}$ independently is Hal, $-C(O)NR_{17}R_{18}$, $C_1$-$C_6$ alkoxy;
each $R_{15}$, $R_{16}$, $R_{19}$, $R_{20}$, $R_{21}$, $R_{22}$, $R_{23}$, $R_{24}$, $R_{23a}$, $R_{24a}$ independently is H, $C_1$-$C_6$ alkyl;
each $R_{17}$, $R_{18}$ independently is H, $C_1$-$C_6$ alkyl; aryl, unsubstituted or substituted by one or several substituents selected from group consisting of Hal, $C_1$-$C_6$ alkyl, $C_1$-$C_6$ alkoxy;
------ each independently is a single bond or a double bond;
Hal is chloro, bromo, iodo, fluoro.

In another one embodiment, the present invention relates to the compound of formula **I**, where each $R_3$ independently is H, Hal.

In another one embodiment, the present invention relates to the compound of formula **I**, where each $L_1$, $L_2$ independently is a chemical bond, -C(O)-, -C(O)-O-, -NH-, -C(=NH)-

In another one embodiment, the present invention relates to the compound of formula **I.1**:

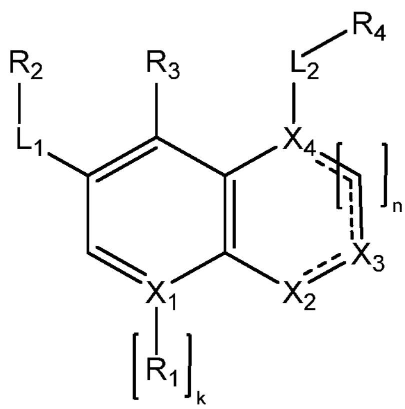

**I.1**,

wherein $X_4$ is $C(H)_m$, N;

m is 0, 1;
$X_1$ is N, C, CH;
each $X_2$, $X_3$ independently is $C(H)_m$, NH, N, $CR_{13}$, $CHR_{13}$;
each $L_1$, $L_2$ independently is a chemical bond, $-C(R_{6b})_2-$, -O-, -C(O)-, -C(O)-O-, -NH-, $-C(=NR_{19})-$;
each n, k independently is selected from 0, 1;
each $R_1$, $R_3$, $R_{13}$ independently is H, Hal, cyano, $C_1$-$C_6$ alkyl, $NH_2$;
each $R_2$, $R_4$ independently is selected from group, consisting of:

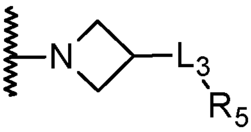,
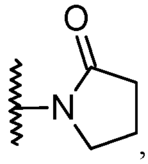,
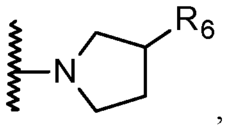,
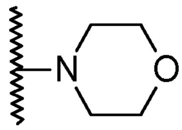,

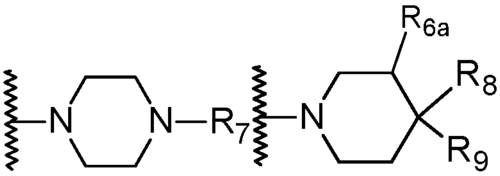,
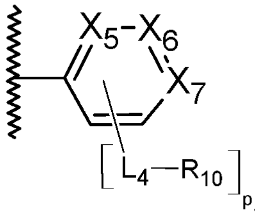,

$-NR_{11}R_{12}$; $C_1$-$C_6$ alkyl, unsubstituted or substituted by one or several substituents $R_{14}$;
each $X_5$, $X_6$, $X_7$ independently is C, CH, N;
each $L_3$, $L_4$ independently is a chemical bond, -C(O)-, -O-, $-CH_2-$, -NH-, $-C(O)-NR_{7a}$ -, -C(=NH)-;
p = 0, 1, 2, 3, 4;
$R_5$ is H; Hal; cyano; $C_1$-$C_6$ alkyl; $C_1$-$C_6$ alkyloxy; $C_1$-$C_6$ alkyloxy $C_1$-$C_6$ alkyl; $NR_{15}R_{16}$; aryl, unsubstituted or substituted by one or several substituents, selected from group, consisting of Hal, $C_1$-$C_6$ alkyl, $C_1$-$C_6$ alkyloxy, $NR_{15}R_{16}$; 5-6 membered heterocyclyl with 1-2 heteroatoms, selected from N and/or O, unsubstituted or substituted by one or several substituents, selected from group, consisting of Hal, $C_1$-$C_6$ alkyl, $C_1$-$C_6$ alkyloxy, $NR_{15}R_{16}$ ;
each $R_6$, $R_{6a}$, $R_{6b}$ independently is H, Hal, hydroxy, $C_1$-$C_6$ alkyl, $C_1$-$C_6$ alkyloxy; each $R_7$, $R_{7a}$ independently is H, $C_1$-$C_6$ alkyl;
each $R_8$, $R_9$ independently is H, $C_1$-$C_6$ alkyl, $-C(O)-NR_{21}R_{22}$, -CN, $-C(O)-OR_{20}$; or
$R_8$ and $R_9$ together with the carbon atom they are attached to, form 5-6-membered heterocyclic ring with 1-2 heteroatoms, selected from nitrogen and/or oxygen, wherein heterocyclic ring, formed by $R_8$ and $R_9$, could be unsubstituted or substituted by 1 or 2 substituents, selected from oxo group, $C_1$-$C_6$ alkyl;
each $R_{10}$ independently is selected from group, consisting of H, Hal, $C_1$-$C_6$ alkyl, hydroxy, cyano, $C_1$-$C_6$ alkyloxy, $C_1$-$C_6$ alkyloxy $C_1$-$C_6$ alkyl, $-NR_{23}R_{24}$; 5-6 membered heterocyclyl with 1-2 heteroatoms, selected from N, O and/or S, unsubstituted or substituted by one or several $C_1$-$C_6$ alkyl; 5-6 membered heteroaryl with 1-2 heteroatoms, selected from N, O and/or S, unsubstituted or substituted by one or several $C_1$-$C_6$ alkyl; $-S(O)_2-C_1$-$C_6$ alkyl;
each $R_{11}$, $R_{12}$ independently is H; $C_1$-$C_6$ alkyl unsubstituted or substituted by hydroxy, $C_3$-$C_6$ cycloalkyl, $-NR_{23a}R_{24a}$; $C_1$-$C_6$ alkoxy $C_1$-$C_6$ alkyl; $C_3$-$C_6$ cycloalkyl;
each $R_{14}$ independently is Hal, $-C(O)NR_{17}R_{18}$, $C_1$-$C_6$ alkoxy;
each $R_{15}$, $R_{16}$, $R_{19}$, $R_{20}$, $R_{21}$, $R_{22}$, $R_{23}$, $R_{24}$, $R_{23a}$, $R_{24a}$ independently is H, $C_1$-$C_6$ alkyl;
each $R_{17}$, $R_{18}$ independently is H, $C_1$-$C_6$ alkyl; aryl, unsubstituted or substituted by one or several substituents selected from group consisting of Hal, $C_1$-$C_6$ alkyl, $C_1$-$C_6$ alkoxy;
------ each independently is a single bond or a double bond;
Hal is chloro, bromo, iodo, fluoro.

In another one embodiment, the present invention relates to the compound of formula **I.1**, where each $R_3$ independently is H, Hal.

In another one embodiment, the present invention relates to the compound of formula **I.1**, where each $L_1$, $L_2$ independently is chemical bond, -C(O)-, -C(O)-O-, -NH-, -C(=NH)-

In another one embodiment, the present invention relates to the compound of formula **I.2**:

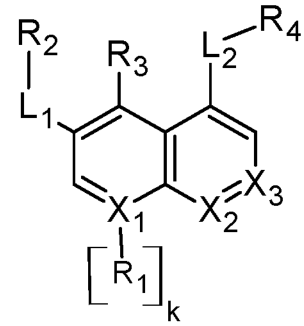

**I.2**,

wherein $X_1$ is C, CH, N;

each $X_2$, $X_3$ independently is N, $CR_{13}$;
each $L_1$, $L_2$ independently is a chemical bond, $-C(R_{6b})_2-$, -O-, -C(O)-, -C(O)-O-, -NH-, $-C(=NR_{19})-$;
k is 0, 1;
each $R_1$, $R_3$, $R_{13}$ independently is H, Hal, cyano, $C_1$-$C_6$ alkyl, $NH_2$;
each $R_2$, $R_4$ independently is selected from group, consisting of:

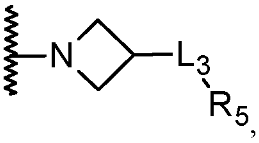, 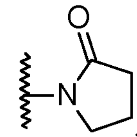, 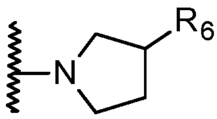, 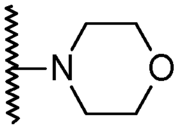,

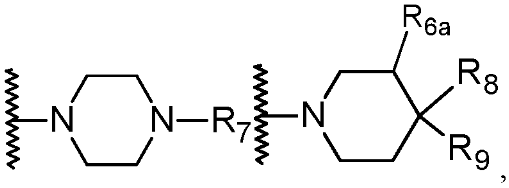, 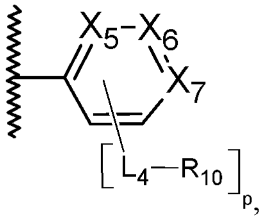,

$-NR_{11}R_{12}$; $C_1$-$C_6$ alkyl, unsubstituted or substituted by one or several substituents $R_{14}$;
each $X_5$, $X_6$, $X_7$ independently is C, CH, N;
each $L_3$, $L_4$ independently is a chemical bond, -C(O)-, -O-, $-CH_2-$, -NH-, $-C(O)-NR_{7a}-$, -C(=NH)-;
p = 0, 1, 2, 3, 4;
$R_5$ is H; Hal; cyano; $C_1$-$C_6$ alkyl; $C_1$-$C_6$ alkyloxy; $C_1$-$C_6$ alkyloxy $C_1$-$C_6$ alkyl; $NR_{15}R_{16}$; aryl, unsubstituted or substituted by one or several substituents, selected from group, consisting of Hal, $C_1$-$C_6$ alkyl, $C_1$-$C_6$ alkyloxy, $NR_{15}R_{16}$; 5-6 membered heterocyclyl with 1-2 heteroatoms, selected from N and/or O, unsubstituted or substituted by one or several substituents, selected from group, consisting of Hal, $C_1$-$C_6$ alkyl, $C_1$-$C_6$ alkyloxy, $NR_{15}R_{16}$ ;
each $R_6$, $R_{6a}$, $R_{6b}$ independently is H, Hal, hydroxy, $C_1$-$C_6$ alkyl, $C_1$-$C_6$ alkyloxy;
each $R_7$, $R_{7a}$ independently is H, $C_1$-$C_6$ alkyl;
each $R_g$, $R_9$ independently is H, $C_1$-$C_6$ alkyl, $-C(O)-NR_{21}R_{22}$, -CN, $-C(O)-OR_{20}$; or
$R_8$ and $R_9$ together with the carbon atom they are attached to, form 5-6-membered heterocyclic ring with 1-2 heteroatoms, selected from nitrogen and/or oxygen, wherein heterocyclic ring, formed by $R_8$ and $R_9$, could be unsubstituted or substituted by 1 or 2 substituents, selected from oxo group, $C_1$-$C_6$ alkyl;
each $R_{10}$ independently is selected from group, consisting of H, Hal, $C_1$-$C_6$ alkyl, hydroxy, cyano, $C_1$-$C_6$ alkyloxy, $C_1$-$C_6$ alkyloxy $C_1$-$C_6$ alkyl, $-NR_{23}R_{24}$; 5-6 membered heterocyclyl with 1-2 heteroatoms, selected from N, O and/or S, unsubstituted or substituted by one or several $C_1$-$C_6$ alkyl; 5-6 membered heteroaryl with 1-2 heteroatoms, selected from N, O and/or S, unsubstituted or substituted by one or several $C_1$-$C_6$ alkyl; $-S(O)_2-C_1$-$C_6$ alkyl;
each $R_{11}$, $R_{12}$ independently is H; $C_1$-$C_6$ alkyl unsubstituted or substituted by hydroxy, $C_3$-$C_6$ cycloalkyl, $-NR_{23a}R_{24a}$; $C_1$-$C_6$ alkoxy $C_1$-$C_6$ alkyl; $C_3$-$C_6$ cycloalkyl;
each $R_{14}$ independently is Hal, $-C(O)NR_{17}R_{18}$;
each $R_{15}$, $R_{16}$, $R_{21}$, $R_{22}$, $R_{23}$, $R_{24}$, $R_{23a}$, $R_{24a}$ independently is H, $C_1$-$C_6$ alkyl;
each $R_{17}$, $R_{18}$ independently is H, $C_1$-$C_6$ alkyl; aryl, unsubstituted or substituted by one or several substituents

selected from group consisting of Hal, $C_1$-$C_6$ alkyl, $C_1$-$C_6$ alkyloxy;
each $R_{19}$, $R_{20}$ independently is H, $C_1$-$C_6$ alkyl.

In another one embodiment, the present invention relates to the compound of formula **I.4**:

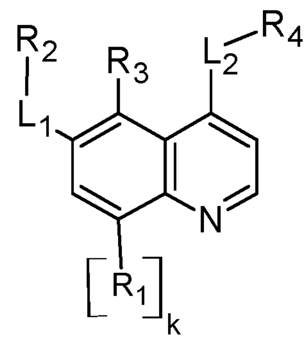

**I.4**,

wherein $L_1$, $L_2$, $R_1$, $R_2$, $R_3$, $R_4$, k have the above meanings.

In another one embodiment, the present invention relates to the compound of formula **I.5a**:

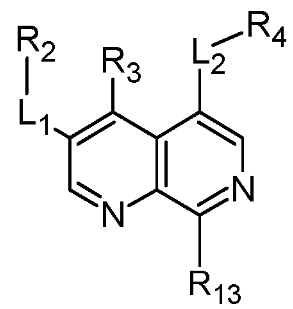

**I.5a**,

wherein $L_1$, $L_2$, $R_2$, $R_3$, $R_4$, $R_{13}$ have the above meanings.

In another one embodiment, the present invention relates to the compound of formula **I.5**:

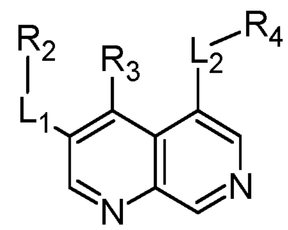

**I.5**,

wherein $L_1$, $L_2$, $R_2$, $R_3$, $R_4$ have the above meanings.

In another one embodiment, the present invention relates to the compound of formula **I.6**:

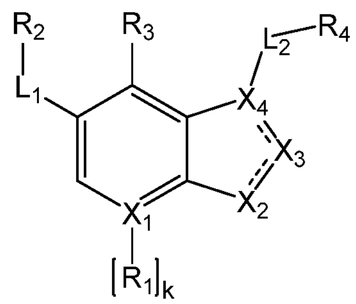

**I.6**,

wherein each $X_2$, $X_3$ independently is $C(H)_m$, NH, N, $CR_{13}$, $CHR_{13}$;

each $X_1$, $X_4$ independently is C, CH, N; each $L_1$, $L_2$ independently is a chemical bond, $-C(R_{6b})_2-$, -O-, -C(O)-, -NH-, $-C(=NR_{19})-$;
k is 0, 1;
m is 1, 2;
each $R_1$, $R_3$, $R_{13}$ independently is H, Hal, cyano, $C_1$-$C_6$ alkyl, $NH_2$;
each $R_2$, $R_4$ independently is selected from group, consisting of:

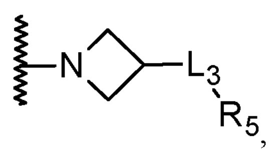,
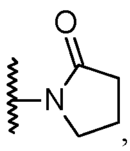,
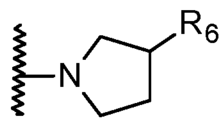,
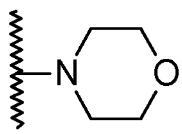,

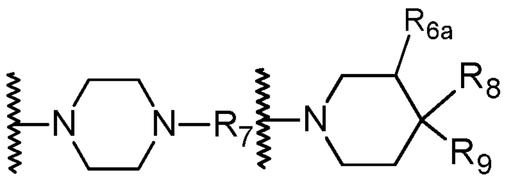,
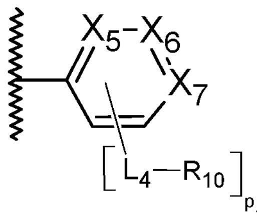,

$-NR_{11}R_{12}$; $C_1$-$C_6$ alkyl, unsubstituted or substituted by one or several substituents $R_{14}$;
each $X_5$, $X_6$, $X_7$ independently is C, CH, N;
each $L_3$, $L_4$ independently is a chemical bond, -C(O)-, -O-, $-CH_2-$, -NH-, $-C(O)-NR_{7a}-$, -C(=NH)-;
p = 0, 1, 2, 3, 4;
$R_5$ is H; Hal; cyano; $C_1$-$C_6$ alkyl; $C_1$-$C_6$ alkyloxy; $C_1$-$C_6$ alkyloxy $C_1$-$C_6$ alkyl; $NR_{15}R_{16}$; aryl, unsubstituted or substituted by one or several substituents, selected from group, consisting of Hal, $C_1$-$C_6$ alkyl, $C_1$-$C_6$ alkyloxy, $NR_{15}R_{16}$; 5-6 membered heterocyclyl with 1-2 heteroatoms, selected from N and/or O, unsubstituted or substituted by one or several substituents, selected from group, consisting of Hal, $C_1$-$C_6$ alkyl, $C_1$-$C_6$ alkyloxy, $NR_{15}R_{16}$ ;
each $R_6$, $R_{6a}$, $R_{6b}$ independently is H, Hal, hydroxy, $C_1$-$C_6$ alkyl, $C_1$-$C_6$ alkyloxy; each $R_7$, $R_{7a}$ independently is H, $C_1$-$C_6$ alkyl;
each $R_8$, $R_9$ independently is H, $C_1$-$C_6$ alkyl, $-C(O)-NR_{21}R_{22}$, -CN, $-C(O)-OR_{20}$; or
$R_8$ and $R_9$ together with the carbon atom they are attached to, form 5-6-membered heterocyclic ring with 1-2 heteroatoms, selected from nitrogen and/or oxygen, wherein heterocyclic ring, formed by $R_8$ and $R_9$, could be unsubstituted or substituted by 1 or 2 substituents, selected from oxo group, $C_1$-$C_6$ alkyl;
each $R_{10}$ independently is selected from group, consisting of H, Hal, $C_1$-$C_6$ alkyl, hydroxy, cyano, $C_1$-$C_6$ alkyloxy, $C_1$-$C_6$ alkyloxy $C_1$-$C_6$ alkyl, $-NR_{23}R_{24}$; 5-6 membered heterocyclyl with 1-2 heteroatoms, selected from N, O and/or S, unsubstituted or substituted by one or several $C_1$-$C_6$ alkyl; 5-6 membered heteroaryl with 1-2 heteroatoms, selected from N, O and/or S, unsubstituted or substituted by one or several $C_1$-$C_6$ alkyl; $-S(O)_2-C_1-C_6$ alkyl;
each $R_{11}$, $R_{12}$ independently is H; $C_1$-$C_6$ alkyl unsubstituted or substituted by hydroxy, $C_3$-$C_6$ cycloalkyl, $-NR_{23a}R_{24a}$; $C_1$-$C_6$ alkoxy $C_1$-$C_6$ alkyl; $C_3$-$C_6$ cycloalkyl; each $R_{14}$ independently is Hal, $-C(O)NR_{17}R_{18}$, $C_1$-$C_6$ alkoxy;
each $R_{15}$, $R_{16}$, $R_{21}$, $R_{22}$, $R_{23}$, $R_{24}$ independently is H, $C_1$-$C_6$ alkyl;
each $R_{17}$, $R_{18}$ independently is H, $C_1$-$C_6$ alkyl; aryl, unsubstituted or substituted by one or several substituents

selected from group consisting of Hal, $C_1$-$C_6$ alkyl, $C_1$-$C_6$ alkyloxy;
each $R_{19}$, $R_{20}$ independently is H, $C_1$-$C_6$ alkyl;
------ each independently is single a bond or a double bond.

In another one embodiment, the present invention relates to the compound of formula **I.7**:

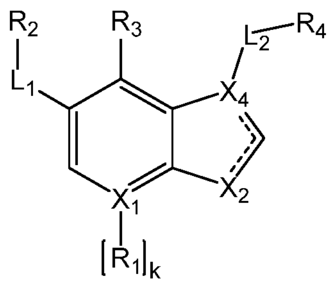

**I.7**,

wherein $X_1$, $X_2$, $X_4$, $L_1$, $L_2$, $R_1$, $R_2$, $R_3$, $R_4$, $R_{13}$, k ------ have the above meanings.

In another one embodiment, the present invention relates to the compound of formula **I.8**:

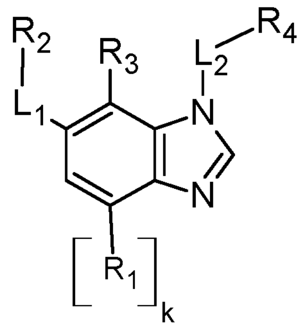

**I.8**,

wherein $L_1$, $L_2$, $R_1$, $R_2$, $R_3$, $R_4$ have the above meanings.

In another one embodiment, the present invention relates to the compound of formula **I.9**:

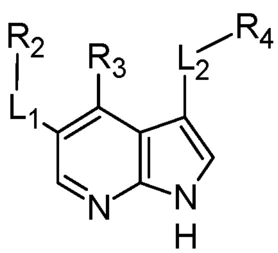

**I.9**,

wherein $L_1$, $L_2$, $R_2$, $R_3$, $R_4$ have the above meanings.

In another one embodiment, the present invention relates to the compound of formula **I.10**:

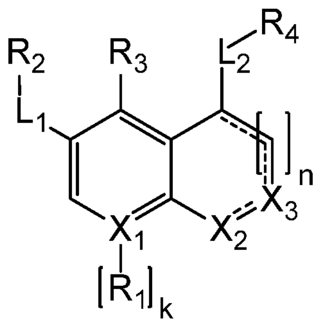

**I.10**,

wherein $X_1$, $X_2$, $X_3$, $L_1$, $L_2$, $R_1$, $R_2$, $R_3$, $R_4$, n, k have the above meanings.

In another one embodiment, the present invention relates to the compound of formula **I, I.1- I.10**, wherein $R_5$ is H; Hal; cyano; $C_1$-$C_6$ alkyl; $C_1$-$C_6$ alkyloxy; $C_1$-$C_6$ alkyloxy $C_1$-$C_6$ alkyl; $NR_{15}R_{16}$; aryl, wherein aryl is phenyl, unsubstituted or substituted by one or several substituents, selected from group, consisting of Hal, $C_1$-$C_6$ alkyl, $C_1$-$C_6$ alkyloxy, $NR_{15}R_{16}$; 5-6 membered heterocyclyl with 1-2 heteroatoms, selected from N and/or O, wherein heterocyclyl is 4-morpholinyl, 1-piperidinyl, 1-pyrrolidinyl, 1-piperazinyl, unsubstituted or substituted by one or several substituents, selected from group, consisting of Hal, $C_1$-$C_6$ alkyl, $C_1$-$C_6$ alkyloxy, $NR_{15}R_{16}$;

In another one embodiment, the present invention relates to the compound of formula **I**, **I.1**- **I.10,** wherein $R_{10}$ independently is selected from group, consisting of H, Hal, $C_1$-$C_6$ alkyl, hydroxy, cyano, $C_1$-$C_6$ alkyloxy, $C_1$-$C_6$ alkyloxy $C_1$-$C_6$ alkyl, $-NR_{23}R_{24}$; 5-6 membered heterocyclyl with 1-2 heteroatoms, selected from N, O and/or S, wherein heterocyclyl is 4-morpholinyl, 1-piperidinyl, 1-pyrrolidinyl, 1-piperazinyl, unsubstituted or substituted by one or several $C_1$-$C_6$ alkyl; 5-6 membered heteroaryl with 1-2 heteroatoms, selected from N, O and/or S, wherein heteroaryl is thienyl, imidazolyl, pyrazolyl, unsubstituted or substituted by one or several $C_1$-$C_6$ alkyl; $-S(O)_2$-$C_1$-$C_6$ alkyl.

Hal is chloro, bromo, iodo, fluoro.

In another one embodiment, the present invention relates to the compound of formula **Ia**:

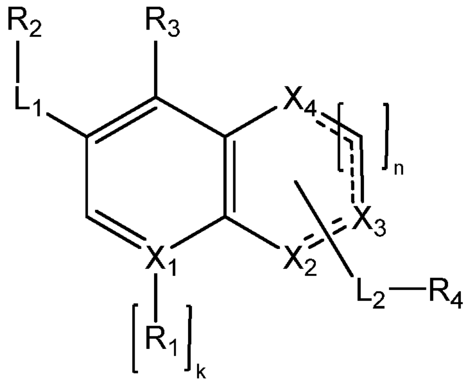

**Ia**,

or pharmaceutically acceptable salt or stereoisomer thereof,
wherein $X_1$ is N, C, CH;

each $X_2$, $X_3$, $X_4$ independently is $C(H)_m$, NH, N, $CR_{13}$;
each $L_1$, $L_2$ independently is a chemical bond, $-C(R_6)_2-$, -O-, -C(O)-, -NH-, $-C(=NR_{19})-$;
each n, k independently is selected from 0, 1;
m is 0, 1, 2;
each $R_1$, $R_3$, $R_{13}$ independently is H, Hal, cyano, $C_1$-$C_6$ alkyl;
each $R_2$, $R_4$ independently is selected from group, consisting of:

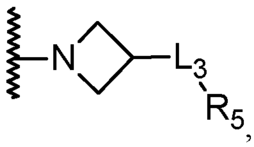, 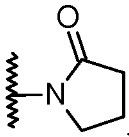, 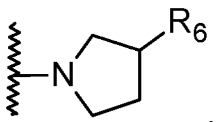, 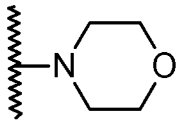,

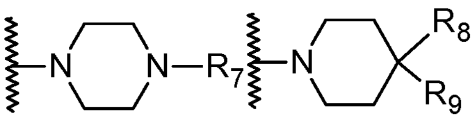, 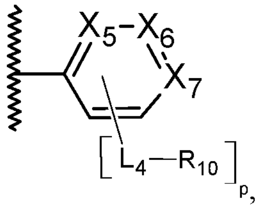,

$-NR_{11}R_{12}$; $C_1$-$C_6$ alkyl, unsubstituted or substituted by one or several substituents $R_{14}$;
each $X_5$, $X_6$, $X_7$ independently is C, CH, N;
each $L_3$, $L_4$ independently is a chemical bond, -C(O)-, -O-, $-CH_2-$, -NH-, $-C(O)-NR_7-$, -C(=NH)-;
p = 0, 1, 2, 3, 4;
$R_5$ is H; Hal; cyano; $C_1$-$C_6$ alkyl; $C_1$-$C_6$ alkyloxy; $C_1$-$C_6$ alkyloxy $C_1$-$C_6$ alkyl; $NR_{15}R_{16}$; aryl, unsubstituted or substituted by one or several substituents, selected from group, consisting of Hal, $C_1$-$C_6$ alkyl, $C_1$-$C_6$ alkyloxy, $NR_{15}R_{16}$; 5-6 membered heterocyclyl with 1-2 heteroatoms, selected from N and/or O, unsubstituted or substituted by one or several substituents, selected from group, consisting of Hal, $C_1$-$C_6$ alkyl, $C_1$-$C_6$ alkyloxy, $NR_{15}R_{16}$ ;
each $R_6$ independently is H, Hal, hydroxy, $C_1$-$C_6$ alkyloxy;
each $R_7$ independently is H, $C_1$-$C_6$ alkyl;
each $R_8$, $R_9$ independently is H, $C_1$-$C_6$ alkyl, $-C(O)-NR_{21}R_{22}$, -CN, $-C(O)-OR_{20}$; or
$R_8$ and $R_9$ together with the carbon atom they are attached to, form 5-6-membered heterocyclic ring with 1-2 heteroatoms, selected from nitrogen and/or oxygen, wherein heterocyclic ring, formed by $R_8$ and $R_9$, could be unsubstituted or substituted by 1 or 2 substituents, selected from oxo group, $C_1$-$C_6$ alkyl;
each $R_{10}$ independently is selected from group, consisting of H, Hal, $C_1$-$C_6$ alkyl, hydroxy, cyano, $C_1$-$C_6$ alkyloxy, $C_1$-$C_6$ alkyloxy $C_1$-$C_6$ alkyl, $-NR_{23}R_{24}$; 5-6 membered heterocyclyl with 1-2 heteroatoms, selected from N, O and/or S, unsubstituted or substituted by one or several $C_1$-$C_6$ alkyl; 5-6 membered heteroaryl with 1-2 heteroatoms, selected from N, O and/or S, unsubstituted or substituted by one or several $C_1$-$C_6$ alkyl; $-S(O)_2-C_1-C_6$ alkyl;
each $R_{11}$, $R_{12}$ independently is H, $C_1$-$C_6$ alkyl, $C_1$-$C_6$ alkoxy, $C_1$-$C_6$ alkyloxy $C_1$-$C_6$ alkyl;
each $R_{14}$ independently is Hal, $-C(O)NR_{17}R_{18}$;
each $R_{15}$, $R_{16}$, $R_{21}$, $R_{22}$, $R_{23}$, $R_{24}$ independently is H, $C_1$-$C_6$ alkyl;
each $R_{17}$, $R_{18}$ independently is H, $C_1$-$C_6$ alkyl; aryl, unsubstituted or substituted by one or several substituents selected from group consisting of Hal, $C_1$-$C_6$ alkyl, $C_1$-$C_6$ alkyloxy;
each $R_{19}$, $R_{20}$ independently is H, $C_1$-$C_6$ alkyl;
------ each independently is single bond or double bond;
Hal is chloro, bromo, iodo, fluoro.

In another one embodiment, the present invention relates to the compound of formula **Ia**, where each $R_1$, $R_3$ independently is H, Hal.

In another one embodiment, the present invention relates to the compound of formula **Ia**, where each $L_1$, $L_2$ independently is chemical bond, -C(O)-, -NH-, - C(=NH)-.

In another one embodiment, the present invention relates to the compound of formula **Ia.1**:

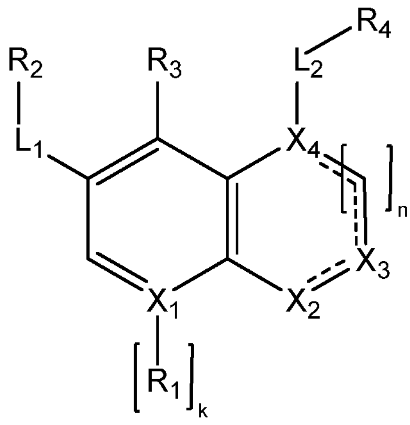

**Ia.1**,

wherein $X_4$ is $C(H)_m$, NH, N;

m is 0, 1;

$X_1$, $X_2$, $X_3$, $L_1$, $L_2$, $R_1$, $R_2$, $R_3$, $R_4$, $R_{13}$, n, k, ------ have the above meanings.

In another one embodiment, the present invention relates to the compound of formula **Ia.1**, where each $R_1$, $R_3$ independently H, Hal.

In another one embodiment, the present invention relates to the compound of formula **Ia.1**, where each $L_1$, $L_2$ independently is a chemical bond, -C(O)-, - NH-, -C(=NH)-.

The compounds, described in the present invention, may be formed as, and/or used as, pharmaceutically acceptable salts. The type of pharmaceutical acceptable salts, include, but are not limited to: acid addition salts, formed by reacting the free base form of the compound with a pharmaceutically acceptable inorganic acid such as hydrochloric acid, hydrobromic acid, sulfuric acid, nitric acid, phosphoric acid, metaphosphoric acid, and the like; or with an organic acid such as acetic acid, propionic acid, hexanoic acid, cyclopentanepropionic acid, glycolic acid, pyruvic acid, lactic acid, malonic acid, succinic acid, malic acid, maleic acid, fumaric acid, trifluoroacetic acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, toluenesulfonic acid, 2-naphthalenesulfonic acid, 4-methylbicyclo-[2.2.2]oct-2-ene-1-carboxylic acid, glucoheptonic acid, 4,4'-methylenebis-3-hydroxy-2-ene-1-carboxylic acid, 3-phenylpropionic acid, trimethylacetic acid, tert-butylacetic acid, lauryl sulfuric acid, gluconic acid, glutamic acid, hydroxynaphthoic acid, salicylic acid, stearic acid, muconic acid, and the like; base addition salts formed by reacting in the free base form of the compound with a pharmaceutically acceptable inorganic base such as metal or ammonium cation hydroxide, carbonate or bicarbonate, or with an organic base. Cations of pharmaceutically acceptable salts include, but are not limited to, sodium, potassium, lithium, magnesium, calcium, iron, zinc, copper, manganese, aluminum; examples of organic bases include, but are not limited to, primary, secondary or tertiary amine (e. g., isopropylamine, trimethylamine, diethylamine, triethylamine, tripropylamine), substituted amine, including naturally-occurring substituted amine (e.g., lysine, arginine, histidine, caffeine, choline), cyclic amine, dicyclohexylamine, procaine, ethanolamine, 2-diethylaminoethanol, hydrabamine, ethylenediamine, glucosamine, methylglucamine, theobromine, purine, piperazine, piperidine, N-ethylpiperidine.

The corresponding counterions of the pharmaceutically acceptable salts may be analyzed and identified using various methods including, but not limited to, ion exchange chromatography, ion chromatography, capillary electrophoresis, inductively coupled plasma, atomic absorption spectroscopy, mass spectrometry, or any combination thereof.

The salts are recovered by using at least one of the following techniques: filtration, precipitation with a non-solvent followed by filtration, evaporation of the solvent, or, in the case of aqueous solutions, lyophilization. It should be understood that a reference to a pharmaceutically acceptable salt includes the solvent addition forms or crystal forms thereof, particularly solvates or polymorphs. Solvates contain either stoichiometric or non-stoichiometric amounts of a solvent and may be formed during the process of crystallization with pharmaceutically acceptable solvents such as water, ethanol, and the like. Hydrates are formed when the solvent is water, or alcoholates are formed when the solvent is alcohol. Solvates of compounds described herein can be conveniently prepared or formed during the processes described herein. In addition, the compounds provided herein can exist in unsolvated as well as solvated forms. In general, the solvated forms are considered equivalent to the unsolvated forms for the purposes of the compounds and methods provided by present invention.

Compounds described herein may be in various forms, including but not limited to, amorphous forms, milled forms and nano-particulate forms. In addition, compounds described herein include crystalline forms, also known as

polymorphs. Polymorphs include the different crystal packing arrangements of the same elemental composition of a compound. Polymorphs usually have different X-ray diffraction patterns, infrared spectra, melting points, density, hardness, crystal shape, optical and electrical properties, stability, and solubility. Various factors such as the recrystallization solvent, rate of crystallization, and storage temperature may cause one crystal form to dominate.

The screening and characterization of the pharmaceutically acceptable salts, polymorphs and/or solvates may be accomplished using a variety of techniques including, but not limited to, thermal analysis, X-ray diffraction, spectroscopy, vapor sorption, and microscopy. Thermal analysis methods address to analysis of thermo chemical degradation or thermo physical processes including, but not limited to, polymorphic transitions, and such methods are used to analyze the relationships between polymorphic forms, to determine weight loss, to find the glass transition temperature, or for excipient compatibility studies. Such methods include, but are not limited to, Differential scanning calorimetry (DSC), Modulated Differential Scanning Calorimetry (MDCS), Thermogravimetric analysis (TGA), Thermogravi-metric and Infrared analysis (TG/IR). Crystallographic methods include, but are not limited to, single crystal and powder diffractometers and synchrotron sources. The various spectroscopic techniques used include, but are not limited to, Raman, FTIR, UVIS, and NMR (liquid and solid state). The various microscopy techniques include, but are not limited to, polarized light microscopy, Scanning Electron Microscopy (SEM) with Energy Dispersive X-Ray Analysis (EDX), Environmental Scanning Electron Microscopy with EDX (in gas or water vapor atmosphere), IR microscopy, and Raman microscopy.

In another embodiment of the present invention relates to the compounds selected from the group including:

| Formula | Name | Code |
|---|---|---|
| 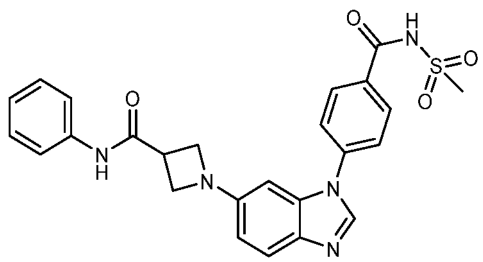 | 1-(1-(4-((Methylsulfonyl) carbamoyl)phenyl)-1*H*-benzo[*d*]imidazol-6-yl)-*N*-phenylazetidine-3-carboxamide | **BCD-CDK8-1-1** |
| 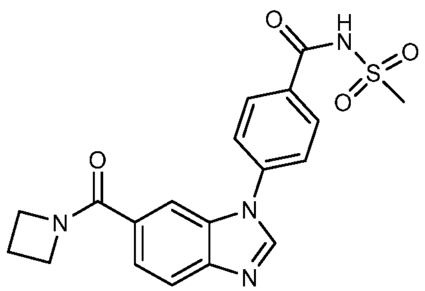 | 4-(6-Azetidine-1-carbonyl)-1*H*-benzo[*d*] imidazol-1-yl)-*N*-(methylsulfonyl) benzamide | **BCD-CDK8-1-2** |
| 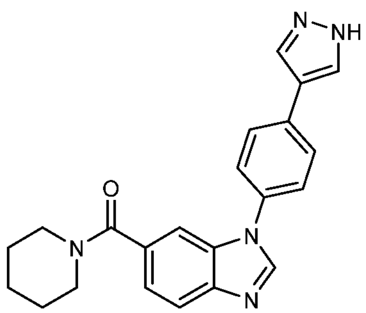 | (1-(4-(1*H*-Pyrazol-4-yl) phenyl)-1*H*-benzo[*d*] imidazol-6-yl)(piperidin-1-yl) methanone | **BCD-CDK8-1-3** |

(continued)

| Formula | Name | Code |
|---|---|---|
| 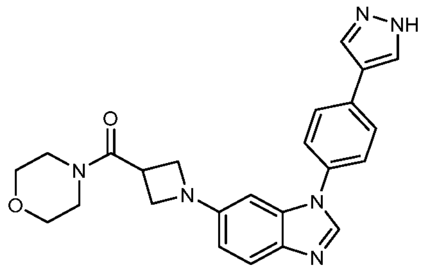 | (1-(1-(4-(1*H*-pyrazol-4-yl) phenyl)-1*H*-benzo[*d*] imidazol-6-yl)azetidin-3-yl) (morpholino) methanone | **BCD-CDK8-1-4** |
| 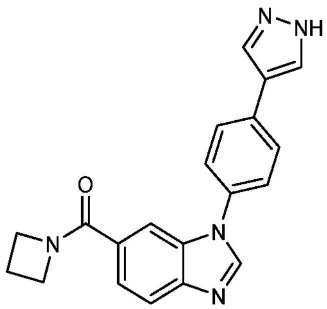 | (1-(4-(1*H*-Pyrazol-4-yl) phenyl)-1*H*-benzo[*d*] imidazol-6-yl)(azetidin-1-yl) methanone | **BCD-CDK8-1-5** |
| 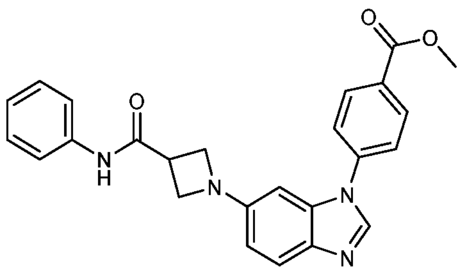 | Methyl 4-(6-(3-(phenylcarbamoyl)azetidin-1-yl)-1*H*-benzo[*d*]imidazol-1-yl) benzoate | **BCD-CDK8-1-6E** |
| 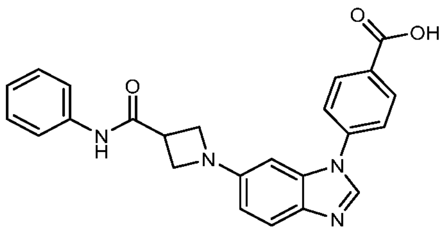 | 4-(6-(3-(phenylcarbamoyl) azetidin-1-yl)-1*H*-benzo[*d*]imidazol-1-yl)benzoic acid | **BCD-CDK8-1-6A** |
| 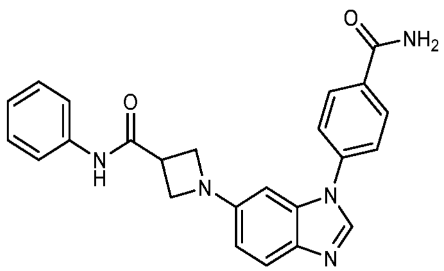 | 1-(1-(4-Carbamoylphenyl)-1*H*-benzo[*d*]imidazol-6-yl)-*N*-phenylazetidine-3-carboxamide | **BCD-CDK8-1-6** |

(continued)

| Formula | Name | Code |
|---|---|---|
| 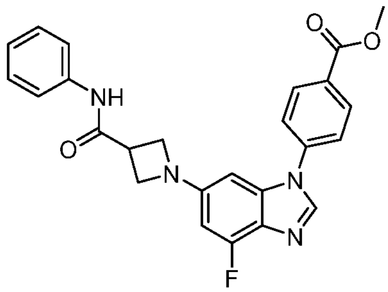 | Methyl 4-(6-(3-(phenylcarbamoyl)azetidin-1-yl)-4-fluoro-1*H*-benzo[*d*]imidazol-1-yl)benzoate | **BCD-CDK8-1-7E** |
| 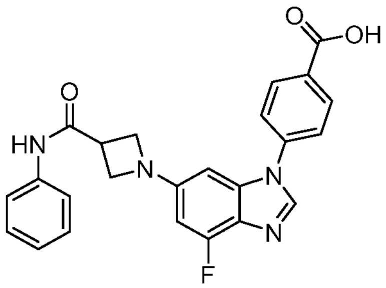 | 4-(-6-(3-(phenylcarbamoyl)azetidin-1-yl)-4-fluoro-1*H*-benzo[*d*]imidazol-1-yl) benzoic acid | **BCD-CDK8-1-7A** |
| 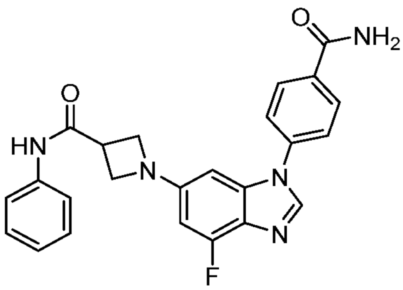 | 1-(1-(4-Carbamoylphenyl)-4-fluoro-1*H*-benzo[*d*] imidazol-6-yl)-*N*-phenylazetidine-3-carboxamide | **BCD-CDK8-1-7** |
| 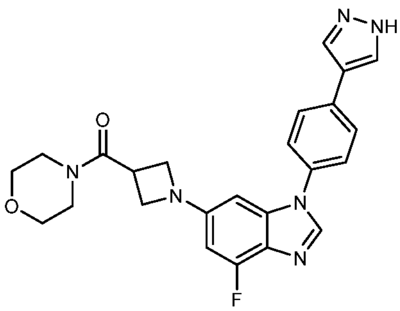 | (1-(1-(4-(1*H*-Pyrazol-4-yl) phenyl)-4-fluoro-1*H*-benzo[*d*]imidazol-6-yl)azetidin-3-yl)(morpholino) methanone | **BCD-CDK8-1-8** |
| 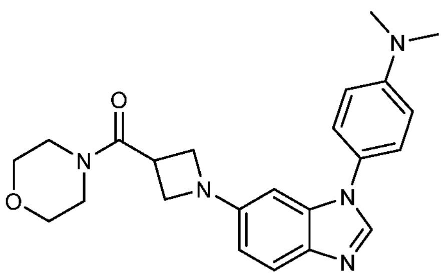 | (1-(1-(4-(Dimethylamino) phenyl)-1*H*-benzo[*d*] imidazol-6-yl)azetidin-3-yl) (morpholino) methanone | **BCD-CDK8-1-9** |

(continued)

| Formula | Name | Code |
|---|---|---|
| 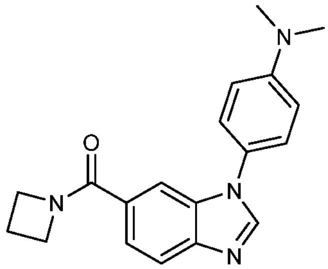 | Azetidin-1-yl(1-(4-(dimethylamino)phenyl)-1*H*-benzo[*d*]imidazol-6-yl) methanone | **BCD-CDK8-1-10** |
| 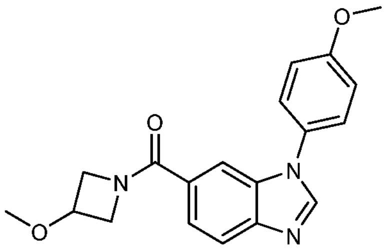 | (3-Methoxyazetidin-1-yl)(1-(4-methoxyphenyl)-1*H*-benzo[*d*]imidazol-6-yl) methanone | **BCD-CDK8-1-11** |
| 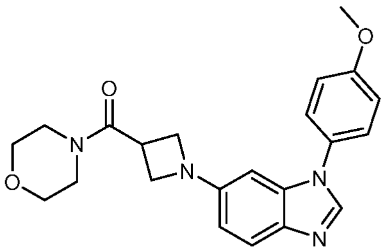 | (1-(1-(4-Methoxyphenyl)-1*H*-benzo[*d*]imidazol-6-yl)azetidin-3-yl)(morpholino) methanone | **BCD-CDK8-1-12** |
| 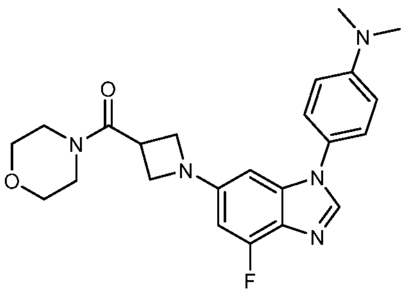 | (1-(1-(4-(Dimethylamino) phenyl)-4-fluoro-1*H*-benzo[*d*]imidazol-6-yl)azetidin-3-yl)(morpholino) methanone | **BCD-CDK8-1-13** |
| 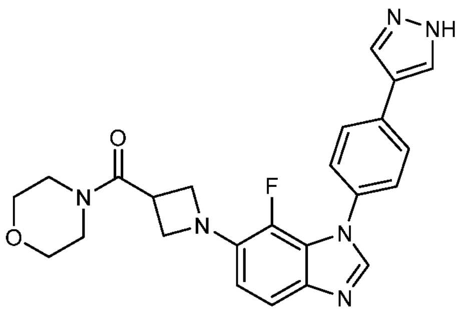 | (1-(1-(4-(1*H*-Pyrazol-4-yl) phenyl)-7-fluoro-1*H*-benzo[d]imidazol-6-yl)azetidin-3-yl)(morpholino) methanone | **BCD-CDK8-1-14** |

(continued)

| Formula | Name | Code |
|---|---|---|
| 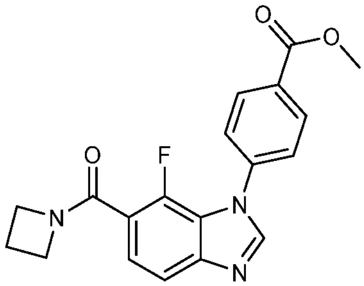 | Methyl-4-(6-(azetidine-1-carbonyl)-7-fluoro-1*H*-benzo[*d*]imidazol-1-yl)benzoate | **BCD-CDK8-1-15E** |
| 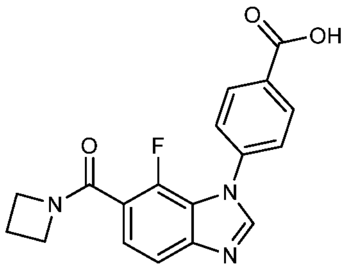 | 4-(6-(Azetidine-1-carbonyl)-7-fluoro-1*H*-benzo [*d*]imidazol-1-yl)benzoic acid | **BCD-CDK8-1-15A** |
| 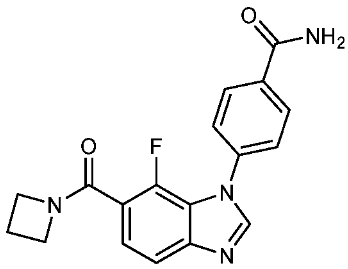 | 4-(6-(Azetidine-1-carbonyl)-7-fluoro-1*H*-benzo [*d*]imidazol-1-yl)benzamide | **BCD-CDK8-1-15** |
| 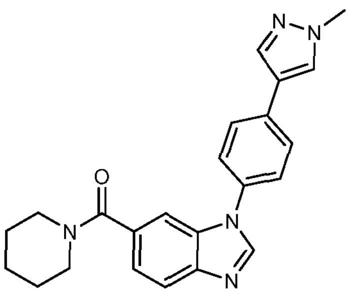 | (1-(4-(1-Methyl-1*H*-pyrazol-4-yl)phenyl)-1*H*-benzo[*d*]imidazol-6-yl) (piperidin-1-yl) methanone | **BCD-CDK8-1-16** |
| 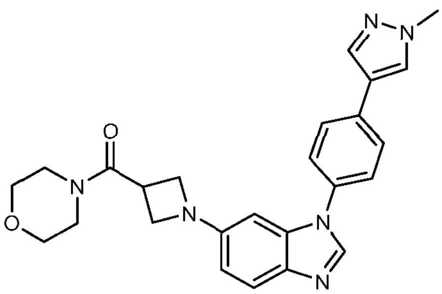 | (1-(1-(4-(1-Methyl-1H-pyrazol-4-yl)phenyl)-1*H*-benzo[*d*]imidazol-6-yl)azetidin-3-yl) (morpholino)methanone | **BCD-CDK8-1-17** |

(continued)

| Formula | Name | Code |
|---|---|---|
| 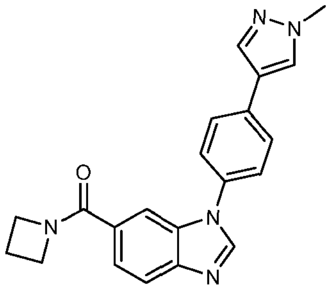 | Azetidin-1-yl(1-(4-(1-methyl-1*H*-pyrazol-4-yl) phenyl)-1*H*-benzo[*d*]imidazol-6-yl) methanone | **BCD-CDK8-1-18** |
| 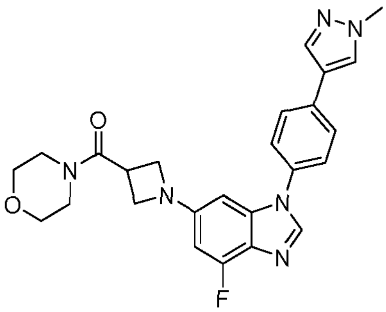 | (1-(1-(4-(1-methyl-1*H*-pyrazol-4-yl)phenyl)-4-fluoro-1*H*-benzo[*d*]imidazol-6-yl) azetidin-3-yl)(morpholino)methanone | **BCD-CDK8-1-19** |
| 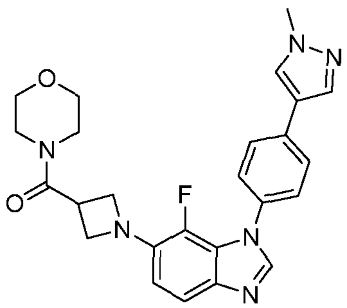 | (1-(1-(4-(1-methyl-1*H*-pyrazol-4-yl)phenyl)-7-fluoro-1*H*-benzo[*d*]imidazol-6-yl) azetidin-3-yl)(morpholino)methanone | **BCD-CDK8-1-20** |
| 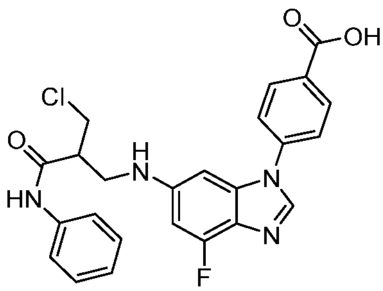 | 4-(6-((2-(Chloromethyl)-3-oxo-3-(phenylamino) propyl)amino)-4-fluoro-1*H*-benzo[d]imidazol-1-yl)benzamide | **BCD-CDK8-1-21A** |
| 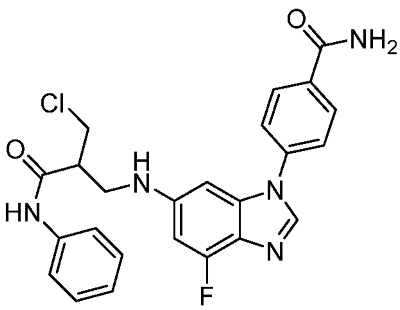 | 4-(6-((2-(Chloromethyl)-3-oxo-3-(phenylamino) propyl)amino)-4-fluoro-1*H*-benzo[*d*]imidazol-1-yl)benzoic acid | **BCD-CDK8-1-21** |

(continued)

| Formula | Name | Code |
|---|---|---|
| 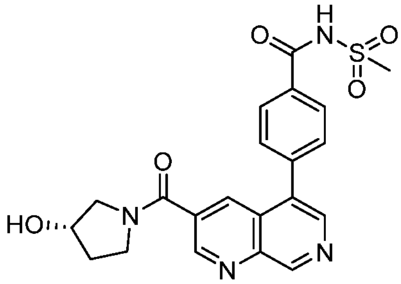 | (*S*)-4-(3-(3-Hydroxypyrrolidine-1-carbonyl)-1,7-naphthyridin-5-yl)-N-(methylsulfonyl) benzamide | **BCD-CDK8-3-1** |
| 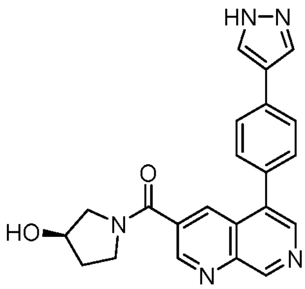 | (*R*)-(5-(4-(1*H*-Pyrazol-4-yl) phenyl)-1,7 -naphthyridin-3 -yl) (3-hydroxypyrrolidin-1-yl) methanone | **BCD-CDK8-3-2** |
| 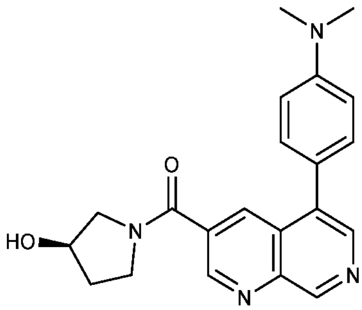 | (*R*)-(5-(4-(dimethylamino) phenyl)-1,7-naphthyridin-3-yl)(3-hydroxypyrrolidin-1-yl) methanone | **BCD-CDK8-3-3** |
| 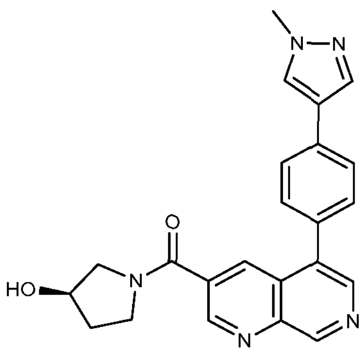 | (*R*)-(3-Hydroxypyrrolidin-1-yl) (5-(4-(1-methyl-1*H*-pyrazol-4-yl)phenyl)-1,7-naphthyridin-3-yl) methanone | **BCD-CDK8-3-4** |
| 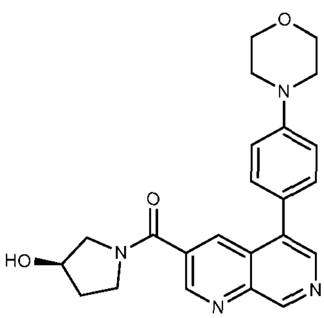 | (*R*)-(3 -Hydroxypyrrolidin-1-yl) (5-(4-morpholinophenyl)-1,7-naphthyridin-3-yl) methanone | **BCD-CDK8-3-5** |

(continued)

| Formula | Name | Code |
|---|---|---|
| 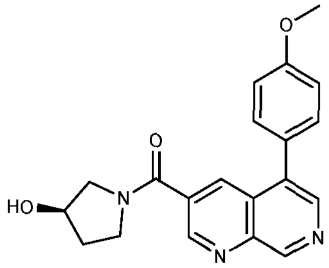 | (*R*)-(3-hydroxypyrrolidin-1-yl) (5-(4-methoxyphenyl)-1,7-naphthyridin-3-yl) methanone | **BCD-CDK8-3-6** |
| 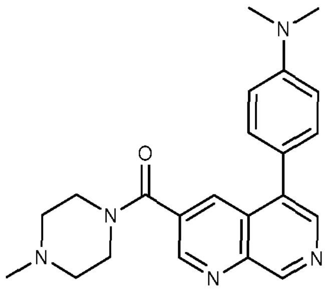 | (5-(4-(Dimethylamino)phenyl)-1,7-naphthyridin-3-yl)(4-methylpiperazin-1-yl) methanone | **BCD-CDK8-3-8** |
| 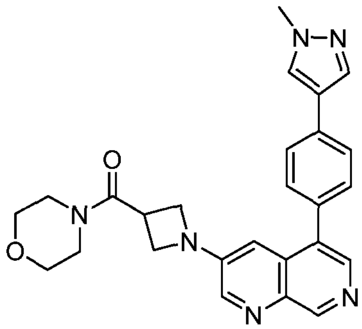 | (1-(5-(4-(1-Methyl-1*H*-pyrazol-4-yl)phenyl)-1,7-naphthyridin-3-yl)azetidin-3-yl) (morpholino) methanone | **BCD-CDK8-3-9** |
| 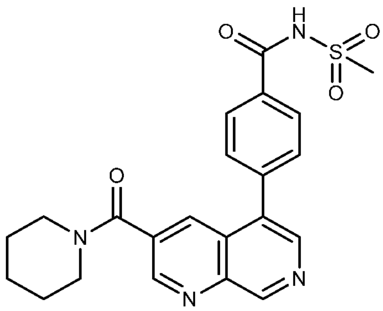 | *N*-(Methyl sulfonyl)-4-(3-(piperidine-1-carbonyl)-1,7-naphthyridin-5-yl)benzamide | **BCD-CDK8-3-11** |
| 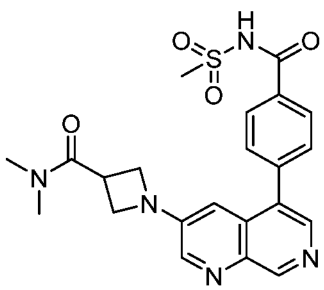 | *N*,*N*-Dimethyl-1-(5-(4-((methylsulfonyl) carbamoyl)phen yl)-1,7-naphthyridin-3-yl) azetidine-3-carboxamide | **BCD-CDK8-3-12** |

(continued)

| Formula | Name | Code |
|---|---|---|
| 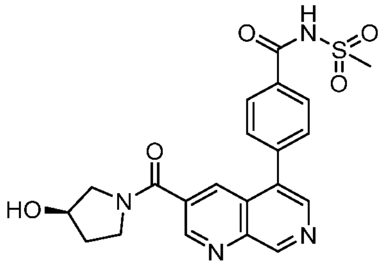 | (*R*)-4-(3-(3-Hydroxypyrrolidine-1-carbonyl)-1,7-naphthyridin-5-yl)-*N*-(methylsulfonyl) benzamide | **BCD-CDK8-3-13** |
| 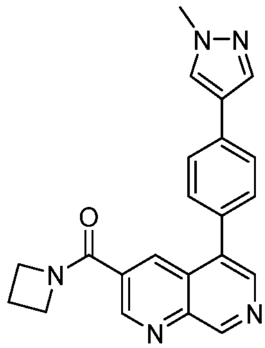 | Azetidin-1-yl-(5-(4-(1-methyl-1*H*-pyrazol-4-yl) phenyl)-1,7-naphthyridin-3-yl) methanone | **BCD-CDK8-3-14** |
| 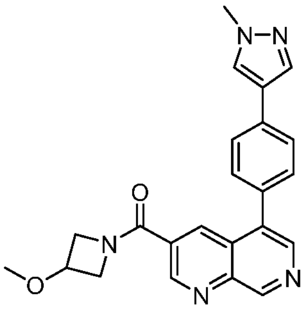 | (3-Methoxyazetidin-1-yl)(5-(4-(1-methyl-1*H*-pyrazol-4-yl) phenyl)-1,7-naphthyridin-3-yl) methanone | **BCD-CDK8-3-15** |
| 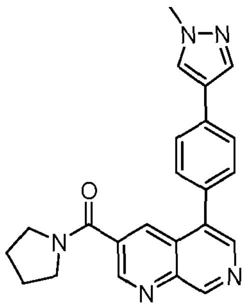 | (5-(4-(1-Methyl-1*H*-pyrazol-4-yl)phenyl)-1,7-naphthyridin-3 - yl)(pyrrolidin-1-yl) methanone | **BCD-CDK8-3-16** |
| 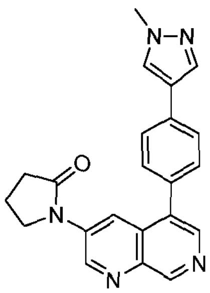 | 1-(5-(4-(1-Methyl-1*H*-pyrazol-4-yl)phenyl)-1,7-naphthyridin-3-yl)pyrrolidin-2-one | **BCD-CDK8-3-17** |

(continued)

| Formula | Name | Code |
|---|---|---|
| 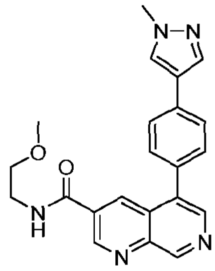 | *N*-(2-Methoxyethyl)-5-(4-(1-methyl-1*H*-pyrazol-4-yl) phenyl)-1,7-naphthyridine-3-carboxamide | **BCD-CDK8-3-18** |
| 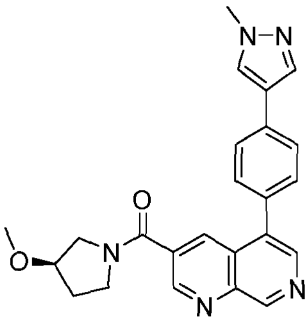 | (*R*)-(3-Methoxypyrrolidin-1-yl)(5-(4-(1-methyl-1*H*-pyrazol-4-yl)phenyl)-1,7-naphthyridin-3-yl) methanone | **BCD-CDK8-3-19** |
| 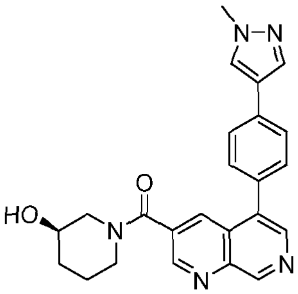 | (*R*)-3-(Hydroxypiperidin-1-yl)(5-(4-(1-methyl-1*H*-pyrazol-4-yl)phenyl)-1,7-naphthyridin-3-yl) methanone | **BCD-CDK8-3-20** |
| 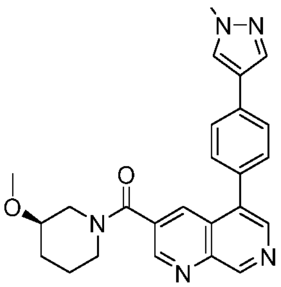 | (*R*)-(3-Methoxypiperidin-1-yl)(5-(4-(1-methyl-1*H*-pyrazol-4-yl)phenyl)-1,7-naphthyridin-3-yl) methanone | **BCD-CDK8-3-21** |
| 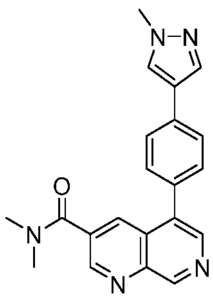 | *N*,*N*-Methyl-5-(4-(1-methyl-1H-pyrazol-4-yl) phenyl)-1,7-naphthyridine-3-carboxamide | **BCD-CDK8-3-22** |

(continued)

| Formula | Name | Code |
|---|---|---|
| 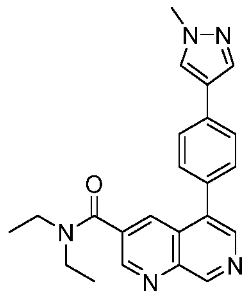 | *N*,*N*-Diethyl-5-(4-(1-methyl-1H-pyrazol-4-yl) phenyl)-1,7-naphthyridine-3-carboxamide | **BCD-CDK8-3-23** |
| 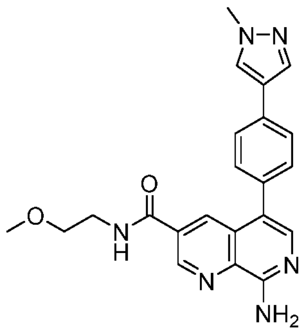 | 8-Amino-*N*-(2-methoxyethyl)-5-(4-(1-methyl-1*H*-pyrazol-4-yl)phenyl)-1,7-naphthyridine-3-carboxamide | **BCD-CDK8-3-24** |
| 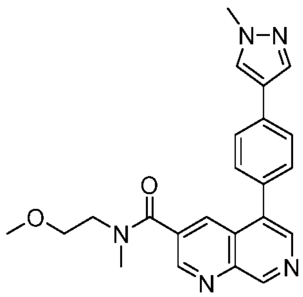 | *N*-(2-Methoxyethyl)-*N*-methyl-5-(4-(1-methyl-1*H*-pyrazol-4-yl)phenyl)-1,7-naphthyridine-3-carboxamide | **BCD-CDK8-3-25** |
| 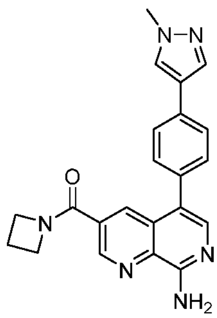 | (8-Amino-5-(4-(1-methyl-1*H*-pyrazol-4-yl) phenyl)-1,7-naphthyridin-3-yl) (azetidin-1-yl) methanone | **BCD-CDK8-3-26** |
| 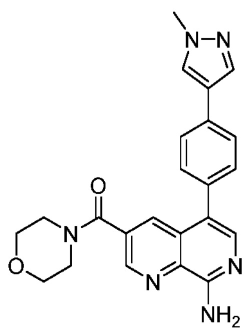 | (8-Amino-5-(4-(1-methyl-1*H*-pyrazol-4-yl) phenyl)-1,7-naphthyridin-3-yl) (morpholino) methanone | **BCD-CDK8-3-27** |

(continued)

| Formula | Name | Code |
|---|---|---|
| 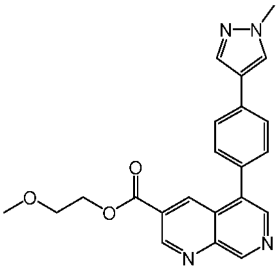 | 2-Methoxyethyl-5-[4-(methyl-1-*H*-pyrazol-4-yl) phenyl]-1,7-naphthyridine-3-carboxylate | **BCD-CDK8-3-28** |
| 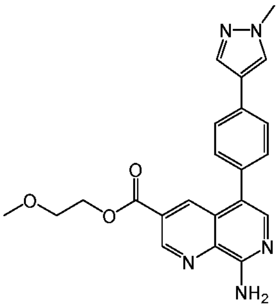 | 2-Methoxyethyl-8-amino-5-(4-(1-methyl-1*H*-pyrazol-4-yl) phenyl)-1,7-naphthyridine-3-carboxylate | **BCD-CDK8-3-29** |
| 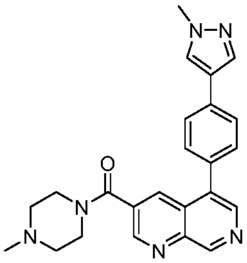 | (4-Methylpiperazin-1-yl)(5-(4-(1-methyl-1*H*-pyrazol-4-yl)phenyl)-1,7-naphthyridin-3-yl) methanone | **BCD-CDK8-3-30** |
| 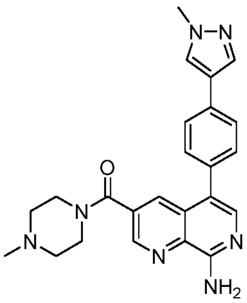 | (8-Amino-5-(4-(1-methyl-1*H*-pyrazol-4-yl) phenyl)-1,7-naphthyridin-3-yl)(4-methylpiperazin-1-yl) methanone | **BCD-CDK8-3-31** |
| 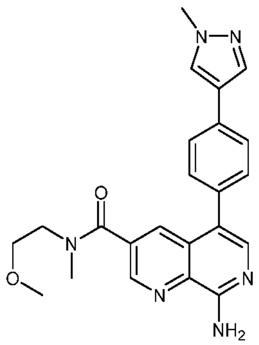 | 8-Amino-*N*-(2-methoxyethyl)-*N*-methyl-(5-(4-(1-methyl-1*H*-pyrazol-4-yl)phenyl)-1,7-naphthyridine-3-carboxamide | **BCD-CDK8-3-32** |

(continued)

| Formula | Name | Code |
|---|---|---|
| 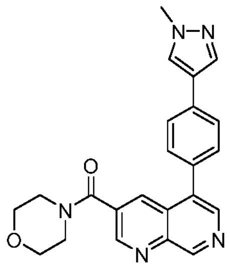 | (5-(4-(1-Methyl-1*H*-pyrazol-4-yl)phenyl)-1,7-naphthyridin-3-yl)(morpholino)methanone | **BCD-CDK8-3-33** |
| 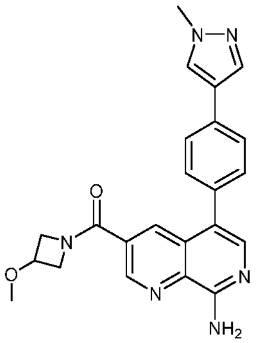 | (8-Amino-5-(4-(1-methyl-1*H*-pyrazol-4-yl) phenyl)-1,7-naphthyridin-3-yl)(3-methoxyazetidin)methanone | **BCD-CDK8-3-34** |
| 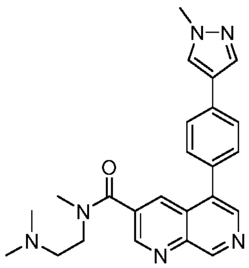 | N-(2-(Dimethylamino)ethyl)-*N*-methyl-5-(4-(1-methyl-1*H*-pyrazol-4-yl)phenyl)-1,7-naphthyridine-3-carboxamide | **BCD-CDK8-3-35** |
| 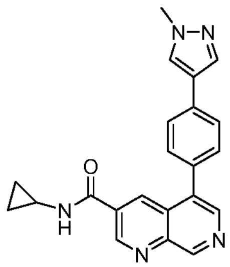 | *N*-Cyclopropyl-5-(-methyl-1*H*-pyrazol-4-yl) phenyl)-1,7-naphthyridine-3-carboxamide | **BCD-CDK8-3-37** |
| 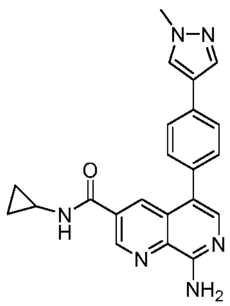 | 8-Amino-*N*-cyclopropyl-5-(4-(1-methyl-1*H*-pyrazol-4-yl)phenyl)-1,7-naphthyridine-3-carboxamide | **BCD-CDK8-3-38** |

(continued)

| Formula | Name | Code |
|---|---|---|
| 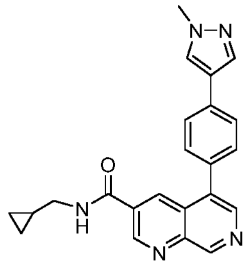 | *N*-(Cyclopropylmethyl)-5-(-methyl-1*H*-pyrazol-4-yl)phenyl)-1,7-naphthyridine-3-carboxamide | **BCD-CDK8-3-39** |
| 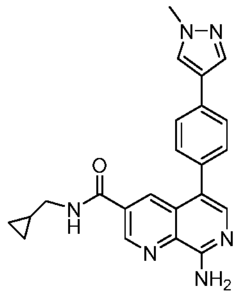 | 8-Amino-*N*-(cyclopropylmethyl)-5-(4-(1-methyl-1*H*-pyrazol-4-yl) phenyl)-1,7-naphthyridine-3-carboxamide | **BCD-CDK8-3-40** |
| 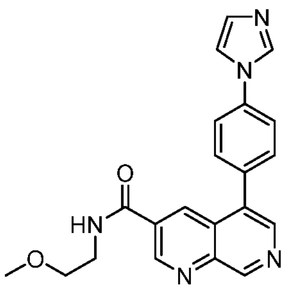 | 5-(4(1*H*-Imidazol-1-yl)phenyl)-N-(2-methoxyethyl)-1,7-naphthyridine-3-yl) carboxamide | **BCD-CDK8-3-41** |
| 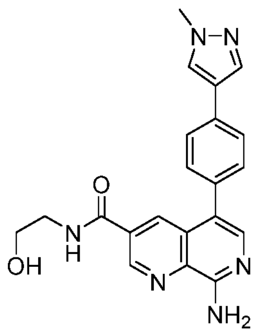 | 8-Amino-*N*-(2-hydroxyethyl)-5-(4-(1-methyl-1*H*-pyrazol-4-yl)phenyl)-1,7-naphthyridine-3-carboxamide | **BCD-CDK8-3-42** |
| 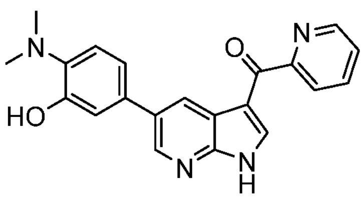 | (5-(4-(Dimethylamino)-3-hydroxyphenyl)-1*H*-pyrrolo[2,3-*b*]pyridin-3-yl)(pyridin-2-yl) methanone | **BCD-CDK8-4-1** |

(continued)

| Formula | Name | Code |
|---|---|---|
| 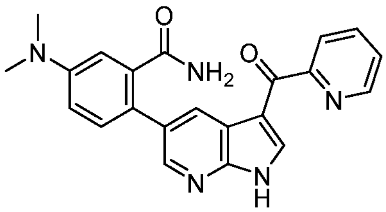 | 5-(Dimethylamino)-2-(3-picolinoyl-1*H*-pyrrolo [2,3-*b*] pyridin-5-yl)benzamide | **BCD-CDK8-4-2** |
| 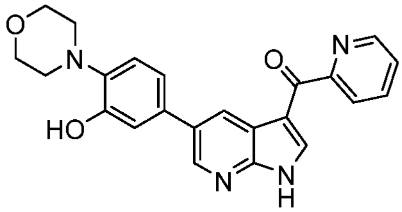 | (5-(3-Hydroxy-4-morpholinophenyl)-1*H*-pyrrolo [2,3-*b*]pyridin-3-yl) (pyridin-2-yl) methanone | **BCD-CDK8-4-3** |
| 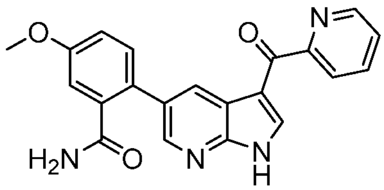 | 5-Methoxy-2-(3-picolinoyl-1*H*-pyrrolo[2,3-*b*] pyridin-5-yl) benzamide | **BCD-CDK8-4-4** |
| 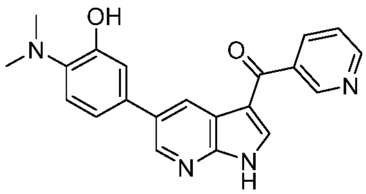 | 5-(4-(Dimethylamino)-3-hydroxyphenyl)-1*H*-pyrrolo[2,3-*b*]pyridin-3-yl) (pyridin-3-yl) methanone | **BCD-CDK8-4-5** |
| 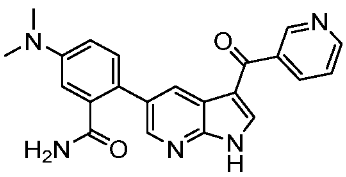 | 5-(Dimethylamino)-2-(3-picolinoyl-1*H*-pyrrolo [2,3-*b*] pyridin-5-yl)benzamide | **BCD-CDK8-4-6** |
| 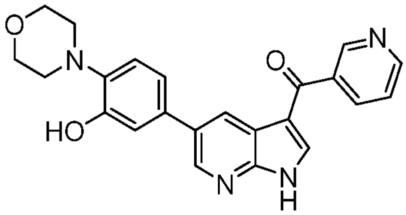 | (5-(3-Hydroxy-4-morpholinophenyl)-1*H*-pyrrolo [2,3-*b*]pyridin-3-yl) (pyridin-3-yl) methanone | **BCD-CDK8-4-7** |
| 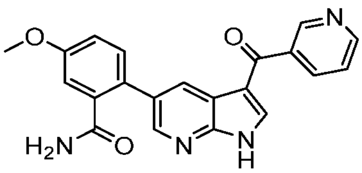 | 5-Methoxy-2-(3-nicotinoyl-1*H*-pyrrolo[2,3-*b* ] pyridin-5-yl) benzamide | **BCD-CDK8-4-8** |

(continued)

| Formula | Name | Code |
|---|---|---|
| 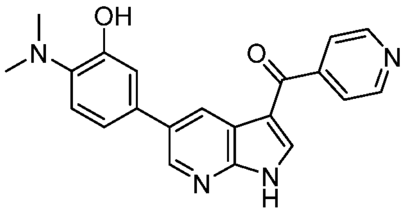 | (5-(4-(Dimethylamino)-3-hydroxyphenyl)-1*H*-pyrrolo.[2,3-*b*]pyridin-3-yl) (pyridin-4-yl) methanone | **BCD-CDK8-4-9** |
| 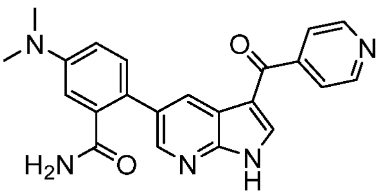 | 5-(Dimethylamino)-2-(3-isonicotinoyl-1*H*-pyrrolo [2,3-*b*] pyridin-5-yl)benzamide | **BCD-CDK8-4-10** |
| 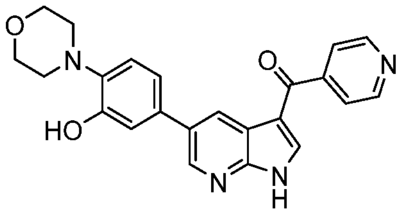 | (5-(3-Hydroxy-4-morpholinophenyl)-1*H*-pyrrolo [2,3-*b*]pyridin-3-yl) (pyridin-4-yl) methanone | **BCD-CDK8-4-11** |
| 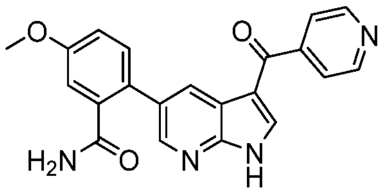 | 2-(3-Isonicotinoyl-1*H*-pyrrolo[2,3-*b*]pyridin-5-yl )-5-methoxybenzamide | **BCD-CDK8-4-12** |
| 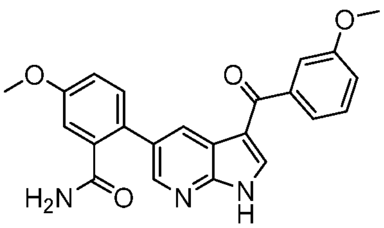 | 5-Methoxy-2-(3-(3-methoxybenzoyl)-1*H*-pyrrolo [2,3-*b*]pyridin-5-yl) benzamide | **BCD-CDK8-4-13** |
| 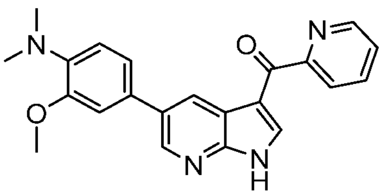 | (5-(4-(Dimethylamino)-3-methoxyphenyl)-1*H*-pyrrolo[2,3-*b*]pyridin-3-yl)(pyridin-2-yl) methanone | **BCD-CDK8-4-21** |
| 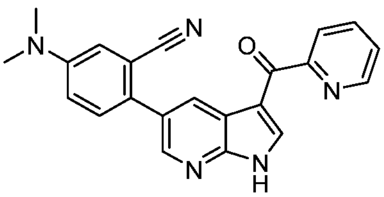 | 5-(Dimethylamino)-2-(3-picolinoyl-1*H*-pyrrolo [2,3-*b*] pyridin-5-yl)benzonitrile | **BCD-CDK8-4-22** |

(continued)

| Formula | Name | Code |
|---|---|---|
| 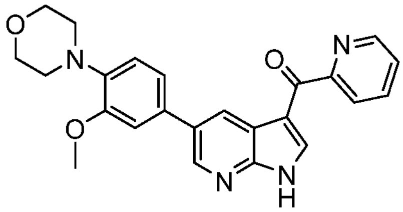 | (5-(3-Methoxy-4-morpholinophenyl)-1*H*-pyrrolo [2,3-*b*]pyridin-3-yl) (pyridin-2-yl) methanone | **BCD-CDK8-4-23** |
| 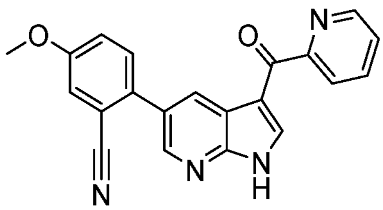 | 5-Methoxy-2-(3-picolinoyl-1*H*-pyrrolo[2,3-*b*] pyridin-5-yl) benzonitrile | **BCD-CDK8-4-24** |
| 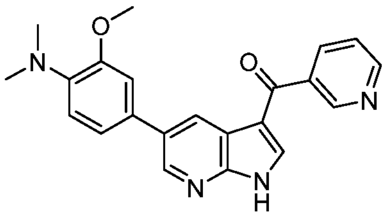 | (5-(4-(Dimethylamino)-3-methoxyphenyl)-1*H*-pyrrolo[2,3-*b*]pyridin-3-yl)(pyridin-3-yl) methanone | **BCD-CDK8-4-25** |
| 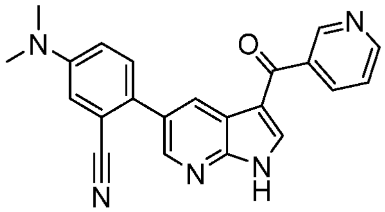 | 5-(Dimethylamino)-2-(3-nicotinoyl-1*H*-pyrrolo [2,3-*b*] pyridin-5-yl)benzonitrile | **BCD-CDK8-4-26** |
| 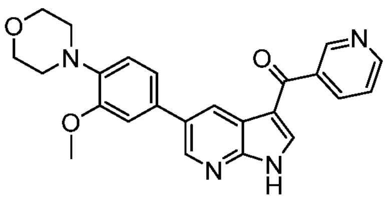 | (5-(3-Methoxy-4-morpholinophenyl)-1*H*-pyrrolo [2,3-*b*]pyridin-3-yl) (pyridin-3-yl) methanone | **BCD-CDK8-4-27** |
| 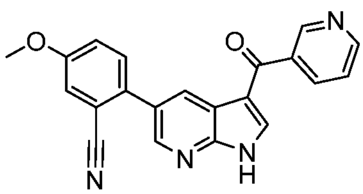 | 5-Methoxy-2-(3-nicotinoyl-1*H*-pyrrolo[2,3-*b*] pyridin-5-yl) benzonitrile | **BCD-CDK8-4-28** |
| 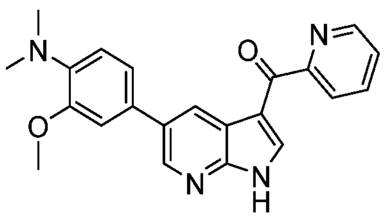 | (5 -(4-(Dimethylamino)-3-methoxyphenyl)-1*H*-pyrrolo[2,3-*b*]pyridin-3-yl)(pyridin-2-yl) methanone | **BCD-CDK8-4-29** |

(continued)

| Formula | Name | Code |
|---|---|---|
| 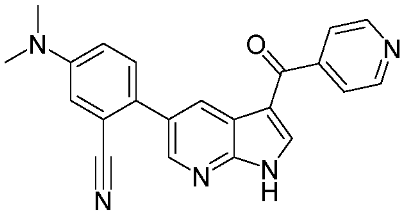 | 5-(Dimethylamino)-2-(3-isonicotinoyl-1*H*-pyrrolo [2,3-*b*] pyridin-5-yl)benzonitrile | **BCD-CDK8-4-30** |
| 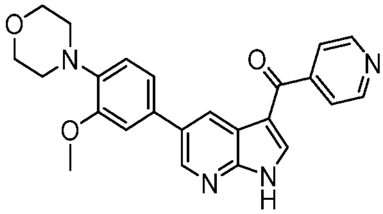 | (5-(3-Methoxy-4-morpholinophenyl)-1*H*-pyrrolo [2,3-*b*]pyridin-3-yl) (pyridin-4-yl) methanone | **BCD-CDK8-4-31** |
| 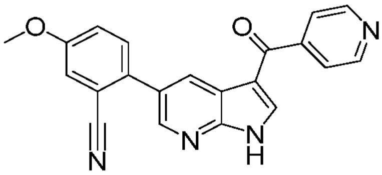 | 2-(3-Isonicotinoyl-1*H*-pyrrolo[2,3-*b*]pyridin-5-yl)-5-methoxybenzonitrile | **BCD-CDK8-4-32** |
| 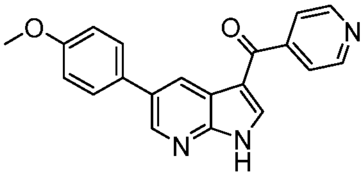 | (5-(4-Methoxyphenyl)-1*H*-pyrrolo[2,3-*b*]pyridin-3-yl) (pyridin-4-yl)methanone | **BCD-CDK8-4-33** |
| 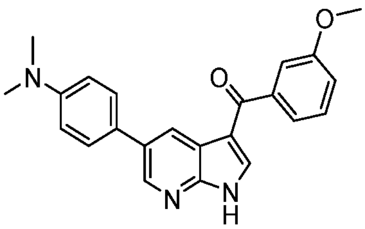 | (5-(4-(Dimethylamino)phenyl)-1*H*-pyrrolo[2,3-*b*] pyridin-3-yl) (3-methoxyphenyl) methanone | **BCD-CDK8-4-34** |
| 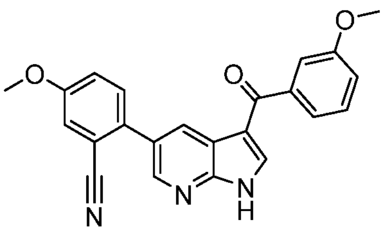 | 5-Methoxy-2-(3-(3-methoxybenzoyl)-1*H*-pyrrolo [2,3-*b*]pyridin-5-yl)benzonitrile | **BCD-CDK8-4-35** |
| 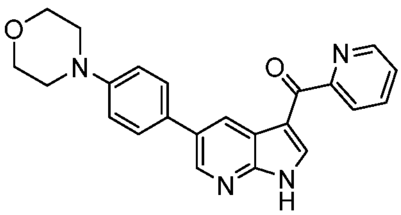 | (5-(4-Morpholinophenyl)-1*H*-pyrrolo[2,3-*b*] pyridin-3-yl) (pyridin-2-yl)methanone | **BCD-CDK8-4-36** |

(continued)

| Formula | Name | Code |
|---|---|---|
| 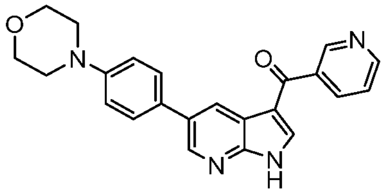 | (5-(4-Morpholinophenyl)-1*H*-pyrrolo[2,3-*b*] pyridin-3-yl) (pyridin-3 -yl)methanone | **BCD-CDK8-4-37** |
| 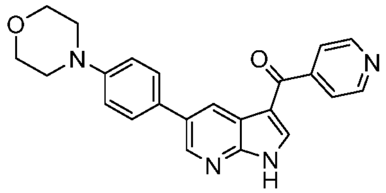 | (5-(4-Morpholinophenyl)-1*H*-pyrrolo[2,3-*b*] pyridin-3-yl) (pyridin-4-yl)methanone | **BCD-CDK8-4-38** |
| 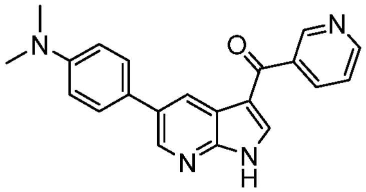 | (5-(4-(Dimethylamino)phenyl)-1*H*-pyrrolo[2,3-*b*] pyridin-3-yl) (pyridin-3-yl)methanone | **BCD-CDK8-4-39** |
| 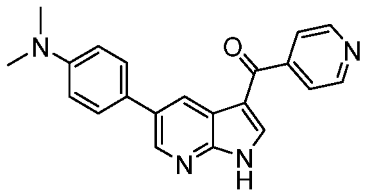 | (5-(4-(Dimethylamino)phenyl)-1*H*-pyrrolo[2,3-*b*] pyridin-3-yl) (pyridin-4-yl) methanone | **BCD-CDK8-4-40** |
| 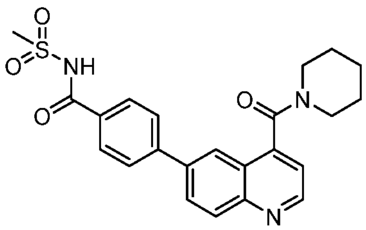 | *N*-(Methylsulfonyl)-4-(4-(pi peridine-1-carbonyl) quinolin-6-yl)benzamide | **BCD-CDK8-5-1** |
| 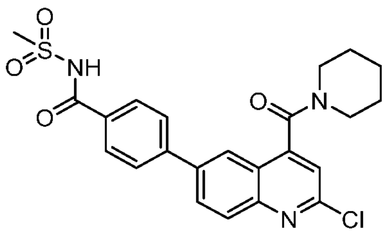 | 4-(4-(Piperidine-1-carbonyl)-2-chloroquinolin-6-yl)-*N*-(methylsulfonyl)benzamide | **BCD-CDK8-5-1Cl** |
| 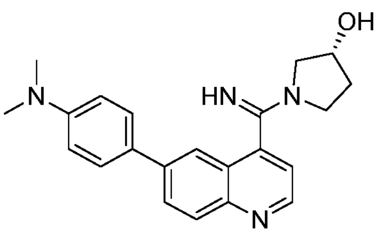 | (*R*)-1-((6-(4-(Dimethylamino) phenyl)quinolin-4-yl)(imino) methyl)pyrrolidin-3-ol | **BCD-CDK8-5-2**i |

(continued)

| Formula | Name | Code |
|---|---|---|
| 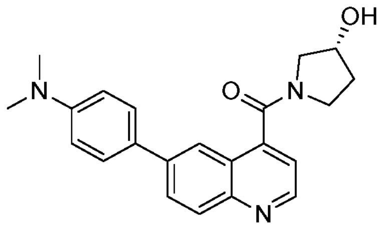 | (*R*)-(6-(4-(Dimethylamino) phenyl)quinolin-4-yl) (3-hydroxypyrrolidin-1-yl) methanone | **BCD-CDK8-5-2** |
| 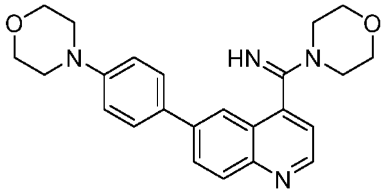 | Morpholino(6-(4-morpholinophenyl)quinolin-4-yl)methanimine | **BCD-CDK8-5-3i** |
| 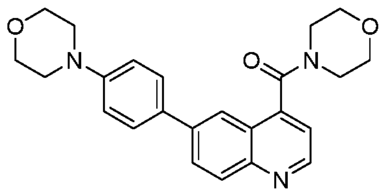 | Morpholino(6-(4-morpholinophenyl)quinolin-4-yl)methanone | **BCD-CDK8-5-3** |
| 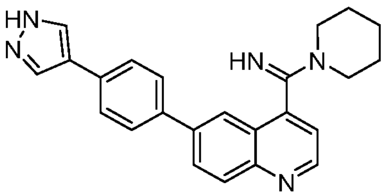 | (6-(4-(1*H*-Pyrazol-4-yl) phenyl)quinolin-4-yl) (piperidin-1-yl)methanimine | **BCD-CDK8-5-4i** |
| 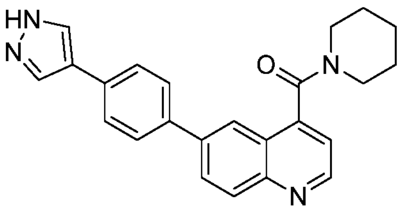 | (6-(4-(1*H*-Pyrazol-4-yl) phenyl)quinolin-4-yl) (piperidin-1-yl)methanone | **BCD-CDK8-5-4** |
| 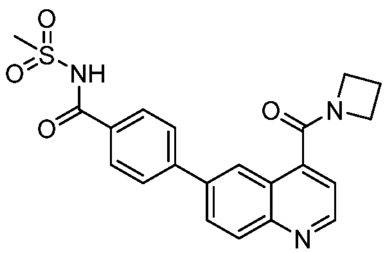 | 4-(4-(Azetidine-1-carbonyl)quinolin-6-yl)-*N*-(methylsulfonyl)benzamide | **BCD-CDK8-5-5** |
| 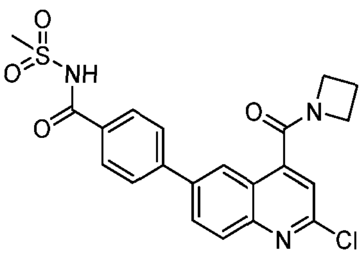 | 4-(4-(Azetidine-1-carbonyl)-2-chloroquinolin-6-yl)-*N*-(methylsulfonyl)benzamide | **BCD-CDK8-5-5Cl** |

(continued)

| Formula | Name | Code |
|---|---|---|
| 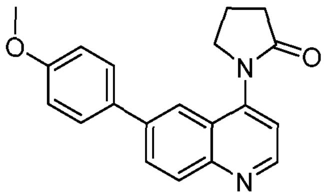 | 1-(6-(4-Methoxyphenyl) quinolin-4-yl)pyrrolidin-2-one | **BCD-CDK8-5-6** |
| 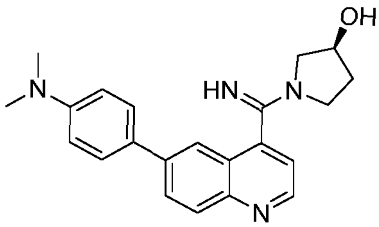 | (*S*)-1-((6-(4-(dimethylamino) phenyl)quinolin-4-yl) (imino)methyl)pyrrolidin-3-ol | **BCD-CDK8-5-7i** |
| 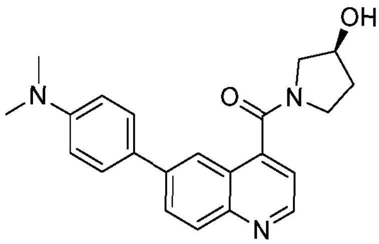 | (S)-(6-(4-(Dimethylamino) phenyl)quinolin-4-yl) (3-hydroxypyrrolidin-1-yl) methanone | **BCD-CDK8-5-7** |
| 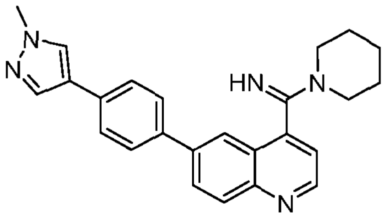 | (6-(4-(1-Methyl-1*H*-pyrazol-4-yl)phenyl)quinolin-4-yl) (piperidin-1-yl) methanimine | **BCD-CDK8-5-8i** |
| 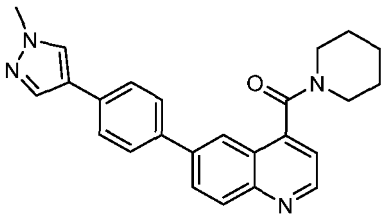 | (6-(4-(1-Methyl-1*H*-pyrazol-4-yl)phenyl)quinolin-4-yl) (piperidin-1-yl)methanone | **BCD-CDK8-5-8** |
| 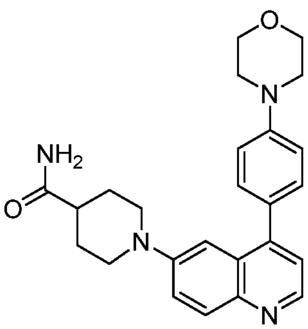 | 1-(4-(4-Morpholinophenyl) quinolin-6-yl) piperidine-4-carboxamide | **BCD-CDK8-6-1** |

(continued)

| Formula | Name | Code |
|---|---|---|
| 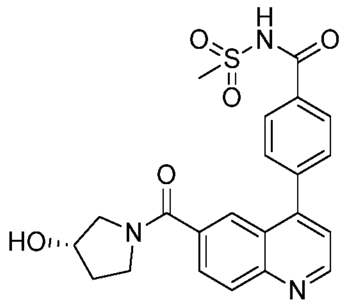 | (*S*)-4-(6-(3-Hydroxypyrrolidine-1-carbonyl) quinolin-4-yl)-*N*-(methylsulfonyl)benzamide | **BCD-CDK8-6-2** |
| 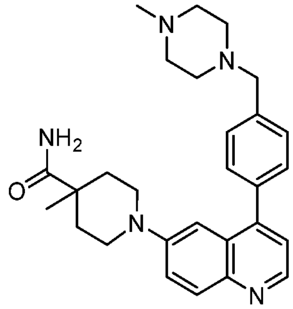 | 4-Methyl-1-(4-(4-((4-methylpiperazin-1-yl) methyl)phenyl)quinolin-6-yl) piperidine-4-carboxamide | **BCD-CDK8-6-3** |
| 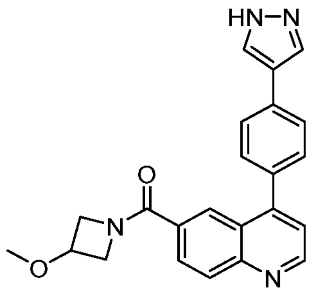 | (4-(4-(1*H*-Pyrazol-4-yl) phenyl)quinolin-6-yl)(3-methoxyazetidin-1-yl) methanone | **BCD-CDK8-6-4** |
| 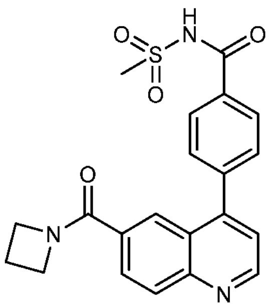 | 4-(6-(Azetidine-1-carbonyl)quinolin-4-yl)-*N*-(methylsulfonyl)benzamide | **BCD-CDK8-6-5** |
| 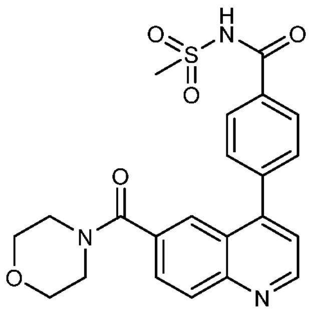 | N-(Methylsulfonyl)-4-(6-(morpholine-4-carbonyl) quinolin-4-yl) benzamide | **BCD-CDK8-6-6** |

(continued)

| Formula | Name | Code |
|---|---|---|
| 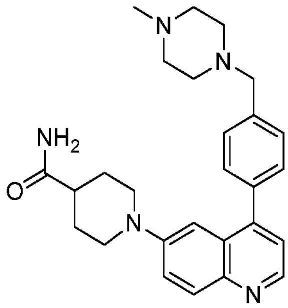 | 1-(4-(4-((4-Methylpiperazin-1-yl)methyl)phenyl) quinolin-6-yl)piperidine-4-carboxamide | **BCD-CDK8-6-7** |
| 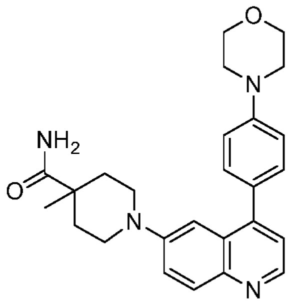 | 4-Methyl-1-(4-(4-morpholinopheny1)quinolin-6-yl)piperidine-4-carboxamide | **BCD-CDK8-6-8** |
| 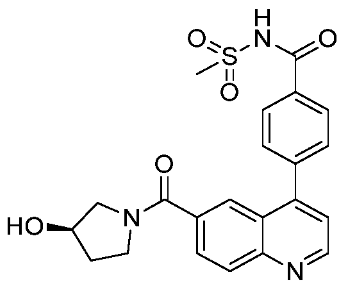 | (*R*)-4-(6-(3-Hydroxypyrrolidine-1-carbonyl) quinolin-4-yl)-*N*-(methylsulfonyl)benzamide | **BCD-CDK8-6-9** |
| 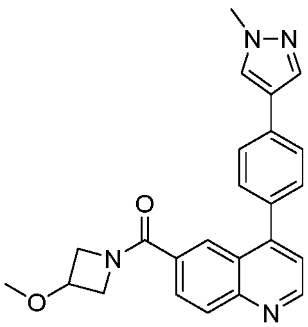 | (3-Methoxyazetidin-1-yl)(4-(4-(1-methyl-1*H*-pyrazol-4-yl) phenyl)quinolin-6-yl) methanone | **BCD-CDK8-6-10** |
| 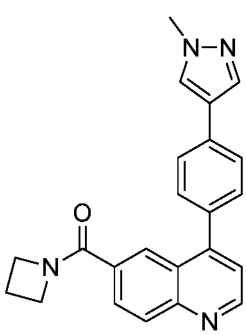 | Azetidin-1-yl(4-(4-(1-methyl-1*H*-pyrazol-4-yl) phenyl) quinolin-6-yl)methanone | **BCD-CDK8-6-11** |

(continued)

| Formula | Name | Code |
|---|---|---|
| 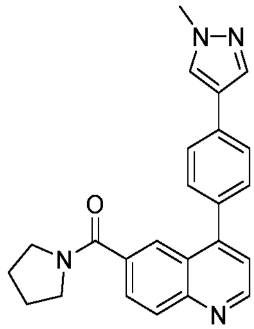 | (4-(4-(1-Methyl-1*H*-pyrazol-4-yl)phenyl)quinolin-6-yl) (pyrrolidin-1-yl)methanone | **BCD-CDK8-6-12** |
| 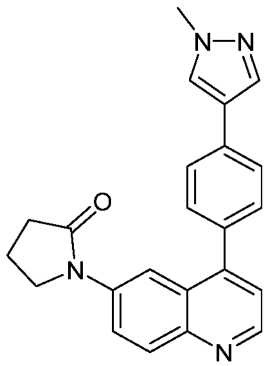 | 1-(4-(4-(1-Methyl-1*H*-pyrazol-4-yl)phenyl) quinolin-6-yl) pyrrolidin-2-one | **BCD-CDK8-6-13** |
| 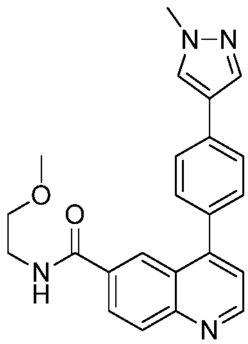 | *N*-(2-Methoxyethyl)-4-(4-(1-methyl-1*H*-pyrazol-4-yl) phenyl)quinoline-6-carboxamide | **BCD-CDK8-6-14** |

The compounds and processes of the present invention will be better understood in conjunction with the following synthesis schemes , which demonstrate the methods by which the compounds of the present invention can be obtained. Initial products can be obtained commercially or by conventional techniques of the prior art, which are known to those having ordinary skill in the art. It will also be obvious to a person having ordinary skill in the art that the steps of selective introduction of protection and deprotection, as well as the order of said steps, can be carried out in different order, depending on the nature of substituents, for the purpose of successful completion of synthesis described below.

Abbreviations in this description, including those shown in the illustrative schemes and the examples described below are well-know for an average person skilled in the art. Some of the abbreviations are as follows:

dimethyl sulfoxide - DMSO
(±)-2.2'-*bis*(diphenylphosphino)-1. 1'-dinaphthalene - BINAP
4-methyl-benzene sulfonic acid - PTSA
4-dimethylaminopyridine - DMAP
*N*-(3-dimethylaminopropyl)-*N*'-ethylcarbodiimide hydrochloride - EDC'HCl
1-hydroxybenzotriazole hydrate - HOBt
diisopropylethylamine - DIPEA
*N*,*N*-dimethylformamide - DMF
tetrakis(triphenylphosphine)palladium(0) - $Pd(PPh_3)_4$

tetrahydrofuran - THF
2-dicyclohexylphosphino-2',4',6'-triisopropylbiphenyl - XPhos
methyl- *tert*-butyl ether - MTBE
diphenylphosphoryl azide - DPPA
2-(trimethylsilyl)ethoxymethyl chloride - SEMCl.

The compounds of the present invention can be prepared according to Scheme 1.

Scheme 1.

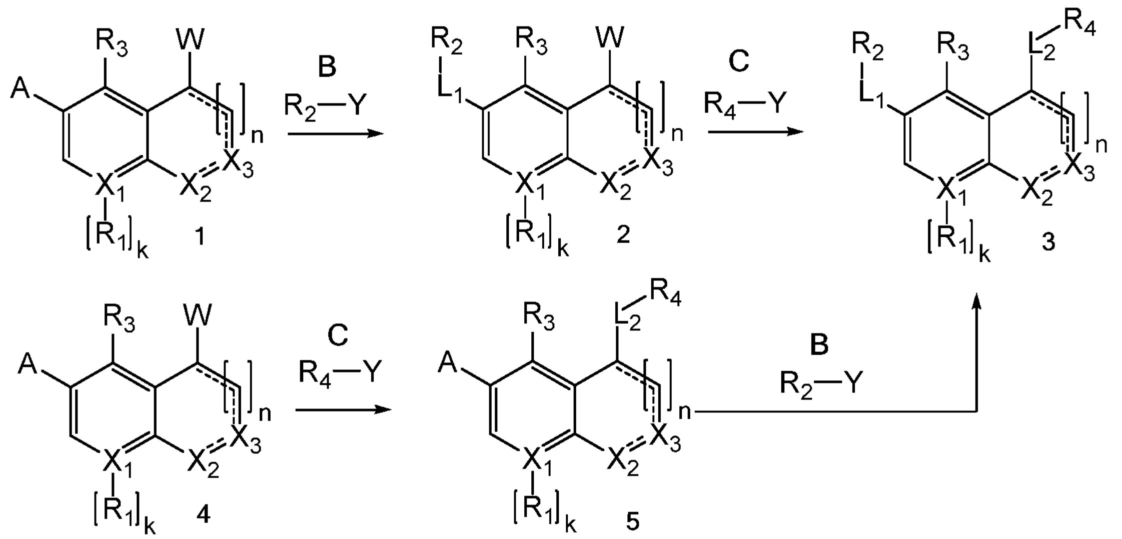


wherein each $X_2$, $X_3$ independently is $C(H)_m$, N, NH;

each $X_1$ independently is C, CH, N;
each $L_1$, $L_2$ independently is chemical bond, $-C(R_6)_2-$, -O-, -C(O)-, -NH-, $-C(=NR_{19})-$;
n is 0, 1;
k is 0, 1;
m is 1, 2;
each $R_1$, $R_3$ independently is H, Hal, cyano, $C_1$-$C_6$ alkyl;
each $R_2$, $R_4$ independently is selected from group, consisting of:

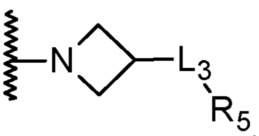, 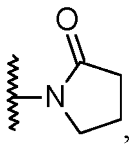, 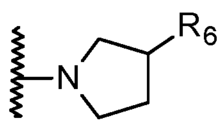, 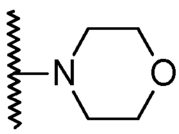,

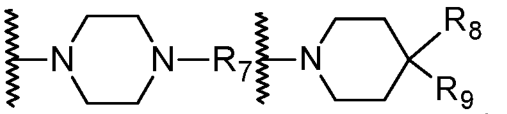, 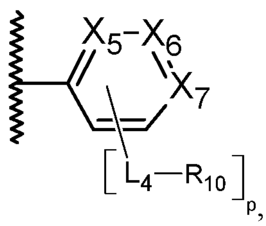,

$-NR_{11}R_{12}$; $C_1$-$C_6$ alkyl, unsubstituted or substituted by one or several substituents $R_{14}$;
each $X_5$, $X_6$, $X_7$ independently is C, CH, N;
each $L_3$, $L_4$ independently is a chemical bond, -C(O)-, -O-, $-CH_2-$, -NH-, $-C(O)-NR_7-$, -C(=NH)-;

p = 0, 1, 2, 3, 4;
$R_5$ is H; Hal; cyano; $C_1$-$C_6$ alkyl; $C_1$-$C_6$ alkyloxy; $C_1$-$C_6$ alkyloxy $C_1$-$C_6$ alkyl; $NR_{15}R_{16}$; aryl, unsubstituted or substituted by one or several substituents, selected from group, consisting of Hal, $C_1$-$C_6$ alkyl, $C_1$-$C_6$ alkyloxy, $NR_{15}R_{16}$; 5-6 membered heterocyclyl with 1-2 heteroatoms, selected from N and/or O, unsubstituted or substituted by one or several substituents, selected from group, consisting of Hal, $C_1$-$C_6$ alkyl, $C_1$-$C_6$ alkyloxy, $NR_{15}R_{16}$ ;
each $R_6$ independently is H, Hal, hydroxy, $C_1$-$C_6$ alkyloxy;
each $R_7$ independently is H, $C_1$-$C_6$ alkyl;
each $R_8$, $R_9$ independently is H, $C_1$-$C_6$ alkyl, -C(O)-$NR_{21}R_{22}$, -CN, -C(O)-$OR_{20}$; or
$R_8$ and $R_9$ together with the carbon atom they are attached to, form 5-6-membered heterocyclic ring with 1-2 heteroatoms, selected from nitrogen and/or oxygen, wherein heterocyclic ring, formed by $R_8$ and $R_9$, could be unsubstituted or substituted by 1 or 2 substituents, selected from oxo group, $C_1$-$C_6$ alkyl;
each $R_{10}$ independently is selected from group, consisting of H, Hal, $C_1$-$C_6$ alkyl, hydroxy, cyano, $C_1$-$C_6$ alkyloxy, $C_1$-$C_6$ alkyloxy $C_1$-$C_6$ alkyl, -$NR_{23}R_{24}$; 5-6 membered heterocyclyl with 1-2 heteroatoms, selected from N, O and/or S, unsubstituted or substituted by one or several $C_1$-$C_6$ alkyl; 5-6 membered heteroaryl with 1-2 heteroatoms, selected from N, O and/or S, unsubstituted or substituted by one or several $C_1$-$C_6$ alkyl; -$S(O)_2$-$C_1$-$C_6$ alkyl;
each $R_{11}$, $R_{12}$ independently is H, hydroxy, $C_1$-$C_6$ alkyl, $C_1$-$C_6$ alkoxy, $C_1$-$C_6$ alkyloxy $C_1$-$C_6$ alkyl;
each $R_{14}$ independently is Hal, -C(O)$NR_{17}R_{18}$;
each $R_{15}$, $R_{16}$, $R_{21}$, $R_{22}$, $R_{23}$, $R_{24}$ independently is H, $C_1$-$C_6$ alkyl;
each $R_{17}$, $R_{18}$ independently is H, $C_1$-$C_6$ alkyl; aryl, unsubstituted or substituted by one or several substituents selected from group consisting of Hal, $C_1$-$C_6$ alkyl, $C_1$-$C_6$ alkyloxy;
each $R_{19}$, $R_{20}$ independently is H, $C_1$-$C_6$ alkyl;
each Y independently is H, Hal, $NH_2$, OH, -$B(OH)_2$, -CON(Me)-OMe, -C(O)Cl; A, W is H, Hal, CN, -C(O)OH, -C(O)Cl

In the case when OH, NH groups are present in the initial reagents, intermediate compounds, said groups can be protected by (2-(trimethylsilyl)ethoxy)methylacetal protecting group, trialkylsilyl protecting group or alkyl(diaryl)silyl protecting group.

The reaction between compound **1** and compound **B** with the formation of compound **2** can be carried out by the following reactions: Suzuki reaction between aryl halide **1** and arylboronic acid **B** in the presence of a palladium catalyst and base (A = Hal, Y = -B $(OH)_2$); Buchwald-Hartwig reaction between aryl halide **1** and amine **B** in the presence of palladium catalyst and base (A = Hal, Y = H); between carboxylic acid **1** and amine **B** according to the methodology for obtaining an amide bond with the participation of a carbodiimide (A = COOH, Y = H); between acyl chloride **1** and amine **B** according to the methodology for obtaining an amide bond in the presence of a base, such as trialkylamine (A = C (O) Cl, Y = H). Compound **3** can be synthesized from compound **2** and compound **C** by the following reactions: Suzuki reaction between aryl halide **2** and aryl boronic acid **C** in the presence of a palladium catalyst and base (W = Hal, Y = -B $(OH)_2$); Buchwald-Hartwig reaction between aryl halide **2** and amine **C** in the presence of a palladium catalyst and base (W = Hal, Y = H); between carboxylic acid **2** and amine **C** according to the methodology for obtaining an amide bond with the participation of a carbodiimide (W = COOH, Y = H); between acyl chloride **2** and amine **C** according to the methodology for obtaining an amide bond in the presence of a base, such as trialkylamine (W = C (O) Cl, Y = H); between carbonitrile **2** and amine **C** according to the methodology for obtaining amidines using organomagnesium reagents (W = CN, Y = H). Compound **5** can be synthesized from compound **4** and compound **C** by the following reactions: Suzuki reaction between aryl halide **4** and arylboronic acid **C** in the presence of a palladium catalyst and base (W = Hal, Y = -B $(OH)_2$); Buchwald-Hartwig reaction between aryl halide **4** and amine **C** in the presence of a palladium catalyst and base (W = Hal, Y = H); between carboxylic acid **4** and amine **C** according to the methodology for obtaining an amide bond with the participation of a carbodiimide (W = COOH, Y = H); between acyl chloride **4** and amine **C** according to the methodology for obtaining an amide bond in the presence of a base, such as trialkylamine (W = C (O) Cl, Y = H); between carbonitrile **4** and amine **C** according to the methodology for obtaining amidines using organomagnesium reagents (W = CN, Y = H); between compound **4** and acid chloride **C** by acylation reaction (W = H, Y = C (O) Cl); between aryl halide **4** and Weinreb amide **C** by the Grignard reaction (W =Hal, Y = C(O)N(Me)-OMe). The reaction between compound **5** and compound **B** with the formation of compound **3** can be carried out by the following reactions: Suzuki reaction between aryl halide **5** and arylboronic acid **B** in the presence of a palladium catalyst and base (A = Hal, Y = -B $(OH)_2$); Buchwald-Hartwig reaction between aryl halide **5** and amine **B** in the presence of palladium catalyst and base (A = Hal, Y = H); between carboxylic acid **5** and amine **B** according to the methodology for obtaining an amide bond with the participation of a carbodiimide (A = COOH, Y = H); between acyl chloride **5** and amine **B** according to the methodology for obtaining an amide bond in the presence of a base, such as trialkylamine (A = C (O) Cl, Y = H).

Compound **2'** is a variant of compound 2, wherein W is halogen, n is 1, and can be prepared according to Scheme 2.

Scheme 2.

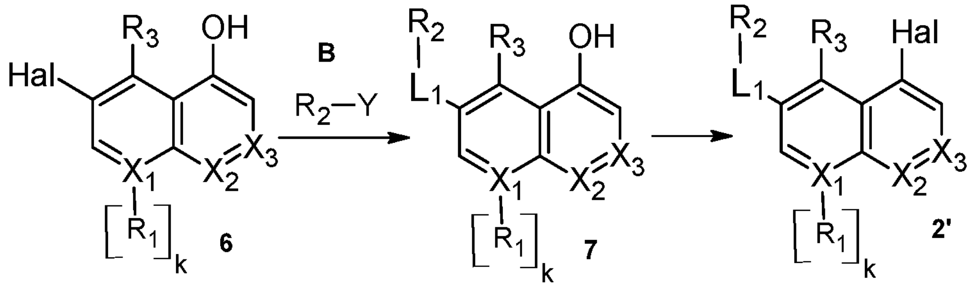


6 7 2' ,

wherein $X_1$, $X_2$, $X_3$, $R_1$, $R_2$, $R_3$, $L_1$, k, Hal, Y have the above meanings.

The reaction between compound **6** and compound **B** with the formation of compound 7 can be carried out analogically to the methods described above and used to obtain compound **2** from compound **1** and compound **B.** Compound **2'** can be prepared by reacting compound 7 with halide or phosphorus oxyhalide, such as $POCl_3$, $PBr_3$.

Compound **5'** is a variant of compound **5,** wherein A is halogen, and can be prepared from compound **8** according to Scheme 3.

Scheme 3.

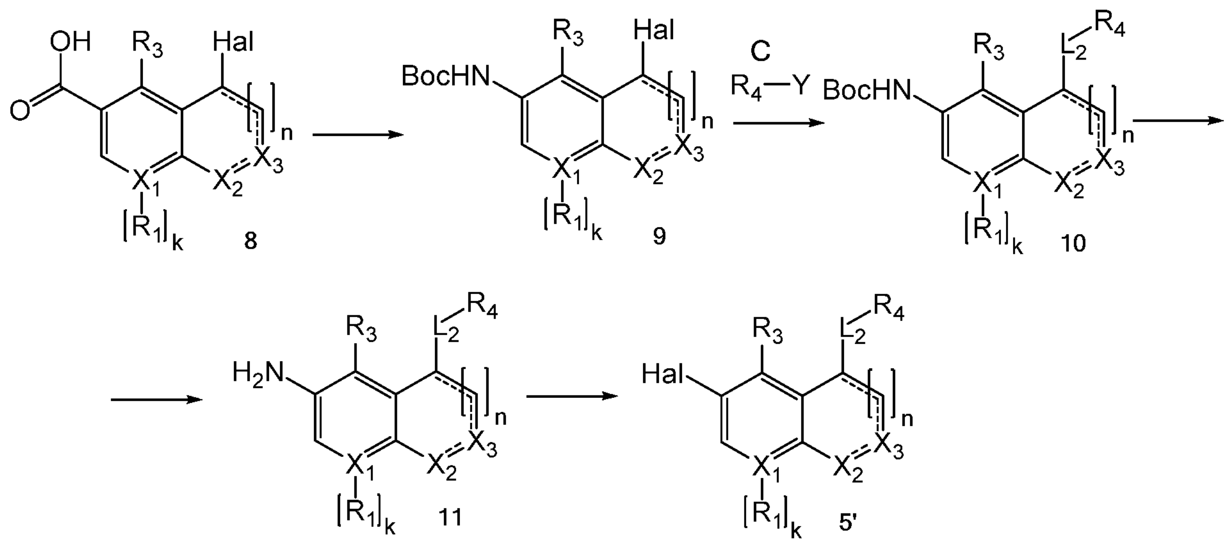


8 9 10 11 5'

, wherein $X_1$, $X_2$, $X_3$, $R_1$, $R_3$, $R_4$, $L_2$, n, k, Hal, Y have the above meanings.

Compound **9** can be prepared by reacting **8** with diphenylphosphoryl azide in *tert*-butanol while being heated. Compound **10** can be synthesized from compound **9** and compound **C** by the following reactions: Suzuki reaction between aryl halide **9** and arylboronic acid **C** in the presence of a palladium catalyst and base (Y = $-B(OH)_2$); Buchwald-Hartwig reaction between aryl halide **9** and amine **C** in the presence of a palladium catalyst and base (Y = H). Compound **11** can be prepared by reacting compound **10** with a strong acid, such as HCl or trifluoroacetic acid. Compound **5'** can be prepared from compound **11** by the diazotization reaction with sodium nitrite, followed by the substitution of a diazonium group for halogen by the reaction with an appropriate metal halide, such as potassium iodide.

Compound **3'** is a variant of compound **3,** wherein $L_2$ is -C(O)-; and compound **2'** can be converted into compound **3'** according to Scheme 4.

Scheme 4.

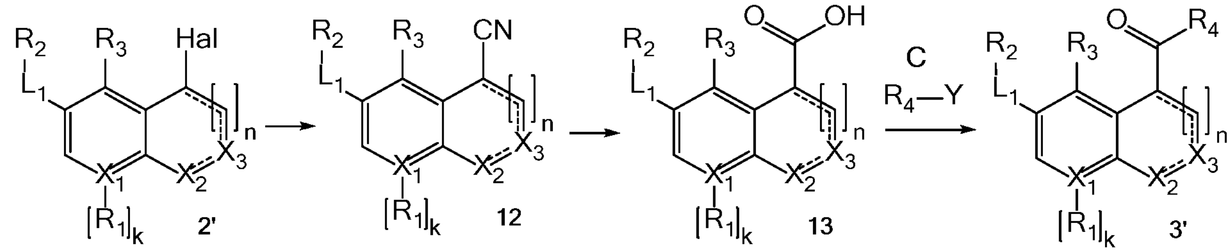


, wherein $X_1$, $X_2$, $X_3$, $R_1$, $R_2$, $R_3$, $R_4$, $L_1$, n, k, Hal, Y have the above meanings.

Compound **12** can be prepared from compound **2’** by the cyanidation reaction in the presence of zinc cyanide and palladium catalyst. Compound **12** can be hydrolyzed to compound **13** under the action of water-alcoholic alkali solution while being heated. Compound 3’ can be prepared by reacting carboxylic acid **13** with amine **C** according to the methodology for obtaining an amide bond with the participation of carbodiimide.

Compound **3"** is a variant of compound **3,** wherein $L_2$ is -C(N=$R_{19}$)-; and compound **12** can be converted into compound **3"** according to Scheme 5.

Scheme 5.

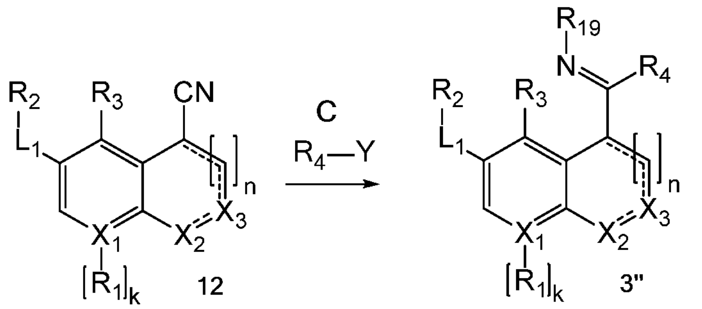


,

wherein $X_1$, $X_2$, $X_3$, $R_1$, $R_2$, $R_3$, $R_4$, $R_{19}$, $L_1$, n, k, Y have the above meanings.

Amidine 3" can be prepared by reacting nitrile **12** with amine **C** in the presence of organomagnesium reagents.

Compound **3’"** is a variant of compound **3,** wherein $L_1$ is -C(O)-; and compound **14** can be converted into compound **3’"** according to Scheme 6.

Scheme 6.

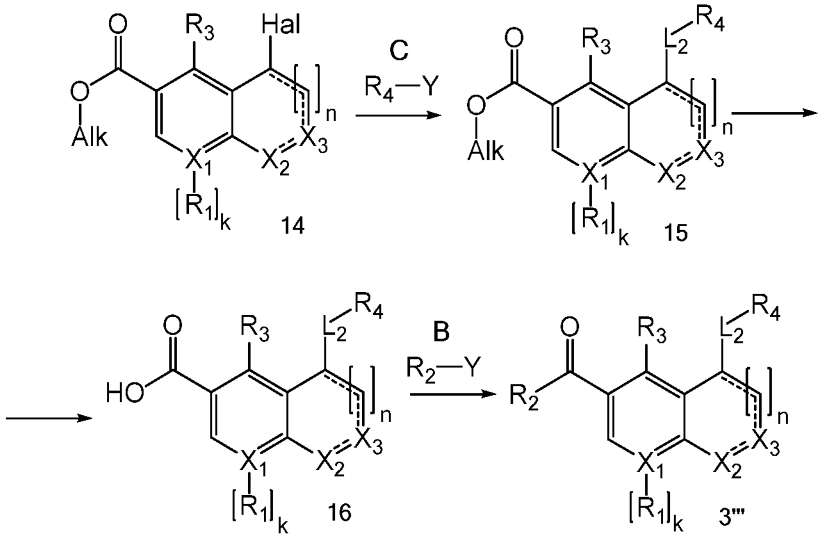


,

wherein $X_1$, $X_2$, $X_3$, $R_1$, $R_2$, $R_3$, $R_4$, $L_2$, n, k, Y, Hal have the above meanings.

Compound **15** can be prepared from compound **14** analogically to the methods described above and used to prepare compound **10** from compound **9** and compound **C.** Compound **16** can be synthesized by hydrolysis of compound **15** by a water-alcohol alkali solution. The reaction between compound **16** and compound **B** with the formation of compound **3'"** can be carried out in the presence of carbodiimide and a base.

The compounds of the present invention can be prepared according to Scheme 7.

Scheme 7.

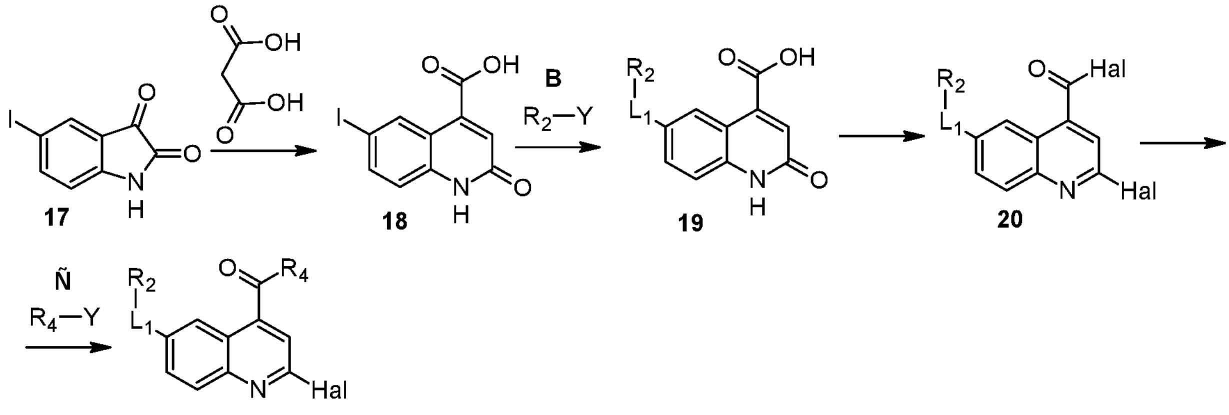


**17** **18** **19** **20** **21**

,

wherein $R_2$, $R_4$, $L_1$, Y, Hal have the above meanings.

Compound **18** can be prepared by reacting compound **17** with malonic acid while heating. Compound 19 can be prepared by the Suzuki reaction between aryl halide **18** and arylboronic acid **B** in the presence of a palladium catalyst and base (Y = $-B(OH)_2$) or the Buchwald-Hartwig reaction between aryl halide **18**and amine **B** in the presence of a palladium catalyst and base (Y = H). Compound **20** can be prepared by reacting with a water-removing halogenating agent such as thionyl chloride. Compound **21** can be prepared by reacting compond **20** with amine **C.**

Said method of preparation may further include a step of transforming compound **21** into compound **22** in the presence of trialkylamine, Pd/C under hydrogen atmospheric conditions.

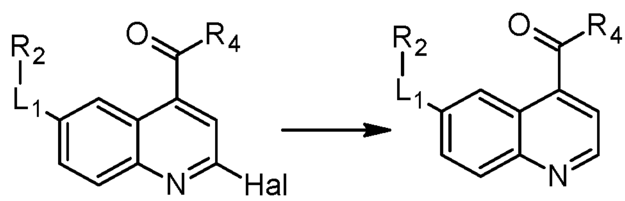

**21** **22**

,

wherein $R_2$, $R_4$, $L_1$, Hal have the above meanings.

Compounds of the present invention can be prepared according to Scheme 8.

Scheme 8.

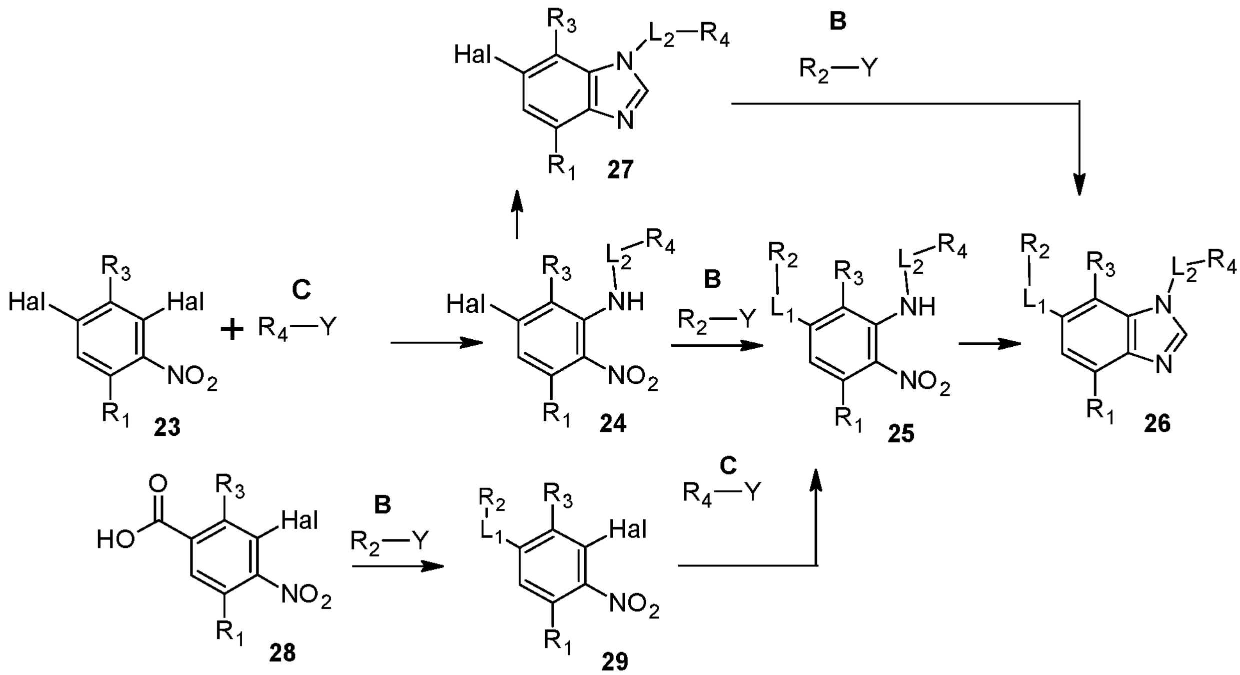

27 23 24 25 26 28 29

,

wherein $R_1$, $R_2$, $R_3$, $R_4$, $L_1$, $L_2$, Y, Hal have the above meanings.

The reaction of compound **23** with amine **C** can be carried out in the presence of a base such as potassium *tert*-butoxide or diisopropylethylamine in DMSO, resulting in compound **24.** Compound **24** can be converted into compound **25** by reacting with compound **B** in the presence of a catalyst such as complex palladium compound with organophosphorus ligands. Compound **26** can be prepared from compound **25** by the hydrogen reduction reaction on Pd/C, followed by cyclization under the action of a trialkyl orthoester of formic acid with acid catalysis. The reaction of substituted nitrobenzoic acid **28** with compound **B** can be carried out in the presence of a non-nucleophilic base, such as trialkylamine, and carbodiimide, resulting in compound **29.** The reaction between compound **29** and amine **C** with the formation of compound **25** is carried out analogically to the methods described above and used to obtain compound **24** from compound **23** and compound **C.** Compound **24** can be converted into compound **27** analogically to the methods described above and used to obtain compound **26** from compound **25.** Compound **27** can be converted into compound **26** analogically to the methods described above and used to prepare compound **25** from compound **24.**

The compounds of the present invention can be prepared according to Scheme 9.

Scheme 9.

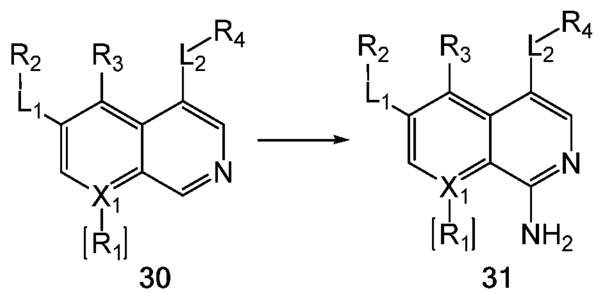

30 31

Compound **30** can be converted into compound **31** by reacting with an oxidizer, such as *m*-chloroperbenzoic acid or hydrogen peroxide with the formation of *N*-oxide, followed by the reaction of said *N*-oxide with sulfonic acid halide, such as benzenesulfonyl chloride or tosyl chloride, followed by the reaction with an amine, such as ethanolamine or isopropylamine.

In the case when OH, NH groups are present in the initial reagents, intermediate compounds, said groups can

be protected by (2-(trimethylsilyl)ethoxy)methylacetal protecting group, trialkylsilyl protecting group or alkyl(diaryl)silyl protecting group. The protecting group can be removed in the final steps of the synthesis by a strong acid, such as HCl or trifluoroacetic acid.

In cases when radicals $R_2$, $R_4$ in the target structures comprise an ester group -COO$C_1$-$C_6$ alkyl, said ester group can be converted into -COOH group by reacting with a strong base, such as lithium hydroxide, sodium hydroxide or potassium hydroxide, followed by treatment with an acid, such as hydrochloric acid or citric acid. Said -COOH group can then be converted into -C(O)$NH_2$ functional group by reacting with ammonium chloride in the presence of carbodiimide, or can be converted into functional group -C(O)-NH-$S(O)_2$-$C_1$-$C_6$alkyl by reacting with $C_1$-$C_6$ alkyl-$S(O)_2$-$NH_2$. In case when radicals $R_2$, $R_4$ in the target structures contain CN functional group, said functional group can be hydrolyzed into -C(O)$NH_2$ functional group. NH group in a heterocyclic fragment, e. g., in pyrazole, can be converted into N-$C_1$-$C_6$ alkyl group using alkylating agents, such as methyl iodide, ethyl bromide, isopropyl iodide, in the presence of a base, such as sodium hydride. -O$C_1$-$C_6$ alkyl group can be converted into OH group by reacting with $AlCl_3$ while heating. Ring opening of the azetidine cycle can be catalyzed by a hydrohalic acid, such as hydrochloric acid, with the production of hydrogen halide as an additive. It is no necessary for intermediate compounds to be prepared and isolated as acid addition salts, e. g., hydrochloride, trifluoroacetate and others, or with bases, such as a sodium salt, potassium salt, ammonium salt, trialkylammonium salt and other salts.

The present invention also relates to a method for inhibiting of biological activity of cyclin-dependent protein kinases CDK8/19 in a subject, comprising contacting the cyclin-dependent protein kinases CDK8/19 with the compound described herein.

In one embodiment, the present invention relates to a pharmaceutical composition that comprises a therapeutically effective amount of at least one of the compounds described herein, or pharmaceutically acceptable salt, solvate thereof, and one or more pharmaceutically acceptable excipients. In another one embodiment, the pharmaceutical composition of the present invention is intended to treat or prevent a disease or disorder mediated by the activation of cyclin-dependent protein kinases CDK8/19. In another one embodiment, the present invention relates to a pharmaceutical composition for the prevention or treatment of a disease or disorder mediated by the activation of cyclin-dependent protein kinases CDK8/19, wherein the disease or disorder mediated by the activation of cyclin-dependent protein kinases CDK8/19 is oncological or haemato-oncological disease. In another one embodiment, the pharmaceutical composition of the present invention is intended to treat or prevent colorectal cancer, melanoma, metastatic melanoma, breast cancer, triple-negative breast cancer, prostate cancer, metastatic ovarian cancer, metastatic stomach cancer, leucosis, acute myeloid leukemia, pancreatic cancer.

The pharmaceutical composition of the present invention comprises, by way of example, from about 10% to about 100% of active ingredients, preferably from about 20% to about 60% of active ingredients. It is to be understood that each dosage unit may not comprise an effective amount of an active ingredient or ingredients, because the sufficient effective amount can be achieved by multiple dosing.

A typical composition is prepared by mixing the compound described herein with a carrier, diluent or excipient. Suitable carriers, diluents and excipients are well known to those skilled in the art and include materials such as carbohydrates, waxes, water soluble and/or swellable polymers, hydrophilic or hydrophobic materials, gelatin, oils, solvents, water, and the like. The particular carrier, diluent or excipient used will depend upon the means and purpose for which compound of the present invention is being applied. Solvents are generally selected based on solvents recognized by persons skilled in the art as safe to be administered to a mammal. In general, safe solvents are non-toxic aqueous solvents such as water and other non-toxic solvents that are soluble or miscible in water. Suitable aqueous solvents include water, ethanol, propylene glycol, polyethylene glycols (e.g., PEG400, PEG300), etc. and mixtures thereof. The compositions may also include one or more buffers, stabilizing agents, surfactants, wefting agents, lubricating agents, emulsifiers, suspending agents, preservatives, antioxidants, opaquing agents, glidants, processing aids, colorants, sweeteners, perfuming agents, flavoring agents and other known additives to provide an elegant presentation of the drug (i.e., compound of the invention or pharmaceutical composition thereof) or aid in the manufacturing of the pharmaceutical product (i.e., medicament).

The pharmaceutical compositions also may contain salts, solvates and hydrates of compounds of the present invention, or stabilized form of the compound (e.g., complex with a cyclodextrin derivative or another known complexation agent).

The pharmaceutical compositions of the present invention may be formulated for an oral route administration. Oral administration is a route of administration, where a medicine is taken through the mouth, by virtue of swallowing. The compounds of the present invention may also be administered by buccal, lingual, or sublingual route by which the compound enters the blood stream directly from the mouth.

Formulations suitable for oral, buccal, lingual, or sublingual administration include solid, semi-solid and liquid systems such as tablets; granules; soft or hard capsules containing multi- or nano-particulates, liquids, or powders; lozenges (including liquid-filled); chews; gels; fast dispersing dosage forms; films; ovules; sprays; and buccal/mucoadhesive patches.

Liquid formulations include suspensions, solutions, syrups and elixirs. Such formulations may be employed as fillers in soft or hard capsules (made, for example, from gelatin or hydroxypropylmethylcellulose) and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The pharmaceutical compositions of the invention could be used for parenteral administration. As used herein, "parenteral administration" of a pharmaceutical composition includes any route of administration characterized by physical breaching of a tissue of a subject and administration of the pharmaceutical composition through the breach in the tissue, thus generally resulting in the direct administration into the blood stream, into muscle, or into an internal organ. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration is contemplated to include, but is not limited to, subcutaneous, intraperitoneal, intramuscular, intravenous, intraarterial, intrathecal, intraventricular, intraurethral, intracranial, intrasynovial injection or infusions; and kidney dialytic infusion techniques. Intratumoral delivery, e.g. intratumoral injection, may also be advantageous. Regional perfusion is also contemplated.

Formulations of a pharmaceutical composition suitable for parenteral administration typically comprise the active ingredient combined with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampoules or in multi-dose containers containing a preservative. Formulations for parenteral administration include, but are not limited to, suspensions, solutions, emulsions in oily or aqueous vehicles, pastes, and the like.

Formulations may be formulated to be immediate and/or modified release. Modified release formulations include delayed-, sustained-, pulsed-, controlled-, targeted and programmed release.

In one embodiment, the present invention relates to the method for treating a disease or disorder mediated by the activation of cyclin-dependent protein kinases CDK8/19 that comprises the step of administering a therapeutically effective amount of the compound of the present invention, or pharmaceutically acceptable salt thereof, or the pharmaceutical composition of the present invention in a subject in need thereof.

In another one embodiment, the present invention relates to the method for treating a disease or disorder mediated by the activation of cyclin-dependent protein kinases CDK8/19, which is either oncological or haemato-oncological, that comprises the step of administering a therapeutically effective amount of any compound described herein, or a pharmaceutical composition of the present invention to a subject in need of such treatment.

In another one embodiment, the present invention relates to the described above method wherein oncological and haemato-oncological disease is selected from the group comprising colorectal cancer, melanoma, metastatic melanoma, breast cancer, triple-negative breast cancer (TNBC), prostate cancer, metastatic ovarian cancer, metastatic stomach cancer, leucosis, acute myeloid leukemia, pancreatic cancer.

It is understood that the compounds of the invention may be used in methods for treating, as described above, in treatment, as described above, and/or in the manufacture of a medicament for the therapeutic applications described above.

As used herein, the terms "co-administration", "co-administered" and "in combination with" referring to the compounds with one or more other therapeutic agents, is intended to mean, and does refer to and include the following:

- simultaneous administration of such combination of compound of the invention and therapeutic agent(s) to a patient in need of treatment, when such components are formulated together into a single dosage form which releases said components at substantially the same time to said patient,
- substantially simultaneous administration of such combination of compound of the invention and therapeutic agent(s) to a patient in need of treatment, when such components are formulated apart from each other into separate dosage forms which are taken at substantially the same time by said patient, whereupon said components are released at substantially the same time to said patient,
- sequential administration of such combination of compound of the invention and therapeutic agent(s) to a patient in need of treatment, when such components are formulated apart from each other into separate dosage forms which are taken at consecutive times by said patient with a significant time interval between each administration, whereupon said components are released at substantially different times to said patient; and
- sequential administration of such combination of compound of the invention and therapeutic agent(s) to a patient in need of treatment, when such components are formulated together into a single dosage form which releases said components in a controlled manner whereupon they are concurrently, consecutively, and/or overlappingly released at the same and/or different times to said patient, where each part may be administered by either the same or a different route.

As well known to those skilled in the art, therapeutically effective dosages may vary when the drugs are used in combination treatment. Methods for experimentally determining therapeutically effective dosages of drugs and other agents for use in combination treatment regimens are described in the literature. For example, the use of metronomic dosing, i.e., providing more frequent, lower doses in order to minimize toxic side effects, has been described in the literature. Combination treatment further includes periodic treatments that start and stop at various times to assist with the clinical management of the patient. For combination therapies described herein, dosages of the co-administered compounds will of course vary depending on the type of co-drug employed, on the specific drug employed, on the condition or disorder being treated and so forth.

In addition, compounds described herein may also be used in combination with procedures that may provide additional or synergistic benefit to the subject. By way of example only, subjects are expected to find therapeutic and/or prophylactic benefit in the methods described herein, wherein pharmaceutical composition of the present invention and /or combinations with other therapeutics are combined with genetic testing to determine whether that individual is a carrier of a mutant gene that is known to be correlated with certain diseases or conditions.

Compounds which are inhibitors of CDK8/19 can be used in the described above treatment methods in the form of monotherapy, or in combination with surgery, or radiation therapy, or drug therapy.

Such drug therapy may comprise administration of one or more of the anti-cancer agents. Examples of anti-cancer agents include, but are not limited to, any of the following: alkylating agents, alkyl sulfonates, nitrosoureas or triazenes; antimetabolites hormones and antagonists; platinum compounds; anticancer antibiotics; topoisomerase inhibitors.

Examples of antimetabolites include, but are not limited to, folic acid analogs (such as methotrexate, trimetrexate, pemetrexed, pralatrexate, raltitrexed, calcium levofolinate) or pyrimidine analogs (such as cytarabine, tegafur, fluorouracil, capecitabine, floxuridine, azacitidine, enocitabine, carmofur, gemcitabine, sapacitabine, elacytarabine, doxifluridine), or purine analogs (such as mercaptopurine, thioguanine, pentostatin, fludarabine, cladribine, nelarabine, azathioprine, clofarabine), or asparaginase.

Examples of alkylating agents include, but are not limited to, mechloretamine, cyclophosphamide, chlorambucil, melphalan, bendamustine, hexamethylmelamine, thiotepa, busulfan, carmustine, lomustine, laromustine, ceMycTИH, streptozocin, dacarbazine, ifosfamide, improsulfan, mitobronitol, mitolactol, nimustine, ranimustine, temozolomide, treosulfan, carboquone, apaziquone, fotemustine, altretamine, glufosfamide, pipobroman, trofosfamide, uramustine, evofosfamide, VAL-083.

Examples of hormones and antagonists include, but are not limited to, prednisone, prednisolone, hydroxyprogesterone caproate, megestrol acetate, medroxyprogesterone acetate, diethlystilbestrol, estradiol, tamoxifen, testosterone propionate, fluoxymesterone, flutamide, leuprolide, abarelix, abiraterone, bicalutamide, buserelin, calusterone, chlorotrianisene, degarelix, dexamethasone, fluocortolone, fulvestrant, goserelin, histrelin, leuprorelin, mitotane, nafarelin, nandrolone, nilutamide, octreotide, raloxifene, thyrotropin alfa, toremifene, triptorelin, diethylstilbestrol; acolbifene, danazol, deslorelin, epitiostanol, orteronel, enzalutamide, aminoglutethimide, anastrozole, exemestane, fadrozole, letrozole, testolactone; formestane.

Examples of platinum compounds include, but are not limited to, cisplatin, carboplatin, oxaliplatin, eptaplatin, miriplatine hydrate, lobaplatin, nedaplatin, picoplatin, satraplatin.

Examples of anticancer antibiotics include, but are not limited to, doxorubicin, daunurobicin, idarubicin, carubicin, valrubicin, zorubicin, aclarubicin, pirarubicin, nemorubicin, amrubicin, epirubicin, bleomycin, dactinomycin, plicamycin, peplomycin, mitomycin C, zinostatin, streptozocin.

Examples of topoisomerase inhibitors include, but are not limited to, irinotecan, topotecan, belotecan, teniposide, etoposide, voreloxin, amonafide.

Examples of anti-cancer agents include, but are not limited to, any of the following agents: microtubule-directed drugs, such as taxanes (e. g., paclitaxel, docetaxel, cabazitaxel, tezetaxel), vinca alkaloids (e. g., vinorelbine, vinblastine, vincristine, vindesine, vinflunine); mitogen-activated protein kinase signaling inhibitors (e. g., U0126, PD98059, PD184352, PD0325901, ARRY-142886, SB239063, SP600125, BAY 43-9006, wortmannin or LY294002); mTOR inhibitors (e. g., sirolimus, temsirolimus, everolimus, ridaforolimus); antibodies (e. g., rituximab, trastuzumab, alemtuzumab, besilesomab, cetuximab, denosumab, ipilimumab, bevacizumab, pertuzumab, nivolumab, ofatumumab, panitumumab, tositumomab, katumaksomab, elotuzumab, epratuzumab, farletuzumab, mogamulizumab, necitumumab, nimotuzumab, obinutuzumab, okaratuzumab, oregovomab, ramucirumab, rilotumumab, siltuximab, tocilizumab, zalutumumab, zanolimumab, matuzumab, dalotuzumab, onartuzumab, racotumomab, tabalumab, EDM-525797); kinase inhibitors (fostamatinib, entospletenib, erlotinib, imatinib, lapatinib, nilotinib, pazopanib, vemurafenib, gefitinib, crizotinib, dasatinib, regorafenib, ruxolitinib, sorafenib, sunitinib, vandetanib, bosutinib, axitinib, afatinib, alisertib, dabrafenib, dacomitinib, dinaciklib, dovitinib , nintedanib, lenvatinib, linifanib, linsitinib, masitinib, motesanib, neratinib, orantinib, ponatinib, radotinib, tipifarnib, tivantinib, tivozanib, trametinib, apatinib, ibrutinib, acalabrutinib, cobimetinib, fedratinib, brivanib alaninate, cediranib, cabozantinib, icotinib, cipatinib, rigosertib, pimasertib, buparlisib, idelalisib, midostaurin, perifosine, XL-647); photosensitizers (e. g., talaporfin, temoporfin, porfimer sodium); cytokines (e. g., aldesleukin, interferon alfa, interferon

alfa-2a, interferon alfa-2b, celmoleukin, tasonermin, recombinant interleukin-2, oprelvekin, recombinant interferon beta-la); vaccines (e. g., picibanil, sipuleucel-T, vitespen, emepepimut-S, oncoVAX, rindopepimut, troVAX, MGN-1601, MGN-1703); bisanthrene, decitabine, mitoxantrone, procarbazine, trabectedin, amsacrine, brostallicin, miltefosine, romidepsin, plitidepsin, eribulin, Ixabepilone, fosbretabulin, denileukin diftitox, ibritumomab tiuxetan, prednimustine, trastuzumab emtansine, estramustine, gemtuzumab ozogamicin, aflibercept, oportuzumab monatox, cintredekin besudotox, edotreotide, inotuzumab ozogamicin, naptumomab estafenatox, vintafolide, brentuximab vedotin, bortezomib, Ixazomib, carfilzomib, lenalidomide, thalidomide, pomalidomide, zoledronic acid, ibandronic acid, pamidronic acid, alitretinoin, tretinoin, peretinoin, bexarotene, tamibarotene, imiquimod, lentinan, mifamurtide, romurtide, pegaspargase, pentostatin, endostatin, sizofiran, vismodegib, vorinostat, entinostat, panobinostat, celecoxib, cilengitide, ethanidazole, ganetespib, idronoksil, Iniparib, lonidamine, nimorazole, procodazole, tasquinimod, telotrystat, belinostat, thymalfasin, tirapazamine, tosedostat, trabedersen, ubenimex, valspodar, gendicine, reolysin, retaspimycin, trebananib, virulizin.

In one embodiment, the present invention relates to use of the compound described herein or pharmaceutically acceptable salt thereof, or a pharmaceutical composition of the present invention in the treatment of a disease or disorder mediated by the activation of cyclin-dependent protein kinases CDK8/19 in a subject in need thereof.

In another one embodiment, the present invention relates to the use of the compound described herein or pharmaceutically acceptable salt thereof, or the pharmaceutical composition of the present invention in the treatment of a disease or disorder mediated by the activation of cyclin-dependent protein kinases CDK8/19 in a subject in need thereof, which is either oncological or haemato-oncological.

In another one embodiment, the present invention relates to the use of the compound described herein or pharmaceutically acceptable salt thereof, or the pharmaceutical composition of the present invention in the treatment of a subject with colorectal cancer, melanoma, metastatic melanoma, breast cancer, triple-negative breast cancer, prostate cancer, metastatic ovarian cancer, metastatic stomach cancer, leucosis, acute myeloid leukemia, pancreatic cancer. In all these embodiments, the subject may be human.

The compounds of the present invention will be administered in an effective amount for treatment of the condition in question, i.e., at dosages and for periods of time necessary to achieve a desired result. A therapeutically effective amount may vary according to factors such as the particular condition being treated, the age, sex and weight of the patient, and whether the compounds are being administered as a stand-alone treatment or in combination with one or more additional treatments.

Dosage regimens may be adjusted to provide the optimum desired response. For example, a single dose may be administered, several divided doses may be administered over time or the dose may be proportionally reduced or increased as indicated by the exigencies of the therapeutic situation. It is especially advantageous to formulate oral compositions in dosage unit form for ease of administration and uniformity of dosage. Dosage unit form, as used herein, refers to physically discrete units suited as unitary dosages for the patients/subjects to be treated; each unit containing a predetermined quantity of active compound calculated to produce the desired therapeutic effect in association with the required pharmaceutical carrier.

It is to be further understood that for any particular subject, specific dosage regimens should be adjusted over time according to the individual need and the professional discretion of the person administering or supervising the administration of the compositions, and that dosage ranges set forth herein are exemplary only and are not intended to limit the scope or practice of the embodied composition. Further, the dosage regimen with the compositions of this invention may be based on a variety of factors, including the type of disease, the age, weight, sex, medical condition of the patient, the severity of the condition, the route of administration, and the specific compound used according to the present invention. Thus, the dosage regimen can vary widely, but can be determined routinely using standard methods. For example, doses may be adjusted based on pharmacokinetic or pharmacodynamic parameters, which may include clinical effects such as toxic effects and/or laboratory values. Thus, the present invention encompasses an individual dose increase, which is determined by the person skilled in the art. The determination of the required dose and regimens is well known in the relevant field of technology and will be understood by a specialist in this field after reading the ideas disclosed in this document.

Generally, standard daily dosage for an adult human is in the range from 0.02 mg to 5000 mg or from about 1 mg to about 1500 mg.

Once improvement of the patient’s conditions has occurred, a maintenance dose is administered, if necessary. Subsequently, the dosage or the frequency of administration, or both, can be reduced, as a function of the symptoms, to a level at which the improved disease or disorder is retained. Patients may be required periodic treatment on a long-term basis upon any relapse of symptoms.

The above spectrum is only an assumption, since the number of variables in relation to the individual treatment regimen is large, and significant deviations from these recommended values are very common. Such dosages may be altered depending on a number of variables, not limited to the activity of the compound used, the disorder or condition to be treated, the method of administration, the requirements of the individual subject, the severity of the disorder or condition being treated, and the judgment of the physician.

In order that this invention may be better understood, the following examples are set forth. These examples are for purposes of illustration only and are not to be construed as limiting the scope of the invention in any manner.

All publications, patents, and patent applications cited in this specification are incorporated herein by reference. Although the above-mentioned invention has been described in some detail by way of illustration and example in order to avoid ambiguous interpretation, it will be quite clear to those skilled in the art, based on the ideas disclosed in this invention, that certain changes and modifications can be made without deviating from the nature and scope of the attached embodiments of the invention .

## Examples

**Example 1.** Method for preparation of compound **BCD-CDK8-1-6e.**

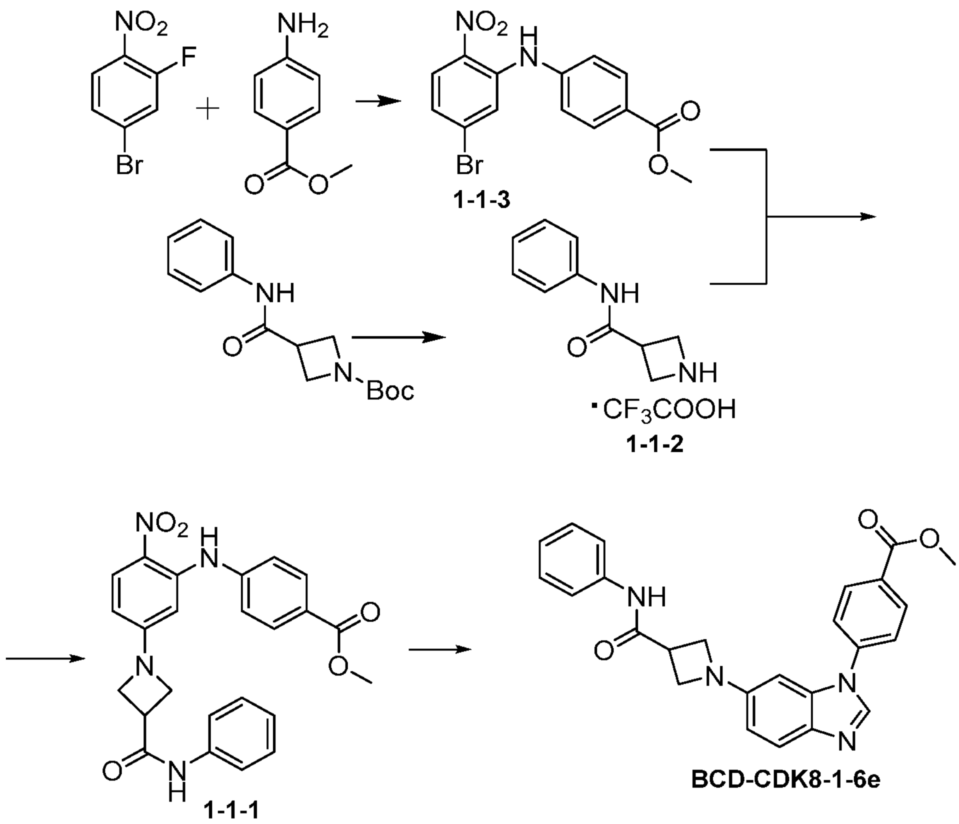

**1-1-3**

**1-1-2**

**1-1-1**

**BCD-CDK8-1-6e**

**Step 1.** Potassium tert-butylate (1.15 g, 10.2 mmol) was added to a solution of 2-fluoro-4-bromonitrobenzene (1.50 g, 6.82 mmol) and methyl 4-aminobenzoate (891 mg, 6.50 mmol) in 100 mL DMSO. Reaction mixture was stirred at room temperature for 6 hours, then poured into 500 mL of water. Suspension was filtered, precipitate was washed with water, dissolved in ethyl acetate, washed with 0.1M HCl solution. Solvent was evaporated by vacuum distillation. Product **1-1-3** was isolated as orange powder by column chromatography on silica gel using hexane-ethyl acetate (8:2) as eluent. Yield: 1.33 g (55%).

**Step 2.** Preparation of compound **1-1-2.**

Trifluoroacetic acid (0.70 mL, 7.5 mmol) was added to a solution of *tert*-butyl-3-(phenylcarbamoyl)azetidine-1-carboxylate (500 mg, 1.81 mmol) (prepared as described in WO 2000/071518) in 5 mL of dichloromethane. Mixture was stirred for 12 hours, solvent was then evaporated by vacuum distillation. Residue was dried by vacuum rotary evaporator at 45 °C, and used in the next step without additional purification.

**Step 3.** Mixture of compounds **1-1-3** (776 mg, 2.22 mmol), **1-1-2** (1.28 g, 4.42 mmol), $Cs_2CO_3$ (2.52 g, 7.73 mmol), BINAP (138 mg, 0.222 mmol) and Palladium(II) acetate (25 mg, 0.111 mmol) in 15 mL of 1,4-dioxane was stirred under inert atmosphere for 15 hours at 80°C. Ethyl acetate was added to reaction mixture, washed with 0.1M HCl solution, saturated solutions of $NaHCO_3$ and NaCl. Organic phase was concentrated under vacuum. Product **1-1-1** was isolated by column chromatography on silica gel using dichloromethane-ethyl acetate-methanol (100:5:1) as

eluent. Yield: 664 mg (67%).

**Step 4.** Hydrogen was passed at atmospheric pressure and 25°C for 4 hours through a mixture of compound **1-1-1** (200 mg, 0.44 mmol), trimethyl orthoformate (5 mL, 46 mmol), *p*-Toluenesulfonic acid monohydrate (20 mg, 0.1 mmol) and 10 Wt. % Pd/C (40 mg) in 30 mL of methanol. Suspension was then filtered through Celite, and filtrate was concentrated under vacuum. Product was isolated by column chromatography on silica gel using hexane-ethyl acetate (1:1) as eluent. Reaction product **BCD-CDK8-1-6e** was obtained as colorless powder. Yield: 159 mg (85%).

**Example 2.** Method for preparation of **BCD-CDK8-1-6a.**

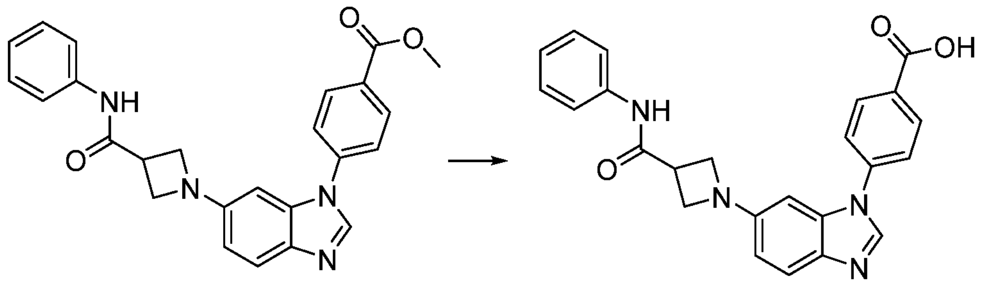

**BCD-CDK8-1-6e** **BCD-CDK8-1-6a**

Solution of LiOH·$H_2O$ (40 mg, 0.95 mmol) in 10 mL of water was added to a solution of **BCD-CDK8-1-6e** (270 mg, 0.63 mmol) in 20 mL of methanol. Solution was stirred at 100 °C for 2 hours. Methanol was evaporated by vacuum distillation, aqueous solution was washed with ethyl acetate, aqueous phase was adjusted to a neutral medium with 0.1M HCl solution. Suspension was filtered. Product **BCD-CDK8-1-6a** was obtained as colorless powder. Yield: 164 mg (62%).

**Example 3.** Method for preparation of **BCD-CDK8-1-1.**

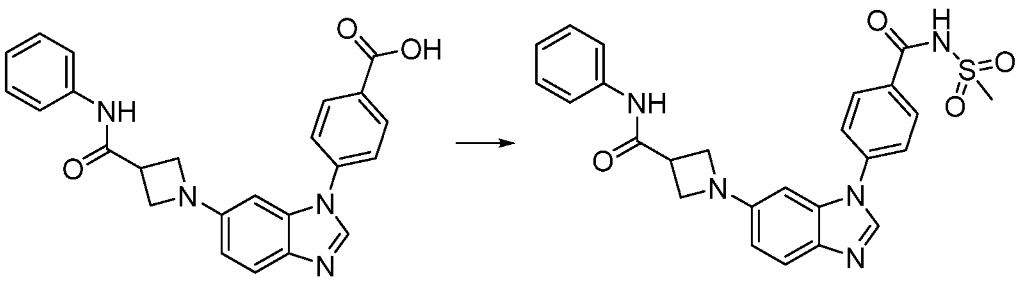

**BCD-CDK8-1-6a** **BCD-CDK8-1-1**

EDC'HCl (42 mg, 0.22 mmol) was added to suspension of **BCD-CDK8-1-6a** (70 mg, 0.17 mmol), methanesulfonamide (17 mg, 0.18 mmol) and DMAP (27 mg, 0.22 mmol) in 15 mL of dichloromethane. Reaction mixture was stirred at room temperature for 16 hours. Solvent was evaporated by vacuum distillation. Product was isolated as colorless powder by column chromatography on silica gel using dichloromethane-methanol-formic acid (9:1:0.1) as eluent. Yield: 55 mg (66%). Compound was additionally purified by preparative chromatography.

**Example 4.** Method for preparation of compound **BCD-CDK8-1-6.**

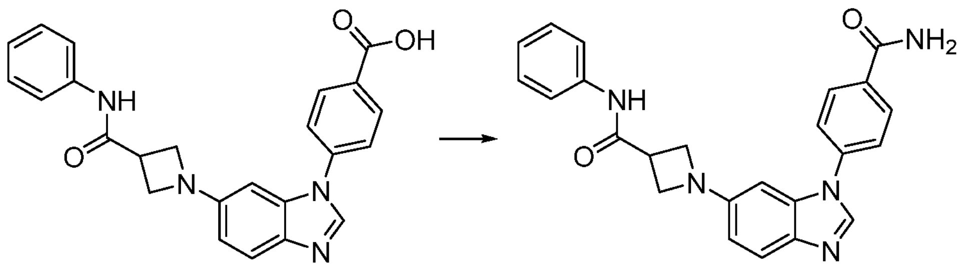

BCD-CDK8-1-6a BCD-CDK8-1-6

EDC'HCl (71 mg, 0.17 mmol) was added to a solution of **BCD-CDK8-1-6a** (71 mg, 0.17 mmol), HOBt (37 mg, 0.24 mmol), $NH_4Cl$ (28 mg, 0.53 mmol), DIPEA (45 $\mu$L, 0.26 mmol) in 5 mL of DMF. Mixture was stirred at room temperature for 48 hours. Reaction mixture was concentrated under vacuum. The product was isolated as colorless powder by column chromatography on silica gel using dichloromethane-methanol (95:5 → 92:8) as eluent. Yield: 60 mg (85%). Compound was additionally purified by preparative chromatography.

**Example 5.** Method for preparation of compound **BCD-CDK8-1-2.**

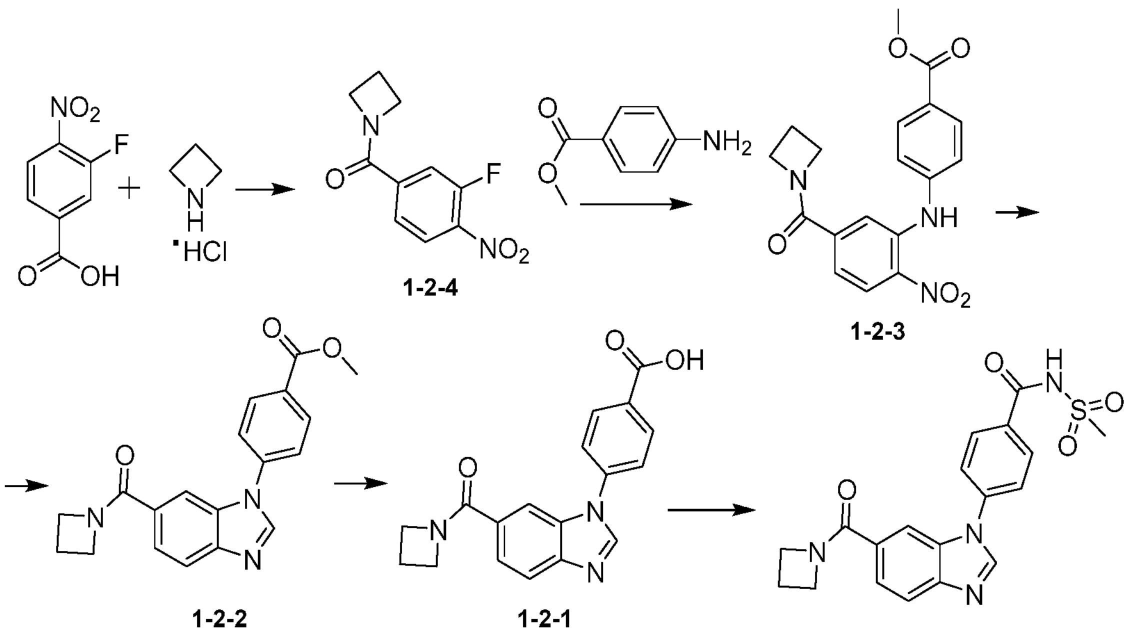

1-2-4

1-2-3

1-2-2

1-2-1

BCD-CDK8-1-2

**Step 1.** EDC'HCl (1.45 g, 7.56 mmol) was added to a solution of 3-fluoro-4-nitrobenzoic acid (1.00 g, 5.40 mmol), azetidine hydrochloride (556 mg, 5.94 mmol), HOBt (1.16 g, 7.56 mmol) and DIPEA (3.30 mL, 18.9 mmol) in 50 mL of dichloromethane. Reaction mixture was stirred at room temperature for 12 hours, washed with 1M HCl solution, saturated $NaHCO_3$ and NaCl solutions. Organic phase was concentrated under vacuum, **1-2-4** was obtained as colorless powder. Yield: 1.0 g (82%).

**Step 2.** Compound **1-2-3** was prepared in a similar fashion to compound **1-1-3** (example 1, step 1) using compound **1-2-4** instead of 2-fluoro-4-bromonitrobenzene.

**Step 3.** Compound **1-2-2** was prepared in a similar fashion to compound **BCD-CDK8-1-6e** (example 1, step 4) using compound **1-2-3** instead of compound **1-1-1.**

**Step 4.** Compound **1-2-1** was prepared analogously to compound **BCD-CDK8-1-6a** (example 2) using compound **1-2-2** instead of compound **BCD-CDK8-1-6e.**

**Step 5:** Preparation of compound **BCD-CDK8-1-2.**

Compound **BCD-CDK8-1-2** was prepared in a similar fashion to compound **BCD-CDK8-1-1** (example 3) using **1-2-1** instead of compound **BCD-CDK8-1-6a.**

**Example 6.** Method of preparation of compound **BCD-CDK8-1-3.**

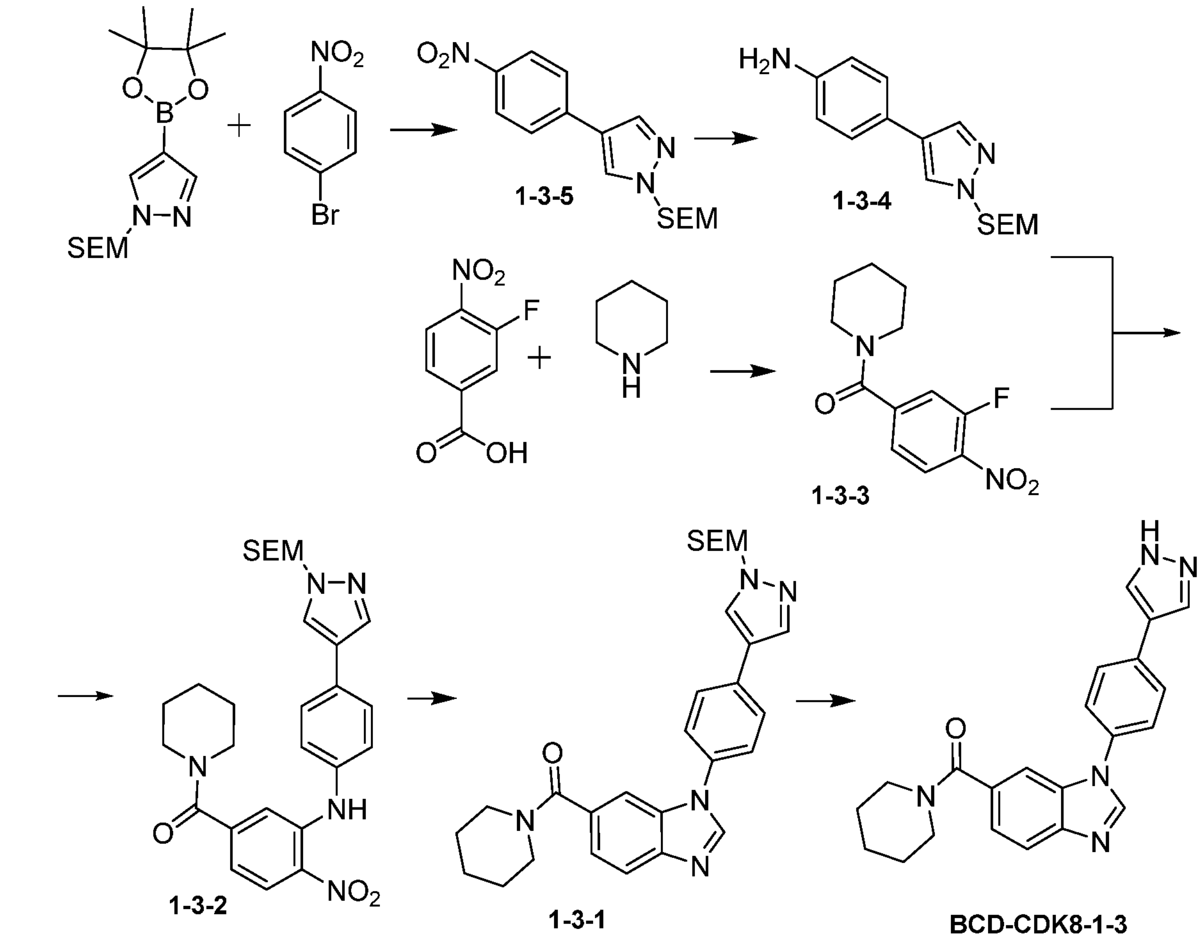

1-3-5

1-3-4

1-3-3

1-3-2

1-3-1

BCD-CDK8-1-3

**Step 1.** 4-bromonitrobenzene (6.50 g, 32.2 mmol), 4- (4,4,5,5-tetramethyl-1,3,2-dioxoborolan-2-yl)-1-((2-(trimethylsilyl)ethoxy)methyl) -1*H*-pyrazole (11.5 g, 35.4 mmol) (prepared as described in WO 2011/130146), $Na_2CO_3$ (6.82 g, 64.3 mmol) and $Pd(PPh_3)_4$ (743 mg, 0.64 mmol) in a mixture of 100 mL of THF and 50 mL of water was stirred at 70 °C under inert atmospheric conditions for 10 hours. THF was evaporated by vacuum distillation, ethyl acetate was added. The layers were separated, the organic layer was washed with saturated solutions of $Na_2CO_3$ and NaCl, and then concentrated under vacuum. Compound **1-3-5** was isolated as colorless powder by column chromatography on silica gel using hexane-ethyl acetate (8:2) as eluent. Yield: 7.82 g (76%).

**Step 2.** A mixture of **1-3-5** (6.32 g, 19.8 mmol) and 10 Wt. % Pd/C (800 mg) in 100 mL of methanol was stirred at room temperature in hydrogen at 5 atm. After 5 hours, reaction mixture was filtered through Celite and concentrated under vacuum. Product **1-3-4** was used without additional purification. Yield: 5.33 g (93%).

**Step 3.** Compound **1-3-3** was prepared in a similar fashion to compound **1-2-4** (example 5, step 1) using piperidine instead of azetidine hydrochloride.

**Step 4.** A solution of **1-3-3** (650 mg, 2.58 mmol), **1-3-4** (746 mg, 2.58 mmol) and DIPEA (0.675 mL, 3.87 mmol) in 20 mL of DMSO was heated at 90 °C for 30 hours. Reaction mixture was poured into 100 mL of water. Suspension was filtered, precipitate was washed with water, dissolved in ethyl acetate, washed with 0.1 M HCl solution. Organic phase was concentrated under vacuum. Product **1-3-2** was isolated as orange powder by column chromatography on silica gel using hexane-ethyl acetate (2:1) as eluent. Yield: 712 mg (53%).

**Step 5:** Compound **1-3-1** was prepared in a similar fashion to compound **BCD-CDK8-1-6e** (example 1, step 4) using compound **1-3-2** instead of compound **1-1-1.**

Step 6. Preparation of compound **BCD-CDK8-1-3.**

Solution of **1-3-1** (338 mg, 0.676 mmol) and tetrabutylammonium fluoride (3.38 mL, 3.38 mmol, 1 M in THF) in 10 mL of THF was stirred at 70°C for 14 hours. Reaction mixture was concentrated under vacuum, residue was dissolved in ethyl acetate, washed with water and concentrated. Product was isolated as colorless powder by column chromatography on silica gel using dichloromethane-ethyl acetate-methanol (20:5:1) as eluent. Yield: 132 mg (56%).

**Example 7.** Method of preparation of compound **BCD-CDK8-1-16.**

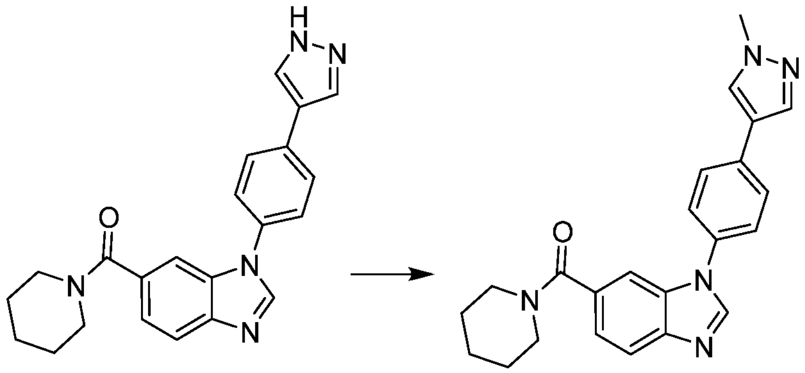

BCD-CDK8-1-3 BCD-CDK8-1-16

Sodium hydride (10 mg, 0.251 mmol) was added to a solution of **BCD-CDK8-1-3** (85 mg, 0.228 mmol) in 4 mL of DMF at -20°C. Mixture was brought to room temperature. Methyl iodide (17 μl, 0.251 mmol) was added after 15 minutes at -20 °C. Reaction mixture was stirred at room temperature for 6 hours, then concentrated under vacuum. Product was isolated as colorless powder by column chromatography on silica gel using dichloromethane-methanol (96:4) as eluent. Yield: 52 mg (61%).

**Example 8.** Method of preparation of compound **BCD-CDK8-1-4.**

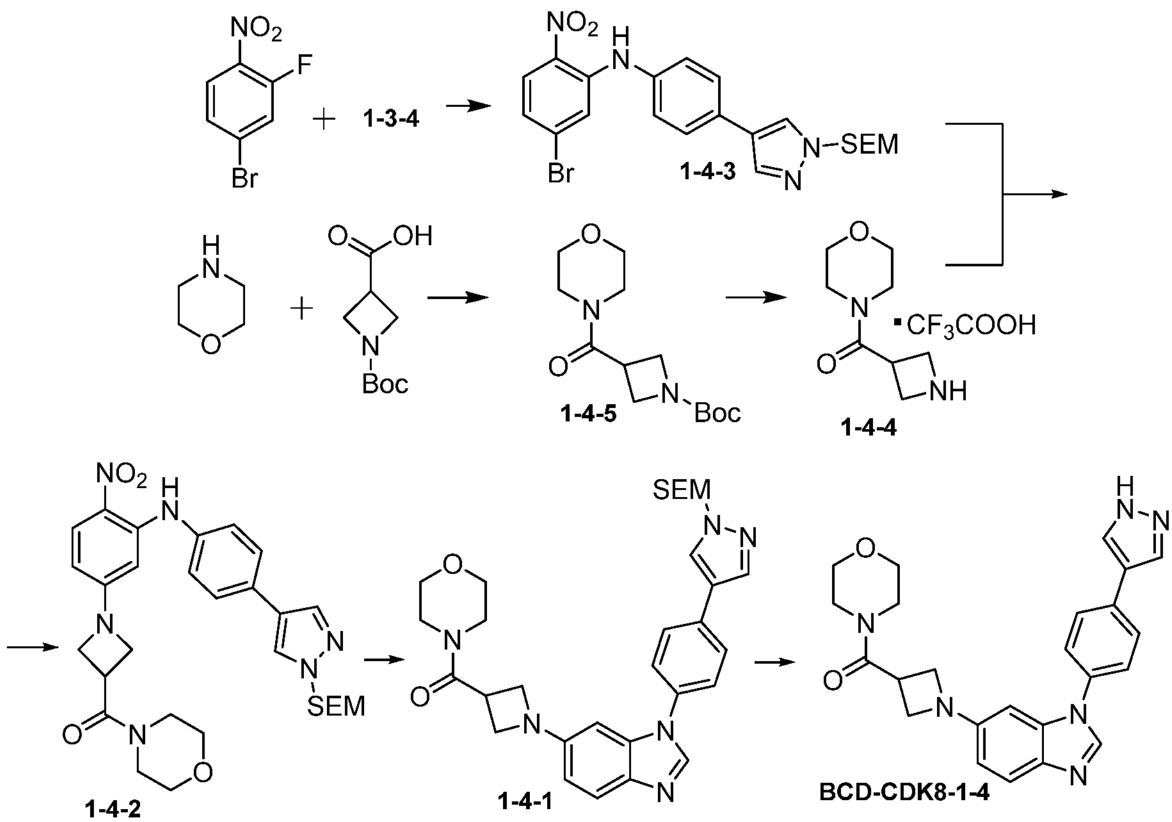

1-3-4 1-4-3 1-4-5 1-4-4 1-4-2 1-4-1 BCD-CDK8-1-4

**Step 1.** Compound **1-4-3** was prepared in a similar fashion to compound **1-3-2** (example 6, step 4) using 2-fluoro-4-bromonitrobenzene instead of compound **1-3-3.**

Step 2. Compound **1-4-5** was prepared in a similar fashion to compound **1-2-4** (example 5, step 1) using 1-(*tert*-butoxycarbonyl)azetidine-3-carboxylic acid instead of 3-fluoro-4-nitrobenzoic acid and morpholine instead of azetidine hydrochloride.

**Step** 3. Compound **1-4-4** was prepared in a similar fashion to compound **1-1-2** (example 1, step 2) using compound **1-4-5** instead of *tert*-butyl-3-(phenylcarbamoyl)azetidine-1-carboxylate.

**Step 4.** Compound **1-4-2** was prepared in a similar fashion to compound **1-1-1** (example 1, step 3) using compound **1-4-3** instead of **1-1-3** and **1-4-4** instead of **1-1-2.**

**Step 5:** Compound **1-4-1** was prepared in a similar fashion to compound **BCD-CDK8-1-6e** (example 1, step 4) using compound **1-4-2** instead of compound **1-1-1.**

**Step 6.** Compound **BCD-CDK8-1-4** was prepared analogously to **BCD-CDK8-1-3** (example 6, step 6) using compound **1-4-1** instead of compound **1-3-1.**

**Example 9.** Method of preparation of compound **BCD-CDK8-1-17.**

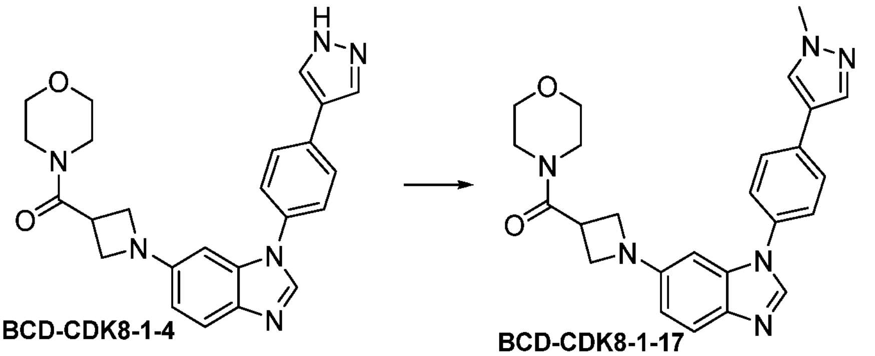

**BCD-CDK8-1-4** **BCD-CDK8-1-17**

Compound **BCD-CDK8-1-17** was prepared in a similar fashion to compound **BCD-CDK8-1-16** (example 7) using compound **BCD-CDK8-1-4** instead of compound **BCD-CDK8-1-3.**

**Example 10.** Method of preparation of compound **BCD-CDK8-1-5.**

**1-2-4** + **1-3-4**

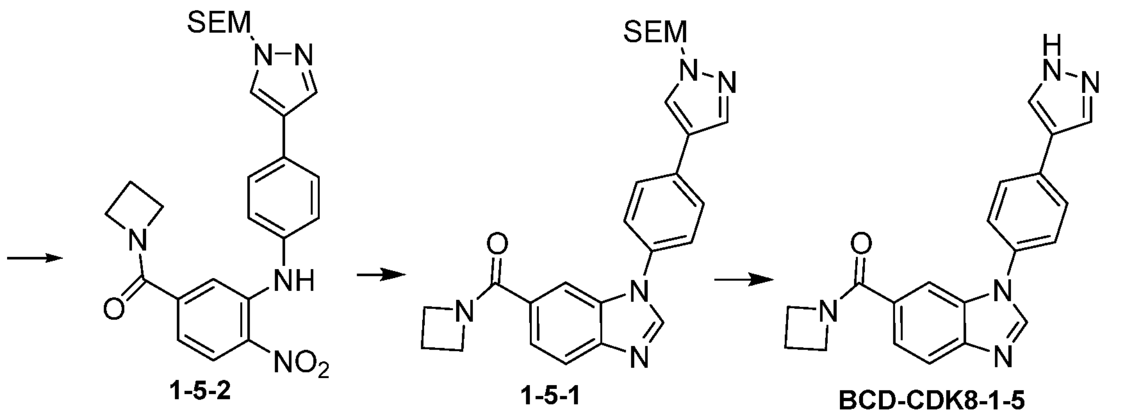

**1-5-2** **1-5-1** **BCD-CDK8-1-5**

**Step 1.** Compound **1-5-2** was obtained in a similar fashion to **1-3-2** (example 6, step 4) using **1-2-4** instead of **1-3-3.**

**Step 2.** Compound **1-5-1** was prepared in a similar fashion to compound **BCD-CDK8-1-6e** (example 1, step 4) using compound **1-5-2** instead of compound **1-1-1.**

**Step 3.** Compound **BCD-CDK8-1-5** was prepared in a similar fashion to **BCD-CDK8-1-3** (example 6, step 6) using compound **1-5-1** instead of compound **1-3-1.**

**Example 11.** Method of preparation of compound **BCD-CDK8-1-18.**

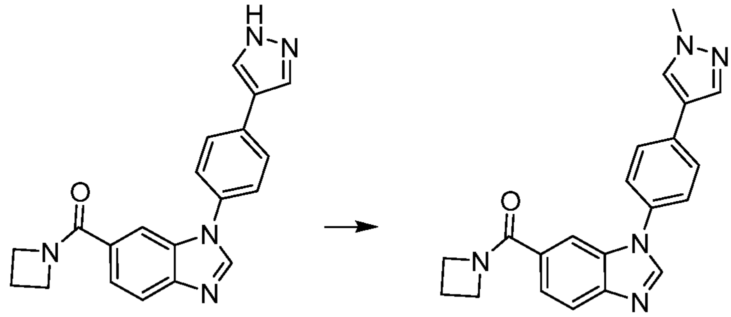

**BCD-CDK8-1-5** **BCD-CDK8-1-18**

Compound **BCD-CDK8-1-18** was prepared in a similar fashion to compound **BCD-CDK8-1-16** (example 7) using compound **BCD-CDK8-1-5** instead of compound **BCD-CDK8-1-3.**

**Example 12.** Method of preparation of compound **BCD-CDK8-1-9.**

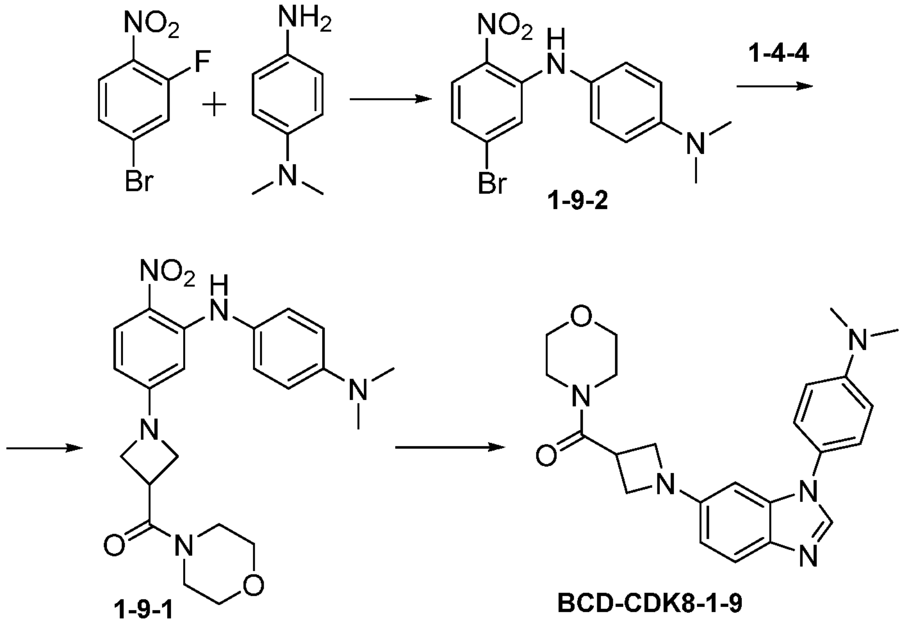


**1-9-2**

**1-9-1** **BCD-CDK8-1-9**

**Step 1.** Compound 1-9-2 was prepared in a similar fashion to **1-3-2** (example 6, step 4) using 2-fluoro-4-bromonitrobenzene instead of 1-3-3 and *N,N*-dimethyl-*p*-phenylenediamine instead of **1-3-4.**

**Step 2.** Compound **1-9-1** was prepared in a similar fashion to **1-1-1** (example 1, step 3) using **1-9-2** instead of **1-1-3** and **1-4-4** instead of **1-1-2.**

**Step 3.** Compound **BCD-CDK8-1-9** was prepared in a similar fashion to compound **BCD-CDK8-1-6e** (example 1, step 4) using compound **1-9-1** instead of compound **1-1-1.**

**Example 13.** Method of preparation of compound **BCD-CDK8-1-10.**

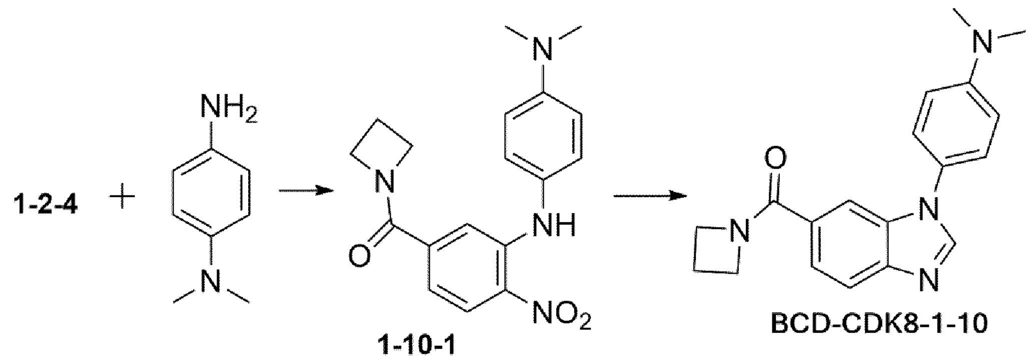

1-2-4

1-10-1

BCD-CDK8-1-10

**Step 1.** Compound **1-10-1** was prepared in a similar fashion to **1-3-2** (example 6, step 4) using **1-2-4** instead of **1-3-3** and *N*,*N*-dimethyl-*p*-phenylenediamine instead of **1-3-4.**

**Step 2.** Compound **BCD-CDK8-1-10** was prepared in a similar fashion to compound **BCD-CDK8-1-6e** (example 1, step 4) using compound **1-10-1** instead of compound **1-1-1.**

**Example 14.** Method of preparation of compound **BCD-CDK8-1-11.**

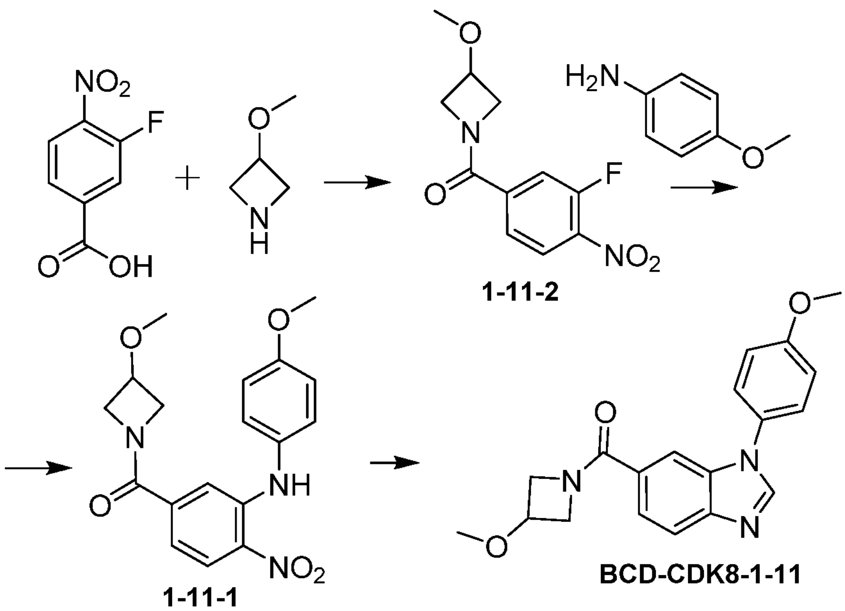

1-11-2

1-11-1

BCD-CDK8-1-11

**Step 1.** Compound **1-11-2** was prepared in a similar fashion to **1-2-4** (example 5, step 1) using 3-methoxyazetidine instead of azetidine hydrochloride.

**Step 2.** Compound **1-11-1** was prepared in a similar fashion to **1-3-2** (example 6, step 4) using **1-11-2** instead of **1-3-3** and 4-methoxyaniline instead of **1-3-4.**

**Step 3.** Compound **BCD-CDK8-1-11** was prepared in a similar fashion to compound **BCD-CDK8-1-6e** (example 1, step 4) using compound **1-11-1** instead of compound **1-1-1.**

**Example 15.** Method of preparation of compound **BCD-CDK8-1-12.**

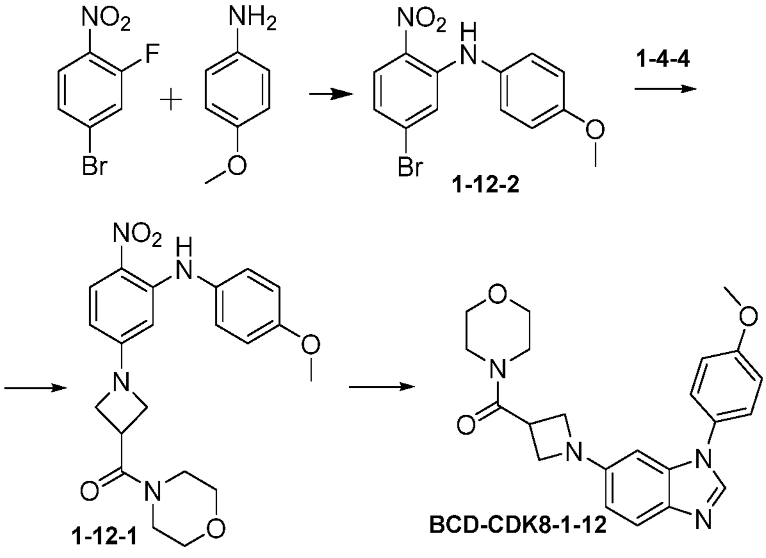

1-12-2

1-12-1

BCD-CDK8-1-12

**Step 1.** Compound **1-12-2** was prepared in a similar fashion to **1-3-2** (example 6, step 4) using 2-fluoro-4-bromonitrobenzene instead of **1-3-3** and 4-methoxyaniline instead of **1-3-4.**

**Step 2.** Compound **1-12-1** was prepared in a similar fashion to **1-1-1** (example 1, step 3) using**1-12-2** instead of **1-1-3** and **1-4-4** instead of **1-1-2.**

**Step 3.** Compound **BCD-CDK8-1-12** was prepared in a similar fashion to compound **BCD-CDK8-1-6e** (example 1, step 4) using compound **1-12-1** instead of compound **1-1-1.**

**Example 16.** Method of preparation of compound **BCD-CDK8-1-7e.**

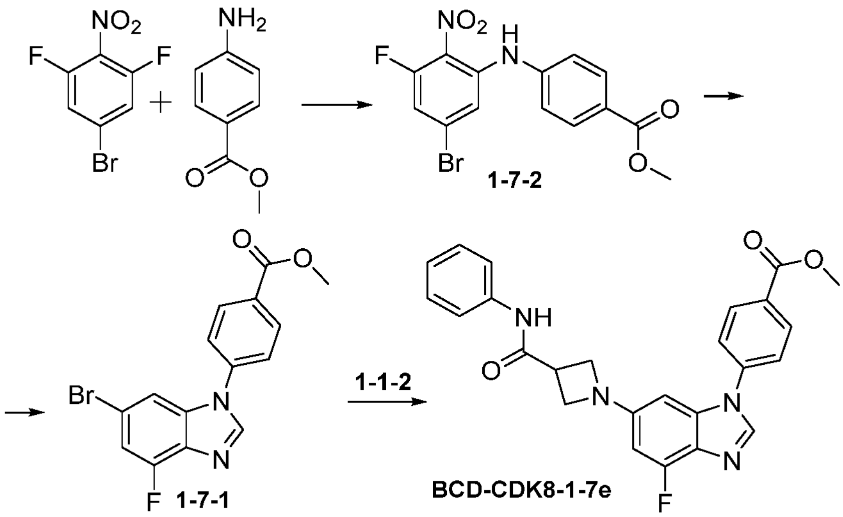

1-7-2

1-7-1

BCD-CDK8-1-7e

**Step 1.** Compound **1-7-2** was prepared in a similar fashion to compound **1-1-3** (example 1, step 1) using 2,6-difluoro-4-bromonitrobenzene instead of 2-Fluoro-4-bromonitrobenzene.

**Step 2.** Compound **1-7-1** was prepared in a similar fashion to compound **BCD-CDK8-1-6e** (example 1, step 4) using compound **1-7-2** instead of compound **1-1-1.**

**Step 3.** Compound **BCD-CDK8-1-7e** was prepared analogously to **1-1-1** (example 1, step 3), using **1-7-1** instead of **1-1-3.**

**Example 17.** Method of preparation of compound **BCD-CDK8-1-7a.**

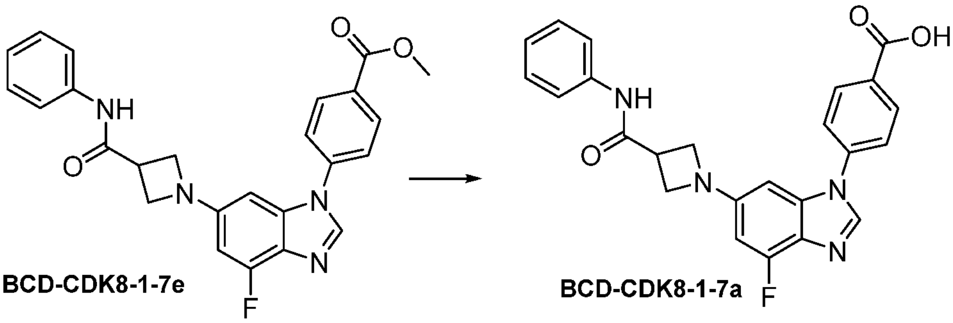

BCD-CDK8-1-7e BCD-CDK8-1-7a

Compound **BCD-CDK8-1-7a** was prepared in a similar fashion to compound **BCD-CDK8-1-6a** (example 2) using compound **BCD-CDK8-1-7e** instead of compound **BCD-CDK8-1-6e.**

**Example 18.** Method of preparation of compound **BCD-CDK8-1-7.**

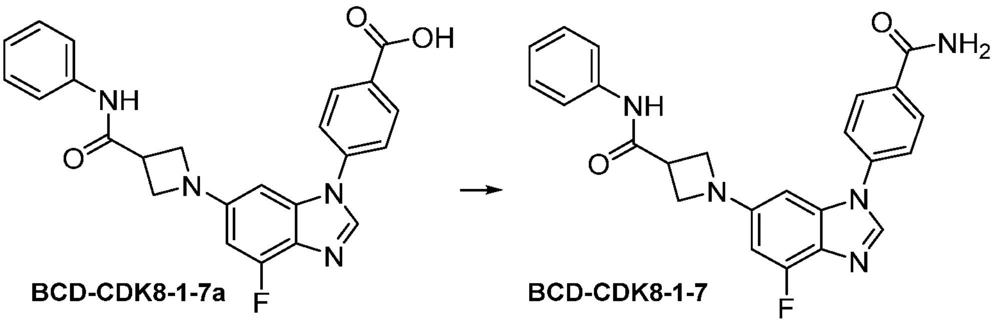

BCD-CDK8-1-7a BCD-CDK8-1-7

The compound **BCD-CDK8-1-7** was prepared in a similar fashion to compound **BCD-CDK8-1-6** (example 4), using **BCD-CDK8-1-7a** instead **of BCD-CDK8-1-6a.**

**Example 19.** Method of preparation of compound **BCD-CDK8-1-8.**

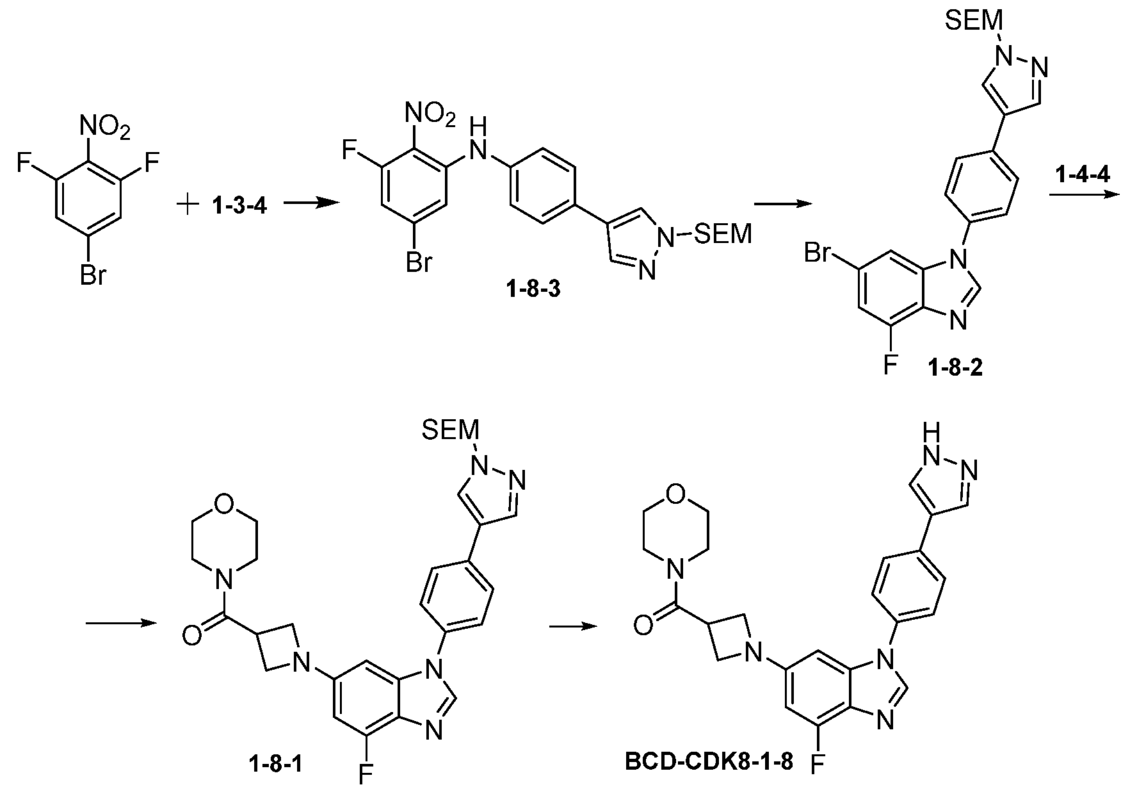


1-8-3 1-8-2 1-8-1 BCD-CDK8-1-8

**Step 1.** Compound **1-8-3** was obtained in a similar fashion to **1-3-2** (example 6, step 4) using 2,6-difluoro-4-bromonitrobenzene instead of **1-3-3.**

**Step 2.** Compound **1-8-2** was prepared in a similar fashion to compound **BCD-CDK8-1-6e** (example 1, step 4) using compound **1-8-3** instead of compound **1-1-1.**

**Step 3.** Compound **1-8-1** was prepared analogously to **1-1-1** (example 1, step 3) using **1-8-2** instead of **1-1-3** and **1-4-4** instead of **1-1-2.**

**Step 4.** Compound **BCD-CDK8-1-8** was prepared analogously to **BCD-CDK8-1-3** (example 6, step 6) using compound **1-8-1** instead of compound **1-3-1.**

**Example 20.** Method of preparation of compound **BCD-CDK8-1-19.**

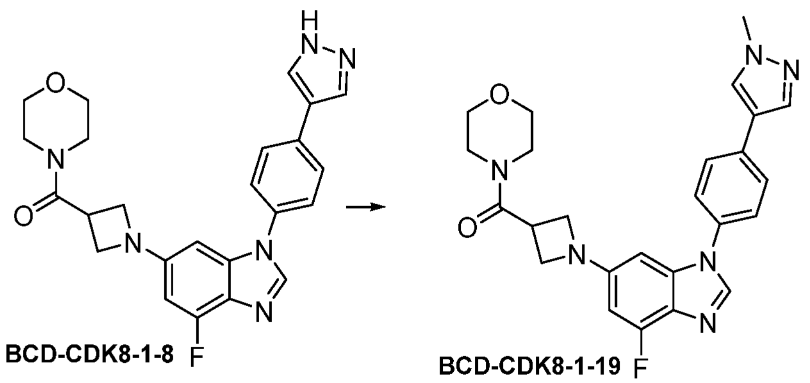

BCD-CDK8-1-8 BCD-CDK8-1-19

Compound **BCD-CDK8-1-19** was prepared analogously to compound **BCD-CDK8-1-16** (example 7) using compound **BCD-CDK8-1-8** instead of compound **BCD-CDK8-1-3.**

**Example 21.** Method of preparation of compound **BCD-CDK8-1-13.**

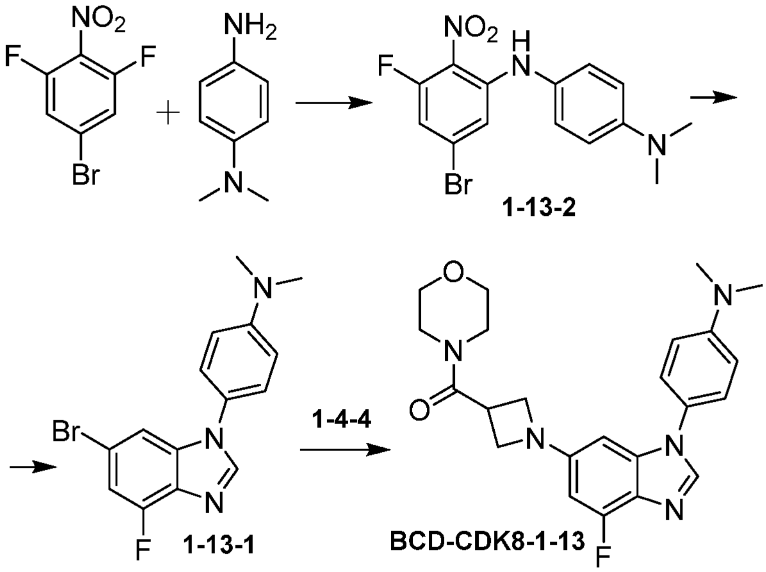


1-13-2, 1-13-1, BCD-CDK8-1-13

**Step 1.** Compound **1-13-2** was prepared in a similar fashion to **1-3-2** (example 6, step 4) using 2,6-difluoro-4-bromonitrobenzene instead of **1-3-3** and *N*,*N*-dimethyl-*n*-phenylenediamine instead of **1-3-4.**
**Step 2.** Compound **1-13-1** was prepared in a similar fashion to compound **BCD-CDK8-1-6e** (example 1, step 4) using compound **1-13-2** instead of compound **1-1-1.**
**Step 3.** Compound **BCD-CDK8-1-13** was prepared analogously to **1-1-1** (example 1, step 3) using **1-13-1** instead of **1-1-3** and **1-4-4** instead of **1-1-2.**

**Example 22.** Method of preparation of compound **BCD-CDK8-1-14.**

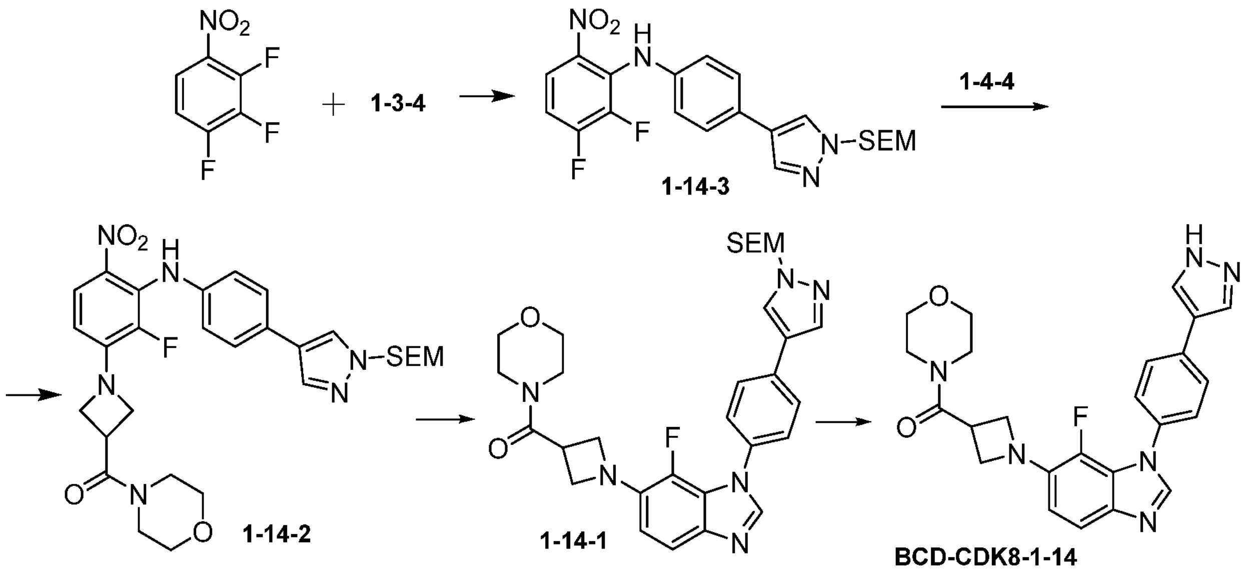


1-14-3, 1-14-2, 1-14-1, BCD-CDK8-1-14

**Step 1.** Compound **1-14-3** was obtained in a similar fashion to **1-3-2** (example 6, step 4) using 2,3,4-trifluoronitrobenzene instead of **1-3-3.**
**Step 2.** Solution of **1-14-3** (735 mg, 1.65 mmol), **1-4-4** (1.03 g, 1.81 mmol), and DIPEA (1.01 mL, 5.77 mmol) in 30

mL of DMSO was heated at 110°C for 5 hours. Reaction mixture was poured into 100 mL of water. Suspension was filtered, precipitate was washed with water, dissolved in ethyl acetate, washed with 0.1 M HCl solution. Organic phase was concentrated under vacuum. Product was isolated as orange powder by column chromatography on silica gel using hexane-ethyl acetate (1:1) as eluent. Yield: 794 mg (81%).

**Step 3.** Compound **1-14-1** was prepared in a similar fashion to compound **BCD-CDK8-1-6e** (example 1, step 4) using compound **1-14-2** instead of compound **1-1-1.**

**Step 4.** Compound **BCD-CDK8-1-14** was prepared analogously to **BCD-CDK8-1-3** (example 6, step 6) using compound **1-14-1** instead of compound **1-3-1.**

**Example 23.** Method of preparation of compound **BCD-CDK8-1-20.**

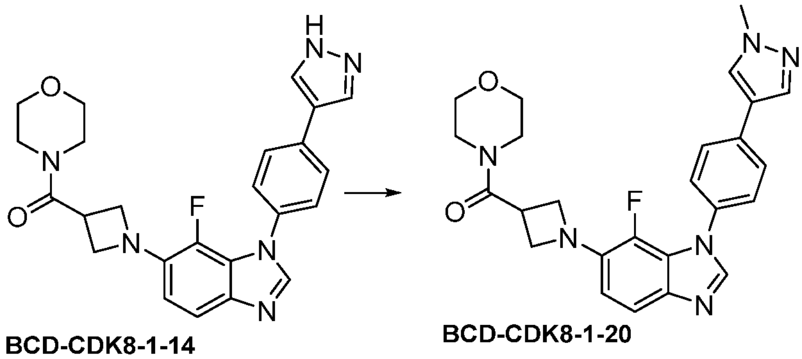

**BCD-CDK8-1-14** **BCD-CDK8-1-20**

Compound **BCD-CDK8-1-20** was prepared analogously to compound **BCD-CDK8-1-16** (example 7) using compound **BCD-CDK8-1-14** instead of compound **BCD-CDK8-1-3.**

**Example 24.** Method of preparation of compound **BCD-CDK8-1-15e.**

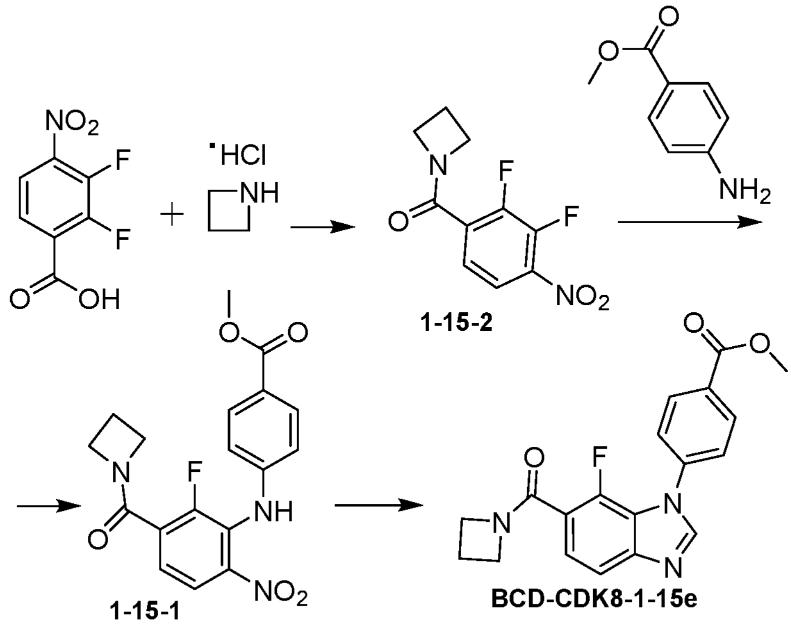

**1-15-2**

**1-15-1**

**BCD-CDK8-1-15e**

**Step 1.** Compound **1-15-2** was prepared in a similar fashion to **1-2-4** (example 5, step 1) using 2,3-difluoro-4-nitrobenzoic acid instead of 3-fluoro-4-nitrobenzoic acid.

**Step 2.** Compound **1-15-1** was prepared in a similar fashion to compound **1-1-3** (example 1, step 1) using **1-15-2** instead of 2-fluoro-4-bromonitrobenzene.

**Step 3.** Compound **BCD-CDK8-1-15e** was prepared analogously to **BCD-CDK8-1-6e** (example 1, step 4) using compound **1-15-1** instead of compound **1-1-1.**

**Example 25.** Method of preparation of compound **BCD-CDK8-1-15a.**

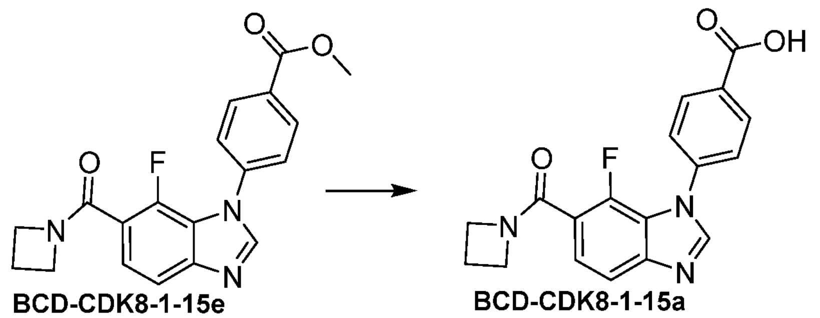

**BCD-CDK8-1-15e** **BCD-CDK8-1-15a**

Compound **BCD-CDK8-1-15a** was prepared analogously to compound **BCD-CDK8-1-6a** (example 2) using compound **BCD-CDK8-1-15e** instead of compound **BCD-CDK8-1-6e.**

**Example 26.** Method of preparation of compound **BCD-CDK8-1-15.**

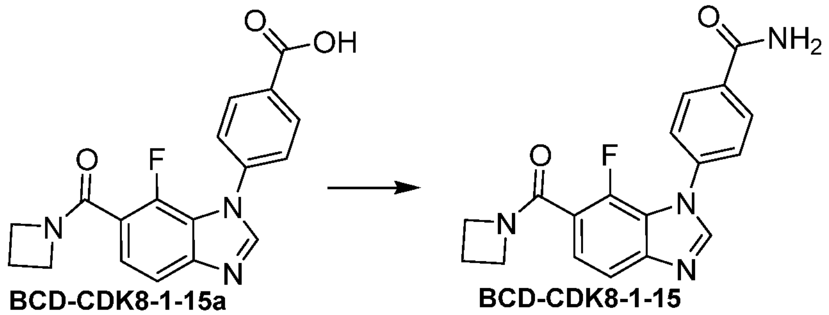

**BCD-CDK8-1-15a** **BCD-CDK8-1-15**

Compound **BCD-CDK8-1-15** was prepared in a similar fashion to compound **BCD-CDK8-1-6** (example 4) using **BCD-CDK8-1-15a** instead of **BCD-CDK8-1-6a.**

**Example 27.** Method for preparation of compound **BCD-CDK8-1-21a.**

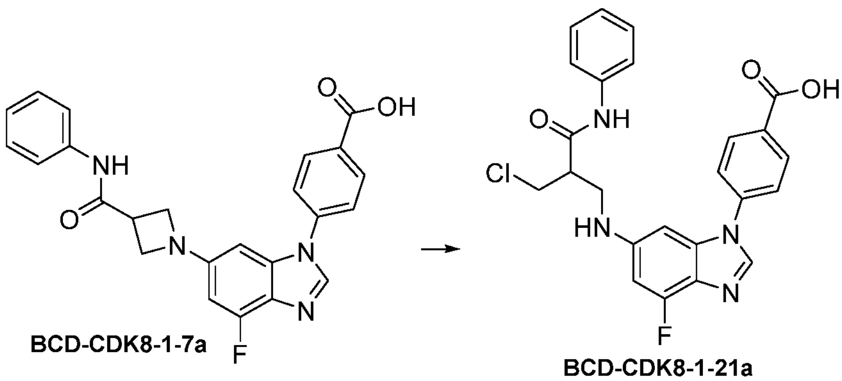

**BCD-CDK8-1-7a** **BCD-CDK8-1-21a**

5 mL of 4M HCl solution in 1,4-dioxane was added to a solution of compound **BCD-CDK8-1-7a** (100 mg, 0.23 mmol) in 10 mL of 1,4-dioxane. Mixture was stirred at room temperature for 12 hours, then concentrated under vacuum. Reaction product was isolated as white powder by column chromatography on silica gel using mixture of dichloromethane-methanol (94:6) as eluent. Yield: 90 mg (83%).

**Example 28.** Method of preparation of compound **BCD-CDK8-1-21.**

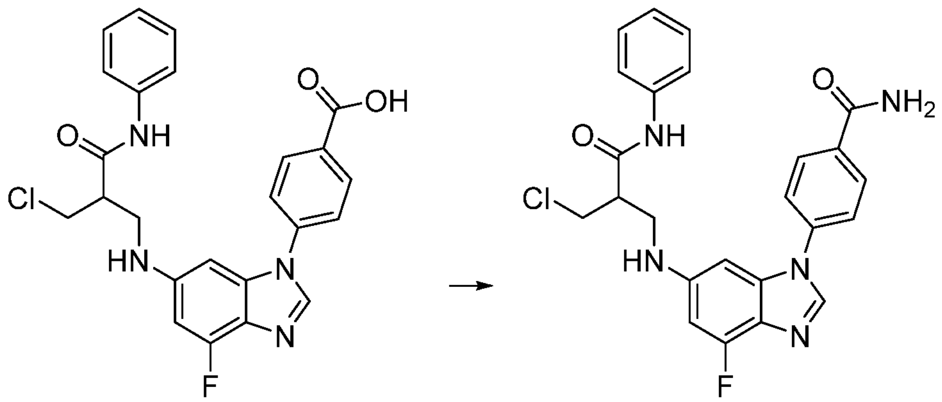

BCD-CDK8-1-21a → BCD-CDK8-1-21

Compound **BCD-CDK8-1-21** was prepared in a similar fashion to compound **BCD-CDK8-1-6** (example 4) using **BCD-CDK8-1-21a** instead of **BCD-CDK8-1-6a.**

**Example 29.** Method of preparation of compound **BCD-CDK8-5-2.**

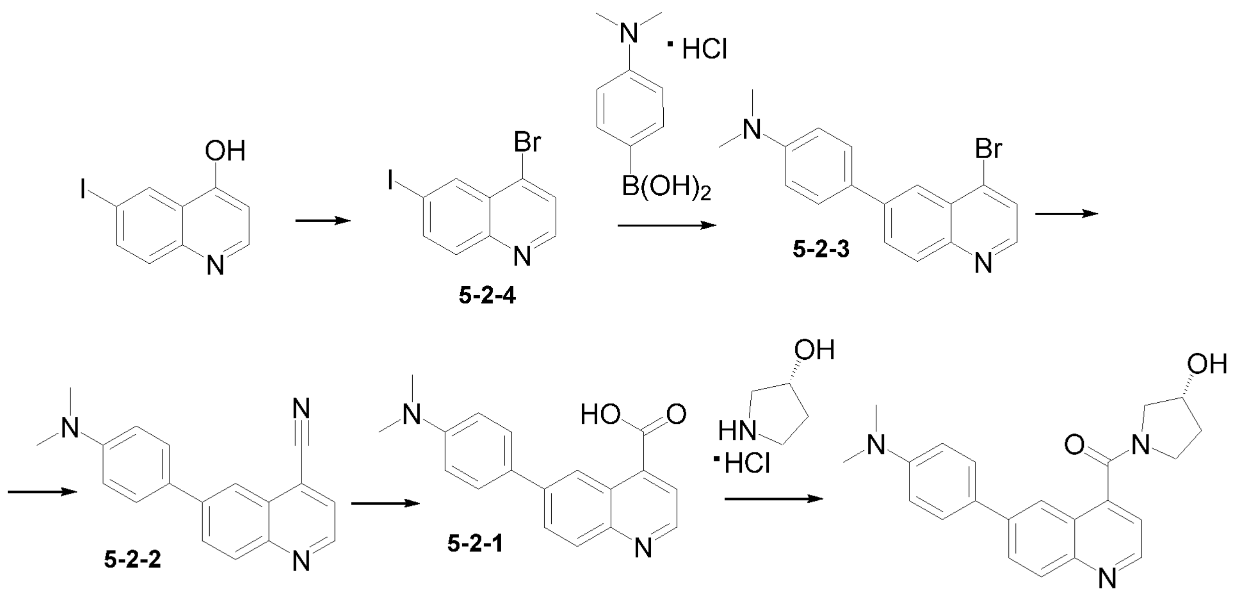

5-2-4, 5-2-3, 5-2-2, 5-2-1, BCD-CDK8-5-2

**Step 1.** $PBr_3$ (0.909 mL, 9.59 mmol) was added to a solution of 4-hydroxy-6-iodoquinoline (2.00 g, 7.38 mmol) in 25 mL of DMF under cooling in an ice bath. Reaction mixture was then adjusted to room temperature and stirred for 3 hours. 300 mL of water was then added, product was extracted with ethyl acetate (3×150 mL). Organic layers were separated, washed with saturated $Na_2CO_3$ and NaCl solutions, and dried with $Na_2SO_4$. Reaction product **5-2-4** was obtained as white powder after concentrating the solution under vacuum. Yield: 2.38 g (97%).

**Step 2. 5-2-4** (700 mg, 2.10 mmol), 4-(dimethylamino)phenylboronic acid hydrochloride (464 mg, 2.31 mmol) and

$CS_2CO_3$ (2.39 g, 7.34 mmol) were dissolved in 10 mL of 1,4-dioxane and 5 mL of water. $Pd(PPh_3)_4$ (121 mg, 0.105 mmol) was added to mixture under inert atmospheric conditions. Reaction mixture was stirred at 80 °C for 6 hours. 250 mL of water was then added, product was extracted with ethyl acetate (3×150 mL). Organic layers were separated, washed with a saturated NaCl solution, and dried with $Na_2SO_4$. Product **5-2-3** was isolated as yellow powder by column chromatography on silica gel using hexane-ethyl acetate (9:1) as eluent. Yield: 514 mg (75%).

**Step 3.** $Pd(PPh_3)_4$ (90 mg, 0.078 mmol) was added to suspension of **5-2-3** (510 mg, 1.56 mmol) and $Zn(CN)_2$ (220 mg, 1.87 mmol) in 5 mL of DMF under inert atmospheric conditions at room temperature. Resulting mixture was then stirred at 100 °C for 1 hour. Mixture was poured into water, precipitated crystals were filtered off, washed with water, thus obtaining product **5-2-2** as yellow powder. Yield: 236 mg (94%).

**Step 4.** KOH solution (264 mg, 4.71 mmol) in 5 mL of water was added to **5-2-2** (430 mg, 1.57 mmol) dissolved in 10 mL of ethanol. Reaction mixture was refluxed for 60 hours. 1M HCl solution was then added to pH 4. Brown precipitate of compound **5-2-1** was filtered off. Yield: 413 mg (90%).

**Step 5: 5-2-1** (150 mg, 0.513 mmol), (*R*)-3-hydroxypyrrolidine hydrochloride (70 mg, 5.64 mmol), HOBt (110 mg, 0.718 mmol) and DIPEA (0.357 mL, 2.05 mmol) were dissolved in 3 mL of DMF. EDC'HCl (138 mg, 0.564 mmol) was added portionwise to mixture under cooling in an ice bath and stirred for 16 hours at room temperature. 100 mL of water was added, product was extracted with ethyl acetate (3×70 mL). Organic layers were separated, washed with a saturated NaCl solution, and dried with $Na_2SO_4$. Product **BCD-CDK8-5-2** was isolated as light brown powder by column chromatography on silica gel using dichloromethane-methanol (97:3) as eluent. Yield: 89 mg (48%).

**Example 30.** Method for preparation of compound **BCD-CDK8-5-2i.**

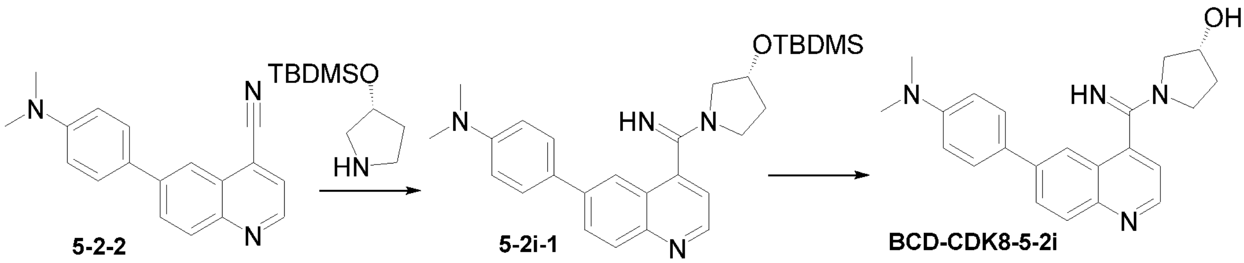

5-2-2 5-2i-1 BCD-CDK8-5-2i

**Step 1.** (*R*)-3-(*tert*-butyldimethylsilyloxy)pyrrolidine solution (766 mg, 3.22 mmol) (prepared as described in WO 2003/045315) in 3 mL of dry THF was added to ethylmagnesium bromide (0.914 mL, 2.93 mmol) under inert atmospheric conditions and cooling in an ice bath. The mixture was incubated for 1 hour at 30°C. The resulting solution was added, being stirred, to a solution of **5-2-2**(200 mg, 0.732 mmol) in 5 mL of dry THF and incubated for 1 hour at 30°C. The reaction was quenched with 50 mL of a saturated $NH_4Cl$ solution, extraction was performed with ethyl acetate (3×30 mL). Organic layers were separated, washed with a saturated NaCl solution, and dried with $Na_2SO_4$. Product **5-2i-1** was isolated as viscous light-yellow liquid by column chromatography on silica gel using dichloromethane-methanol (97:3) as eluent. Yield: 330 mg (95%).

**Step 2. 5-2i-1** (330 mg, 0.694 mmol) was dissolved in 5 mL of dichloromethane and, while cooling in an ice bath, 3 mL of 4M HCl solution in 1,4-dioxane was added, the mixture was incubated for 16 hours at room temperature. At the end of reaction, 0.25 mL of 7M $NH_3$ solution in methanol was added under stirring, and then solvents were evaporated. Product **BCD-CDK8-5-2i** was isolated as yellow powder by column chromatography on silica gel using dichloromethane-$NH_3$in methanol (7M) (15:1) as eluent. Yield: 236 mg (94%).

**Example 31.** Method of preparation of compound **BCD-CDK8-5-3.**

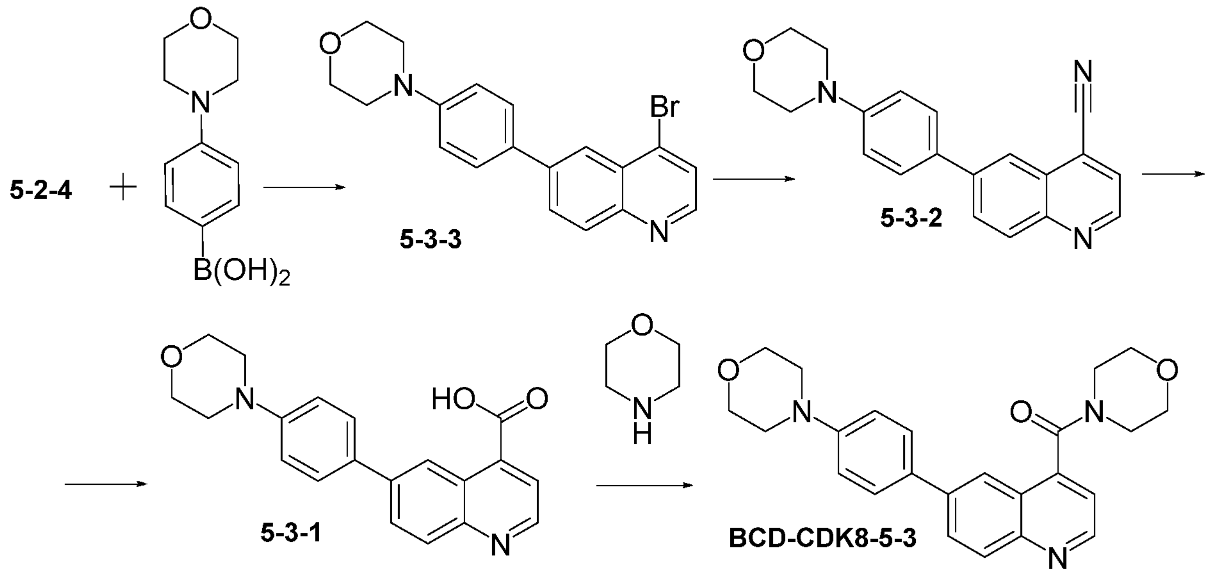

5-2-4 5-3-3 5-3-2 5-3-1 BCD-CDK8-5-3

**Step 1.** Compound **5-3-3** was prepared in a similar fashion to compound **5-2-3** (example 29, step 2) using 4-morpholinophenylboronic acid instead of 4-(dimethylamino) phenylboronic acid hydrochloride.

**Step 2.** Compound **5-3-2** was prepared in a similar fashion to compound **5-2-2** (example 29, step 3) using compound **5-3-3** instead of compound **5-2-3.**

**Step 3.** Compound **5-3-1** was prepared in a similar fashion to compound **5-2-1** (example 29, step 4) using compound **5-3-2** instead of compound **5-2-2.**

**Step 4.** Compound **BCD-CDK8-5-3** was prepared in a similar fashion to compound **BCD-CDK8-5-2** (example 29, step 5) using compound **5-3-1** instead of compound **5-2-1** and morpholine instead of (R)-3 hydroxypyrrolidine.

**Example 32.** Method for preparation of compound **BCD-CDK8-5-3i.**

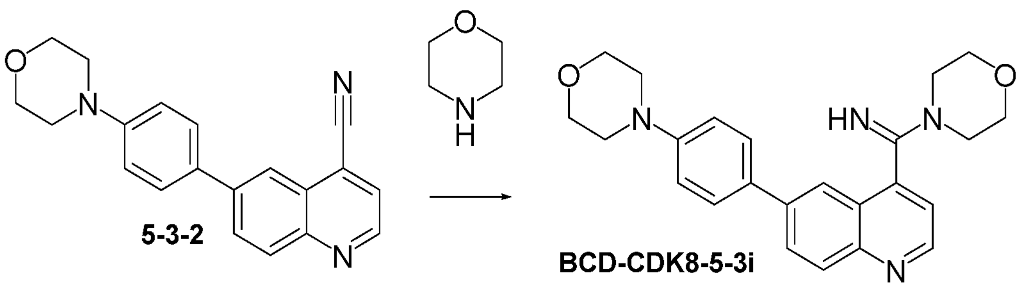

5-3-2 BCD-CDK8-5-3i

Compound **BCD-CDK8-5-3i** was prepared in a similar fashion to compound **5-2i-1** (example 30, step 1) using compound **5-3-2** instead of compound **5-2-2** and morpholine instead of (*R*)-3-(*tert*-butyldimethylsilyloxy)pyrrolidine.

**Example 33.** Method of preparation of compound **BCD-CDK8-5-4.**

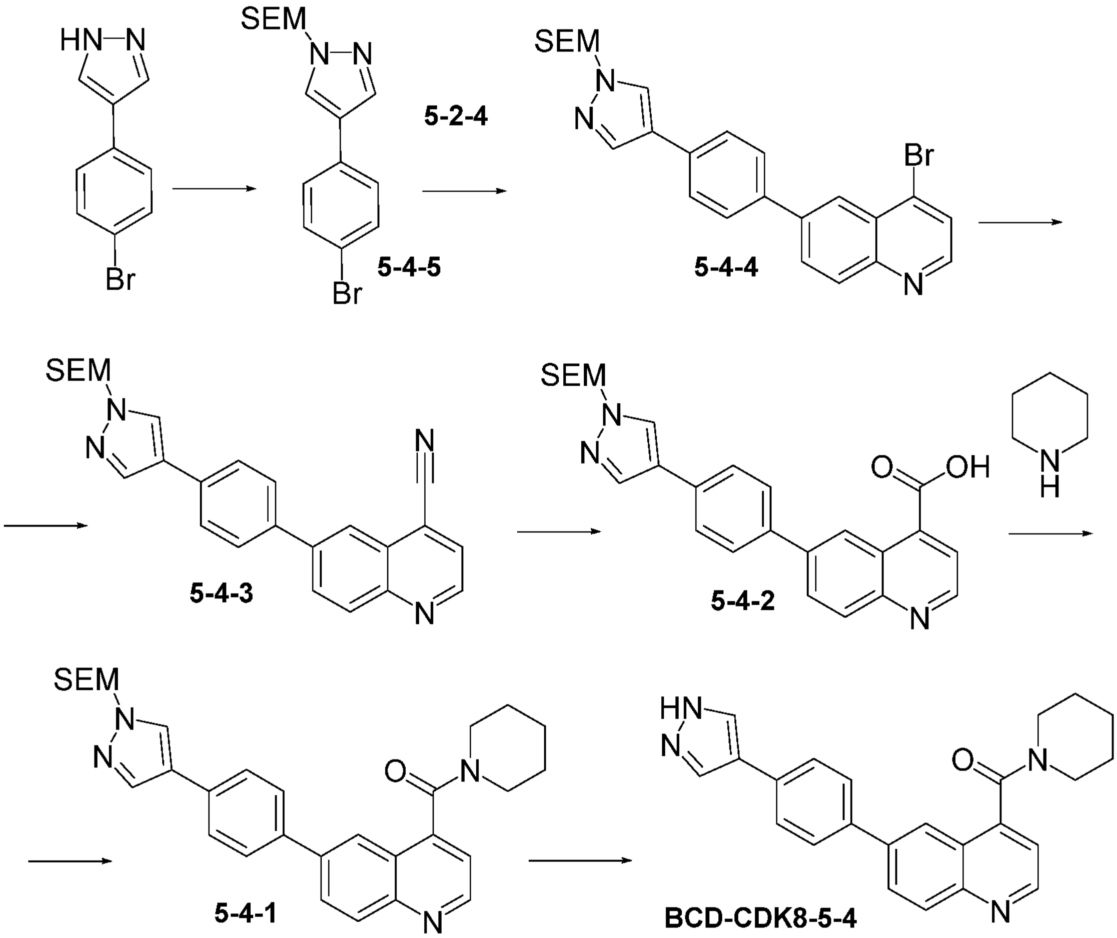

5-2-4

5-4-5

5-4-4

5-4-3

5-4-2

5-4-1

BCD-CDK8-5-4

**Step 1.** NaH (834 mg, 0.021 Mol) was added portionwise to 4-(4-bromophenyl)-1*H*-pyrazole (3.00 g, 0.01 mol) in 10 mL of DMF under stirring and cooling in an ice bath. The reaction mixture was adjusted to room temperature and incubated for 15 minutes. 2-(trimethylsilyl)ethoxymethyl chloride (3.36 g, 0.02 mol) was then added. After 1 hour, the mixture was poured into 300 mL of water, extraction was performed with ethyl acetate (3×100 mL). Organic layers were separated, washed with a saturated NaCl solution, and dried with $Na_2SO_4$. Reaction product **5-4-5** was obtained as light-yellow powder after concentrating the solution under vacuum. Yield: 3.42 g (88%).

**Step 2.** Palladium (II) acetate (35 mg, 0.158 mmol) was added under inert atmospheric conditions to a stirred suspension of **5-4-5** (557 mg, 1.58 mmol), bis(pinacolato)diborane (600 mg, 2.36 mmol), potassium acetate (464 mg, 4.73 mmol), XPhos (150 mg, 0.316 mmol) in 20 mL of 1,4-dioxane. The reaction mixture was incubated at 80 °C for 30 minutes. **5-2-4** (500 mg, 1.50 mmol), $Na_2CO_3$ (835 mg, 7.88 mmol), $Pd(PPh_3)_4$(182 mg, 0.158 mmol) and 10 mL of water were then added. The reaction mixture was stirred at 80 °C for 3 hours. 250 mL of water was then added, product was extracted with ethyl acetate (3×150 mL). Organic layers were separated, washed with a saturated NaCl solution, and dried with $Na_2SO_4$. Product **5-4-4** was isolated as yellow powder by column chromatography on silica gel using hexane-ethyl acetate (9:1) as eluent. Yield: 514 mg (75%).

**Step 3.** Compound **5-4-3** was prepared in a similar fashion to compound **5-2-2** (example 29, step 3) using compound **5-4-4** instead of compound **5-2-3.**

**Step 4.** Compound **5-4-2** was prepared in a similar fashion to compound **5-2-1** (example 29, step 4) using compound **5-4-3** instead of compound **5-2-2.**

**Step 5:** Compound **5-4-1** was prepared in a similar fashion to compound **BCD-CDK8-5-2** (example 29, step 5) using compound **5-4-2** instead of compound **5-2-1** and piperidine instead of (*R*)-3-hydroxypyrrolidine hydrochloride.

**Step 6. 5-4-1** (57 mg, 0.111 mmol) was dissolved in 3 mL of dichloromethane and, under cooling in an ice bath, 1 mL of trifluoroacetic acid was added. The solution was stirred for 16 hours at room temperature. 50 mL of a saturated $Na_2CO_3$ solution was then added, product was extracted with dichloromethane (3×30 mL). Organic layers were separated, washed with a saturated NaCl solution, and dried with $Na_2SO_4$. Product **BCD-CDK8-5-4** was isolated as light-yellow powder by column chromatography on silica gel using dichloromethane-methanol (97:3) as eluent. Yield: 30 mg (71%).

**Example 34.** Method for preparation of compound **BCD-CDK8-5-4i.**

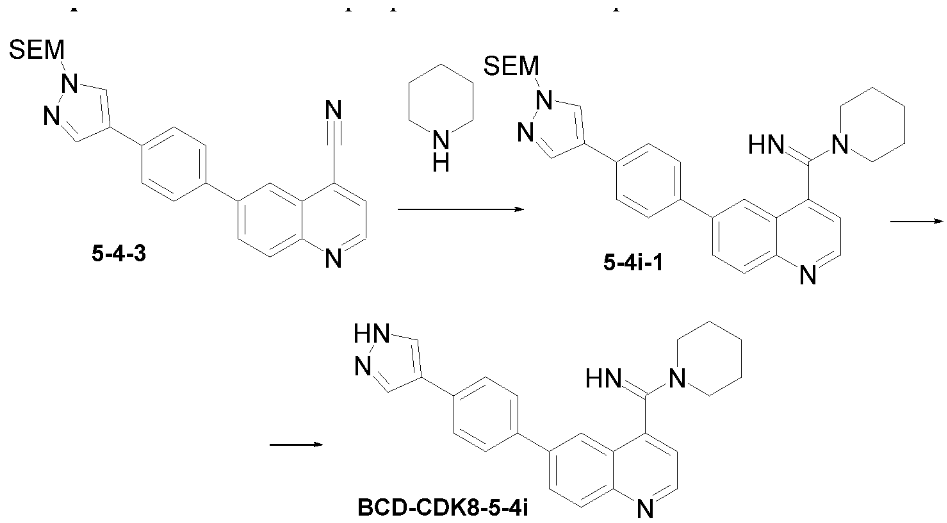

5-4-3

5-4i-1

BCD-CDK8-5-4i

**Step 1.** Compound **5-4i-1** was prepared in a similar fashion to compound **5-2i-1** (example 30, step 1) using compound **5-4-3** instead of compound **5-2-2** and piperidine instead of compound (*R*)-3-(*tert*-butyldimethylsilyloxy)pyrrolidine.
**Step 2.** Compound **BCD-CDK8-5-4i** was prepared in a similar fashion to compound **BCD-CDK8-5-4** (example 33, step 6) using compound **5-4i-1** instead of compound **5-4-1.**

**Example 35.** Method of preparation of compound **BCD-CDK8-5-6.**

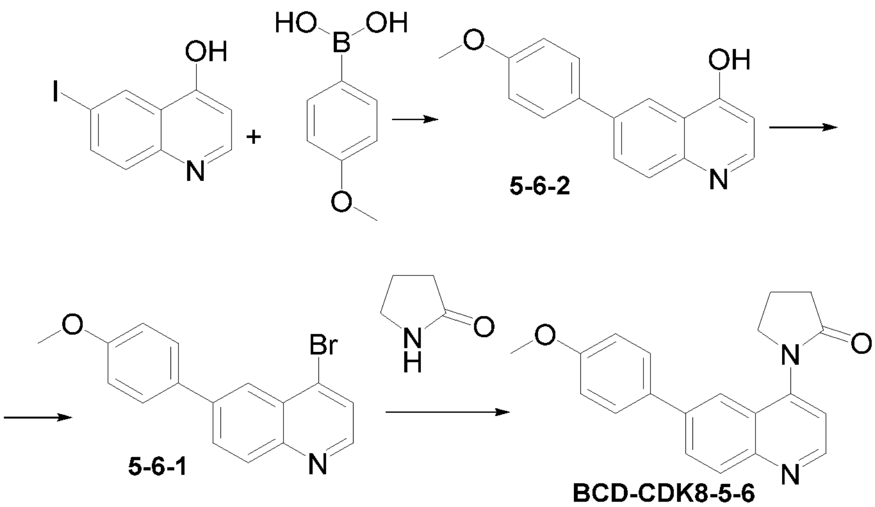

5-6-2

5-6-1

BCD-CDK8-5-6

**Step** 1. Compound 5-6-2 was prepared in a similar fashion to compound 5-2-3 (example 29, step 2) using 4-hydroxy-6-iodoquinoline instead of compound 5-2-4 and 4-methoxyphenylboronic acid instead of 4-(dimethylamino)phenylboronic acid hydrochloride.
**Step 2.** Compound **5-6-1** was prepared in a similar fashion to compound **5-2-4** (example 29, step 1) using compound **5-6-2** instead of 4-hydroxy-6-iodoquinoline.
**Step 3.** Palladium (II) acetate (4 mg, 5 mol%) was added to a suspension of **5-6-1** (100 mg, 0.318 mmol), 2-pyrrolidone (54 mg, 0.637 mmol), BINAP (20 mg, 10 mol%) and $Cs_2CO_3$ (259 mg, 0.796 mmol) in 5 mL of 1,4-dioxane under inert atmospheric conditions. The reaction mixture was stirred at 80 °C for 4 hours. 50 mL of water was then added, product was extracted with ethyl acetate (3×30 mL). Organic layers were separated, washed with

a saturated NaCl solution, and dried with $Na_2SO_4$. Product **BCD-CDK8-5-6** was isolated by column chromatography on silica gel using dichloromethane-methanol (98:2) as eluent. Yield: 76 mg (75%).

**Example 36.** Method of preparation of compound **BCD-CDK8-5-7.**

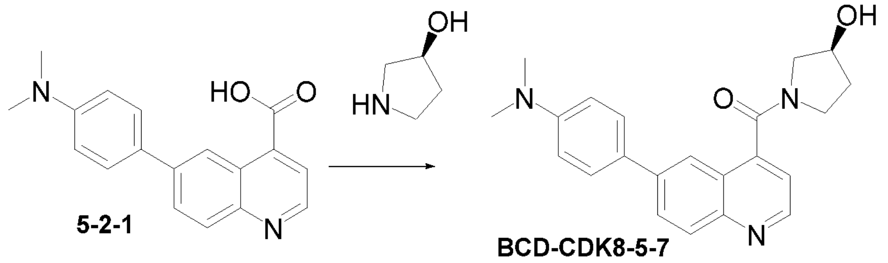

**5-2-1** **BCD-CDK8-5-7**

Compound **BCD-CDK8-5-7** was prepared in a similar fashion to compound **BCD-CDK8-5-2** (example 29, step 5) using (*S*)-3-hydroxypyrrolidine hydrochloride instead of (*R*)-3-hydroxypyrrolidine hydrochloride.

**Example 37.** Method for preparation of compound **BCD-CDK8-5-7i.**

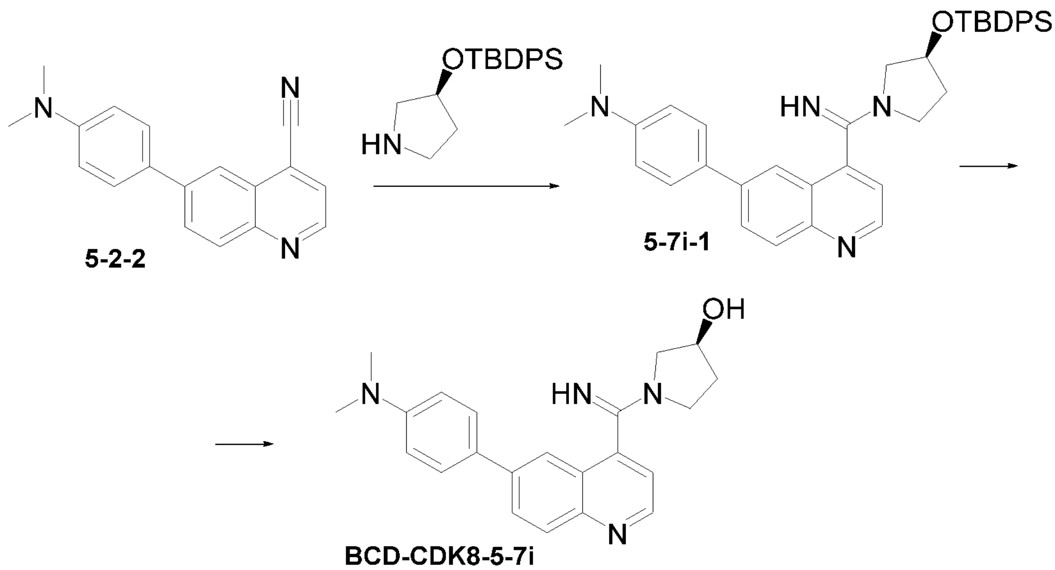

**5-2-2** **5-7i-1** **BCD-CDK8-5-7i**

**Step 1.** Compound **5-7i-1** was prepared in a similar fashion to compound **5-2i-1** (example 30, step 1) using (*S*)-3-(*tert*-butyldiphenylsilyloxy) pyrrolidine (prepared as described in WO 2014/029983) instead of (*R*)-3-(*tert*-butyldimethylsilyloxy) pyrrolidine.

**Step 2. 5-7i-1** (150 mg, 0.250 mmol) was dissolved in 2 mL of dichloromethane, and, under cooling, 4 mL of 4M HCl solution in 1,4-dioxane was added, the mixture was incubated for 72 hours at room temperature. At the end of reaction, 0.25 mL of 7M $NH_3$ solution in methanol was added under stirring, and then solvents were evaporated. Product **BCD-CDK8-5-7i** was isolated by column chromatography on silica gel using dichloromethane-$NH_3$ as eluent in methanol (7M) (15:1). Yield: 70 mg (78%).

**Example 38.** Method of preparation of compound **BCD-CDK8-5-8.**

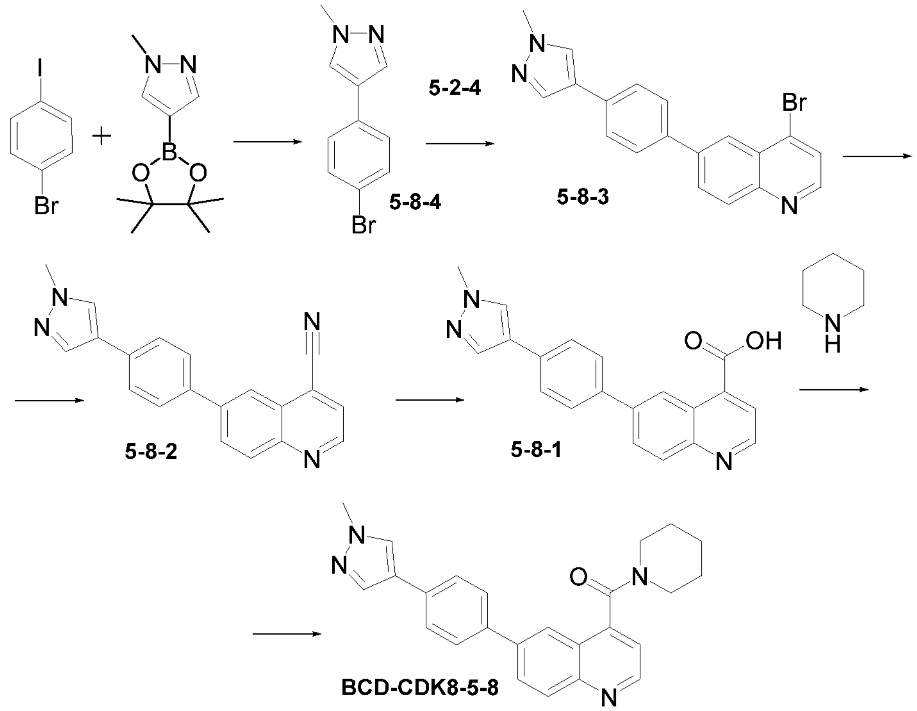

5-8-4

5-8-3

5-8-2

5-8-1

BCD-CDK8-5-8

**Step 1.** Compound **5-8-4** was prepared in a similar fashion to compound **5-2-3** (example 29, step 2) using 1-bromo-4-iodobenzene instead of compound **5-2-4** and 1-methyl-4-(4,4,5,5-tetramethyl-1,3,2-dioxoborolan-2-yl)-1*H*-pyrazole instead of 4-(dimethylamino)phenylboronic acid hydrochloride.

**Step 2.** Compound **5-8-3** was prepared in a similar fashion to compound **5-4-4** (example 33, step 2) using compound **5-8-4** instead of compound **5-4-5.**

**Step 3.** Compound **5-8-2** was prepared in a similar fashion to compound **5-2-2** (example 29, step 3) using compound **5-8-3** instead of compound **5-2-3.**

**Step 4.** Compound **5-8-1** was prepared in a similar fashion to compound **5-2-1** (example 29, step 4) using compound **5-3-2** instead of compound **5-2-2.**

**Step 5:** Compound **BCD-CDK8-5-8** was prepared in a similar fashion to compound **BCD-CDK8-5-2** (example 29, step 5) using compound **5-8-1** instead of compound **5-2-1** and piperidine instead of (*R*)-3 hydroxypyrrolidine.

**Example 39.** Method for preparation of compound **BCD-CDK8-5-8i.**

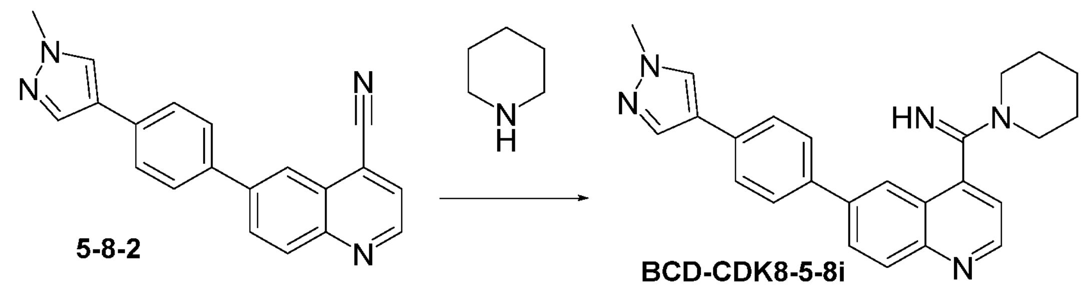
5-8-2

BCD-CDK8-5-8i

Compound **BCD-CDK8-5-8i** was prepared in a similar fashion to compound **5-2i-1** (example 30, step 1) using compound **5-8-2** instead of compound **5-2-2** and piperidine instead of (*R*)-3-(*tert*-butyldimethylsilyloxy) pyrrolidine.

**Example 40.** Method for preparation of compound **BCD-CDK8-5-1Cl.**

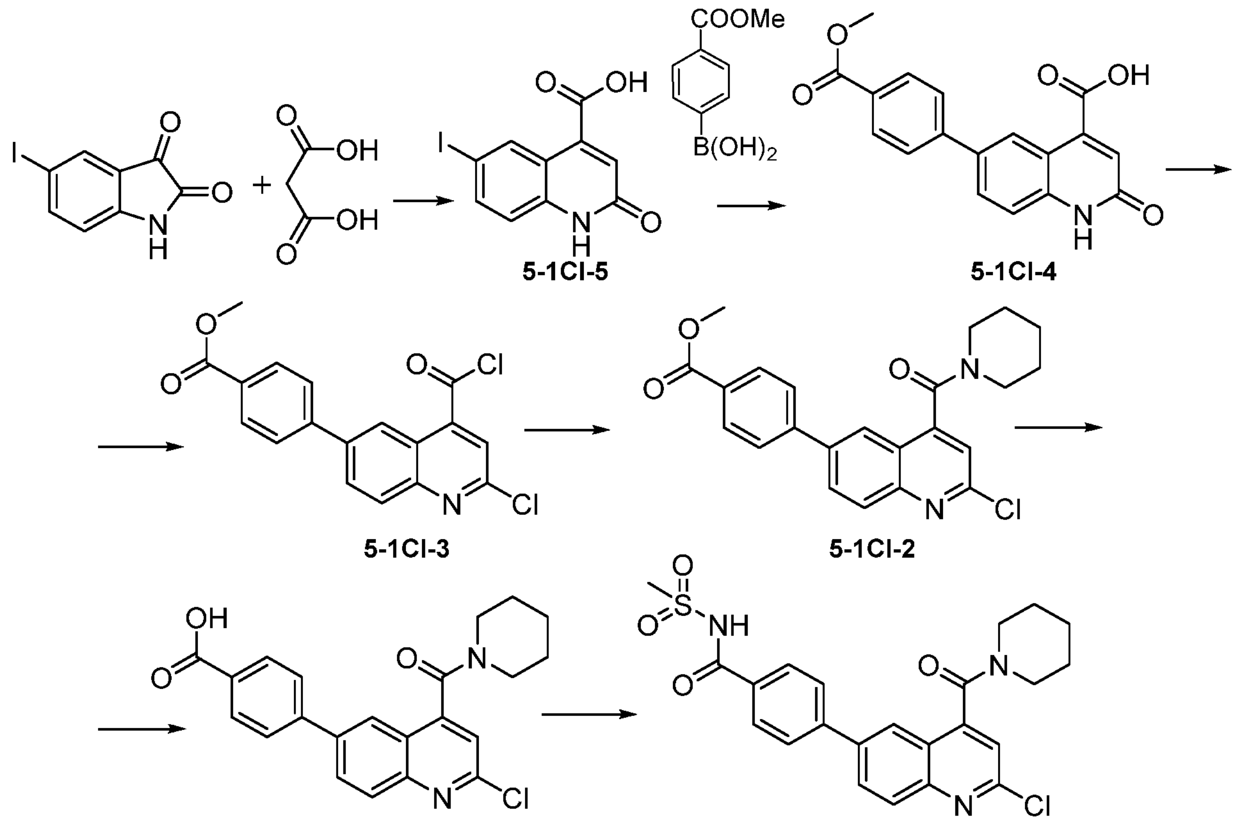

5-1Cl-5

5-1Cl-4

5-1Cl-3

5-1Cl-2

5-1Cl-1

BCD-CDK8-5-1Cl

**Step** 1. A mixture of 5-iodoisatin (2.00 g, 7.32 mmol), malonic acid (838 mg, 8.06 mmol) and sodium acetate (751 mg, 9.16 mmol) was refluxed for 10 hours in 15 mL of acetic acid. The suspension was filtered, precipitate was washed with ethanol and acetone. Product **5-1Cl-5** was obtained as dark gray powder. Yield: 1.35 g (58%).

**Step 2.** Compound **5-1Cl-4** was prepared in a similar fashion to compound **5-2-3** (example 29, step 2) using compound **5-1Cl-5** instead of **5-2-4** and 4-methoxycarbonylphenylboronic acid instead of 4-(dimethylamino)phenylboronic acid hydrochloride.

**Step 3.** $SOCl_2$ (5 mL, 68.9 mmol) was added to **5-1Cl-4** (1.21 g, 3.73 mmol) at 0-5 °C. The reaction mixture was incubated for 1.5 hours at 100 °C. Volatile components were then evaporated under reduced pressure. Product **5-1Cl-3** was obtained as brown powder and was used in next step without additional purification. Yield: 1.34 g (100%).

**Step 4.** A suspension of **5-1Cl-3** (1.34 g, 3.73 mmol) in 10 mL of dry dichloromethane was added under stirring to a solution of piperidine (1.11 g, 13.1 mmol) and triethylamine (5.21 mL, 37.3 mmol) in 10 mL of dry dichloromethane at 0-5 °C. The reaction mixture was incubated for 6 hours at room temperature. After concentrating, product **5-1Cl-2** was isolated as light-yellow powder by column chromatography on silica gel using hexane-ethyl acetate (3:1) as eluent. Yield: 457 mg (30%).

**Step 5:** Compound **5-1Cl-1** was prepared in a similar fashion to compound **BCD-CDK8-1-6a** (example 2) using compound **5-1Cl-2** instead of compound **BCD-CDK8-1-6e.**

**Step 6.** Compound **BCD-CDK8-5-1Cl** was prepared in a similar fashion to compound **BCD-CDK8-1-1** (example 3) using **5-1Cl-1** instead of **BCD-CDK8-1-6a.**

**Example 41.** Method of preparation of compound **BCD-CDK8-5-1.**

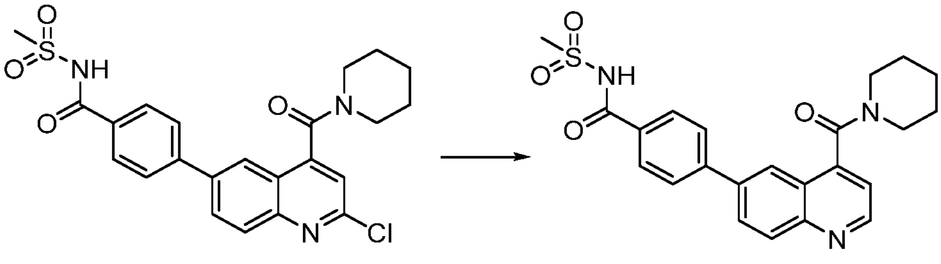

BCD-CDK8-5-1Cl BCD-CDK8-5-1

A mixture of **BCD-CDK8-5-1Cl** (127 mg, 0.27 mmol), triethylamine (0.075 mL, 0.54 mmol) and Pd/C (20 mg) in 5 mL of methanol was stirred under hydrogen at 8 atm for 3 hours. After concentrating, product was isolated as white powder by preparative chromatography. Yield: 100 mg (85%).

**Example 42.** Method for preparation of compound **BCD-CDK8-5-5Cl.**

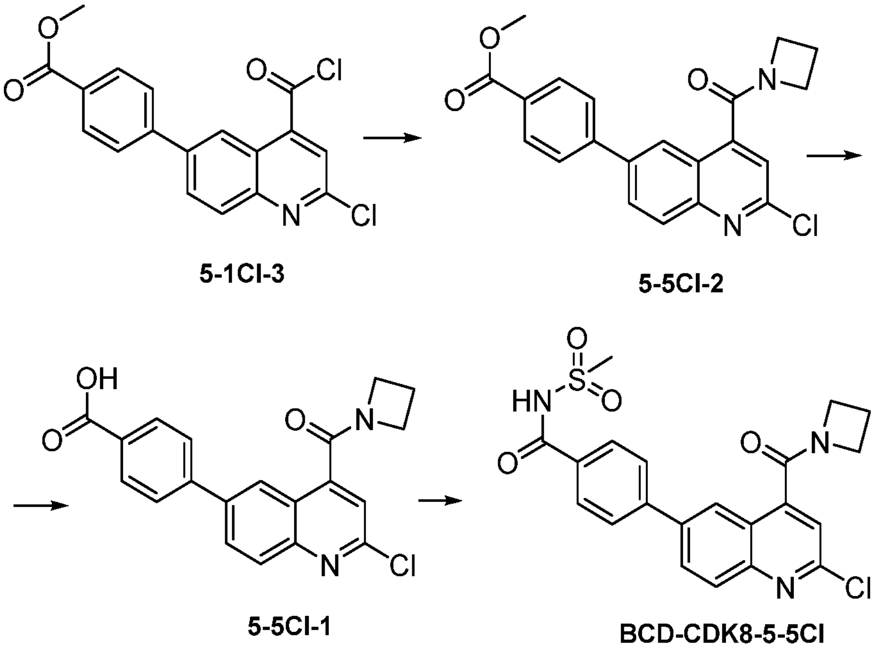

5-1Cl-3 5-5Cl-2

5-5Cl-1 BCD-CDK8-5-5Cl

**Step 1.** Compound **5-5Cl-2** was prepared analogously to **5-1Cl-2** (example 40, step 4) using azetidine hydrochloride instead of piperidine.
**Step 2.** Compound **5-5Cl-1** was prepared in a similar fashion to compound **BCD-CDK8-1-6a** (example 2) using compound **5-5Cl-2** instead of compound **BCD-CDK8-1-6e.**
**Step 3.** Compound **BCD-CDK8-5-5Cl** was prepared in a similar fashion to compound **BCD-CDK8-1-1** (example 3) using **5-5Cl-1** instead of **BCD-CDK8-1-6a.**

**Example 43.** Method of preparation of compound **BCD-CDK8-5-5.**

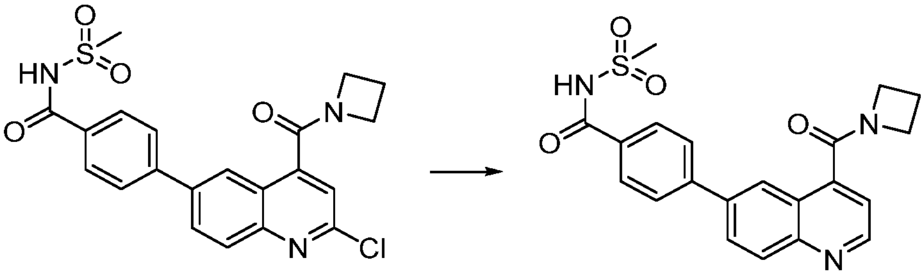

BCD-CDK8-5-5Cl BCD-CDK8-5-5

Compound **BCD-CDK8-5-5** was prepared in a similar fashion to compound **BCD-CDK8-5-1** (example 41) using **BCD-CDK8-5-5Cl** instead of **BCD-CDK8-5-1Cl.**

**Example 44.** Method of preparation of compound **BCD-CDK8-6-1.**

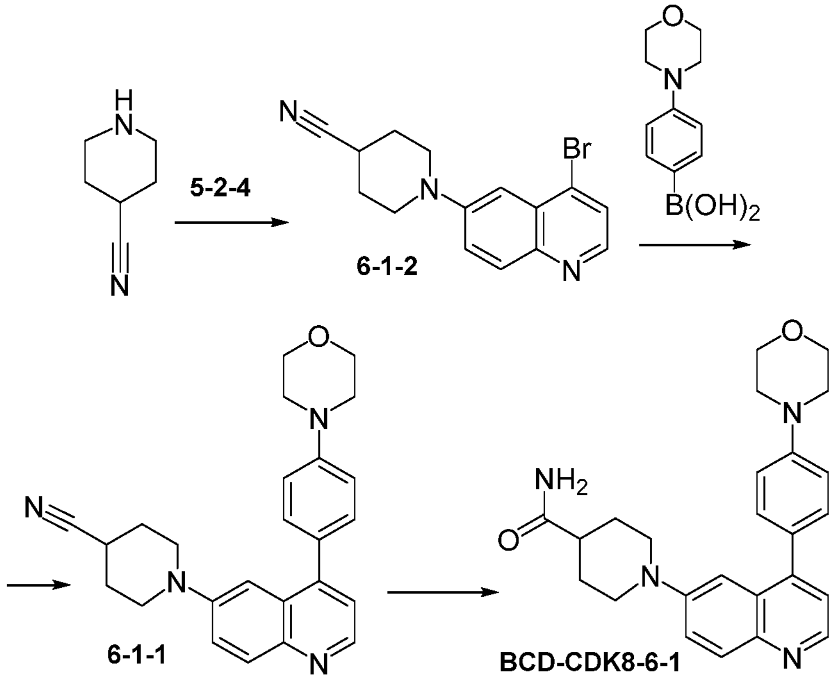


6-1-2

6-1-1

BCD-CDK8-6-1

**Step 1.** Compound **6-1-2** was prepared in a similar fashion to compound **BCD-CDK8-5-6** (example 35, step 3) using compound **5-2-4** instead of compound **5-6-1** and piperidine-4-carbonitrile instead of 2-pyrrolidone.

**Step 2.** Compound **6-1-1** was prepared in a similar fashion to compound **5-2-3** (example 29, step 2) using compound **6-1-2** instead of compound **5-2-4** and 4-morpholinophenylboronic acid instead of 4-(dimethylamino)phenylboronic acid hydrochloride.

**Step 3. 6-1-1** (101 mg, 0.253 mmol) was dissolved in 2 mL of 80 wt % $H_2SO_4$ and incubated for 48 hours at room temperature. The reaction mixture was then poured into 50 mL of water, saturated $Na_2CO_3$ solution was added to pH 8, product was extracted with ethyl acetate (3×50 mL). Combined extract was washed with a saturated NaCl solution and dried with $Na_2SO_4$. Product **BCD-CDK8-6-1** was isolated as yellow-orange powder by column chromatography on silica gel using dichloromethane-methanol (97:3), as eluent. Yield: 61 mg (57%).

**Example 45.** Method of preparation of compound **BCD-CDK8-6-2.**

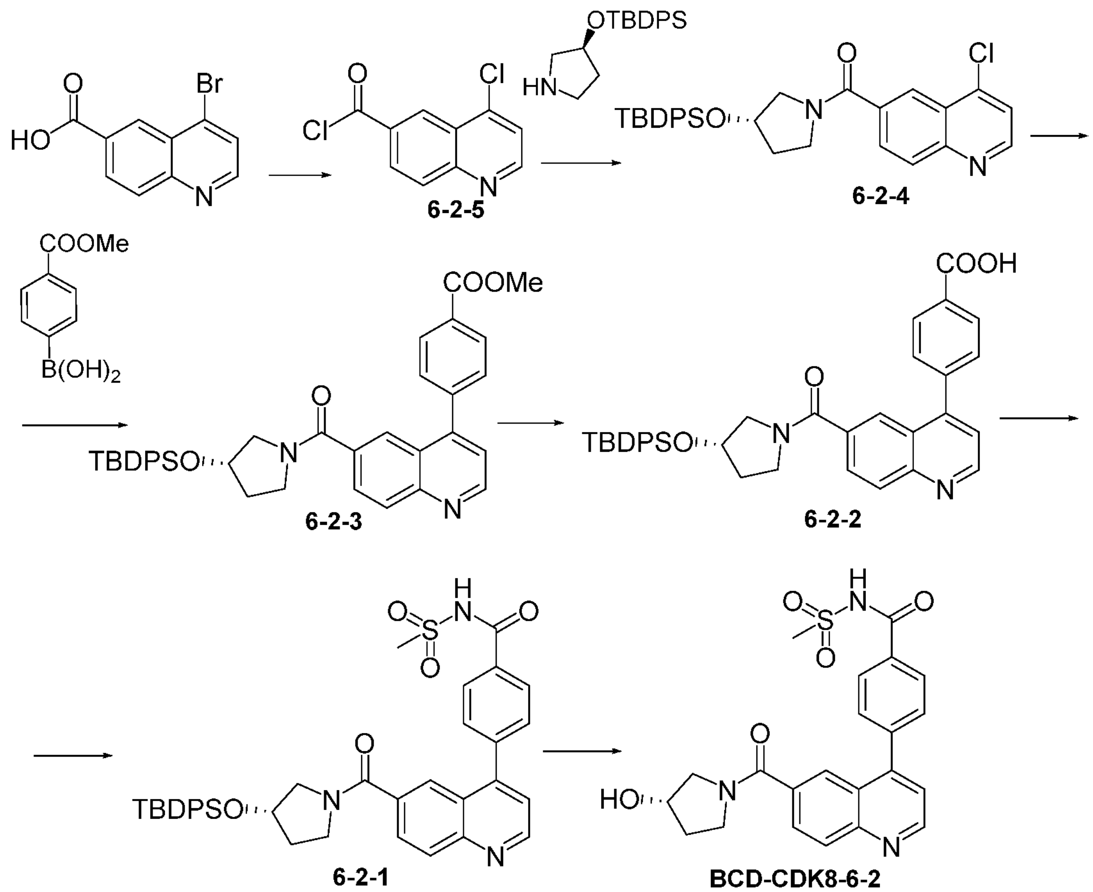

6-2-5 6-2-4 6-2-3 6-2-2 6-2-1 BCD-CDK8-6-2

**Step 1.** $SOCl_2$ (5 mL, 68.5 mmol) was added to 4-bromoquinoline-6-carboxylic acid (530 mg, 2.11 mmol) at 0-5 ° C. The reaction mixture was incubated for 3 hours at 100 °C. Volatile components were then evaporated under reduced pressure. Product **6-2-5** was obtained as beige powder and used in the next step without additional purification. Yield: 476 mg (100%).

**Step 2.** A suspension of **6-2-5** (476 mg, 2.11 mmol) in 4 mL of dry dichloromethane was added under stirring to a solution of (*S*)-3- (*tert*-butyldiphenylsilyloxy)pyrrolidine (755 mg, 2.32 mmol) and triethylamine (0.881 mL, 6.33 mmol) in 10 mL of dry dichloromethane at 0-5 °C. The reaction mixture was incubated for 6 hours at room temperature. After concentrating, product **6-2-4** was isolated as white powder by column chromatography on silica gel using hexane-ethyl acetate-dichloromethane (3:2:2) as eluent. Yield: 850 mg (72%).

**Step 3.** Compound **6-2-3** was prepared in a similar fashion to compound **5-2-3** (example 29, step 2) using compound **6-2-4** instead of compound **5-2-4** and 4-methoxycarbonylphenylboronic acid instead of 4-(dimethylamino) phenylboronic acid hydrochloride.

**Step 4.** A solution of LiOH·H**2**O (96 mg, 2.28 mmol) in 6 mL of water was added to a solution of **6-2-3** (934 mg, 1.52 mmol) in 12 mL of methanol. The reaction mixture was refluxed for 2 hours, methanol was evaporated, 0.1M HCl solution was added to pH 4. The suspension was filtered, precipitate was washed with water, product **6-2-2** was obtained as gray powder. Yield: 795 mg (87%).

**Step 5:** EDC 'HCl (128 mg, 0.67 mmol) was added portionwise under stirring and cooling in an ice bath to a solution of **6-2-2** (350 mg, 0.58 mmol), methanesulfonamide (66 mg, 0.70 mmol) and DMAP (11 mg, 0.09 mmol) in 5 mL of dichloromethane. The reaction mixture was incubated for 16 hours at room temperature. Solvent was then evaporated, product **6-2-1** was isolated as white powder by column chromatography on silica gel using dichloromethane-methanol (94:6) as eluent. Yield: 90 mg (23%).

**Step 6.** Compound **BCD-CDK8-6-2** was prepared in a similar fashion to compound **BCD-CDK8-5-7i** (example 37, step 2) using compound **6-2-1** instead of **5-7i-1.**

**Example 46.** Method of preparation of compound **BCD-CDK8-6-3.**

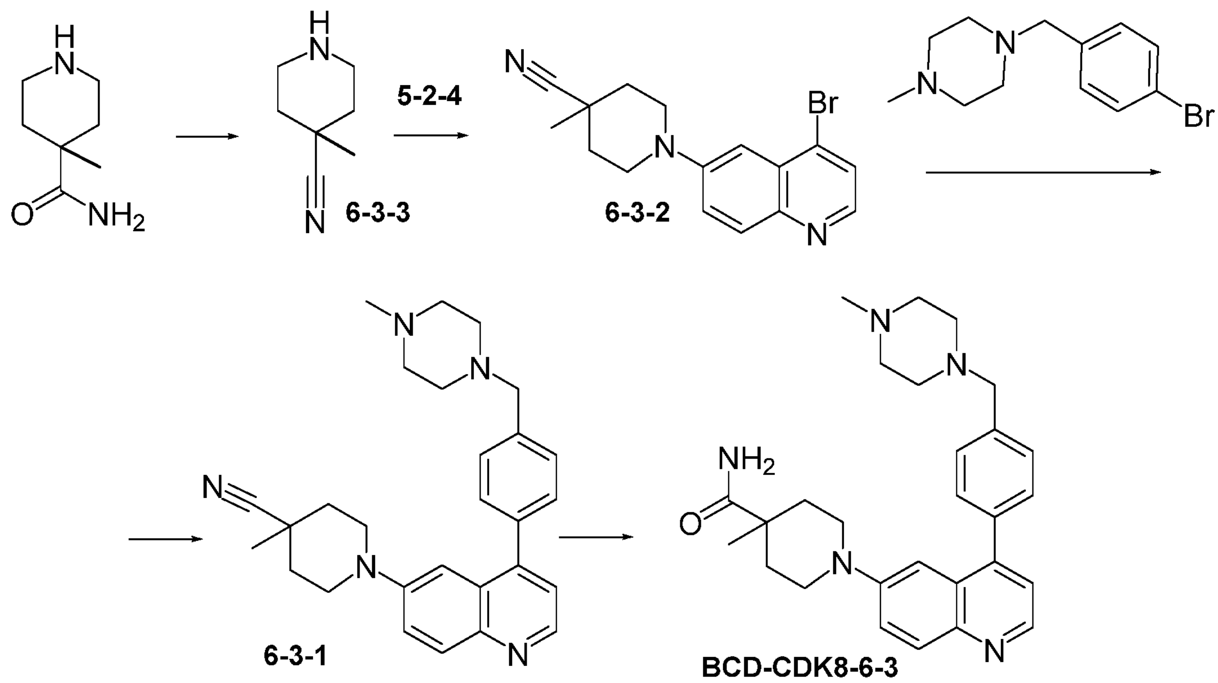


6-3-3 6-3-2 6-3-1 BCD-CDK8-6-3

**Step 1.** $SOCl_2$ (1.22 mL, 16.6 mmol) was added to 4-methylpiperidine-4-carboxamide (786 mg, 5.54 mmol) at 0-5 °C. The reaction mixture was incubated at 60 °C for 10 hours. The solvent was evaporated, 7M $NH_3$ solution in methanol was added to the residue at 0-5 °C. After concentrating, product **6-3-3** was isolated as yellow liquid by column chromatography on silica gel using dichloromethane-$NH_3$ in methanol (7M) (99:1) as eluent. Yield: 433 mg (63%).

**Step 2.** Compound **6-3-2** was prepared in a similar fashion to compound **BCD-CDK8-5-6** (example 35, step 3) using compound **5-2-4** instead of compound **5-6-1** and **6-3-3** instead of 2-pyrrolidone.

**Step 3.** Compound **6-3-1** was prepared in a similar fashion to compound **5-4-4** (example 33, step 2) using compound **6-3-2** instead of compound **5-2-4** and 1-[(4-bromophenyl)methyl]-4-methylpiperazine instead of compound **5-4-5.**

**Step 4.** Compound **BCD-CDK8-6-3** was prepared in a similar fashion to compound **BCD-CDK8-6-1** (example 44, step 3) using compound **6-3-1** instead of compound **6-1-1.**

**Example 47.** Method of preparation of compound **BCD-CDK8-6-4.**

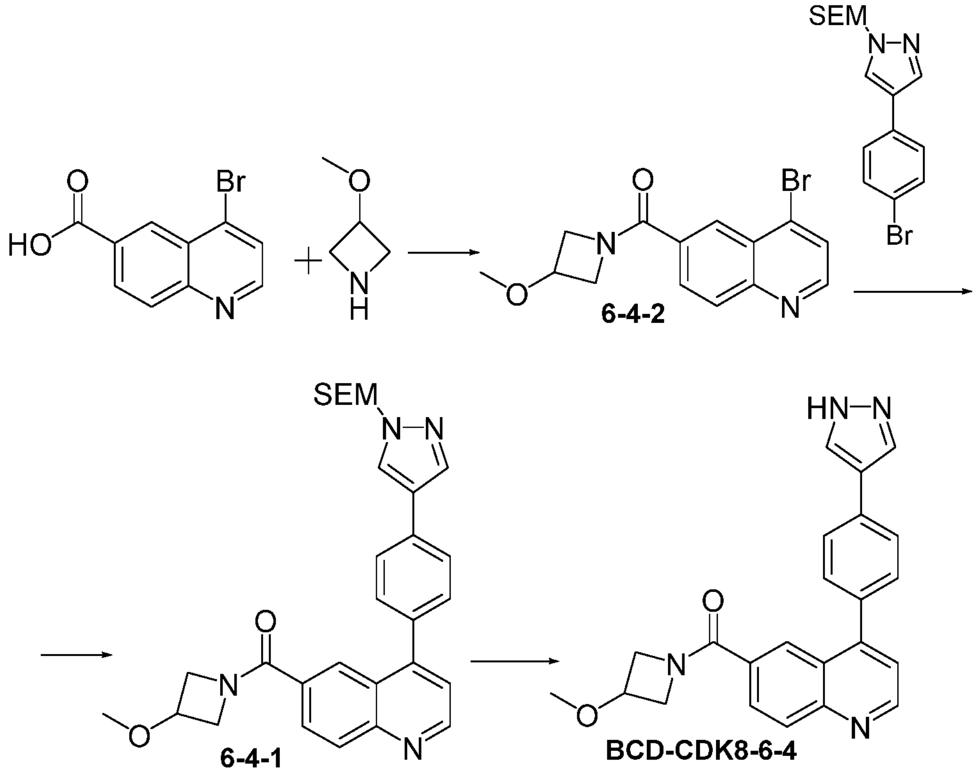

6-4-2

6-4-1

BCD-CDK8-6-4

**Step 1.** Compound **6-4-2** was prepared in a similar fashion to compound **BCD-CDK8-5-2** (example 29, step 5) using 4-bromoquinoline-6-carboxylic acid instead of compound **5-2-1** and 3-methoxyazetidine instead of (*R*)-3-hydroxypyrrolidine hydrochloride.

**Step 2.** Compound **6-4-1** was prepared in a similar fashion to compound **5-4-4** (example 33, step 2) using compound **6-4-2** instead of compound **5-2-4.**

**Step 3.** Compound **BCD-CDK8-6-4** was prepared analogously to **BCD-CDK8-5-4** (example 33, step 6) using compound **6-4-1** instead of compound **5-4-1.**

**Example 48.** Method of preparation of compound **BCD-CDK8-6-5.**

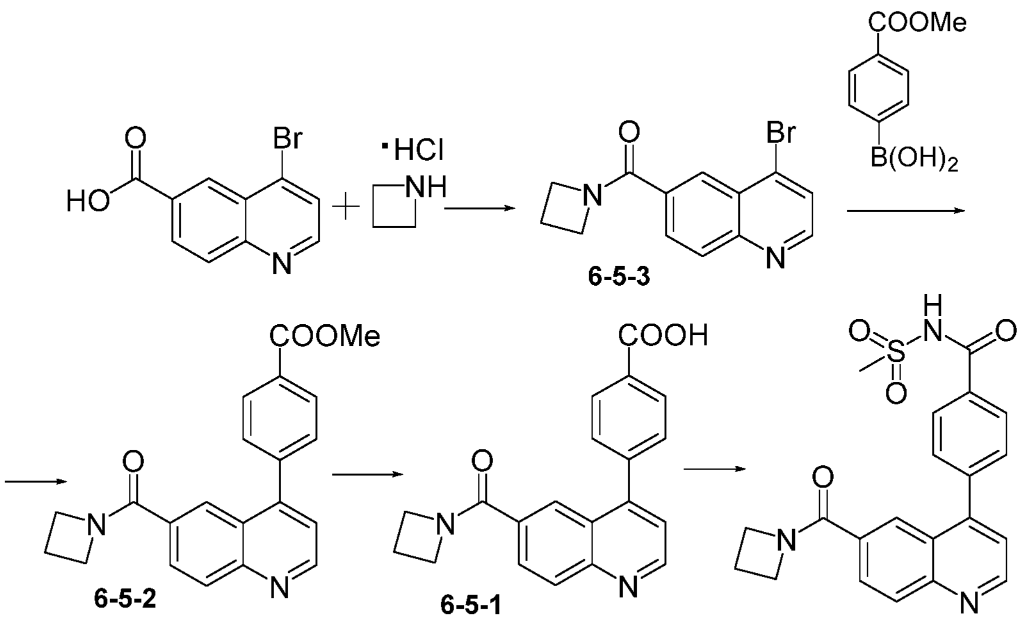

6-5-3

6-5-2

6-5-1

BCD-CDK8-6-5

**Step 1.** Compound **6-5-3** was prepared in a similar fashion to compound **BCD-CDK8-5-2** (example 29, step 5) using 4-bromoquinoline-6-carboxylic acid instead of compound **5-2-1** and azetidine hydrochloride instead of *(R)-3-*hydroxypyrrolidine hydrochloride.

**Step 2.** Compound **6-5-2** was prepared in a similar fashion to compound **5-2-3** (example 29, step 2) using compound **6-5-3** instead of compound **5-2-4** and (4-methoxycarbonylphenyl)boronic acid instead of [4-(dimethylamino)phenyl]boronic acid hydrochloride.

**Step 3.** Compound **6-5-1** was prepared in a similar fashion to compound **6-2-2** (example 45, step 4) using compound **6-5-2** instead of compound **6-2-3.**

**Step 4.** Compound **BCD-CDK8-6-5** was prepared in a similar fashion to compound **6-2-1** (example 45, step 5) using compound **6-5-1** instead of compound **6-2-2.**

**Example 49.** Method of preparation of compound **BCD-CDK8-6-6.**

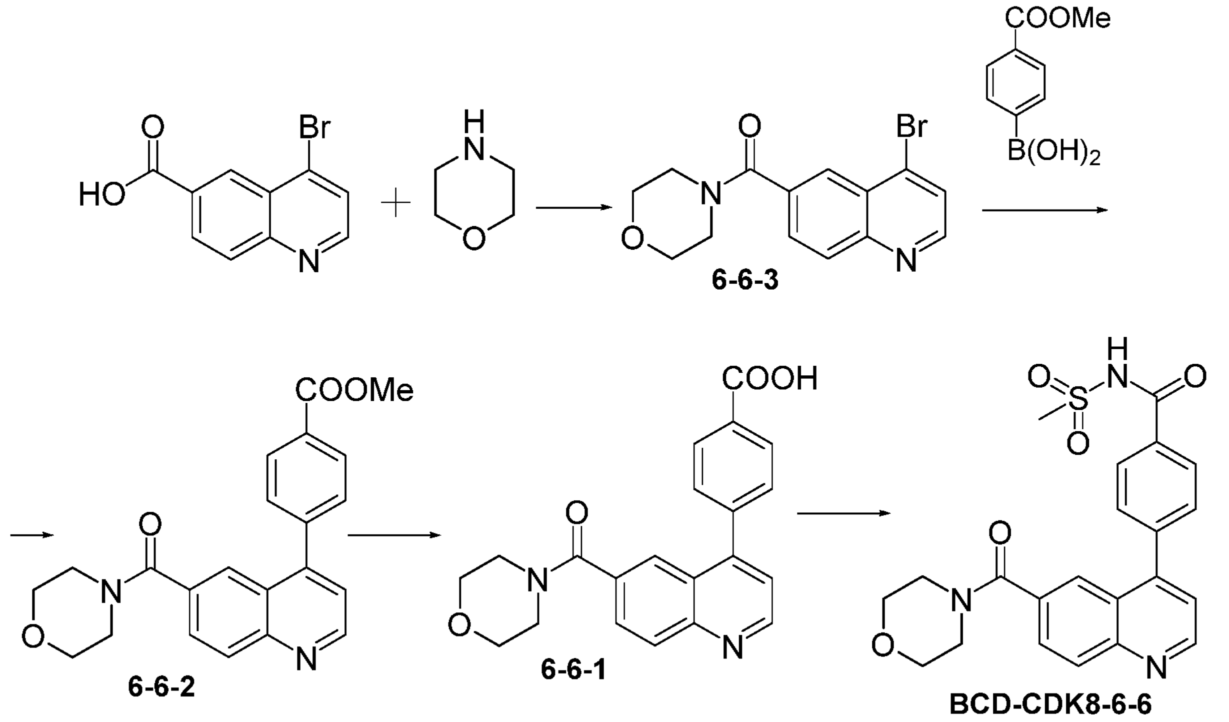

6-6-3

6-6-2

6-6-1

BCD-CDK8-6-6

**Step 1.** Compound **6-6-3** was prepared in a similar fashion to compound **BCD-CDK8-5-2** (example 29, step 5) using 4-bromoquinoline-6-carboxylic acid instead of compound **5-2-1** and morpholine instead of (*R*)-3-hydroxypyrrolidine hydrochloride.
**Step 2.** Compound **6-6-2** was prepared in a similar fashion to compound **5-2-3** (example 29, step 2) using compound **6-6-3** instead of compound **5-2-4** and (4-methoxycarbonylphenyl)boronic acid instead of [4-(dimethylamino)phenyl]boronic acid hydrochloride.
**Step 3.** Compound **6-6-1** was prepared in a similar fashion to compound **6-2-2** (example 45, step 4) using compound **6-6-2** instead of compound **6-2-3.**
**Step 4.** Compound **BCD-CDK8-6-6** was prepared in a similar fashion to compound **6-2-1** (example 45, step 5) using compound **6-6-1** instead of compound **6-2-2.**

**Example 50.** Method of preparation of compound **BCD-CDK8-6-7.**

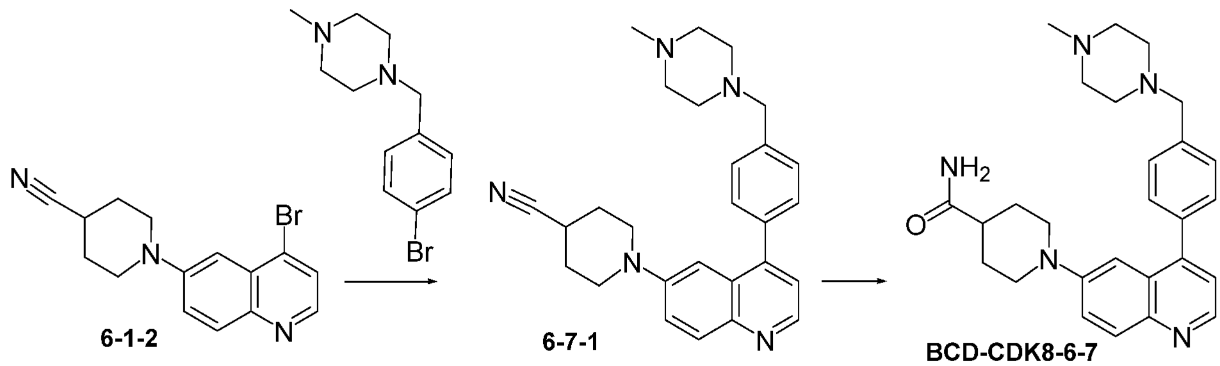

6-1-2

6-7-1

BCD-CDK8-6-7

**Step 1.** Compound **6-7-1** was prepared in a similar fashion to compound **5-4-4** (example 33, step 2) using compound **6-1-2** instead of compound **5-2-4** and 1-[(4-bromophenyl)methyl]-4-methylpiperazine instead of **5-4-5.**
**Step 2.** Compound **BCD-CDK8-6-7** was prepared in a similar fashion to compound **BCD-CDK8-6-1** (example 44, step 3) using compound **6-7-1** instead of compound **6-1-1.**

**Example 51.** Method of preparation of compound **BCD-CDK8-6-8.**

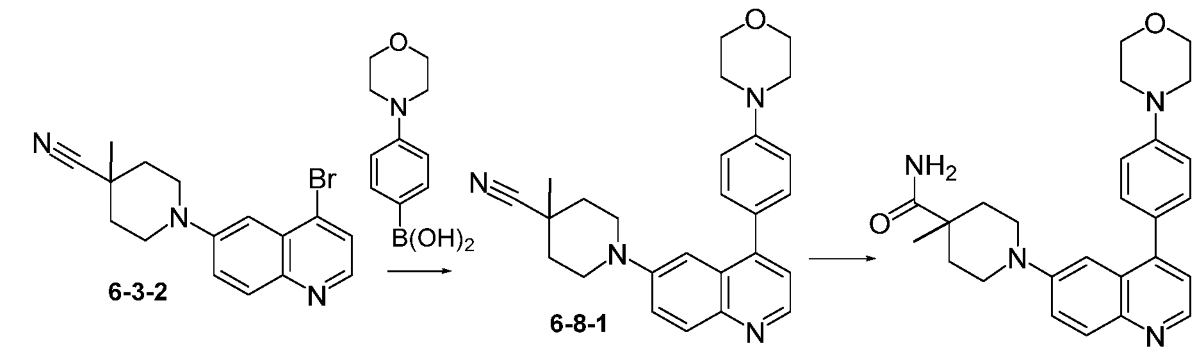

6-3-2

6-8-1

BCD-CDK8-6-8

**Step 1.** Compound **6-8-1** was prepared in a similar fashion to compound **5-2-3** (example 29, step 2) using compound **6-3-2** instead of compound **5-2-4** and 4-morpholinophenylboronic acid instead of [4-(dimethylamino)phenyl]boronic acid hydrochloride.

**Step 2.** Compound **BCD-CDK8-6-8** was prepared in a similar fashion to compound **BCD-CDK8-6-1** (example 44, step 3) using compound **6-8-1** instead of compound **6-1-1.**

**Example 52.** Method of preparation of compound **BCD-CDK8-6-9.**

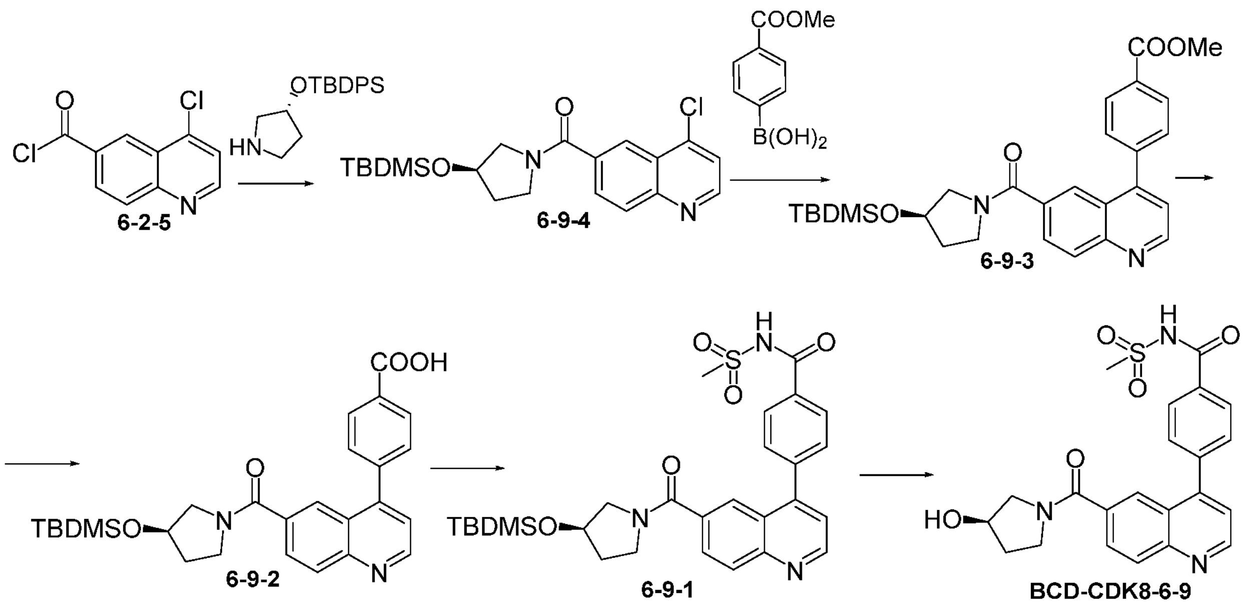

6-2-5

6-9-4

6-9-3

6-9-2

6-9-1

BCD-CDK8-6-9

**Step 1.** Compound **6-9-4** was prepared in a similar fashion to compound **6-2-4** (example 45, step 2) using (*R*)-3-(*tert*-butyldimethylsilyloxy) pyrrolidine instead of (*S*)-3-(*tert*-butyldiphenylsilyloxy) pyrrolidine.

**Step 2.** Compound **6-9-3** was prepared in a similar fashion to compound **5-2-3** (example 29, step 2) using compound **6-9-4** instead of compound **5-2-4** and (4-methoxycarbonylphenyl)boronic acid instead of [4-(dimethylamino)phenyl]boronic acid hydrochloride.

**Step 3.** Compound **6-9-2** was prepared in a similar fashion to compound **6-2-2** (example 45, step 4) using compound **6-9-3** instead of compound **6-2-3.**

**Step 4.** Compound **6-9-1** was prepared in a similar fashion to compound **6-2-1** (example 45, step 5) using compound **6-9-2** in place of compound **6-2-2.**

**Step 5:** Compound **BCD-CDK8-6-9** was prepared in a similar fashion to compound **BCD-CDK8-5-2i** (example 30,

step 2) using compound **6-9-1** instead of compound **5-2i-1.**

**Example 53.** Method of preparation of compound **BCD-CDK8-6-10.**

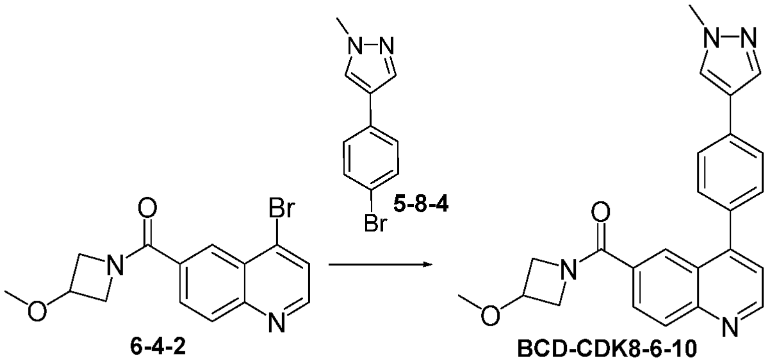

5-8-4

6-4-2

BCD-CDK8-6-10

Compound **BCD-CDK8-6-10** was prepared in a similar fashion to compound **5-4-4** (example 33, step 2) using compound **6-4-2** instead of compound **5-2-4** and **5-8-4** instead of **5-4-5.**

**Example 54.** Method of preparation of compound **BCD-CDK8-6-11.**

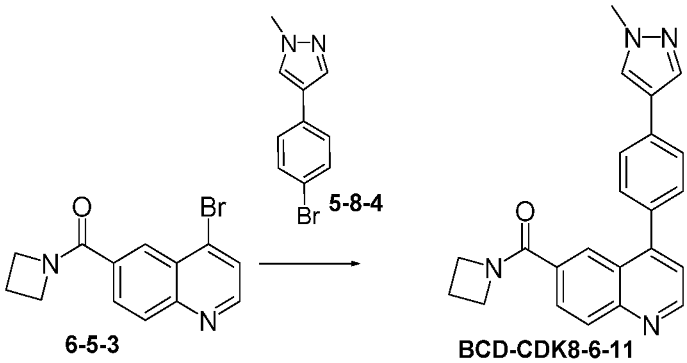

5-8-4

6-5-3

BCD-CDK8-6-11

Compound **BCD-CDK8-6-11** was prepared in a similar fashion to compound **5-4-4** (example 33, step 2) using compound **6-5-3** instead of compound **5-2-4** and **5-8-4** instead of **5-4-5.**

**Example 55.** Method of preparation of compound **BCD-CDK8-6-12.**

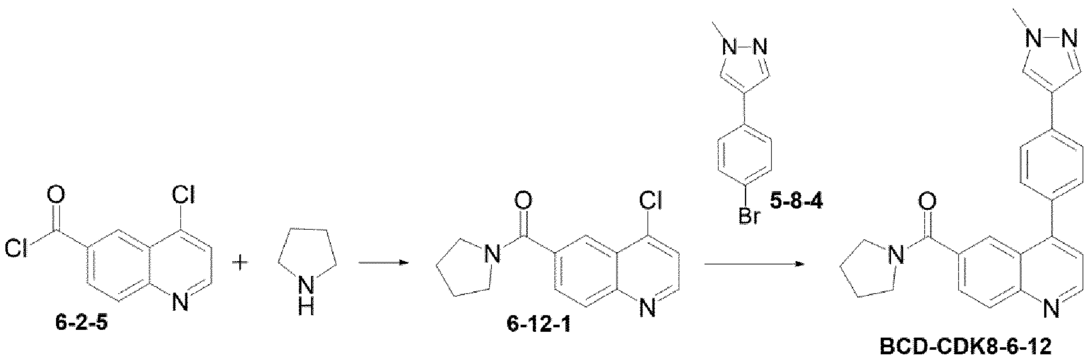

5-8-4

6-2-5

6-12-1

BCD-CDK8-6-12

**Step 1.** Compound **6-12-1** was prepared in a similar fashion to compound **6-2-4** (example 45, step 2) using pyrrolidine instead of (*S*)-3- (*tert*-butyldiphenylsilyloxy) pyrrolidine.
**Step 2.** Compound **BCD-CDK8-6-12** was prepared in a similar fashion to compound **5-4-4** (example 33, step 2) using compound **6-12-1** instead of compound **5-2-4** and **5-8-4** instead of **5-4-5.**

**Example 56.** Method of preparation of compound **BCD-CDK8-6-13.**

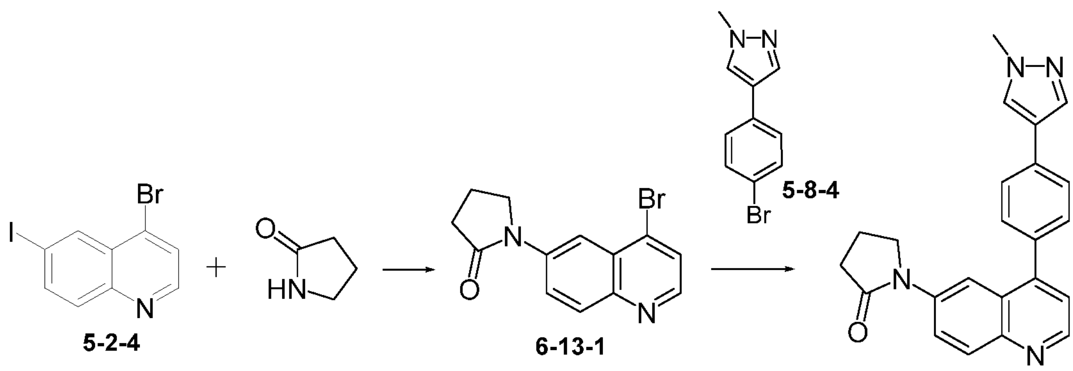

5-2-4 6-13-1 5-8-4

**BCD-CDK8-6-13**

**Step 1.** Compound **6-13-1** was prepared analogously to **BCD-CDK8-5-6** (example 35, step 3) using compound **5-2-4** instead of compound **5-6-1.**
**Step 2.** Compound **BCD-CDK8-6-13** was prepared in a similar fashion to compound **5-4-4** (example 33, step 2) using compound **6-13-1** instead of compound **5-2-4** and **5-8-4** instead of **5-4-5.**

**Example 57.** Method of preparation of compound **BCD-CDK8-6-14.**

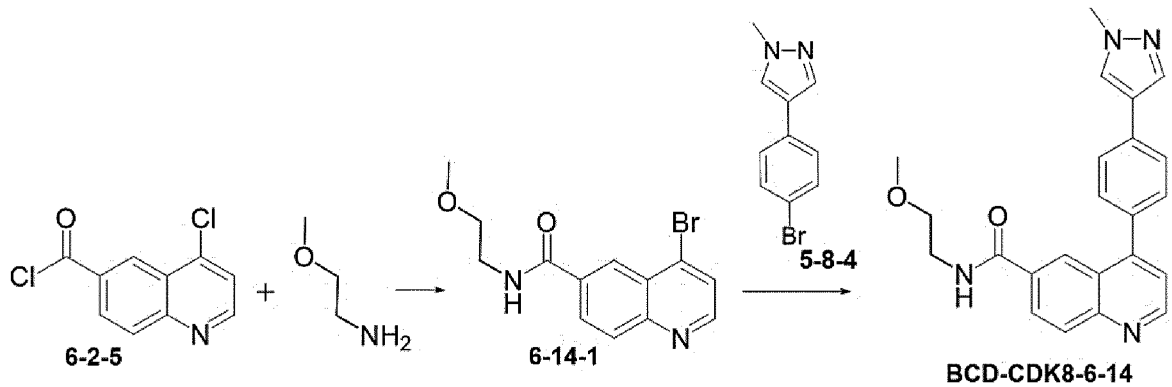

6-2-5 6-14-1 5-8-4

**BCD-CDK8-6-14**

**Step** 1. Compound 6-14-1 was prepared in a similar fashion to compound 6-**2-4** (example 45, step 2) using 2-methoxyethylamine instead of (*S*)-3-(*tert*-butyldiphenylsilyloxy)pyrrolidine.
**Step 2.** Compound **BCD-CDK8-6-14** was prepared in a similar fashion to compound **5-4-4** (example 33, step 2) using compound **6-14-1** instead of compound **5-2-4** and **5-8-4** instead of **5-4-5.**

**Example 58.** Method of preparation of compound **BCD-CDK8-3-1.**

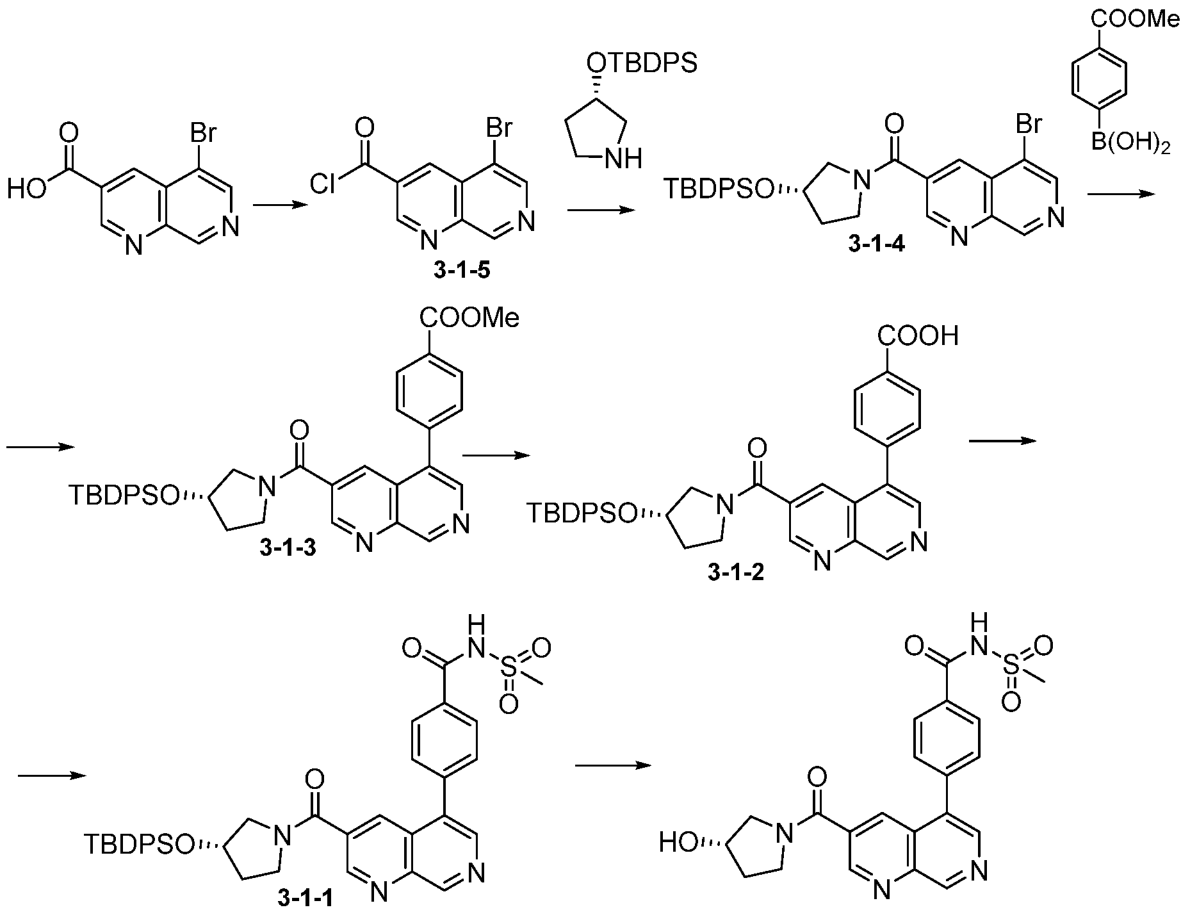

3-1-5

3-1-4

3-1-3

3-1-2

3-1-1

BCD-CDK8-3-1

**Step 1.** $SOCl_2$ (5 mL, 60 mmol) and one drop of DMF were added to 5-bromo-1,7-naphthyridine-3-carboxylic acid (1.00 g, 3.95 mmol) (prepared as described in WO 2015/014768). The reaction mixture was refluxed for 5 hours, after which volatile compounds were evaporated under reduced pressure. 5 mL of MTBE was added to the residue, it was concentrated, and the residue was dried under vacuum conditions in a rotary evaporator. Reaction product **3-1-5** isolated as yellow-green powder was used without additional purification. Yield: 1.04 g (97%).

**Step 2.** A solution of freshly prepared **3-1-5** (400 mg, 1.47 mmol), triethylamine (0.411 mL, 2.95 mmol) in 15 mL of dichloromethane was added dropwise under nitrogen at -5 °C to a solution of (*S*)-3-(*tert*-butyldiphenylsilyloxy)pyrrolidine (959mg, 2.94 mmol), triethylamine (1.234 mL, 8.84 mmol) in 20 mL of dichloromethane.The resulting mixture was stirred at room temperature for 5 hours. The reaction mass was then washed with 1M HCl solution and extracted with dichloromethane. Organic layer was separated, washed with a saturated NaCl solution, and dried with $Na_2SO_4$. Product **3-1-4** was isolated as white powder by column chromatography on silica gel using dichloromethane-methanol (98:2) as eluent. Yield: 644 mg (78%).

**Step 3.** Pd(PPh**3**)$_4$ (66 mg, 0.057 mmol) was added under nitrogen at room temperature to a solution of **3-1-4** (644 mg, 1.15 mmol), (4-methoxycarbonylphenyl)boronic acid (248 mg, 1.37 mmol), $Cs_2CO_3$ (749 mg, 2.23 mmol) in 20 mL of 1,4-dioxane-water mixture (1:1). The reaction mixture was heated at 70 °C for 5 hours. The reaction mass was then concentrated under vacuum. Product **3-1-3** was isolated as white powder by column chromatography on silica gel using dichloromethane-methanol (98:2) as eluent. Yield: 410 mg (58%).

**Step 4.** A solution of LiOH·$H_2O$ (42 mg, 1.00 mmol) in 5 mL of water was added dropwise to a solution of **3-1-3** (410 mg, 0.67 mmol) in 10 mL of methanol. The resulting mixture was stirred for 8 h. 1M HCl solution was then added to pH 4. The suspension was filtered, precipitate was washed with water (3×20 mL), dried under vacuum to constant weight, thereby obtaining product **3-1-2** as white powder. Yield: 360 mg (87%).

**Step 5:** EDC'HCl (91 mg, 0.473 mmol) was added under nitrogen atmosphere at room temperature to a solution of **3-1-2** (219 mg, 0.364 mmol), methanesulfonamide (42 mg, 0.437 mmol) and DMAP (7 mg, 0.05 mmol) in 15 mL of dichloromethane. The resulting mixture was stirred for 24 h. Product **3-1-1** was isolated as white powder by column chromatography on silica gel using dichloromethane-methanol (9:1) as eluent. Yield: 101 mg (41%).

**Step 6.** 4 mL of 4M HCl solution in diethyl ether was added dropwise to a solution of **3-1-1** (94 mg, 0.138 mmol) in 10 mL of dichloromethane. After two days, the reaction mixture was filtered, and washed with diethyl ether (2×20

mL). Product **BCD-CDK8-3-1** was isolated as white powder by column chromatography on silica gel using dichloromethane-methanol (9:1) as eluent. Yield: 59 mg (96%).

**Example 59.** Method of preparation of compound **BCD-CDK8-3-13.**

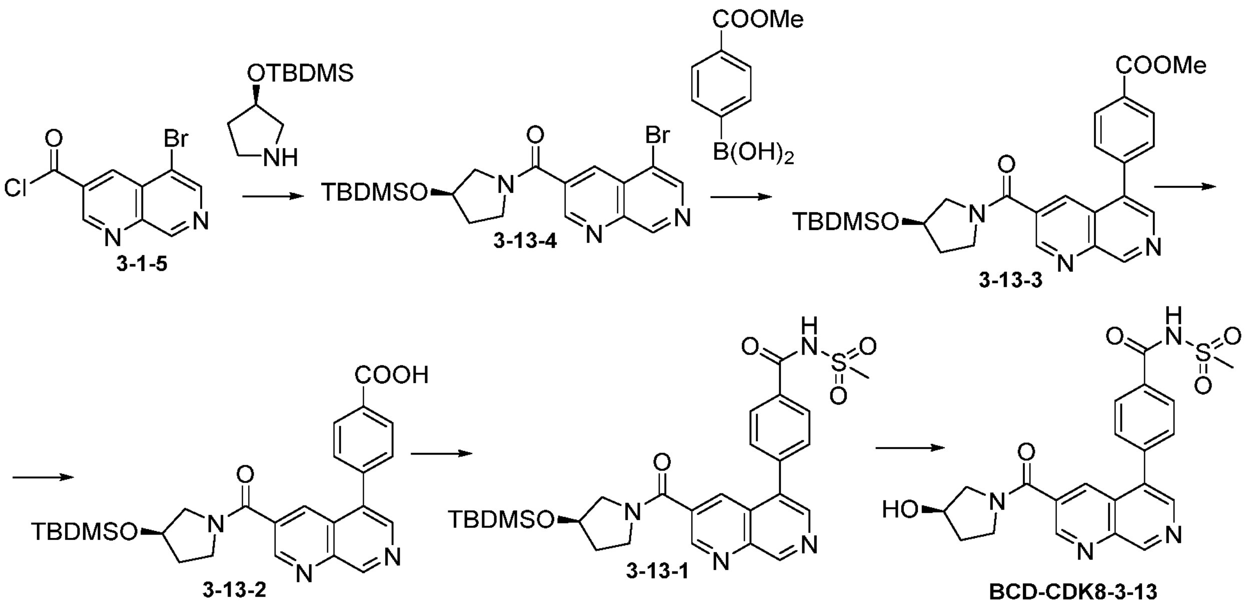

**3-1-5** **3-13-4** **3-13-3** **3-13-2** **3-13-1** **BCD-CDK8-3-13**

**Step 1.** Compound **3-13-4** was prepared in a similar fashion to compound **3-1-4** (example 58, step 2) using (*R*)-3-(*tert*-butyldimethylsilyloxy) pyrrolidine instead of (*S*)-3-(*tert*-butyldiphenylsilyloxy)pyrrolidine.
**Step 2.** Compound **3-13-3** was prepared in a similar fashion to compound **3-1-3** (example 58, step 3) using **3-13-4** instead of **3-1-4.**
**Step 3.** Compound **3-13-2** was prepared in a similar fashion to compound **3-1-2** (example 58, step 4) using **3-13-3** instead of **3-1-3.**
**Step 4.** Compound **3-13-1** was prepared in a similar fashion to compound **3-1-1** (example 58, step 5) using **3-13-2** instead of **3-1-2.**
**Step 5:** Compound **BCD-CDK8-3-13** was prepared analogously to **BCD-CDK8-3-1** (Example 58, step 6) using **3-13-1** instead of **3-1-1.**

**Example 60.** Method of preparation of compound **BCD-CDK8-3-2.**

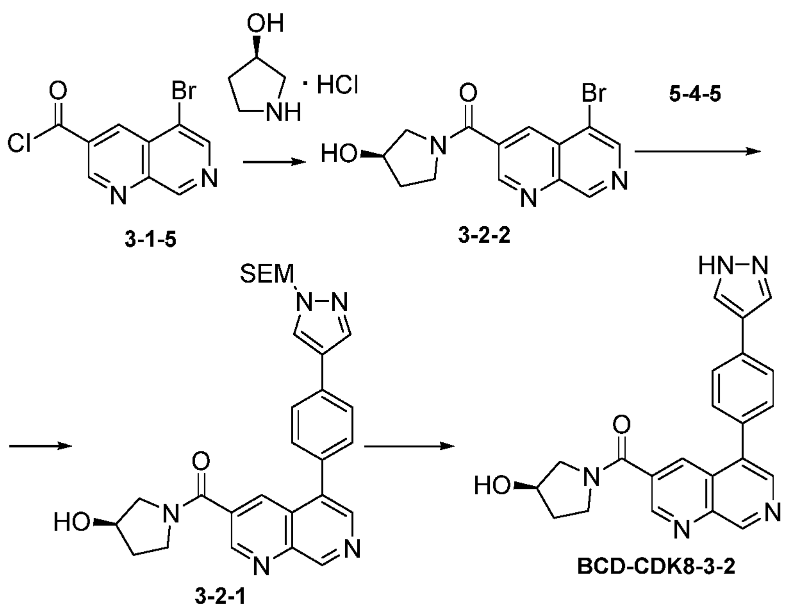


3-1-5

3-2-2

3-2-1

BCD-CDK8-3-2

**Step 1.** Compound **3-2-2** was prepared in a similar fashion to compound **3-1-4** (example 58, step 2) using (*R*)-3-hydroxypyrrolidine hydrochloride instead of (*S*)-3-(*tert*-butyldiphenylsilyloxy)pyrrolidine.

**Step 2.** Compound **3-2-1** was prepared in a similar fashion to compound **5-4-4** (example 33, step 2) using compound **3-2-2** instead of **5-2-4.**

**Step 3.** Compound **BCD-CDK8-3-2** was prepared analogously to **BCD-CDK8-5-4** (example 33, step 6) using **3-2-1** instead of **5-4-1.**

**Example 61.** Method of preparation of compound **BCD-CDK8-3-3.**

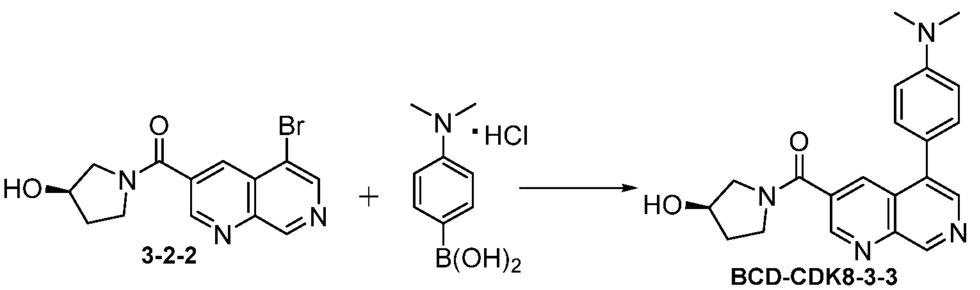

3-2-2

BCD-CDK8-3-3

Compound **BCD-CDK8-3-3** was prepared in a similar fashion to compound **3-1-3** (example 58, step 3) using [4- (dimethylamino)phenyl]boronic acid hydrochloride instead of (4-methoxycarbonylphenyl)boronic acid.

**Example 62.** Method of preparation of compound **BCD-CDK8-3-4.**

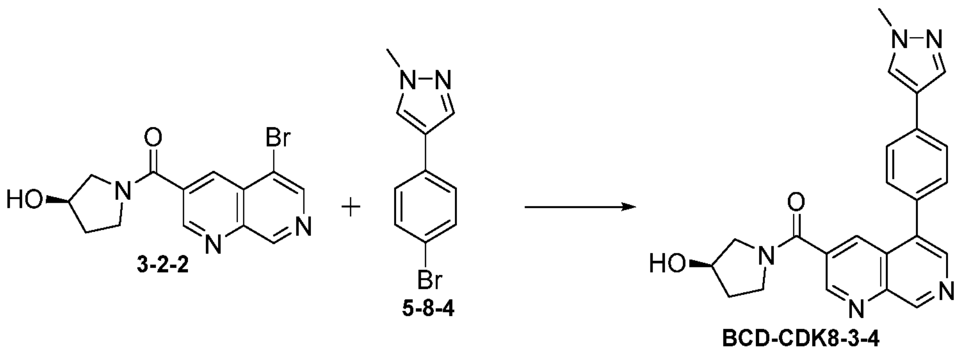

3-2-2 5-8-4 BCD-CDK8-3-4

Compound **BCD-CDK8-3-4** was prepared in a similar fashion to compound **5-4-4** (example 33, step 2) using compound **5-8-4** instead of compound **5-4-5** and compound **3-2-2** instead of compound **5- 2-4.**

**Example 63.** Method of preparation of compound **BCD-CDK8-3-5.**

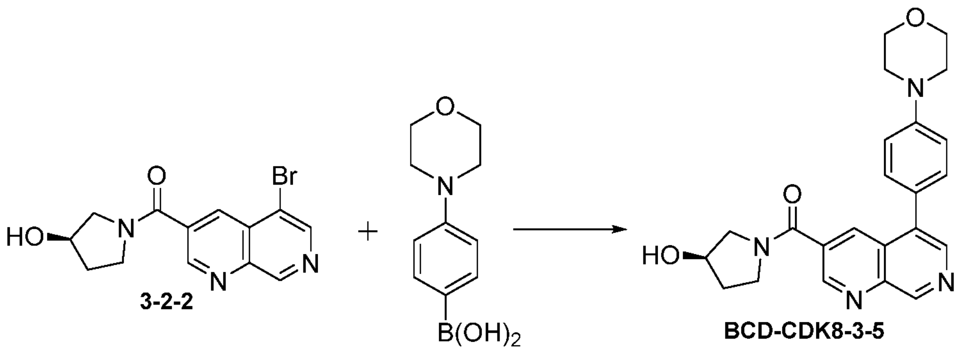

3-2-2 BCD-CDK8-3-5

Compound **BCD-CDK8-3-5** was prepared in a similar fashion to compound **3-1-3** (example 58, step 3) using 4-morpholinophenylboronic acid instead of (4-methoxycarbonylphenyl)boronic acid.

**Example 64.** Method of preparation of compound **BCD-CDK8-3-6.**

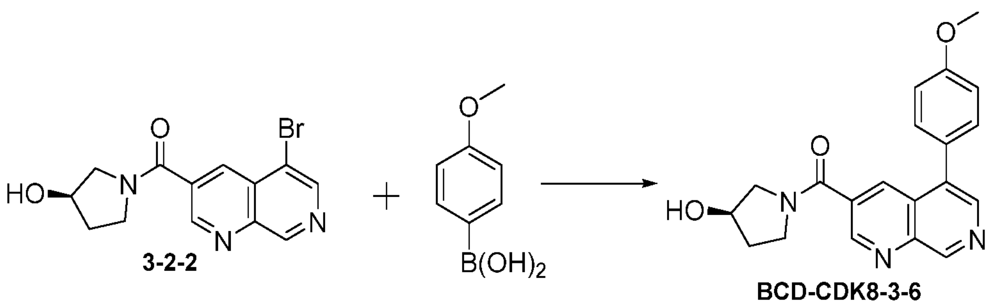

3-2-2 BCD-CDK8-3-6

Compound **BCD-CDK8-3-6** was prepared in a similar fashion to compound **3-1-3** (example 58, step 3) using (4-methoxyphenyl)boronic acid instead of (4-methoxycarbonylphenyl)boronic acid.

**Example 65.** Method of preparation of compound **BCD-CDK8-3-8.**

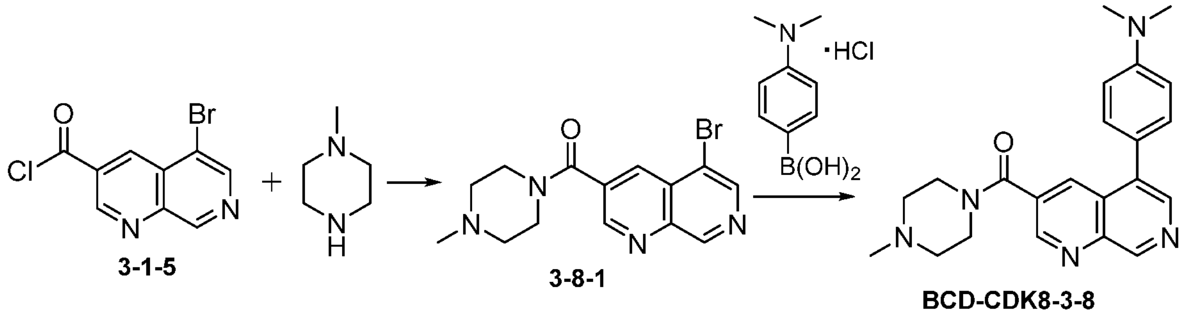

3-1-5 3-8-1 BCD-CDK8-3-8

**Step 1.** Compound **3-8-1** was prepared in a similar fashion to compound **3-1-4** (example 58, step 2) using *N*-methylpiperazine instead of (*S*)-3-(*tert*-butyldiphenylsilyloxy)pyrrolidine.
**Step 2.** Compound **BCD-CDK8-3-8** was prepared in a similar fashion to compound **3-1-3** (example 58, step 3) using compound **3-8-1** instead of compound **3-1-4** and [4-(dimethylamino)phenyl]boronic acid instead of (4-methoxycarbonylphenyl)boronic acid.

**Example 66.** Method of preparation of compound **BCD-CDK8-3-9.**

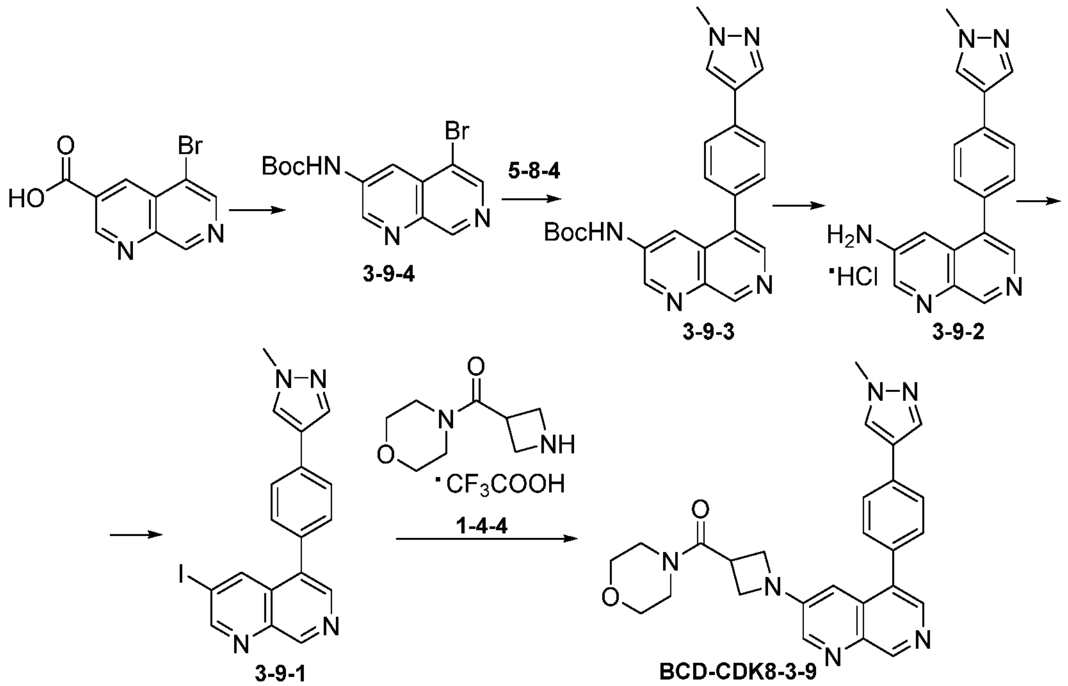


3-9-4 3-9-3 3-9-2 3-9-1 BCD-CDK8-3-9

**Step 1.**
5-bromo-1,7-naphthyridine-3-carboxylic acid (2.22 g, 8.78 mmol) (prepared as described in WO 2015/014768), DPPA (2.27 mL, 10.5 mmol), triethylamine (1.47 mL, 10.5 mmol) was suspended in anhydrous tert-butanol (50 mL). The mixture was refluxed for 8 hours. The reaction mixture was concentrated under vacuum, residue was dissolved in 50 mL of ethyl acetate, washed with water (2×40 mL), saturated $N_3HCO_3$ solution (50 mL) and saturated NaCl solution (50 mL). The organic layer was dried with $Na_2SO_4$. Product **3-9-4** as light yellow powder was isolated by column chromatography on silica gel using dichloromethane-methanol (96:4) as eluent. Yield: 1.64 g (58%).
**Step 2.** Compound **3-9-3** was prepared in a similar fashion to compound **5-4-4** (example 33, step 2) using compound **3-9-4** instead of compound **5-2-4** and compound **5-8-4** instead of **5-4-5.**
**Step 3.** 5 mL of 4M HCl solution in diethyl ether was added under stirring to a solution of compound **3-9-3** (205 mg, 0.51 mmol) in 10 mL of dichloromethane. The reaction mixture was stirred for 1 hour, filtered, washed with diethyl ether, dried under vacuum to constant weight. **3-9-2** was obtained. Yield: 169 mg (98%).
**Step 4.** A solution of $NaNO_2$ (52 mg, 0.75 mmol) in 0.5 mL of $H_2O$ was added at -5 ° C to a solution of compound

**3-9-2** (169 mg, 0.50 mmol) in 1 mL of 6M HCl solution. After 1 h, a solution of KI (415 mg, 2.50 mmol) in 0.5 mL of $H_2O$ was added. After 1 h, the reaction mixture was heated to 80 °C and stirred for 3 hours. The reaction mixture was poured into a saturated $Na_2SO_3$ solution, extracted with ethyl acetate (3×20 mL). The combined organic layers were washed with NaCl solution, dried on $Na_2SO_4$. Product **3-9-1** was isolated as yellow powder by column chromatography on silica gel using dichloromethane-methanol (98:2) as eluent. Yield: 132 mg (64%).

**Step 5:** Compound **BCD-CDK8-3-9** was prepared in a similar fashion to compound **1-1-1** (example 1, step 3) using compound **3-9-1** instead of compound **1-1-3** and compound **1-4-4** instead of compound **1-1-2.**

**Example 67.** Method of preparation of compound **BCD-CDK8-3-11.**

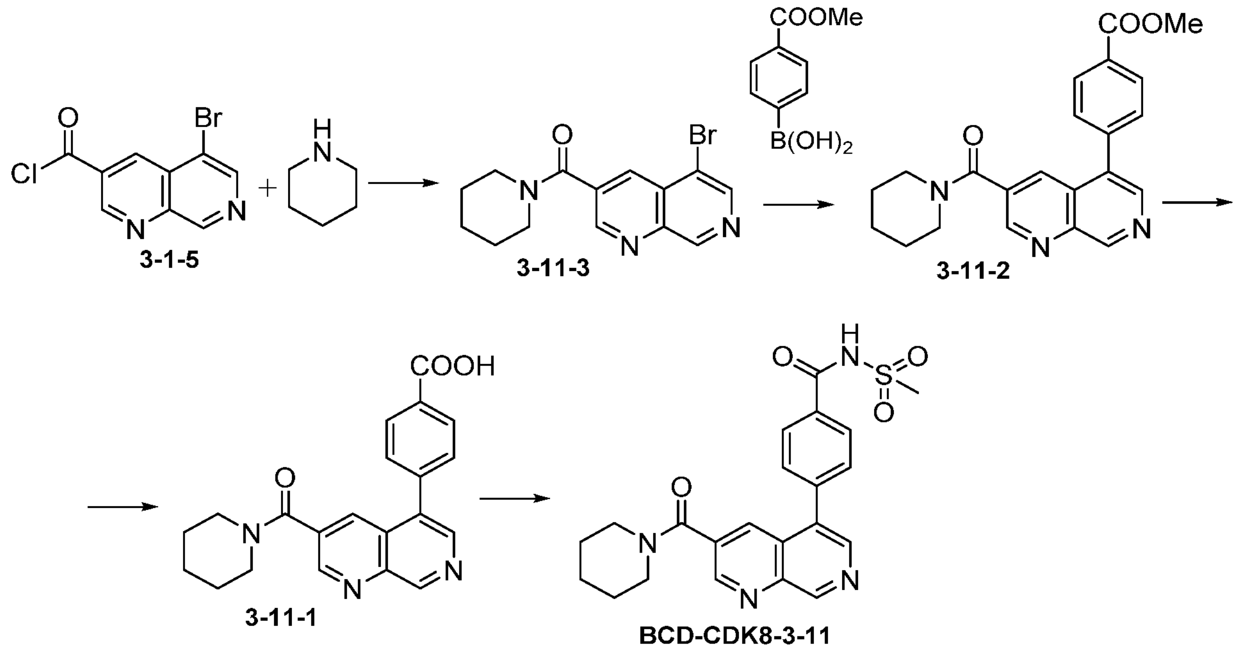

3-1-5 3-11-3 3-11-2

3-11-1 BCD-CDK8-3-11

**Step 1.** Compound **3-11-3** was prepared in a similar fashion to compound **3-1-4** (example 58, step 2) using piperidine instead of (*S*)-3-(*tert*-butyldiphenylsilyloxy)pyrrolidine hydrochloride.

**Step 2.** Compound **3-11-2** was prepared in a similar fashion to compound **3-1-3** (example 58, step 3) using compound **3-11-3** instead of **3-1-4.**

**Step 3.** Compound **3-11-1** was prepared in a similar fashion to compound **3-1-2** (example 58, step 4) using compound **3-11-2** instead of **3-1-3.**

**Step 4.** Compound **BCD-CDK8-3-11** was prepared in a similar fashion to compound **3-1-1** (example 58, step 5) using compound **3-11-1** instead of **3-1-2.**

**Example 68.** Method of preparation of compound **BCD-CDK8-3-12.**

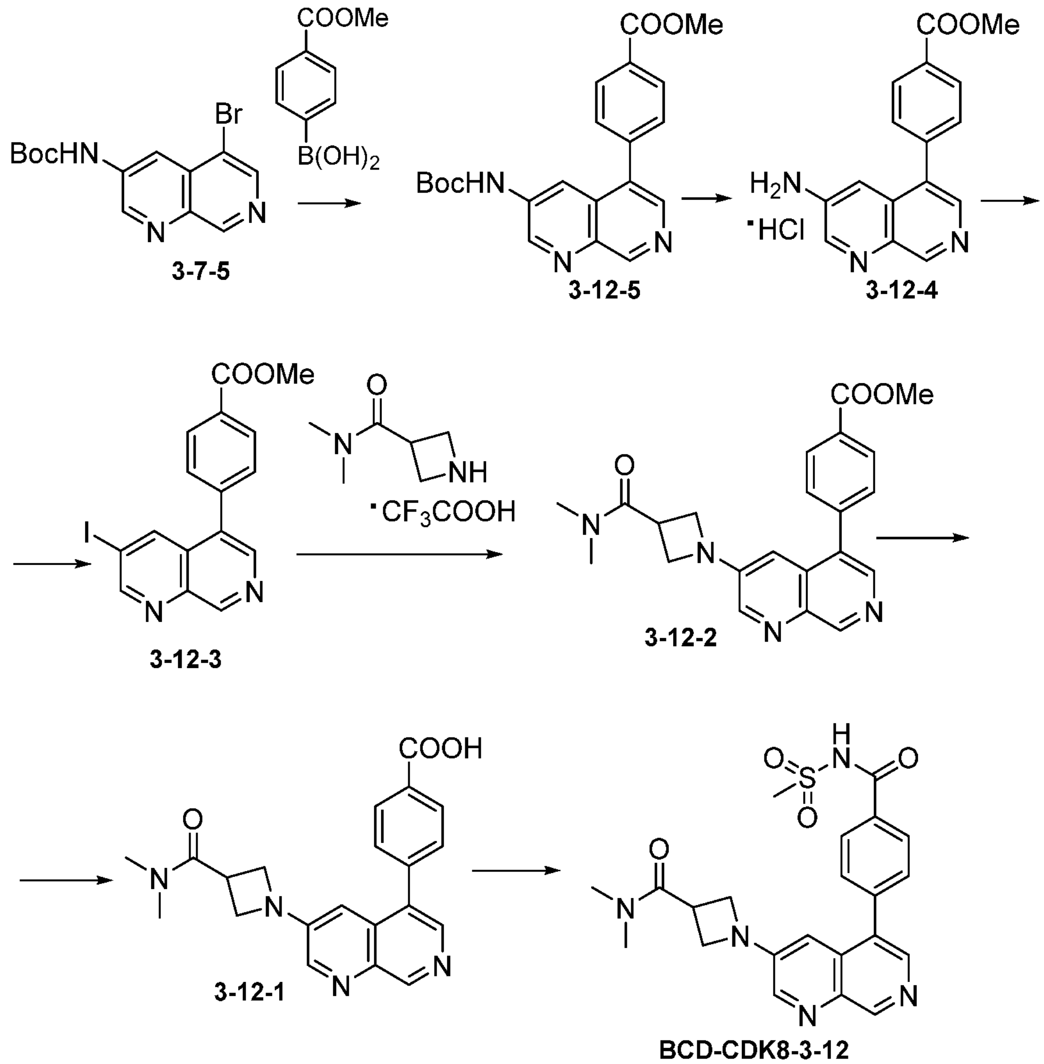

3-7-5

3-12-5

3-12-4

3-12-3

3-12-2

3-12-1

BCD-CDK8-3-12

**Step 1.** Compound **3-12-5** was prepared in a similar fashion to compound **3-1-3** (example 58, step 3) using compound **3-7-5** instead of compound **3-1-4.**

**Step 2.** Compound **3-12-4** was prepared in a similar fashion to compound **3-9-2** (example 66, step 3) using compound **3-12-5** instead of **3-9-3.**

**Step 3.** Compound **3-12-3** was prepared in a similar fashion to compound **3-9-1** (example 66, step 4) using compound **3-12-4** instead of compound **3-9-2.**

**Step 4.** Compound **3-12-2** was prepared in a similar fashion to compound **1-1-1** (example 1, step 3) using compound **3-12-3** instead of **1-1-3** and *N*,*N*-dimethylazetidine-3-carboxamide trifluoroacetic acid (prepared as described in Journal of Medicinal Chemistry, 53 (9), 3645-3674, 2010) instead of compound **1-1-2.**

**Step 5:** Compound **3-12-1** was prepared in a similar fashion to compound **3-1-2** (example 58, step 4) using compound **3-12-2** instead of compound **3-1-3.**

**Step 6.** Compound **BCD-CDK8-3-12** was prepared in a similar fashion to compound **3-1-1** (example 58, step 5) using compound **3-12-1** instead of compound **3-1-2.**

**Example 69.** Method of preparation of compound **BCD-CDK8-3-14.**

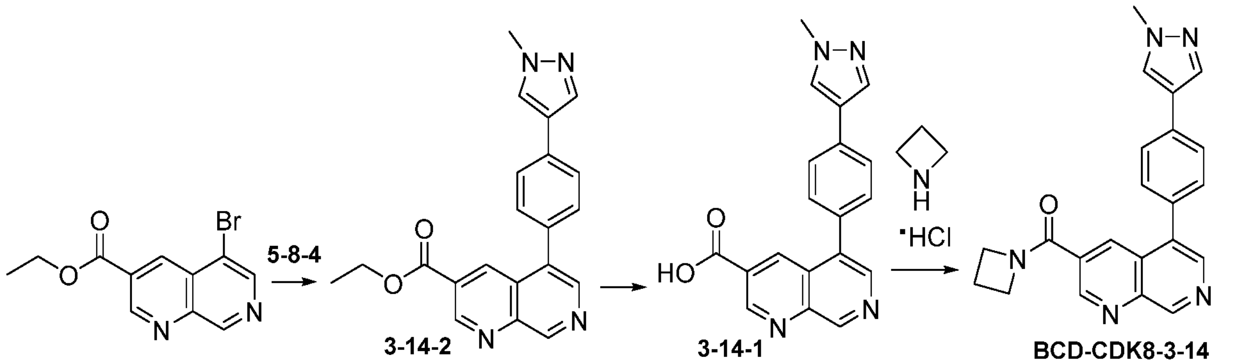


3-14-2 3-14-1 BCD-CDK8-3-14

**Step 1.** Compound **3-14-2** was prepared in a similar fashion to compound **5-4-4** (example 33, step 2) using ethyl 5-bromo-1,7-naphthyridine-3-carboxylate (prepared as described in WO 2015/014768) instead of **5-2-4** and compound **5-8-4** instead of **5-4-5.**

**Step 2.** Compound **3-14-1** was prepared in a similar fashion to compound **3-1-2** (example 58, step 4) using compound **3-14-2** instead of **3-1-3.**

**Step 3.** Compound **BCD-CDK8-3-14** was prepared in a similar fashion to compound **1-2-4** (example 5, step 1) using compound **3-14-1** instead of 3-fluoro-4-nitrobenzoic acid.

**Example 70.** Method of preparation of compound **BCD-CDK8-3-15.**

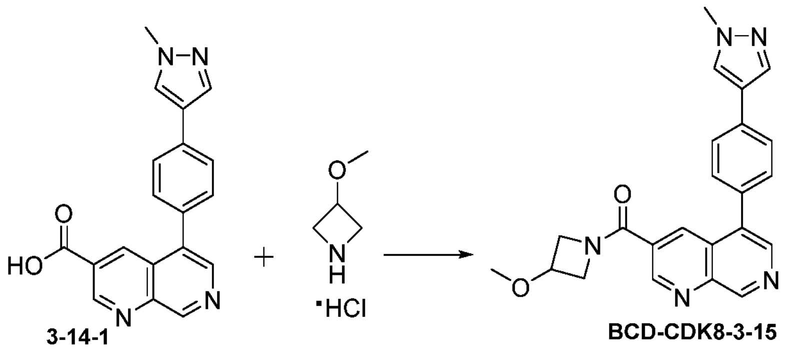


3-14-1 BCD-CDK8-3-15

Compound **BCD-CDK8-3-15** was prepared in a similar fashion to compound **1-2-4** (example 5, step 1) using 3-methoxyazetidine hydrochloride instead of azetidine hydrochloride and compound **3-14-1** instead of 3-fluoro-4-nitrobenzoic acid.

**Example 71.** Method of preparation of compound **BCD-CDK8-3-16.**

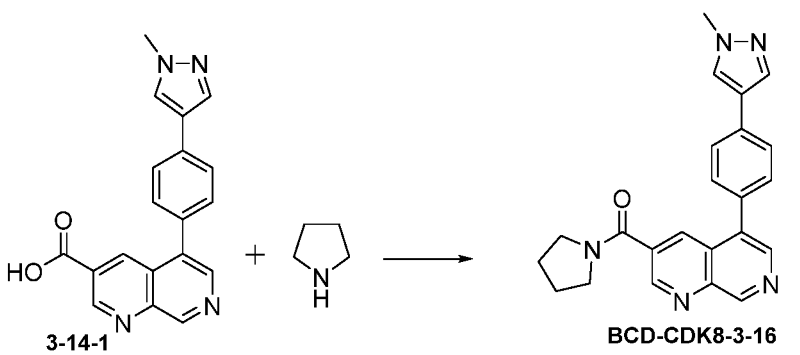

3-14-1 BCD-CDK8-3-16

Compound **BCD-CDK8-3-16** was prepared in a similar fashion to compound **1-2-4** (example 5, step 1) using pyrrolidine instead of azetidine hydrochloride and compound **3-14-1** instead of 3-fluoro-4-nitrobenzoic acid.

**Example 72.** Method of preparation of compound **BCD-CDK8-3-17.**

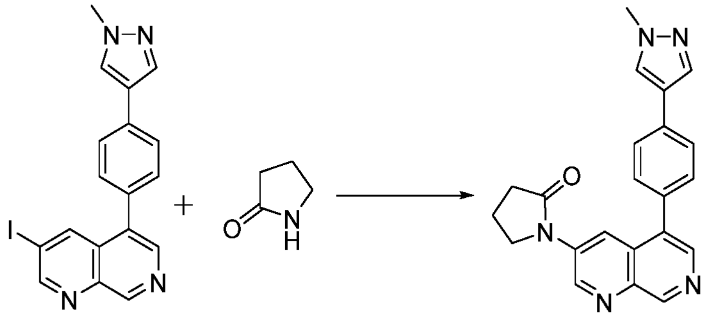

**3-9-1** **BCD-CDK8-3-17**

Compound **BCD-CDK8-3-17** was prepared analogously to compound **1-1-1** (example 1, step 3) using **3-9-1** instead of **1-1-3** and 2-pyrrolidone instead of **1-1-2.**

**Example 73.** Method of preparation of compound **BCD-CDK8-3-18.**

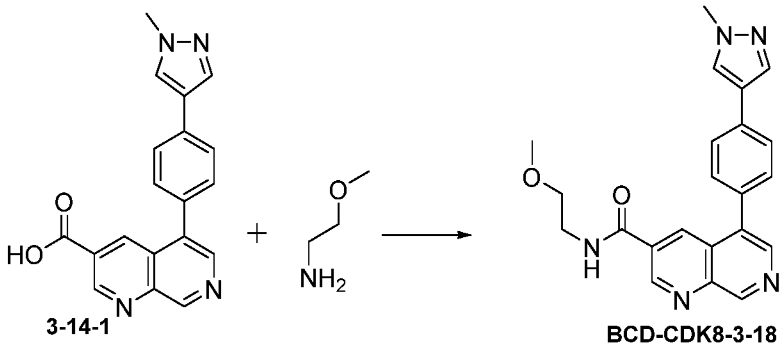

**3-14-1** **BCD-CDK8-3-18**

Compound **BCD-CDK8-3-18** was prepared in a similar fashion to compound **1-2-4** (example 5, step 1) using 2-methoxyethylamine instead of azetidine hydrochloride and compound **3-14-1**instead of 3-fluoro-4-nitrobenzoic acid.

**Example 74.** Method of preparation of compound **BCD-CDK8-4-24.**

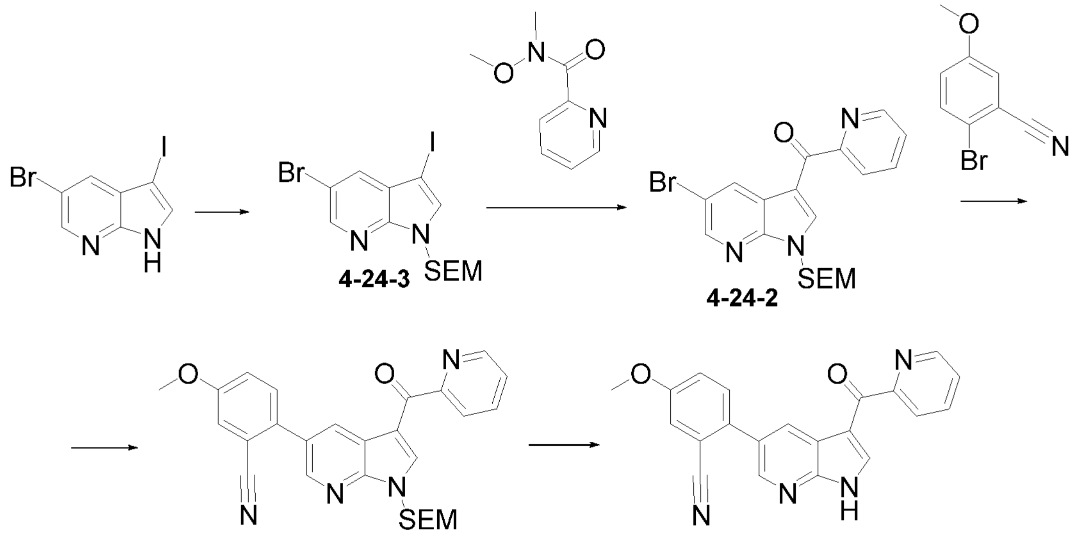

4-24-3

4-24-2

4-24-1

BCD-CDK8-4-24

**Step 1.** NaH (1.51 g, 63 mmol) was added to a solution of 5-bromo-3-iodo-1*H*-pyrrolo[2,3]pyridine (6.78 g, 21 mmol) in 70 mL of THF while cooling in an ice bath. The suspension was stirred for 15 minutes, SEMCl (4.1 mL, 23.1 mmol) was then added. The reaction mass was stirred overnight. 250 mL of water was then added, extraction was performed with ethyl acetate. Organic layer was washed with saturated NaCl solution and dried with $MgSO_4$. Product **4-24-3** was isolated as viscous light-yellow liquid by column chromatography on silica gel using ethyl acetate-hexane (2:8) as eluent. Yield: 9.01 g (95%).

**Step 2.** A solution of compound **4-24-3** (3.00 g, 6.50 mmol) in dry THF was deaerated with argon and cooled to -77°C. 2.5 M butyl lithium solution in hexane (3.1 mL, 7.54 mol) was then added and mixed for 15 minutes at the same temperature. *N*-methoxy-*N*-methylpyridine-2-carboxamide (990 mg, 5.90 mmol) (prepared as described in Bioorganic and Medicinal Chemistry Letters 19(16), 4639-4642; 2009) was added to the resulting solution. After 20 minutes, the reaction mass was diluted with 200 mL of saturated $NH_4Cl$ solution, product was extracted with ethyl acetate. Organic layer was dried with $MgSO_4$. Product **4-24-2** was isolated as light-yellow powder by column chromatography on silica gel using ethyl acetate-hexane (1:9) as eluent. Yield: 1.90 g (64%).

**Step 3.** Bis(pinacolato)diborane (449 mg, 1.77 mmol), potassium acetate (139 mg, 1.41 mmol), X-Phos (112 mg, 0.236 mmol) and $Pd(OAc)_2$ (26 mg, 0.118 mmol) was added to a solution of 2-bromo-5-methoxybenzonitrile (250 mg, 1.18 mmol) dissolved in 1.5 ml of 1,4-dioxane. The reaction mixture was stirred under inert atmosphere at 85 °C for 2.5 hours. Compound **4-24-2** (510 mg, 1.18 mmol), $Cs_2CO_3$ (1.15 g, 3.54 mmol), Pd $(PPh_3)_4$ (115 mg, 0.117 mol) and 0.5 mL of water was added to the resulting suspension. The reaction mass was stirred under inert atmosphere for 6 hours at 90°C. The mixture was diluted with water, extraction was performed with ethyl acetate. Organic layer was dried with $MgSO_4$. Product **4-24-1** was isolated as light-yellow powder by column chromatography on silica gel using acetone-hexane (2:8) as eluent. Yield: 414 mg (91%).

**Step 4.** Trifluoroacetic acid was added to a suspension of compound **4-24-1** (414 mg, 0.85 mmol) in 1.5 mL of dichloromethane while cooling in ice bath. The reaction mixture was incubated for 8 hours at room temperature. The mass was then diluted with saturated $NaHCO_3$ solution and extracted with ethyl acetate. Organic layer was dried with $MgSO_4$. Reaction product **BCD-CDK8-4-24** was isolated as white powder by column chromatography using acetone-hexane (5:5) as eluent. Yield: 157 mg (52%).

**Example 75.** Method of preparation of compound **BCD-CDK8-4-4.**

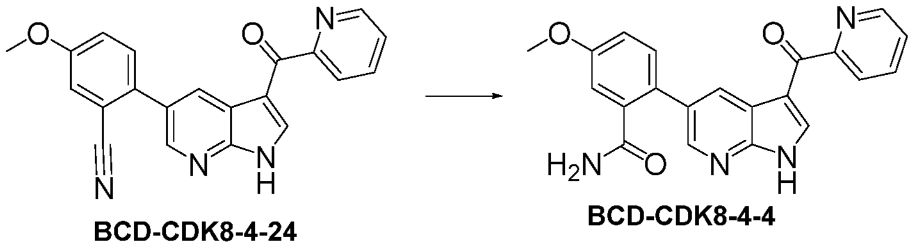

**BCD-CDK8-4-24** **BCD-CDK8-4-4**

$K_2CO_3$ (38 mg, 0.275 mmol) and 0.25 mL of 30% $H_2O_2$ were added to a solution of compound **BCD-CDK8-4-24** (90 mg, 0.25 mol) in 1 mL of DMSO. The reaction mixture was stirred at 50 °C for 5 hours. The mixture was diluted with water, extraction was performed with ethyl acetate. Organic layer was dried with $MgSO_4$. Reaction product was isolated as white powder by column chromatography on silica gel using acetone-hexane (5:5) as eluent. Yield: 35 mg (37%).

**Example 76.** Method of preparation of compound **BCD-CDK8-4-28.**

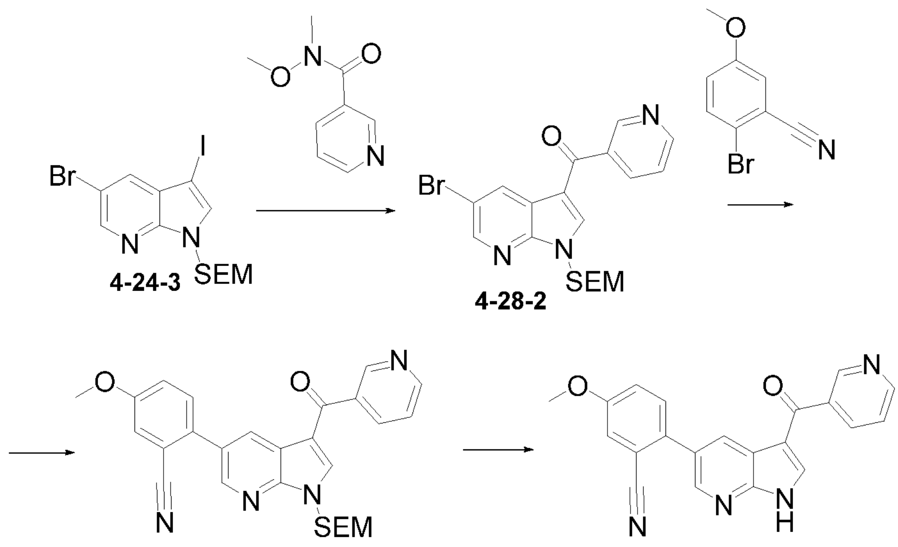

4-24-3 4-28-2

4-28-1 **BCD-CDK8-4-28**

**Step 1.** Compound **4-28-2** was prepared in a similar fashion to compound **4-24-2** (example 74, step 2) using *N*-methoxy-*N*-methylpyridine-3-carboxamide (prepared as described in Bioorganic & Medicinal Chemistry Letters, 19(16), 4639-4642, 2009) instead of *N*-methoxy-*N*-methylpyridine-2-carboxamide.

**Step 2.** Compound **4-28-1** was prepared in a similar fashion to compound **4-24-1** (example 74, step 3) using compound **4-28-2** instead of compound **4-24-2.**

**Step 3.** Compound **BCD-CDK8-4-28** was prepared in a similar fashion to compound **BCD-CDK8-4-24** (example 74, step 4) using compound **4-28-1** instead of compound **4-24-1.**

**Example 77.** Method of preparation of compound **BCD-CDK8-4-8.**

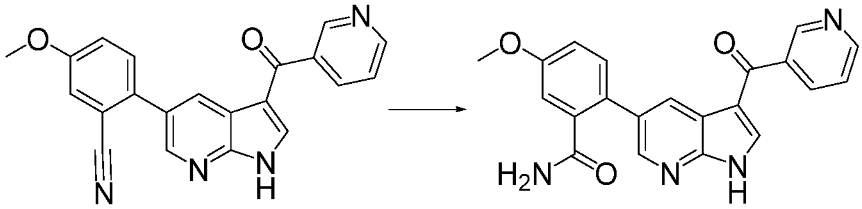

**BCD-CDK8-4-28** **BCD-CDK8-4-8**

Compound BCD-CDK8-4-8 was prepared in a similar fashion to compound BCD-CDK8-4-4 (example 75) using compound BCD-CDK8-4-28instead of compound BCD-CDK8-4-24.

**Example 78.** Method of preparation of compound **BCD-CDK8-4-32.**

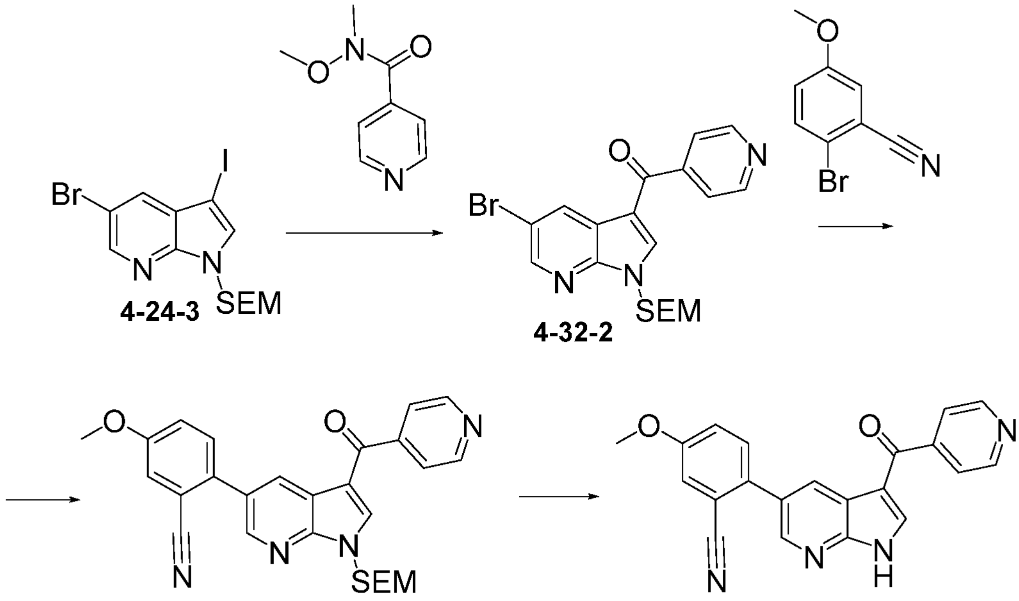

4-24-3 4-32-2 4-32-1 BCD-CDK8-4-32

**Step 1.** Compound **4-32-2** was prepared in a similar fashion to compound **4-24-2** (example 74, step 2) using *N*-methoxy-*N*-methylpyridine-4-carboxamide (prepared as described in Bioorganic & Medicinal Chemistry Letters, 24(3), 790-793, 2014) instead of *N*-methoxy-*N*-methylpyridine-2-carboxamide.

**Step 2.** Compound **4-32-1** was prepared in a similar fashion to compound **4-24-1** (example 74, step 3) using compound **4-32-2** instead of compound **4-24-2.**

**Step 3.** Compound **BCD-CDK8-4-32** was prepared in a similar fashion to compound **BCD-CDK8-4-24** (example 74, step 4) using compound **4-32-1** instead of compound **4-24-1.**

**Example 79.** Method of preparation of compound **BCD-CDK8-4-12.**

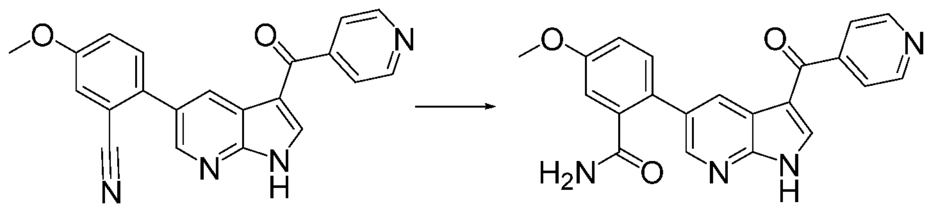

**BCD-CDK8-4-32** **BCD-CDK8-4-12**

Compound **BCD-CDK8-4-12** was prepared in a similar fashion to compound **BCD-CDK8-4-4** (example 75) using compound **BCD-CDK8-4-32** instead of compound **BCD-CDK8-4-24.**

**Example 80.** Method of preparation of compound **BCD-CDK8-4-21.**

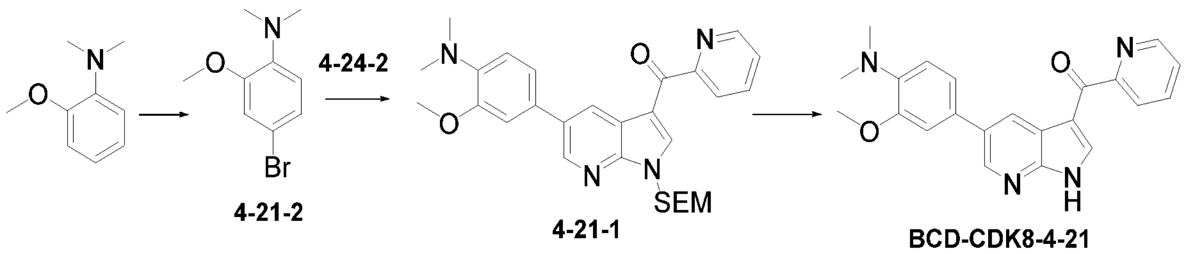


4-21-2 4-21-1 BCD-CDK8-4-21

**Step 1.** *N*-bromosuccinimide (3.71 g, 20.8 mmol) was added while cooling in ice bath to a solution of 2-methoxy-*N*,*N*-dimethylaniline (3.00 g, 19.8 mmol) (prepared as described in Angewandte Chemie International Edition, 55(23),6776-6779, 2016) in 50 mL of acetonitrile. After 30 minutes, the mixture was diluted with 250 mL of water, product was extracted with ethyl acetate, organic layer was washed with water, NaCl solution and dried with $MgSO_4$. Product was used without additional purification. Yield: 3.5 g (77%).

**Step 2.** Compound **4-21-1** was prepared in a similar fashion to compound **4-24-1** (example 74, step 3) using compound **4-21-2** instead of 2-bromo-5-methoxybenzonitrile.

**Step 3.** Compound **BCD-CDK8-4-21** was prepared in a similar fashion to compound **BCD-CDK8-4-24** (example 74, step 4) using compound **4-21-1** instead of compound **4-24-1.**

**Example 81.** Method of preparation of compound **BCD-CDK8-4-1.**

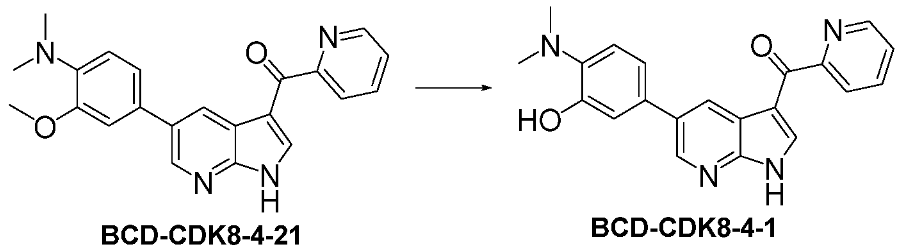

BCD-CDK8-4-21 BCD-CDK8-4-1

$AlCl_3$ (296 mg 2.22 mmol) was added to a solution of compound **BCD-CDK8-4-21** (92 mg, 0.222 mmol) in 20 mL of dichloroethane. The reaction mixture was stirred at 60 °C for 8 hours. Solvent was evaporated by vacuum distillation. Saturated $NaHCO_3$ solution was added to residue, extraction was performed with ethyl acetate, organic layer was dried with $MgSO_4$. Reaction product was isolated as white powder by column chromatography on silica gel using dichloromethane-methanol (95:5) as eluent. Yield: 46 mg (52%).

**Example 82.** Method of preparation of compound **BCD-CDK8-4-25.**

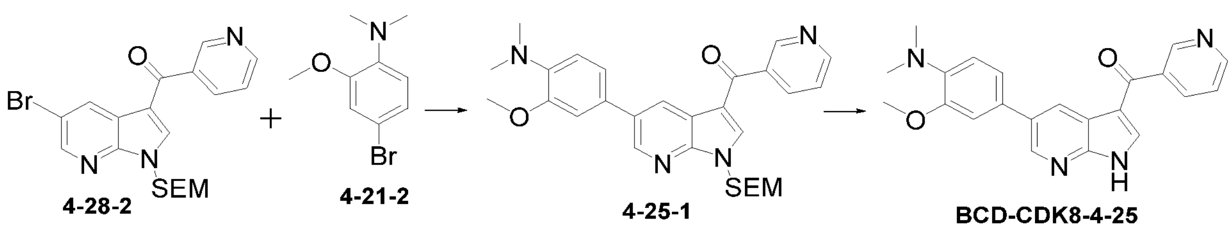

4-28-2 + 4-21-2 4-25-1 BCD-CDK8-4-25

**Step 1.** Compound **4-25-1** was prepared in a similar fashion to compound **4-24-1** (example 74, step 3) using **4-28-2** instead of **4-24-2** and **4-21-2** instead of 2-bromo-5-methoxybenzonitrile.

**Step 2.** Compound **BCD-CDK8-4-25** was prepared in a similar fashion to compound **BCD-CDK8-4-24** (example 74, step 4) using compound **4-25-1** instead of compound **4-24-1.**

**Example 83.** Method of preparation of compound **BCD-CDK8-4-5.**

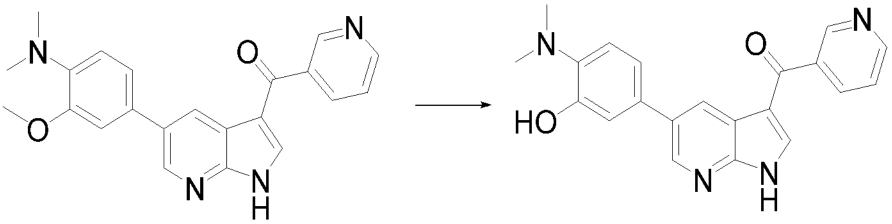

**BCD-CDK8-4-25** **BCD-CDK8-4-5**

Compound **BCD-CDK8-4-5** was prepared analogously to **BCD-CDK8-4-1** (example 81) using compound **BCD-CDK8-4-25** instead of compound **BCD-CDK8-4-21.**

**Example 84.** Method of preparation of compound **BCD-CDK8-4-29.**

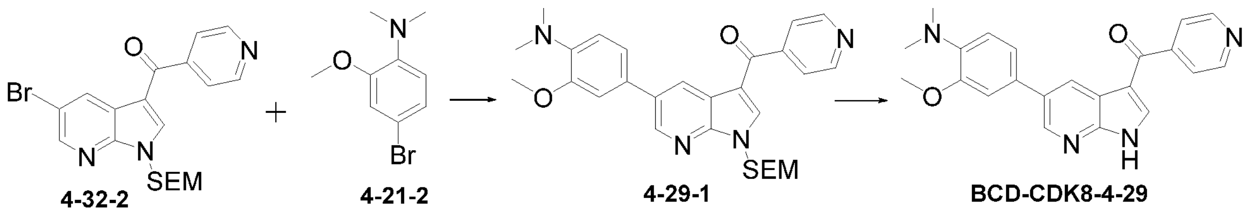

**4-32-2** **4-21-2** **4-29-1** **BCD-CDK8-4-29**

**Step 1.** Compound **4-29-1** was prepared in a similar fashion to compound **4-24-1**(example 74, step 3) using **4-32-2** instead of **4-24-2** and **4-21-2** instead of 2-bromo-5-methoxybenzonitrile.

**Step 2.** Compound **BCD-CDK8-4-29** was prepared in a similar fashion to compound **BCD-CDK8-4-24**(example 74, step 4) using compound **4-29-1** instead of compound **4-24-1.**

**Example 85.** Method of preparation of compound **BCD-CDK8-4-9.**

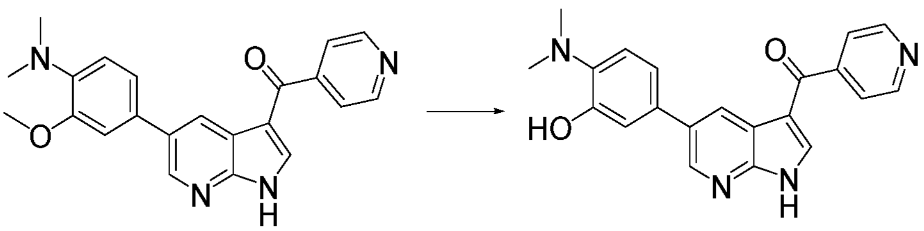

**BCD-CDK8-4-29** **BCD-CDK8-4-9**

Compound **BCD-CDK8-4-9** was prepared analogously to **BCD-CDK8-4-1** (example 81) using compound **BCD-CDK8-4-29** instead of compound **BCD-CDK8-4-21.**

**Example 86.** Method of preparation of compound **BCD-CDK8-4-22.**

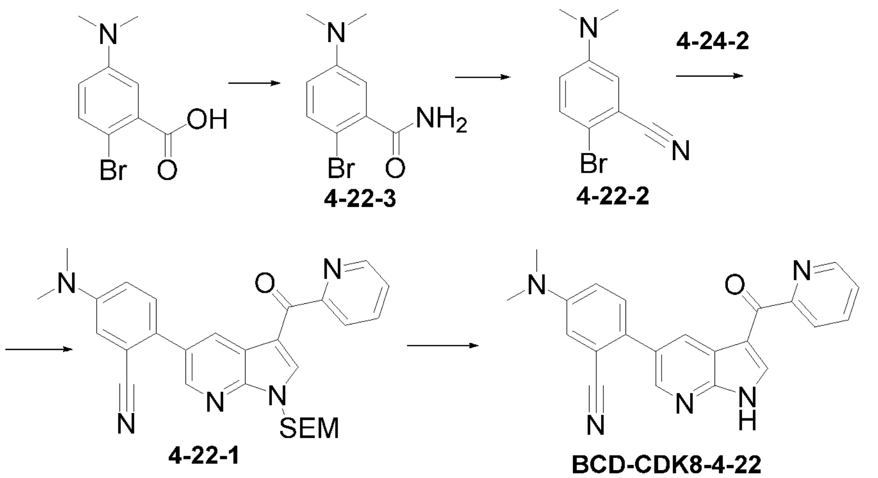


4-22-3 4-22-2

4-22-1 BCD-CDK8-4-22

**Step 1.** $NH_4Cl$ (4.92 g, 91.8 mmol), EDC' HCl (7.34 g, 38.3 mmol), HOBt (5.18 g, 38.3 mmol) and triethylamine (6.4 mL, 45.9 mmol) were added to a solution of 2-bromo-5-(dimethylamino)benzoic acid (7.48 g, 30.6 mmol) (prepared as described in WO 2008/079277) in 50 mL of DMF. The reaction mixture was stirred for 12 hours, then it was diluted with 150 mL of water, extraction was perfromed with ethyl acetate, organic layer was dried with $MgSO_4$. Solvent was evaporated under reduced pressure. Reaction product isolated as white powder was used without additional purification. Yield: 6.85 g (92%).

**Step 2.** $POCl_3$ (1.71 mL, 18 mmol) was added to a suspension of compound **4-22-3** (2.97 g, 12 mmol) in 35 mL of chloroform. The reaction mixture was stirred under boiling. After 2.5 hours, solvent was evaporated under reduced pressure, residue was poured into ice water, $NaHCO_3$ was added. Reaction product was extracted with ethyl acetate, organic layer was dried with $MgSO_4$. Reaction product was isolated as white powder by column chromatography on silica gel using ethyl acetate-hexane (1:9) as eluent. Yield: 1.732 g (63%).

**Step 3.** Compound **4-22-1** was prepared in a similar fashion to compound **4-24-1** (example 74, step 3) using compound **4-22-2** instead of 2-bromo-5-methoxybenzonitrile.

**Step 4.** Compound **BCD-CDK8-4-22** was prepared in a similar fashion to compound **BCD-CDK8-4-24** (example 74, step 4) using compound **4-22-1**instead of compound **4-24-1.**

**Example 87.** Method of preparation of compound **BCD-CDK8-4-2.**

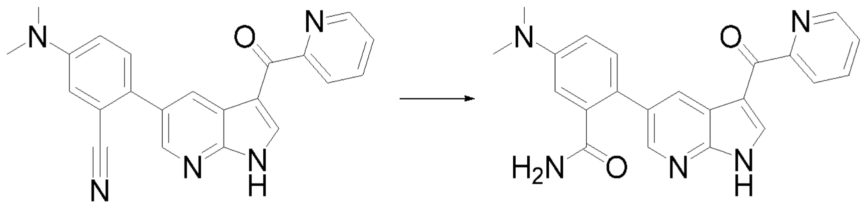

BCD-CDK8-4-22 BCD-CDK8-4-2

Compound **BCD-CDK8-4-2** was prepared in a similar fashion to compound **BCD-CDK8-4-4** (example 75) using compound **BCD-CDK8-4-22** instead of compound **BCD-CDK8-4-24.**

**Example 88.** Method of preparation of compound **BCD-CDK8-4-26.**

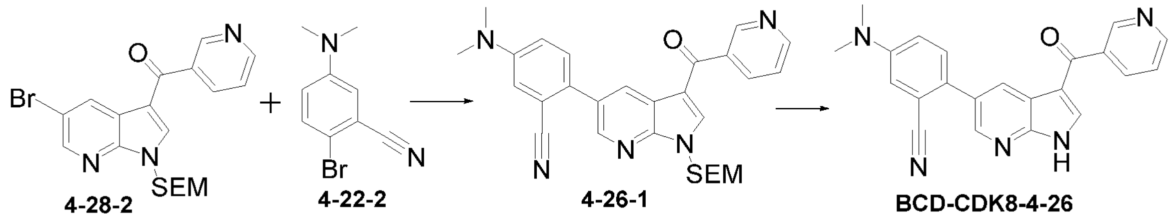

4-28-2 4-22-2 4-26-1 BCD-CDK8-4-26

**Step 1.** Compound **4-26-1** was prepared in a similar fashion to compound **4-24-1** (example 74, step 3) using compound **4-22-2** instead of 2-bromo-5-methoxybenzonitrile and compound **4-28-2** instead of compound **4-24-2.**
**Step 2.** Compound **BCD-CDK8-4-26** was prepared in a similar fashion to compound **BCD-CDK8-4-24**(example 74, step 4) using compound **4-26-1** instead of compound **4-24-1.**

**Example 89.** Method of preparation of compound **BCD-CDK8-4-6.**

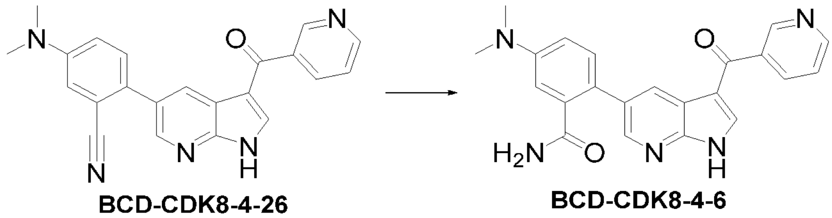

BCD-CDK8-4-26 BCD-CDK8-4-6

Compound **BCD-CDK8-4-6** was prepared in a similar fashion to compound **BCD-CDK8-4-4** (example 75) using compound **BCD-CDK8-4-26** instead of compound **BCD-CDK8-4-24.**

**Example 90.** Method of preparation of compound **BCD-CDK8-4-30.**

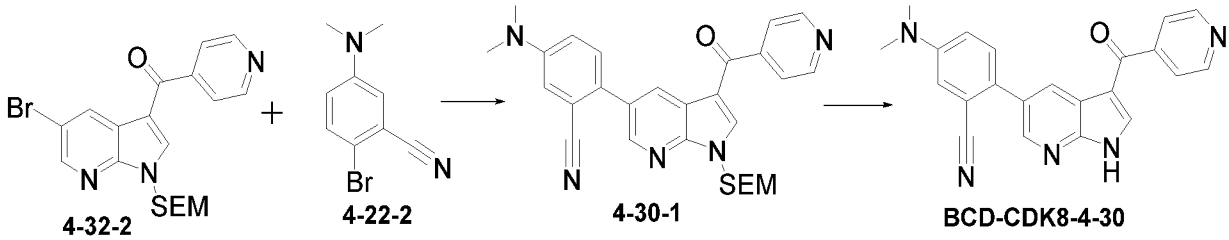

4-32-2 4-22-2 4-30-1 BCD-CDK8-4-30

**Step** 1. Compound 4-30-1 was prepared in a similar fashion to compound 4-24-1 (example 74, step 3) using compound 4-22-2 instead of 2-bromo-5-methoxybenzonitrile and compound 4-32-2 instead of compound 4-24-2.
**Step 2.** Compound **BCD-CDK8-4-30** was prepared in a similar fashion to compound **BCD-CDK8-4-24** (example 74, step 4) using compound **4-30-1**instead of compound **4-24-1.**

**Example 91.** Method of preparation of compound **BCD-CDK8-4-10.**

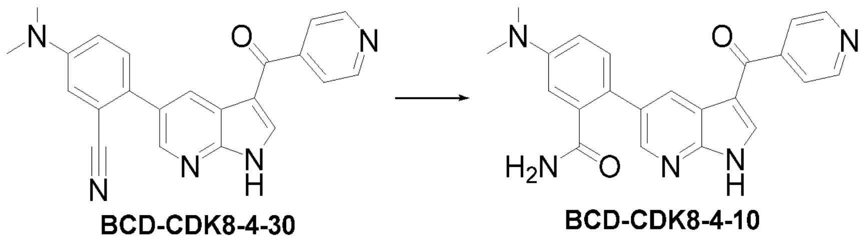

BCD-CDK8-4-30 BCD-CDK8-4-10

Compound **BCD-CDK8-4-10** was prepared in a similar fashion to compound **BCD-CDK8-4-4** (example 75) using compound **BCD-CDK8-4-30** instead of compound **BCD-CDK8-4-24.**

**Example 92.** Method of preparation of compound **BCD-CDK8-4-23.**

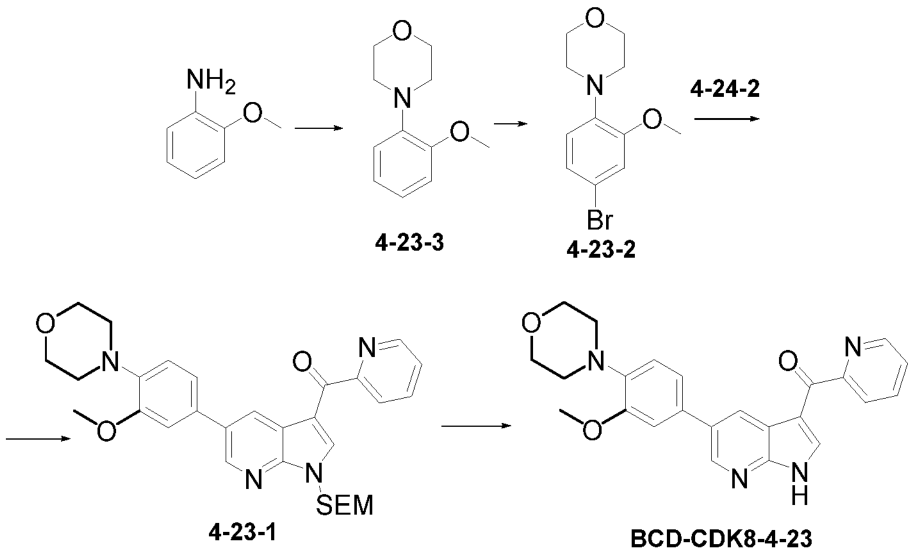


**4-23-3** **4-23-2**

**4-23-1** **BCD-CDK8-4-23**

**Step 1.** 2,2'-Dichlorodiethyl ether (3.00 g, 21 mmol), $K_2CO_3$ (4.30 g, 40 mol) and KI (3.10 g, 20 mmol) were added to a solution of *o*-methoxyaniline (2.50 g, 20mmol) in 20 mL of DMF. The mixture was stirred at 90 °C for 5 hours. 100 mL of water was then added, extraction was performed with ethyl acetate. Organic layer was isolated, washed with water and dried with $Na_2SO_4$. Product was isolated as gray powder by column chromatography on silica gel using hexane-ethyl acetate (95:5) as eluent. Yield: 3.35 g (87%).

**Step 2.** *N*-bromosuccinimide (3.15 g, 18 mmol) was added to a solution of compound **4-23-3** (3.35 g, 17 mmol) in 40 mL of acetonitrile at -10°C. After 1 h, solvent was evaporated under vacuum, residue was dissolved in dichloromethane, washed with water, dried with $Na_2SO_4$. Product was isolated as dark-gray powder by column chromatography on silica gel using hexane-ethyl acetate (95:5) as eluent. Yield: 4.28 g (92%).

**Step 3.** Compound **4-23-1** was prepared in a similar fashion to compound **4-24-1** (example 74, step 3) using compound **4-23-2** instead of 2-bromo-5-methoxybenzonitrile.

**Step 4.** Compound **BCD-CDK8-4-23** was prepared in a similar fashion to compound **BCD-CDK8-4-24** (example 74, step 4) using compound **4-23-1**instead of compound **4-24-1.**

**Example 93.** Method of preparation of compound **BCD-CDK8-4-3.**

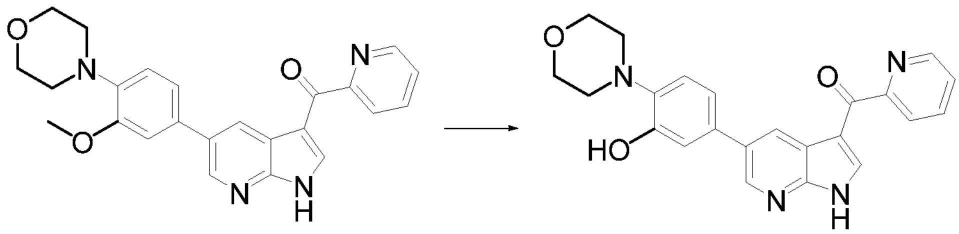

**BCD-CDK8-4-23** **BCD-CDK8-4-3**

Compound **BCD-CDK8-4-3** was prepared analogously to **BCD-CDK8-4-1** compound (example 81), using compound **BCD-CDK8-4-23** instead of compound **BCD-CDK8-4-21.**

**Example 94.** Method of preparation of compound **BCD-CDK8-4-27.**

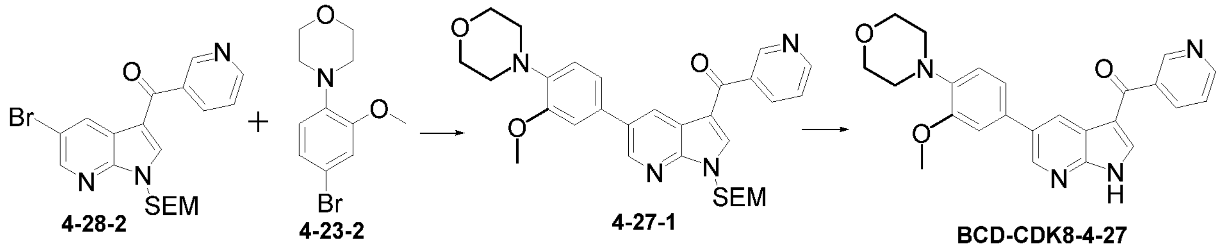

4-28-2 4-23-2 4-27-1 BCD-CDK8-4-27

**Step 1.** Compound **4-27-1** was prepared in a similar fashion to compound **4-24-1** (example 74, step 3) using compound **4-23-2** instead of 2-bromo-5-methoxybenzonitrile and compound **4-28-2** instead of compound **4-24-2.**
**Step 2.** Compound **BCD-CDK8-4-27** was prepared in a similar fashion to compound **BCD-CDK8-4-24** (example 74, step 4) using compound **4-27-1**instead of compound **4-24-1.**

**Example 95.** Method of preparation of compound **BCD-CDK8-4-7.**

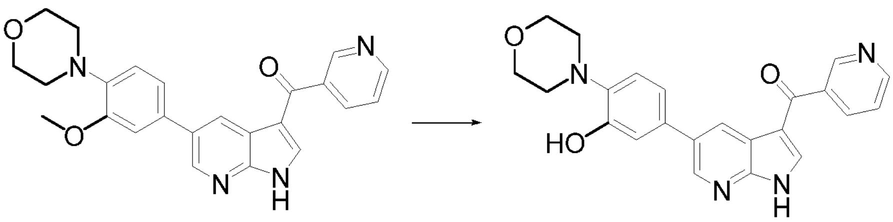

BCD-CDK8-4-27 BCD-CDK8-4-7

Compound **BCD-CDK8-4-7** was prepared analogously to compound **BCD-CDK8-4-1** (example 81) using compound **BCD-CDK8-4-27** instead of compound **BCD-CDK8-4-21.**

**Example 96.** Method of preparation of compound **BCD-CDK8-4-31.**

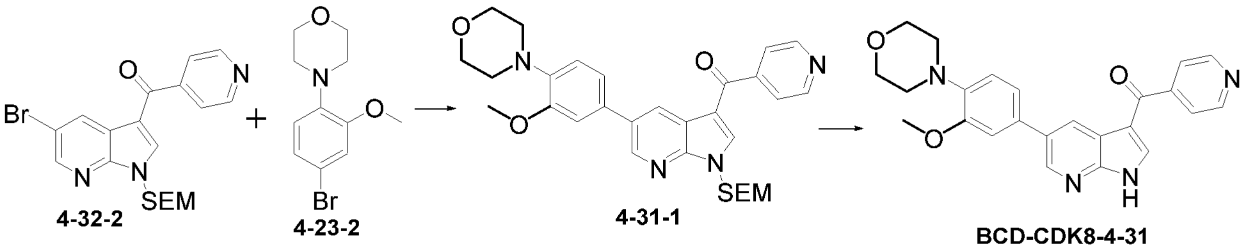

4-32-2 4-23-2 4-31-1 BCD-CDK8-4-31

**Step 1.** Compound **4-31-1** was prepared in a similar fashion to compound **4-24-1** (example 74, step 3) using compound **4-23-2** instead of 2-bromo-5-methoxybenzonitrile and compound **4-32-2** instead of compound **4-24-2.**
**Step 2.** Compound **BCD-CDK8-4-31** was prepared in a similar fashion to compound **BCD-CDK8-4-24**(example 74, step 4) using compound **4-31-1** instead of compound **4-24-1.**

**Example 97.** Method of preparation of compound **BCD-CDK8-4-11.**

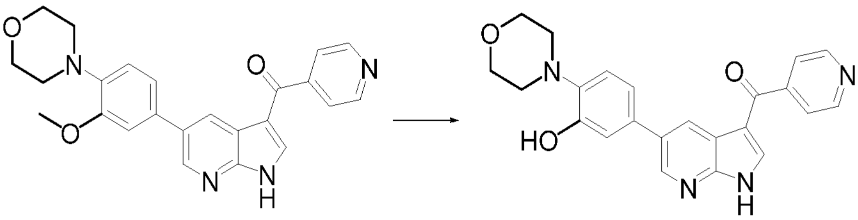

BCD-CDK8-4-31 BCD-CDK8-4-11

Compound **BCD-CDK8-4-11** was prepared analogously to **BCD-CDK8-4-1** compound (example 81) using compound **BCD-CDK8-4-31** instead of compound **BCD-CDK8-4-21.**

**Example 98.** Method of preparation of compound **BCD-CDK8-4-35.**

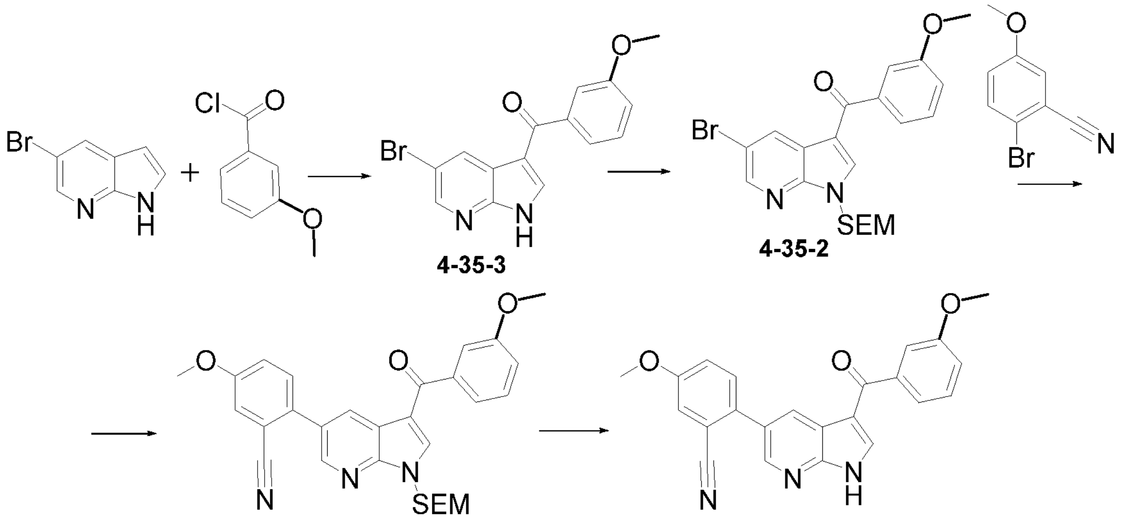

4-35-3 4-35-2

4-35-1 BCD-CDK8-4-35

**Step 1.** $AlCl_3$ (2.71 g, 20.3 mmol) was added to a solution of 5-bromo-1*H*-pyrrolo[2.3]pyridine (1.00 g, 5.07 mmol) in 30 mL of dichloroethane, the resulting suspension was stirred for 15 minutes, a solution of *m*-methoxybenzoyl chloride (862 mg, 5.07 mmol) in 10 mL of dichloroethane was then added. The reaction mixture was stirred at solvent's boiling temperature for 1.5 h. Solvent was evaporated by vacuum distillation. Ice water and saturated $NaHCO_3$ solution were added to the residue. The suspension was filtered, precipitation was dried in vacuum oven, product was isolated as white powder. Yield: 928 mg (56%).

**Step 2.** Compound **4-35-2** was prepared in a similar fashion to compound **4-24-3** (example 74, step 1) using compound **4-35-3** instead of 5-bromo-3-iodo-1*H*-pyrrolo[2,3]pyridine.

**Step 3.** Compound **4-35-1** was prepared in a similar fashion to compound **4-24-1** (example 74, step 3) using compound **4-35-2** instead of compound **4-24-2.**

**Step 4.** Compound **BCD-CDK8-4-35** was prepared in a similar fashion to compound **BCD-CDK8-4-24** (example 74, step 4) using compound **4-35-1**instead of compound **4-24-1.**

**Example 99.** Method of preparation of compound **BCD-CDK8-4-13.**

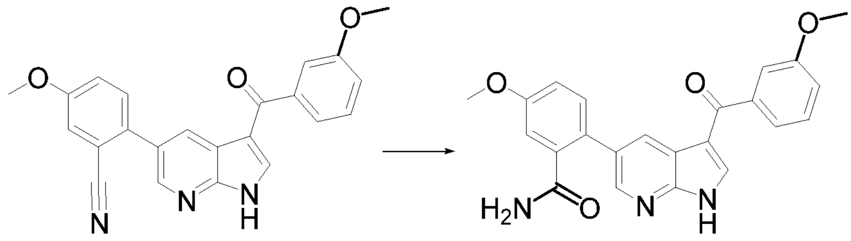

**BCD-CDK8-4-35** **BCD-CDK8-4-13**

Compound **BCD-CDK8-4-13** was prepared in a similar fashion to compound **BCD-CDK8-4-4** (example 75) using compound **BCD-CDK8-4-35** instead of compound **BCD-CDK8-4-24.**

**Example 100.** Method of preparation of compound **BCD-CDK8-4-33.**

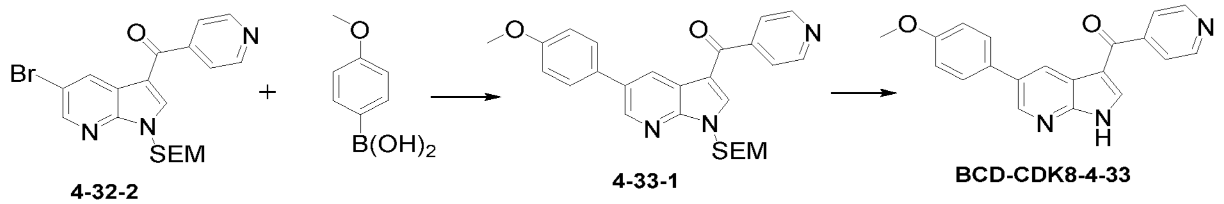

**4-32-2** **4-33-1** **BCD-CDK8-4-33**

**Step 1.** Compound **4-33-1** was prepared in a similar fashion to compound **5-2-3** (example 29, step 2) using compound **4-32-2** instead of compound **5-2-4** and (4-methoxycarbonylphenyl)boronic acid instead of [4-(dimethylamino)phenyl]boronic acid hydrochloride.

**Step 2.** Compound **BCD-CDK8-4-33** was prepared in a similar fashion to compound **BCD-CDK8-4-24** (example 74, step 4) using compound **4-33-1** instead of compound **4-24-1.**

**Example 101.** Method of preparation of compound **BCD-CDK8-4-34.**

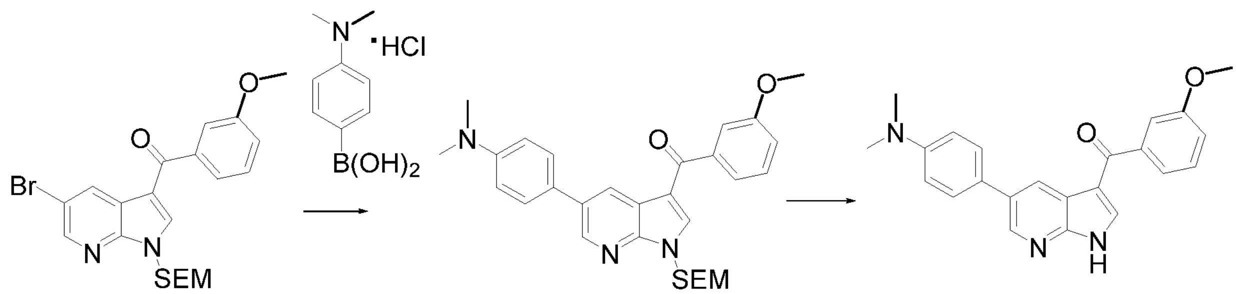

**4-35-2** **4-34-1** **BCD-CDK8-4-34**

**Step** 1. Compound 4-34-1 was prepared in a similar fashion to compound 5-**2-3** (example 29, step 2) using compound **4-35-2** instead of compound **5-2-4.**

**Step 2.** Compound **BCD-CDK8-4-34** was prepared in a similar fashion to compound **BCD-CDK8-4-24** (example 74, step 4) using compound **4-34-1** instead of compound **4-24-1.**

**Example 102.** Method of preparation of compound **BCD-CDK8-4-36.**

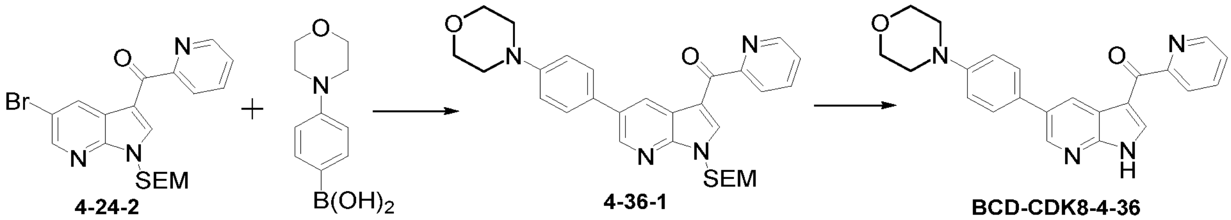

4-24-2 4-36-1 BCD-CDK8-4-36

**Step** 1. Compound 4-36-1 was prepared in a similar fashion to compound 5-2-3 (example 29, step 2) using compound 4-24-2 instead of compound 5-2-4 and (4-morpholin-4-ylphenyl)boronic acid instead of [4-(dimethylamino)phenyl]boronic acid hydrochloride.

**Step 2.** Compound **BCD-CDK8-4-36** was prepared in a similar fashion to compound **BCD-CDK8-4-24** (example 74, step 4) using compound **4-36-1** instead of **4-24-1.**

**Example 103.** Method of preparation of compound **BCD-CDK8-4-37.**

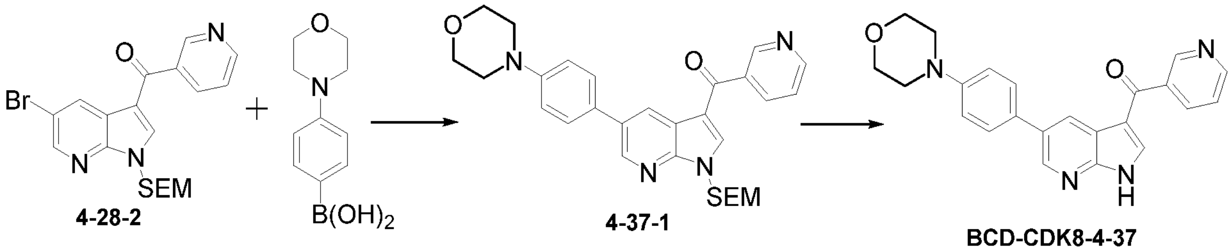

4-28-2 4-37-1 BCD-CDK8-4-37

**Step 1.** Compound **4-37-1** was prepared in a similar fashion to compound **5-2-3** (example 29, step 2) using **4-28-2** instead of **5-2-4** and (4-morpholin-4-ylphenyl)boronic acid instead of [4-(dimethylamino)phenyl]boronic acid hydrochloride.

**Step 2.** Compound **BCD-CDK8-4-37** was prepared in a similar fashion to compound **BCD-CDK8-4-24** (example 74, step 4) using compound **4-37-1** instead of **4-24-1.**

**Example 104.** Method of preparation of compound **BCD-CDK8-4-38.**

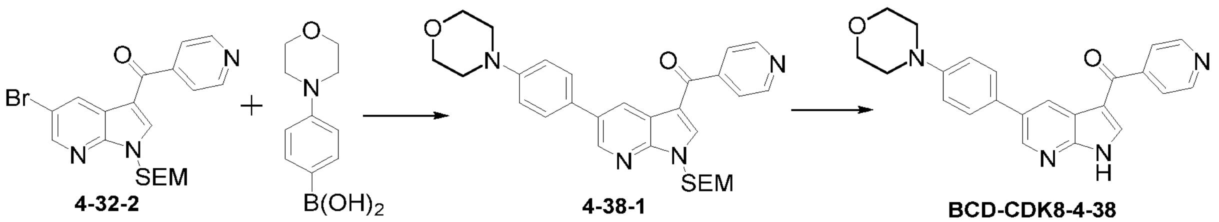

4-32-2 4-38-1 BCD-CDK8-4-38

**Step 1.** Compound **4-38-1** was prepared in a similar fashion to compound **5-2-3** (example 29, step 2) using **4-32-2** instead of **5-2-4** and (4-morpholin-4-ylphenyl)boronic acid instead of [4-(dimethylamino)phenyl]boronic acid hydrochloride.

**Step 2.** Compound **BCD-CDK8-4-38** was prepared in a similar fashion to compound **BCD-CDK8-4-24** (example 74, step 4) using compound **4-38-1** instead of compound **4-24-1.**

**Example 105.** Method of preparation of compound **BCD-CDK8-4-39.**

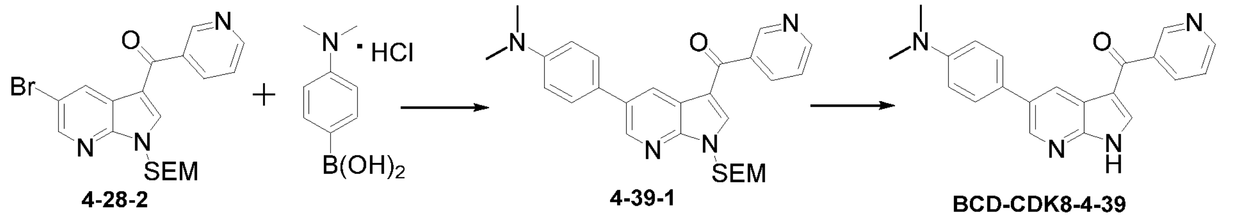

4-28-2 4-39-1 BCD-CDK8-4-39

**Step 1.** Compound **4-39-1** was prepared in a similar fashion to compound **5-2-3** (example 29, step 2) using compound **4-28-2** instead of compound **5-2-4.**

**Step 2.** Compound **BCD-CDK8-4-39** was prepared in a similar fashion to compound **BCD-CDK8-4-24** (example 74, step 4) using compound **4-39-1** instead of compound **4-24-1.**

**Example 106.** Method of preparation of compound **BCD-CDK8-4-40.**

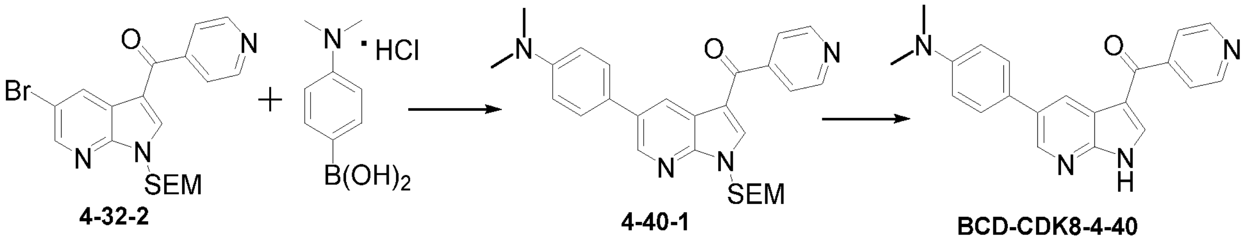

4-32-2 4-40-1 BCD-CDK8-4-40

**Step** 1. Compound 4-40-1 was prepared in a similar fashion to compound 5-2-3 (example 29, step 2) using 4-32-2 instead of 5-2-4.

Step 2. Compound BCD-CDK8-4-40 was prepared in a similar fashion to compound **BCD-CDK8-4-24** (example 74, step 4) using compound **4-40-1** instead of **4-24-1.**

**Example 107.** Method of preparation of compound **BCD-CDK8-3-19.**

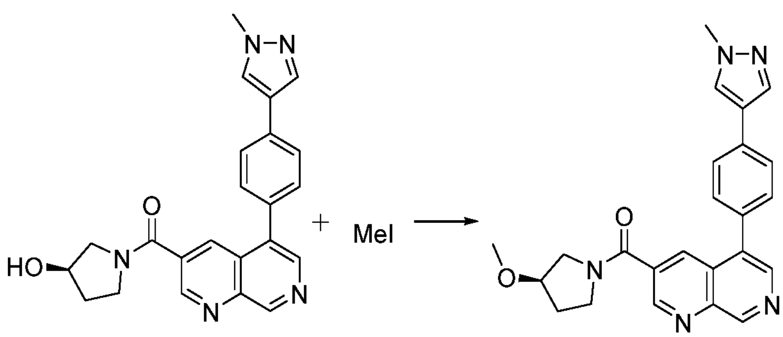

BCD-CDK8-3-4 BCD-CDK8-3-19

**BCD-CDK8-3-4.** (35 mg, 0.088 mmol, 1.00 eq) was dissolved in 2 mL of THF, and NaH (5 mg, 0.114 mmol, 1.30 eq, 60% suspension in mineral oil) was added portionwise while cooling in ice bath. The mixture was stirred for 15 min under cooling and then for 30 min at room temperature. Methyl iodide (9 μl, 0.150 mmol, 1.7 eq) was then added dropwise under stirring and cooling in ice bath; after 5 min, the reaction mixture was heated to room temperature. After 1 hour, 10 mL of water was added, extraction was performed with ethyl acetate (2×15 mL). Organic layers were washed with a saturated NaCl solution and dried with $Na_2SO_4$. Product was isolated as light-yellow powder by column chromatography on silica gel using dichloromethane-methanol (96:4) as eluent. Yield: 18 mg (50%).

**Example 108.** Method of preparation of compound **BCD-CDK8-3-20.**

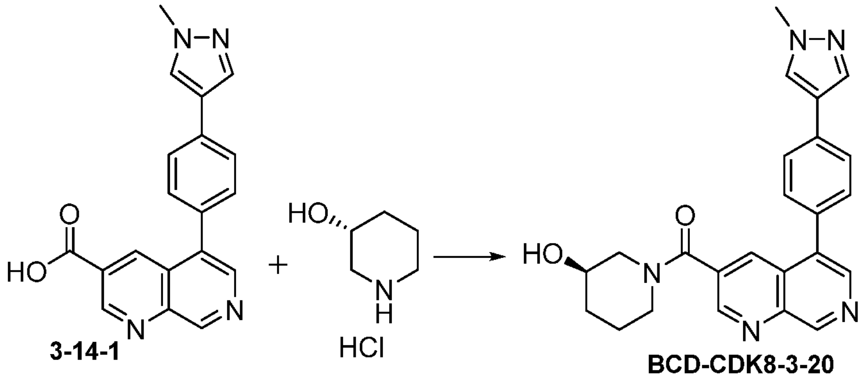


3-14-1

BCD-CDK8-3-20

Compound **BCD-CDK8-3-20** was prepared in a similar fashion to compound **1-2-4** (example 5, step 1) using compound **3-14-1** instead of 3-fluoro-4-nitrobenzoic acid and (*S*)-3-hydroxypiperidin hydrochloride instead of azetidine hydrochloride.

**Example 109.** Method of preparation of compound **BCD-CDK8-3-21.**

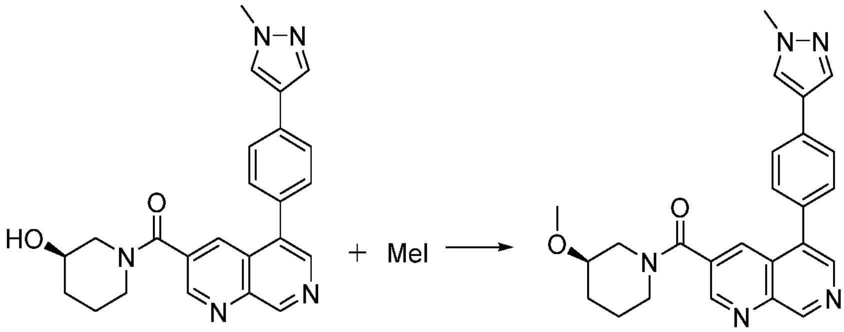


BCD-CDK8-3-20

BCD-CDK8-3-21

Compound **BCD-CDK8-3-21** was prepared in a similar fashion to compound **BCD-CDK8-3-19** (example 107) using compound **BCD-CDK8-3-20** instead of **BCD-CDK8-3-4.**

**Example 110.** Method of preparation of compound **BCD-CDK8-3-22.**

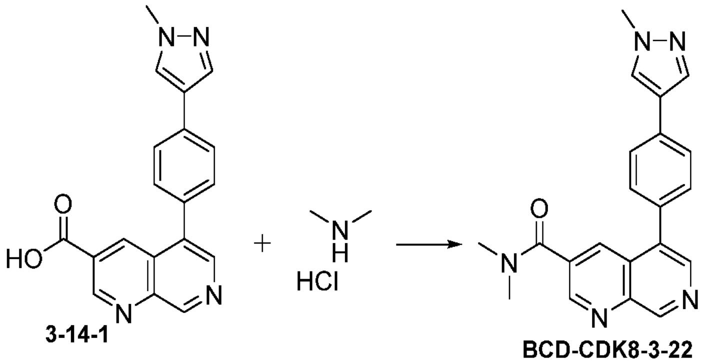


3-14-1

BCD-CDK8-3-22

Compound BCD-CDK8-3-22 was prepared in a similar fashion to compound 1-2-4 (example 5, step 1) using compound 3-14-1 instead of 3-fluoro-4-nitrobenzoic acid and dimethylamine hydrochloride instead of azetidine hydrochloride.

**Example 111.** Method of preparation of compound **BCD-CDK8-3-23.**

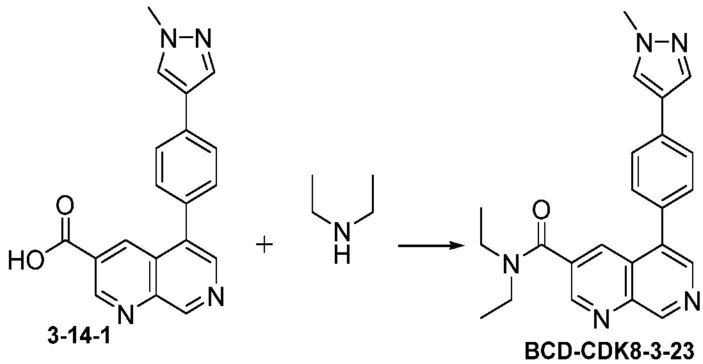

3-14-1 BCD-CDK8-3-23

Compound **BCD-CDK8-3-23** was prepared in a similar fashion to compound **1-2-4** (example 5, step 1) using compound **3-14-1**instead of 3-fluoro-4-nitrobenzoic acid and dimethylamine instead of azetidine hydrochloride.

**Example 112.** Method of preparation of compound **BCD-CDK8-3-24.**

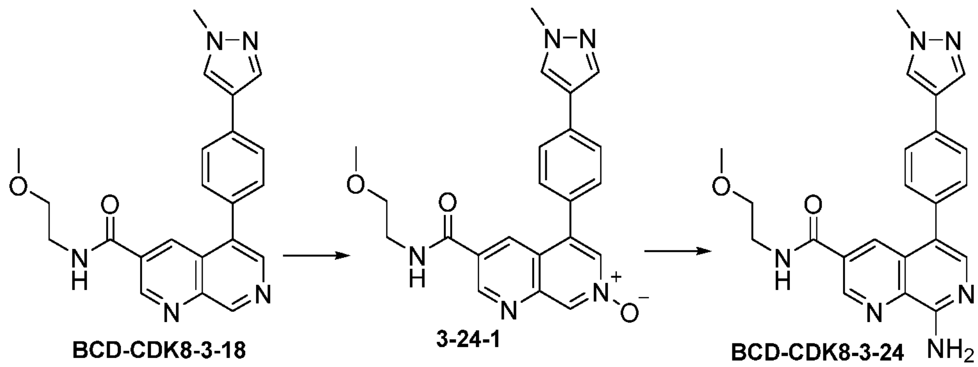

BCD-CDK8-3-18 3-24-1 BCD-CDK8-3-24

**Step 1.** Compound **BCD-CDK8-3-18** (110 mg, 0.283 mmol, 1.00 eq) was dissolved in 5 mL of dichloromethane, and *m*-chloroperbenzoic acid (66 mg, 0.326 mmol, 1.15 eq) was added portionwise. After 1 hour, 15 mL of water and 5 mL of dichloromethane were poured, a solution of 1M sodium hydroxide was added to pH 8. Organic layers were isolated, water layer was further extracted with dichloromethane. The combined organic layers were washed with saturated NaCl solution, dried on $Na_2SO_4$. Reaction product was isolated as yellow powder by column chromatography on silica gel using ethyl acetate-dichloromethane-methanol (5:4.7:0.3) as eluent. Yield: 65 mg (57%).

**Step 2.** Compound **CDK8-3-24-1** (30 mg, 0.074 mmol, 1.00 eq) was dissolved in 2 mL of pyridine, and *n*-tosyl chloride (17 mg, 0.089 mmol, 1.20 eq) was added. The mixture was stirred for 1 h at room temperature. Solvent was evaporated, 3 mL of ethanolamine was added to the reaction mixture, the mixture was stirred for 3 h at 35 °C (in water bath), 50 mL of water was then added to the reaction mixture. Suspension was filtered, precipitation was washed with water, dried under vacuum, product was prepared as yellow powder. Yield: 25 mg (83%).

**Example 113.** Method of preparation of compound **BCD-CDK8-3-25.**

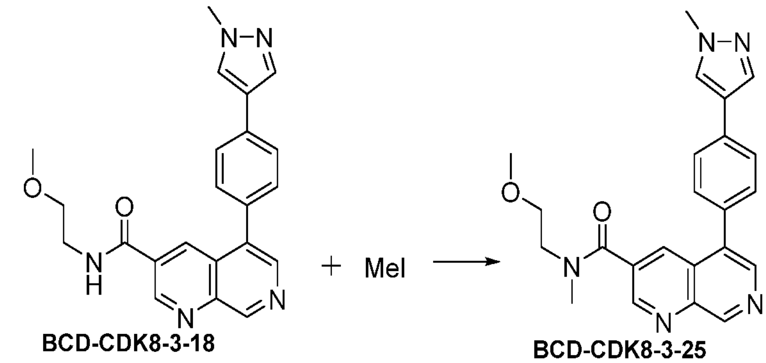

BCD-CDK8-3-18 BCD-CDK8-3-25

Compound **BCD-CDK8-3-25** was prepared in a similar fashion to compound **BCD-CDK8-3-19** (example 107) using compound **BCD-CDK8-3-18** instead of **BCD-CDK8-3-4,** and DMF instead of THF as a solvent.

**Example 114.** Method of preparation of compound **BCD-CDK8-3-26.**

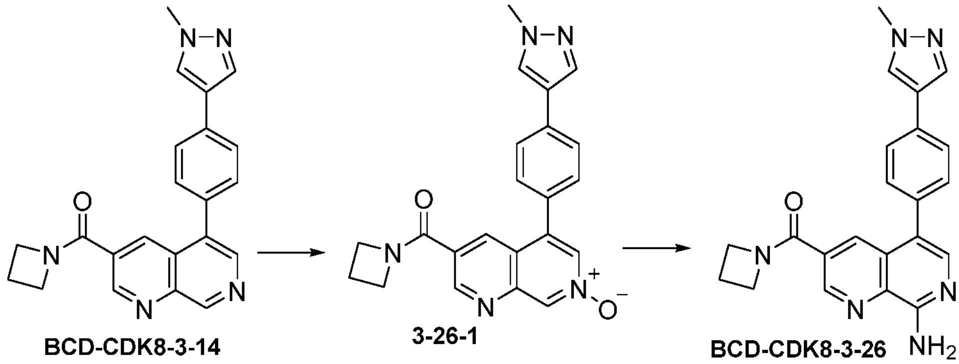

BCD-CDK8-3-14 3-26-1 BCD-CDK8-3-26

**Step 1.** Compound **3-26-1** was prepared in a similar fashion to compound **3-24-1**(example 112, step 1) using compound **BCD-CDK8-3-14** instead of compound **BCD-CDK8-3-18.**

**Step 2.** Compound **BCD-CDK8-3-26** was prepared in a similar fashion to compound **BCD-CDK8-3-24** (example 112, step 2) using compound **3-26-1** instead of compound **3-24-1.**

**Example 115.** Method of preparation of compound **BCD-CDK8-3-33.**

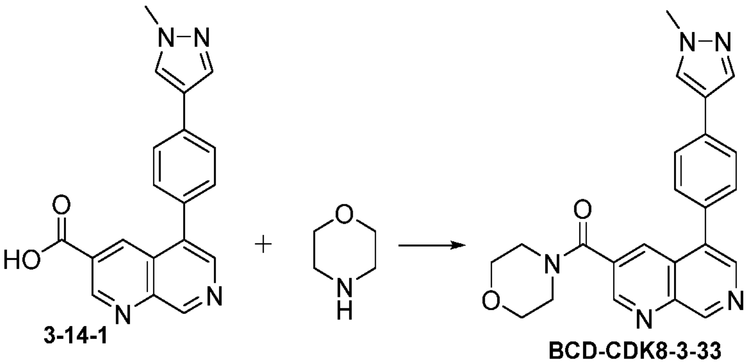

3-14-1 BCD-CDK8-3-33

Compound **BCD-CDK8-3-33** was prepared in a similar fashion to compound **1-2-4** (example 5, step 1) using compound **3-14-1** instead of 3-fluoro-4-nitrobenzoic acid and morpholine instead of azetidine hydrochloride.

**Example 116.** Method of preparation of compound **BCD-CDK8-3-27.**

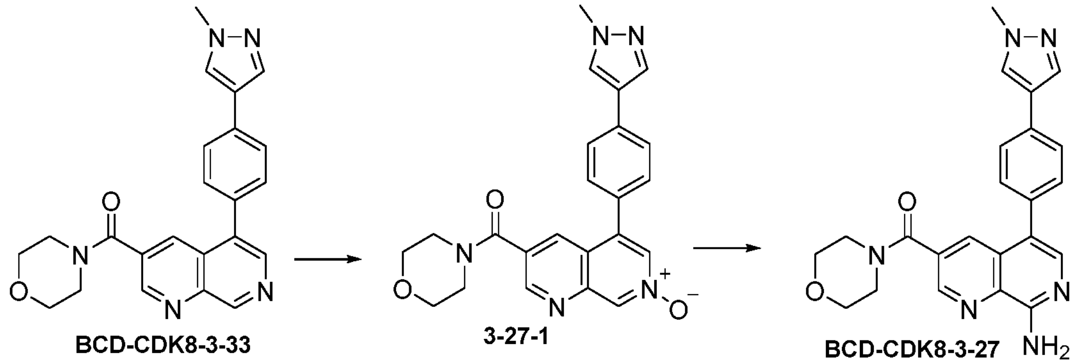

BCD-CDK8-3-33 3-27-1 BCD-CDK8-3-27

**Step 1.** Compound **3-27-1** was prepared in a similar fashion to compound **3-24-1**(example 112, step 1) using compound **BCD-CDK8-3-33** instead of compound **BCD-CDK8-3-18.**

**Step 2.** Compound **BCD-CDK8-3-27** was prepared in a similar fashion to compound **BCD-CDK8-3-24** (example 112, step 2) using compound **3-27-1** instead of compound **3-24-1.**

**Example 117.** Method of preparation of compound **BCD-CDK8-3-28.**

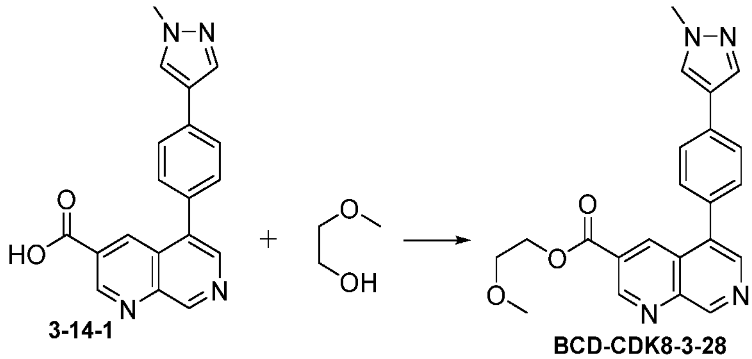

3-14-1 BCD-CDK8-3-28

Compound **BCD-CDK8-3-28** was prepared in a similar fashion to compound **1-2-4** (example 5, step 1) using compound **3-14-1** instead of 3-fluoro-4-nitrobenzoic acid and 2-metoxyethanol instead of azetidine hydrochloride.

**Example 118.** Method of preparation of compound **BCD-CDK8-3-29.**

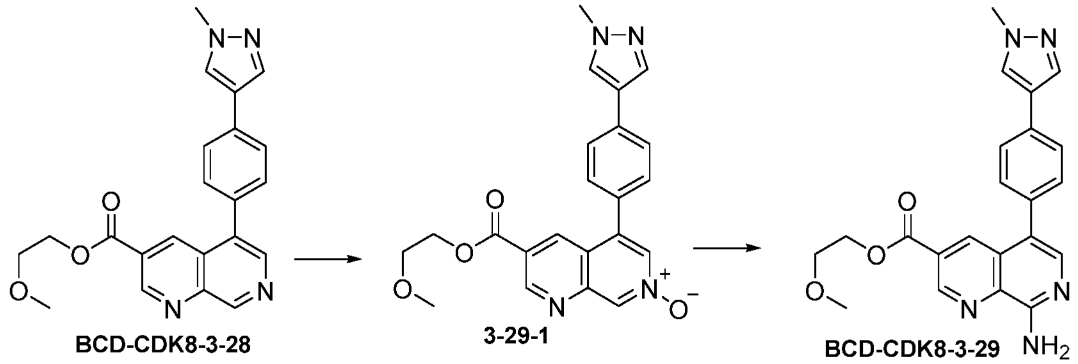

BCD-CDK8-3-28 3-29-1 BCD-CDK8-3-29

**Step 1.** Compound **3-29-1** was prepared in a similar fashion to compound **3-24-1**(example 112, step 1) using compound **BCD-CDK8-3-28** instead of compound **BCD-CDK8-3-18.**

**Step 2.** *n*-tosyl chloride (80 mg, 0.42 mmol) was added to a solution of compound **3-29-1** (132 mg, 0.323 mmol) in 5 mL of pyridine, the reaction mass was stirred at room temperature for 1 hour. Ethanolamine (20 mg 0.323 mmol) was added to the resulting solution. Ethanolamine (30 mg, 0.484 mmol) was additionally added after 1-hour stirring. After 1.5-hour stirring, the reaction mass was poured into water, product was extracted with ethyl acetate, dried with Na $_2SO_4$. Reaction product was isolated by column chromatography on silica gel using dichloromethane-methanol (95:5) as eluent. Yield: 48 mg (37%).

**Example 119.** Method of preparation of compound **BCD-CDK8-3-30.**

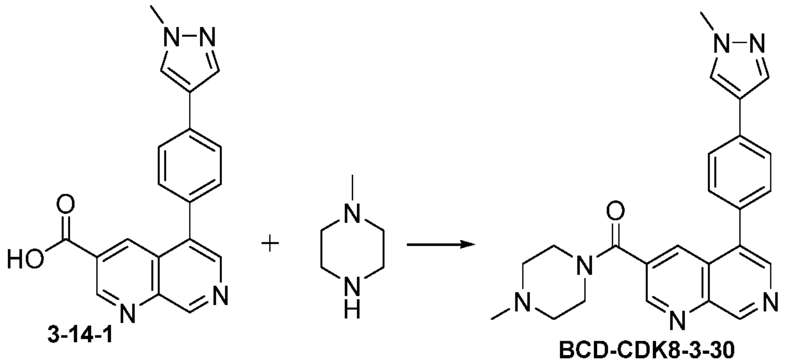

**3-14-1** **BCD-CDK8-3-30**

Compound **BCD-CDK8-3-30** was prepared in a similar fashion to compound **1-2-4** (example 5, step 1) using compound **3-14-1** instead of 3-fluoro-4-nitrobenzoic acid and *N*-methylpiperazin, instead of azetidine hydrochloride.

**Example 120.** Method of preparation of compound **BCD-CDK8-3-31.**

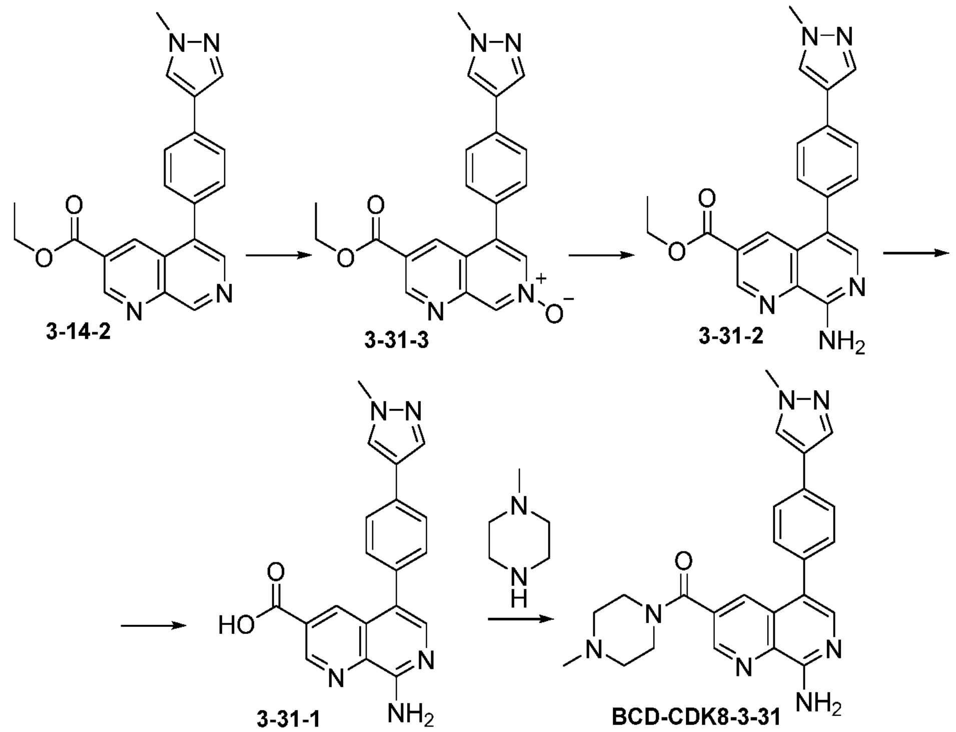

**3-14-2** **3-31-3** **3-31-2** **3-31-1** **BCD-CDK8-3-31**

**Step 1.** Compound **3-31-3** was prepared in a similar fashion to compound **3-24-1**(example 112, step 1) using compound **3-14-2** instead of compound **BCD-CDK8-3-18.**

**Step 2.** Compound **3-31-2** was prepared analogously to **BCD-CDK8-3-29** (example 118, step 2) using compound **3-31-3** instead of compound **3-29-1.**

**Step 3.** Compound **3-31-1** was prepared analogously to **BCD-CDK8-1-6a** (example 2) using compound **3-31-2** instead of compound **BCD-CDK8-1-6e.**

**Step 4.** Compound **BCD-CDK8-3-31** was prepared in a similar fashion to compound **BCD-CDK8-5-2** (example 29, step 5) using compound **3-31-1** instead of compound **5-2-1** and *N*-methylpiperazin instead of (*R*)-3 hydroxypyrrolidine hydrochloride.

**Example 121.** Method of preparation of compound **BCD-CDK8-3-32.**

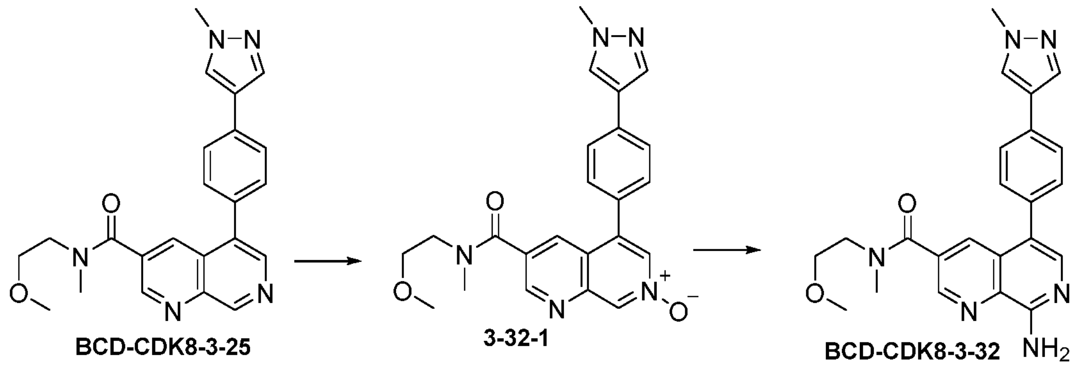

**BCD-CDK8-3-25** **3-32-1** **BCD-CDK8-3-32**

**Step 1.** Compound **3-32-1** was prepared in a similar fashion to compound **3-24-1**(example 112, step 1) using compound **BCD-CDK8-3-25** instead of compound **BCD-CDK8-3-18.**

**Step 2.** Compound **BCD-CDK8-3-32** was prepared in a similar fashion to compound **BCD-CDK8-3-24** (example 112, step 2) using compound **3-32-1** instead of compound **3-24-1.**

**Example 122.** Method of preparation of compound **BCD-CDK8-3-34.**

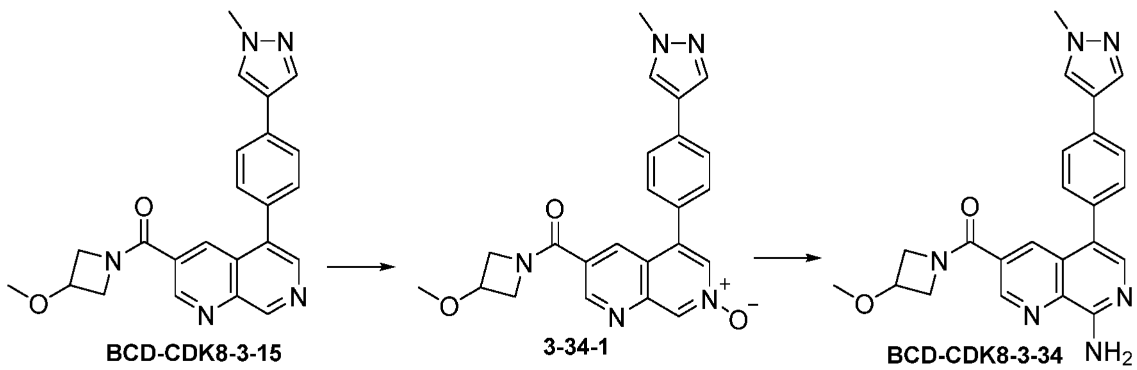

**BCD-CDK8-3-15** **3-34-1** **BCD-CDK8-3-34**

**Step 1.** Compound **3-34-1** was prepared in a similar fashion to compound **3-24-1**(example 112, step 1) using compound **BCD-CDK8-3-15** instead of compound **BCD-CDK8-3-18.**

**Step 2.** Compound **BCD-CDK8-3-34** was prepared in a similar fashion to compound **BCD-CDK8-3-24** (example 112, step 2) using compound **3-34-1** instead of compound **3-24-1.**

**Example 123.** Method of preparation of compound **BCD-CDK8-3-35.**

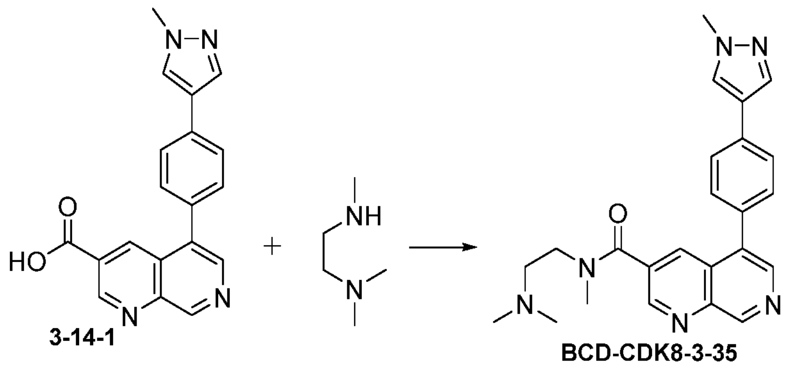

3-14-1 BCD-CDK8-3-35

Compound **BCD-CDK8-3-35** was prepared in a similar fashion to compound **1-2-4** (example 5, step 1) using compound **3-14-1** instead of 3-fluoro-4-nitrobenzoic acid and *N*,*N*,*N*'-3-methylethylenediamine, instead of azetidine hydrochloride.

**Example 124.** Method of preparation of compound **BCD-CDK8-3-37.**

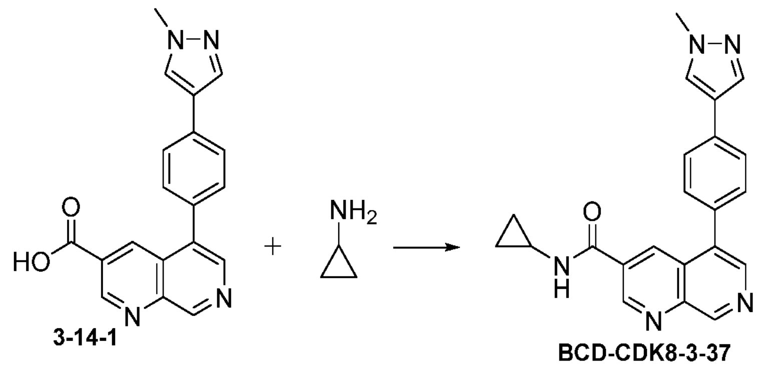

3-14-1 BCD-CDK8-3-37

Compound **BCD-CDK8-3-37** was prepared in a similar fashion to compound **1-2-4** (example 5, step 1) using compound **3-14-1** instead of 3-fluoro-4-nitrobenzoic acid and cyclopropylamine instead of azetidine hydrochloride.

**Example 125.** Method of preparation of compound **BCD-CDK8-3-38.**

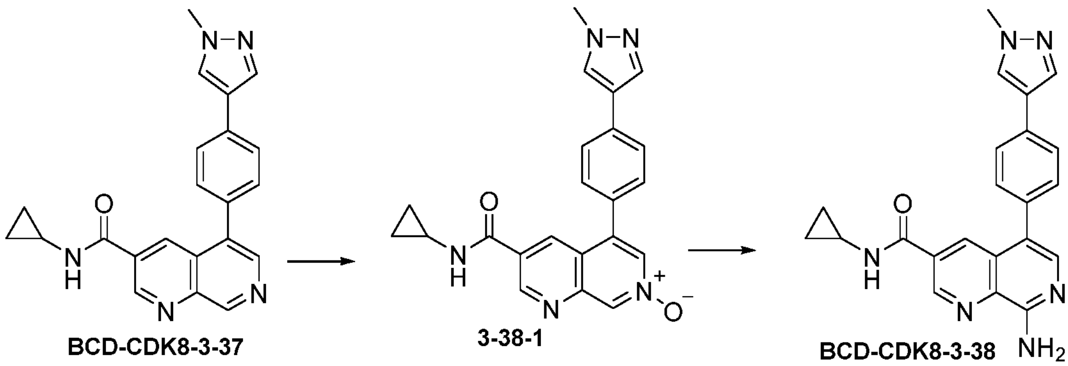

BCD-CDK8-3-37 3-38-1 BCD-CDK8-3-38

**Step 1.** Compound **3-38-1** was prepared in a similar fashion to compound **3-24-1**(example 112, step 1) using compound **BCD-CDK8-3-37** instead of compound **BCD-CDK8-3-18**.
**Step 2.** Compound **BCD-CDK8-3-38** was prepared in a similar fashion to compound **BCD-CDK8-3-24** (example 112, step 2) using compound **3-38-1** instead of compound **3-24-1.**

**Example 126.** Method of preparation of compound **BCD-CDK8-3-39.**

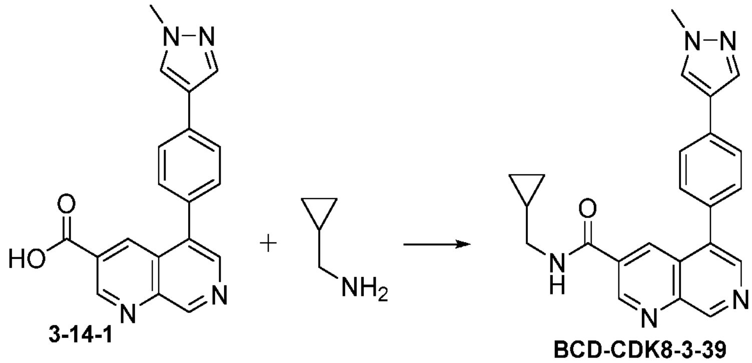

**3-14-1** **BCD-CDK8-3-39**

Compound **BCD-CDK8-3-39** was prepared in a similar fashion to compound **1-2-4** (example 5, step 1) using compound **3-14-1** instead of 3-fluoro-4-nitrobenzoic acid and cyclopropylmethanamine instead of azetidine hydrochloride.

**Example 127.** Method of preparation of compound **BCD-CDK8-3-40.**

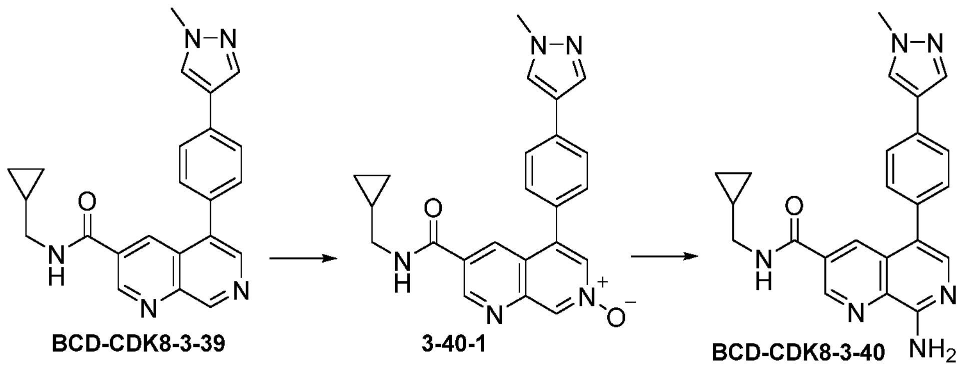

**BCD-CDK8-3-39** **3-40-1** **BCD-CDK8-3-40**

**Step 1.** Compound **3-40-1** was prepared in a similar fashion to compound **3-24-1**(example 112, step 1) using compound **BCD-CDK8-3-39** instead of compound **BCD-CDK8-3-18.**

**Step 2.** Compound **BCD-CDK8-3-40** was prepared in a similar fashion to compound **BCD-CDK8-3-24** (example 112, step 2) using compound **3-40-1** instead of compound **3-24-1.**

**Example 128.** Method of preparation of compound **BCD-CDK8-3-42.**

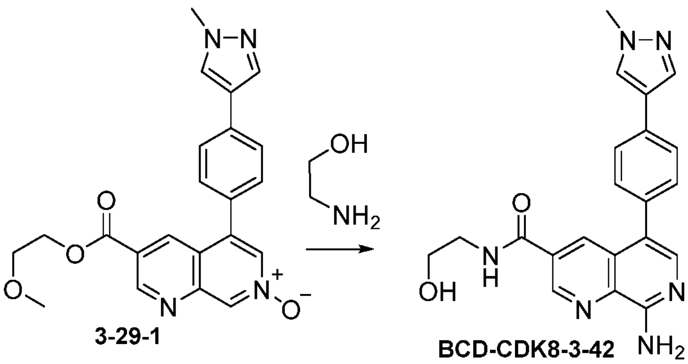

**3-29-1** **BCD-CDK8-3-42**

Compound **BCD-CDK8-3-42** was prepared in a similar fashion to compound **BCD-CDK8-3-24** (example 112, step 2) using compound **3-29-1** instead of compound **3-24-1.**

**Example 129.** Method of preparation of compound **BCD-CDK8-3-41.**

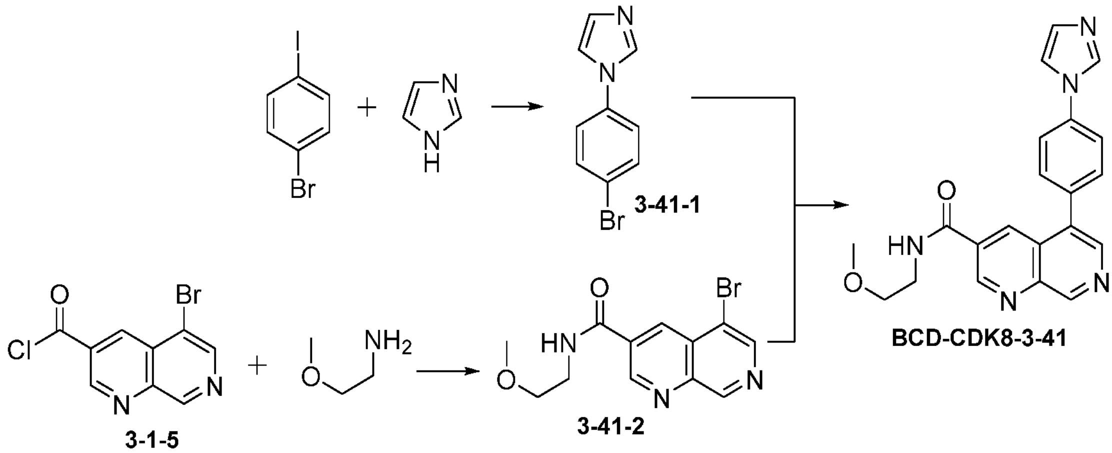

3-41-1

3-1-5

3-41-2

BCD-CDK8-3-41

**Step 1.** Nitrogen was passed through a suspension of benzotriazole (84 mg, 0.7 mmol, 0.2 eq) and copper(I) iodide (135 mg, 0.7 mmol, 0.2 eq) in 20 mL of DMSO for 30 min. 1-bromo-4-iodobenzene (1.00 g, 3.5 mmol, 1 eq), imidazole (265 mg, 3.8 mmol, 1.1 eq) and potassium *tert*-butylate (595 mg, 5.3 mmol, 1.5 eq) were then added. The reaction mixture was stirred in a sealed flask for 3 hours at 140 °C, then overnight at room temperature. The mixture was filtered through Celite, precipitate was washed 3 times with ethyl acetate, mother liquor was washed with water and NaCl solution (2×50 mL). Organic layer was isolated, washed with water and dried with $Na_2SO_4$. Product was isolated as gray powder by column chromatography on silica gel using hexane-ethyl acetate (7:3) as eluent. Yield: 430 g (55%).

**Step 2**. Compound **3-41-2** was prepared in a similar fashion to compound **3-1-4** (example 58, step 2) using 2-methoxyethylamine instead of (*S*)-3-(*tert*-butyldiphenylsilyloxy)pyrrolidine.

**Step 3**. Compound **BCD-CDK8-3-41** was prepared in a similar fashion to compound **5-4-4** (example 33, step 2) using compound **3-41-2** instead of compound **5-2-4** and compound **3-41-1** instead of compound **5-4-5.**

**Example 130.** Analysis of prepared compounds.

Purity and structure of the prepared compounds were confirmed by liquid chromatography-mass spectrometry (LC-MS) and $^1$H NMR spectroscopy (Table 1).

**Equipment Data:**

Liquid chromatography-mass spectrometry

| Name | Manufacturer, country |
|---|---|
| Agilent Triple Quad liquid chromatography/mass spectrometry (LC/MS) system | Agilent, USA |
| Agilent 1200 Autosampler | |
| Agilent 1200 Thermostatted Column | |
| Agilent 1200 Degasser | |
| Agilent 1200 Autosampler Thermostat | |
| Agilent 6410 QQQ MS Detector | |
| Agilent 1200 UV Detector | |
| Agilent 1200 Pump | |

NMR spectrometer

| Name | Manufacturer, country | Model, main characteristics |
|---|---|---|
| NMR spectrometer | Germany | AVANCE III, 400 MHz |

**Table 1.** Analytical data for examples of compounds

| Code | ESI-MS. $[M+H]^+$ | $^1H$ NMR, $\delta$, MD |
|---|---|---|
| **BCD-CDK8-1-1** | 490.2 | $^1H$ NMR (400 MHz, DMSO-$d_6$) $\delta$ 12.95-11.55 (br s, 1H), 10.09 (s, 1H), 8.85 (s, 1H), 8.20 (d, $J$ = 8.6 Hz, 2H), 7.91 (d, $J$ = 8.6 Hz, 2H), 7.67 (d, $J$ = 8.7 Hz, 1H), 7.62 (d, $J$ = 7.7 Hz, 2H), 7.31 (t, $J$ = 7.9 Hz, 2H), 7.05 (t, $J$ = 7.4 Hz, 1H), 6.69-6.60 (m, 2H), 4.16-4.07 (m, 2H), 4.02-3.96 (m, 2H), 3.77-3.68 (m, 1H), 3.42 (s, 3H). |
| **BCD-CDK8-1-2** | 399.0 | $^1H$ NMR (400 MHz, DMSO-$d_6$) $\delta$ 8.66 (s, 1H), 8.22 (d, $J$ = 8.4 Hz, 2H), 7.84 (s, 1H), 7.78 (d, $J$ = 8.4 Hz, 1H), 7.65 (d, $J$ = 8.4 Hz, 2H), 7.56 (dd, $J$ = 8.3, 1.1 Hz, 1H), 4.33 (s, 2H), 4.08 (s, 2H), 2.92 (s, 3H), 2.36-2.25 (m, 2H). |
| **BCD-CDK8-1-3** | 372.1 | $^1H$ NMR (400 MHz, DMSO-$d_6$) $\delta$ 13.04 (s, 1H), 8.66 (s, 1H), 8.33 (s, 1H), 8.04 (s, 1H), 7.87 (d, $J$ = 8.5 Hz, 2H), 7.82 (d, $J$ = 8.3 Hz, 1H), 7.67 (d, $J$ = 8.5 Hz, 2H), 7.59 (s, 1H), 7.32 (dd, $J$ = 8.3, 1.2 Hz, 1H), 3.71-3.25 (m, 4H), 1.60-1.50 (m, 6H). |
| **BCD-CDK8-1-4** | 429.2 | $^1H$ NMR (400 MHz, DMSO-$d_6$) $\delta$ 13.03 (s, 1H), 8.31 (s, 1H), 8.28 (s, 1H), 8.04 (s, 1H), 7.85 (d, $J$ = 8.4 Hz, 2H), 7.62 (d, $J$ = 8.4 Hz, 2H), 7.58 (d, $J$ = 9.0 Hz, 1H) 6.52-6.50 (m, 2H), 4.05 (t, 2H), 3.91 (t, 2H), 3.85-3.78 (m, 1H), 3.58-3.54 (m, 4H), 3.47-3.45 (m, 2H), 3.35-3.32 (m, 2H). |
| **BCD-CDK8-1-5** | 344.1 | $^1H$ NMR (400 MHz, DMSO-$d_6$) $\delta$ 13.05 (s, 1H), 8.69 (s, 1H), 8.28-8.06 (m, 2H), 7.88 (d, $J$ = 8.4 Hz, 2H), 7.85-7.79 (m, 2H), 7.67 (d, $J$ = 8.4 Hz, 2H), 7.58 (dd, $J$ = 8.6, 0.8 Hz, 1H), 4.32 (m, 2H), 4.06 (m, 2H), 2.31-2.20 (m, 2H). |
| **BCD-CDK8-1-6E** | 427.2 | $^1H$ NMR (400 MHz, DMSO-$d_6$) $\delta$ 10.08 (s, 1H), 8.41 (s, 1H), 8.17 (d, $J$ = 8.5 Hz, 2H), 7.86 (d, $J$ = 8.5 Hz, 2H), 7.63 - 7.60 (m, 3H), 7.31 (t, 2H), 7.05 (t, 1H), 6.64 (d, $J$ = 1.8 Hz, 1H), 6.56 (dd, $J$ = 8.6, 1.8 Hz, 1H), 4.09 (t, 2H), 3.97 (t, 2H), 3.90 (s, 3H), 3.76 - 3.65 (m, 1H). |
| **BCD-CDK8-1-6A** | 413.1 | $^1H$ NMR (400 MHz, DMSO-$d_6$) $\delta$ 10.13 (s, 1H), 8.39 (s, 1H), 8.15 (s, 2H), 7.81 (s, 2H), 7.62 (s, 3H), 7.31 (s, 2H), 7.05 (s, 1H), 6.64 (s, 1H), 6.56 (s, 1H), 4.09 (s, 2H), 3.97 (s, 2H), 3.72 (s, 1H). |

(continued)

| Code | ESI-MS. $[M+H]^+$ | $^1H$ NMR, $\delta$, MD |
|---|---|---|
| **BCD-CDK8-1-6** | 412.2 | $^1H$ NMR (400 MHz, DMSO-$d_6$) $\delta$ 10.08 (s, 1H), 8.98 (s, 1H), 8.20-8.10 (m, 3H), 7.84 (dd, *J* = 8.4, 2.4 Hz, 2H), 7.69 (dd, *J* = 8.7, 2.5 Hz, 1H), 7.62 (d, *J* = 7.0 Hz, 2H), 7.54 (s, 1H), 7.31 (t, *J* = 6.6 Hz, 2H), 7.05 (t, *J* = 6.7 Hz, 1H), 6.70 (d, *J* = 8.7 Hz, 1H), 6.61 (s, 1H), 4.19-4.06 (m, 2H), 4.05-3.94 (m, 2H), 3.79-3.67 (s, 1H). |
| **BCD-CDK8-1-7E** | 445.1 | $^1H$ NMR (400 MHz, DMSO-$d_6$) $\delta$ 10.06 (s, 1H), 8.42 (s, 1H), 8.18 (d, *J* = 8.7 Hz, 2H), 7.86 (d, *J* = 8.6 Hz, 2H), 7.62 (d, *J* = 7.7 Hz, 2H), 7.31 (t, *J* = 7.9 Hz, 2H), 7.05 (t, *J* = 7.4 Hz, 1H), 6.49-6.33 (m, 2H), 4.12-4.05 (m, 2H), 4.01-3.94 (m, 2H), 3.91 (s, 3H), 3.75-3.65 (m, 1H). |
| **BCD-CDK8-1-7A** | 431.2 | $^1H$ NMR (400 MHz, DMSO-$d_6$) $\delta$ 13.17 (s, 1H), 10.06 (s, 1H), 8.41 (s, 1H), 8.16 (d, J = 8.7 Hz, 2H), 7.83 (d, J = 8.6 Hz, 2H), 7.61 (d, J = 7.7 Hz, 2H), 7.31 (t, *J* = 7.9 Hz, 1H), 7.05 (t, J = 7.4 Hz, 1H), 6.48-6.36 (m, 2H), 4.11-4.07 (m, 2H), 3.99-3.96 (m, 2H), 3.76-3.65 (m, 1H). |
| **BCD-CDK8-1-7** | 430.1 | $^1H$ NMR (400 MHz, DMSO-$d_6$) $\delta$ 10.06 (s, 1H), 8.38 (s, 1H), 8.15-8.07 (m, 3H), 7.77 (d, *J* = 8.6 Hz, 2H), 7.62 (d, *J* = 7.6 Hz, 2H), 7.48 (s, 1H), 7.31 (t, *J* = 7.9 Hz, 2H), 7.06 (t, *J* = 7.3 Hz, 1H), 6.45-6.37 (m, 2H), 4.09 (m, 2H), 3.97 (m, 2H), 3.75-3.64 (m, 1H). |
| **BCD-CDK8-1-8** | 447.2 | $^1H$ NMR (400 MHz, DMSO-$d_6$) $\delta$ 13.04 (s, 1H), 8.36-8.25 (m, 2H), 8.04 (s, 1H), 7.85 (d, *J* = 8.4 Hz, 2H), 7.62 (d, *J* = 8.5 Hz, 2H), 6.37 (dd, *J* = 12.6, 1.3 Hz, 1H), 6.31 (d, *J* = 1.5 Hz, 1H), 4.10-4.05 (m, 2H), 3.96-3.88 (m, 2H), 3.87-3.75 (m, 1H), 3.61-3.52 (m, 4H), 3.49-3.43 (m, 2H), 3.43-3.38 (m, 2H). |
| **BCD-CDK8-1-9** | 406.3 | $^1H$ NMR (400 MHz, DMSO-$d_6$) $\delta$ 8.11 (s, 1H), 7.55 (d, *J* = 8.6 Hz, 1H), 7.39 (d, *J* = 8.9 Hz, 2H), 6.90 (d, *J* = 9.0 Hz, 2H), 6.48 (dd, *J* = 8.6, 2.1 Hz, 1H), 6.37 (d, *J* = 1.9 Hz, 1H), 4.03 (t, 2H), 3.88 (t, 2H), 3.84-3.77 (m, 1H), 3.58-3.54 (m, 4H), 3.47-3.45 (m, 2H), 3.35 - 3.32 (m, 2H), 2.98 (s, 6H). |
| **BCD-CDK8-1-10** | 321.1 | $^1H$ NMR (400 MHz, DMSO-$d_6$) $\delta$ 8.50 (s, 1H), 7.76 (d, *J* = 8.4 Hz, 1H), 7.69 (d, *J* = 1.1 Hz, 1H), 7.53 (dd, *J* = 8.4, 1.4 Hz, 1H), 7.43 (d, *J* = 8.9 Hz, 2H), 6.92 (d, *J* = 9.0 Hz, 2H), 4.31 (m, 2H), 4.06 (m, 2H), 3.01 (s, 6H), 2.34-2.22 (m, 2H). |
| **BCD-CDK8-1-11** | 338.2 | $^1H$ NMR (400 MHz, DMSO-$d_6$) $\delta$ 8.53 (s, 1H), 7.77 (d, *J* = 8.4 Hz, 1H), 7.72 (s, 1H), 7.59-7.53 (m, 3H), 7.18 (d, *J* = 8.9 Hz, 2H), 4.45 (s, 1H), 4.28-4.20 (m, 2H), 4.09 (s, 1H), 3.88 (s, 4H), 3.24 (s, 3H). |
| **BCD-CDK8-1-12** | 393.2 | $^1H$ NMR (400 MHz, DMSO-$d_6$) $\delta$ 8.18 (s, 1H), 7.57-7.53 (m, 3H), 7.17 (d, *J* = 8.9 Hz, 2H), 6.49 (dd, *J* = 8.6, 2.1 Hz, 1H), 6.40 (d, *J* = 2.0 Hz, 1H), 4.03 (t, 2H), 3.89 (t, 2H), 3.85 (s, 3H), 3.84-3.77 (m, 1H), 3.61-3.53 (m, 4H), 3.49-3.43 (m, 2H), 3.34 (t, 2H). |
| **BCD-CDK8-1-13** | 424.3 | $^1H$ NMR (400 MHz, DMSO-$d_6$) $\delta$ 8.01 (s, 1H), 7.35 (d, *J* = 8.9 Hz, 2H), 6.87 (d, *J* = 8.8 Hz, 2H), 6.24 (dd, *J* = 12.3, 1.3 Hz, 1H), 6.13 (d, *J* = 1.5 Hz, 1H), 4.07-3.98 (m, 2H), 3.94-3.86 (m, 2H), 3.85-3.74 (m, 1H), 3.62-3.54 (m, 4H), 3.51-3.43 (m, 2H), 3.37-3.30 (m, 2H), 3.02 (s, 6H). |
| **BCD-CDK8-1-14** | 447.2 | $^1H$ NMR (400 MHz, DMSO-$d_6$) $\delta$ 13.03 (s, 1H), 8.30 (s, 1H), 8.24 (s, 1H), 8.03 (s, 1H), 7.79 (d, *J* = 8.4 Hz, 2H), 7.57 (dd, *J* = 8.5, 2.1 Hz, 2H), 7.43 (d, *J* = 8.6 Hz, 1H), 6.60 (t, *J* = 8.4 Hz, 1H), 4.13 (t, 2H), 4.01 (t, 2H), 3.85-3.78 (m, 1H), 3.55 (s, 4H), 3.47-3.45 (m, 2H), 3.33 (s, 2H). |
| **BCD-CDK8-1-15E** | 354.2 | $^1H$ NMR (400 MHz, DMSO-$d_6$) $\delta$ 8.68 (s, 1H), 8.16 (d, *J* = 8.6 Hz, 2H), 7.84 (dd, *J* = 8.6, 2.6 Hz, 2H), 7.68 (d, *J* = 8.4 Hz, 1H), 7.38 (dd, *J* = 8.3, 6.0 Hz, 1H), 4.06-4.00 (m, 4H), 3.92 (s, 3H), 2.29-2.18 (m, 2H). |

(continued)

| Code | ESI-MS. [M+H]+ | $^1$H NMR, $\delta$, MD |
|---|---|---|
| BCD-CDK8-1-15A | 340.2 | $^1$H NMR (400 MHz, DMSO-$d_6$) $\delta$ 8.59 (s, 1H), 8.14 (d, $J$ = 8.5 Hz, 2H), 7.76 (dd, $J$ = 8.5, 2.5 Hz, 2H), 7.64 (d, $J$ = 8.4 Hz, 1H), 7.36 (dd, $J$ = 8.3, 6.0 Hz, 1H), 4.08-4.03 (m, 4H), 2.32-2.24 (m, 2H). |
| BCD-CDK8-1-15 | 339.1 | $^1$H NMR (400 MHz, DMSO-$d_6$) $\delta$ 8.57 (s, 1H), 8.14-8.02 (m, 3H), 7.71 (dd, $J$ = 8.5, 2.4 Hz, 2H), 7.63 (d, $J$ = 8.4 Hz, 1H), 7.44 (s, 1H), 7.38-7.31 (m, 1H), 4.12-3.98 (m, 4H), 2.36-2.21 (m, 2H). |
| BCD-CDK8-1-16 | 386.2 | $^1$H NMR (400 MHz, DMSO-$d_6$) $\delta$ 8.66 (s, 1H), 8.26 (s, 1H), 7.98 (d, $J$ = 0.7 Hz, 1H), 7.83-7.81 (m, 3H), 7.68 (d, $J$ = 8.6 Hz, 2H), 7.59 (d, $J$ = 0.8 Hz, 1H), 7.32 (dd, $J$ = 8.3, 1.4 Hz, 1H), 3.97 (s, 3H), 3.70-3.34 (m, 4H), 1.66-1.41 (m, 6H). |
| BCD-CDK8-1-17 | 443.4 | $^1$H NMR (400 MHz, DMSO-$d_6$) $\delta$ 8.26 (d, $J$ = 13.7 Hz, 2H), 7.97 (s, 1H), 7.80 (d, $J$ = 8.5 Hz, 2H), 7.62 (d, $J$ = 8.5 Hz, 2H), 7.58 (d, $J$ = 8.9 Hz, 1H), 6.54-6.48 (m, 2H), 4.09-4.02 (m, 2H), 3.95-3.88 (m, 5H), 3.86-3.76 (m, 1H), 3.61-3.53 (m, 4H), 3.49-3.44 (m, 2H). |
| BCD-CDK8-1-18 | 358.2 | $^1$H NMR (400 MHz, OMSO-$d_6$) $\delta$ 8.69 (s, 1H), 8.27 (s, 1H), 7.98 (s, 1H), 7.87-7.80 (m, 4H), 7.68 (d, $J$ = 8.6 Hz, 2H), 7.58 (dd, $J$ = 8.5, 1.4 Hz, 1H), 4.39-4.27 (m, 2H), 4.12-4.00 (m, 2H), 3.91 (s, 3H), 2.33-2.21 (m, 2H). |
| BCD-CDK8-1-19 | 461.2 | $^1$H NMR (400 MHz, DMSO-$d_6$) $\delta$ 8.30 (s, 1H), 8.25 (d, $J$ = 0.7 Hz, 1H), 7.97 (d, $J$ = 0.8 Hz, 1H), 7.80 (d, $J$ = 8.6 Hz, 2H), 7.62 (d, $J$ = 8.5 Hz, 2H), 6.36 (dd, $J$ = 12.6, 1.8 Hz, 1H), 6.31 (d, $J$ = 1.8 Hz, 1H), 4.09-4.01 (m, 2H), 3.96-3.90 (m, 2H), 3.90 (s, 3H), 3.87-3.76 (m, 1H), 3.61-3.50 (m, 4H), 3.49-3.40 (m, 2H), 3.36-3.30 (m, 2H). |
| BCD-CDK8-1-20 | 461.3 | $^1$H NMR (400 MHz, OMSO-$d_6$) $\delta$ 8.23 (s, 1H), 8.23 (s, 1H), 7.96 (s, 1H), 7.74 (d, $J$ = 8.5 Hz, 2H), 7.60-7.54 (m, 2H), 7.43 (d, $J$ = 8.6 Hz, 1H), 6.60 (t, $J$ = 8.4 Hz, 1H), 4.17-4.09 (m, 2H), 4.05-3.98 (m, 2H), 3.90 (s, 3H), 3.86-3.77 (m, 1H), 3.60-3.53 (m, 4H), 3.49-3.43 (m, 2H), 3.36 - 3.32 (m, 2H). |
| BCD-CDK8-1-21A | 467.1 | $^1$H NMR (400 MHz, DMSO-$d_6$) $\delta$ 13.17 (s, 1H), 10.10 (s, 1H), 8.32 (s, 1H), 8.12 (d, $J$ = 8.5 Hz, 2H), 7.77 (d, $J$ = 8.4 Hz, 2H), 7.56 (d, $J$ = 7.6 Hz, 2H), 7.27 (t, $J$ = 7.9 Hz, 2H), 7.04 (t, $J$ = 7.4 Hz, 1H), 6.62 (s, 1H), 6.56 (d, $J$ = 13.3 Hz, 1H), 6.17 (t, $J$ = 5.7 Hz, 1H), 3.92-3.74 (m, 2H), 3.53-3.40 (m, 1H), 3.28 (s, 1H), 3.18-3.06 (m, 1H). |
| BCD-CDK8-1-21 | 466.2 | $^1$H NMR (400 MHz, DMSO-$d_6$) $\delta$ 10.10 (s, 1H), 8.30 (s, 1H), 8.11-8.09 (m, $J$ = 8.6 Hz, 3H), 7.74 (d, $J$ = 8.6 Hz, 2H), 7.61-7.55 (m, 2H), 7.49 (s, 1H), 7.32-7.25 (m, 2H), 7.05 (t, $J$ = 7.4 Hz, 1H), 6.61 (d, $J$ = 1.7 Hz, 1H), 6.57 (dd, $J$ = 13.3, 1.6 Hz, 1H), 6.15 (m, 1H), 3.93-3.81 (m, 2H), 3.52-3.41 (m, 1H), 3.29 (s, 1H), 3.20-3.08 (m, 1H). |
| BCD-CDK8-3-1 | 441.1 | $^1$H NMR (400 MHz, OMSO-$d_6$) $\delta$ 9.49 (s, 1H), 9.19 (m, 1H), 8.69 (s, 1H), 8.31 (dd, $J$ = 6.0, 1.1 Hz, 1H), 8.15 (d, $J$ = 8.2 Hz, 2H), 7.57 (dd, $J$ = 8.2, 3.0 Hz, 2H), 5.09, 5.02 (2d, $J$ = 3.8 Hz, 1H, rotamers), 4.33, 4.25 (2s, 1H, rotamers), 3.23-3.59 (m, 4H), 2.91 (s, 3H), 1.83-1.99 (m, 2H). |
| BCD-CDK8-3-2 | 386.1 | $^1$H NMR (400 MHz, OMSO-$d_6$) $\delta$ 13.03 (s, 1H), 9.48 (s, 1H), 9.20 (s, 1H), 8.70 (s, 1H), 8.36 (d, $J$ = 2.9 Hz, 1H), 8.32 (s, 1H), 8.05 (s, 1H), 7.85 (d, $J$ = 7.4 Hz, 2H), 7.59 (dd, $J$ = 8.0, 2.1 Hz, 2H), 5.01 (dd, $J$ = 28.6, 3.6 Hz, 1H), 4.40-4.18 (m, 1H), 3.68-3.14 (m, 4H), 2.06-1.66 (m, 2H). |
| BCD-CDK8-3-3 | 363.1 | $^1$H NMR (400 MHz, DMSO-$d_6$) $\delta$ 9.40 (s, 1H), 9.17 (t, 1H), 8.61 (s, 1H), 8.38 (m, 1H), 7.43 (m, 2H), 6.93 (d, $J$ = 8.8 Hz, 2H), 5.01 (dd, $J$ = 32.7, 3.7 Hz, 1H), 4.30 (d, $J$ = 36.4 Hz, 1H), 3.67-3,20 (m, 4H), 3.01 (s, 6H), 2,01-1,76 (d, 2H). |

(continued)

| Code | ESI-MS. [M+H]+ | 1H NMR, δ, MD |
|---|---|---|
| **BCD-CDK8-3-4** | 400.1 | 1H NMR (400 MHz, OMSO-$d_6$) δ 9.46 (s, 1H), 9.17 (t, *J* = 2.1 Hz, 1H), 8.67 (s, 1H), 8.36 (d, *J* = 4.3 Hz, 1H), 8.22 (d, *J* = 2.1 Hz, 1H), 7.93 (d, *J* = 1.2 Hz, 1H), 7.78 (d, *J* = 7.3 Hz, 2H), 7.57 (dd, *J* = 8.2, 2.2 Hz, 2H), 5.00 (dd, *J* = 25.8, 3.7 Hz, 1H), 4.40-4.21 (m, 1H), 3.91 (s, 3H), 3.67-3.40 (m, 4H), 2.04-1.73 (m, 2H). |
| **BCD-CDK8-3-5** | 405.1 | 1H NMR (400 MHz, DMSO-$d_6$) δ 9.41 (s, 1H), 9.16 (s, 1H), 8.61 (s, 1H), 8.36 (s, 1H), 7.46 (d, *J* = 7.5 Hz, 2H), 7.16 (d, *J* = 8.5 Hz, 2H), 5.13-4.88 (br d, 1H), 4.32 (m, 1H), 3.80 (m, 4H), 3.68-3.38 (m, 5H), 3.26 (m, 4H), 2.04-1.79 (m, 2H). |
| **BCD-CDK8-3-6** | 350.1 | 1H NMR (400 MHz, DMSO-$d_6$) δ 9.46 (s, 1H), 9.19 (t, 1H), 8.66 (s, 1H), 8.31 (m, 1H), 7.55 (m, 2H), 7.18 (d, *J* = 8.4 Hz, 2H), 5.05, 4.97 (2d, *J* = 3.8 Hz, 1H, rotamers), 4.35, 4.25 (2s, 1H, rotamers), 3.87 (s, 3H), 3.65-3.19 (m, 4H), 2.03-1.75 (m, 2H). |
| **BCD-CDK8-3-8** | 376.2 | 1H NMR (400 MHz, DMSO-$d_6$) δ 9.39 (s, 1H), 9.07 (d, *J* = 1.9 Hz, 1H), 8.61 (s, 1H), 8.28 (d, *J* = 1.3 Hz, 1H), 7.43 (d, *J* = 8.7 Hz, 2H), 6.93 (d, *J* = 8.8 Hz, 2H), 3.66 (m, 2H), 3.40 (m, 2H), 3.01 (s, 6H), 2.39 (m, 2H), 2.29 (m, 2H), 2.20 (s, 3H). |
| **BCD-CDK8-3-9** | 455.2 | 1H NMR (400 MHz, DMSO-$d_6$) δ 9.11 (s, 1H), 8.53 (d, *J* = 2.7 Hz, 1H), 8.33 (s, 1H), 8.24 (s, 1H), 7.96 (s, 1H), 7.75 (d, *J* = 8.3 Hz, 2H), 7.54 (d, *J* = 8.3 Hz, 2H), 6.80 (d, *J* = 2.4 Hz, 1H), 4.30-4.21 (m, 2H), 4.20-4.12 (m, 2H), 3.96-3.86 (m, 1H), 3.90 (s, 3H), 3.60-3.52 (m, 4H), 3.51-3.44 (m, 2H), 3.36-3.27 (m, 2H). |
| **BCD-CDK8-3-11** | 439.2 | 1H NMR (400 MHz, DMSO-$d_6$) δ 12.30 (s, 1H), 9.53 (s, 1H), 912 (d, *J* = 1.8 Hz, 2H), 8.71 (s, 1H), 8.12-8.20 (m, 3H), 7.77 (d, *J* = 8.2 Hz, 2H), 3.61 (s, 2H), 3.41 (s, 3H), 3.35 (2H in $H_2O$ signal), 1.61 (d, *J* = 3.9 Hz, 4H), 1.47 (d, *J* = 1.7 Hz, 2H). |
| **BCD-CDK8-3-12** | 454.1 | 1H NMR (400 MHz, OMSO-$d_6$) δ 13.12-11.51 (br s, 1H), 9.19 (s, 1H), 8.58 (d, *J* = 2.6 Hz, 1H), 8.37 (s, 1H), 8.13 (d, *J* = 8.4 Hz, 2H), 7.73 (d, *J* = 8.4 Hz, 2H), 6.68 (d, *J* = 2.4 Hz, 1H), 4.33-4.23 (m, 2H), 4.21-4.11 (m, 2H), 3.98-3.86 (m, 1H), 3.41 (s, 3H), 2.89 (s, 3H), 2.85 (s, 3H). |
| **BCD-CDK8-3-13** | 441.2 | 1H NMR (400 MHz, OMSO-$d_6$) δ 12.25 (br s, 1H), 9.49 (s, 1H), 9.22 - 9.14 (m, 1H), 8.69 (s, 1H), 8.29-8.24 (m, 1H), 8.18 (d, *J* = 7.8 Hz, 2H), 7.79-7.71 (m, 2H), 4.40-4.32, 4.30-4.23 (2m, 1H, rotamers), 3.63-3.16 (m, 4H), 3.40 (s, 3H), 2.05-1.77 (m, 2H). |
| **BCD-CDK8-3-14** | 370.2 | 1H NMR (400 MHz, DMSO-$d_6$) δ 9.49 (d, *J* = 0.6 Hz, 1H), 9.26 (d, *J* = 2.0 Hz, 1H), 8.71 (s, 1H), 8.44 (d, *J* = 1.9 Hz, 1H), 8.28 (s, 1H), 8.00 (d, *J* = 0.5 Hz, 1H), 7.81 (d, *J* = 8.3 Hz, 2H), 7.60 (d, *J* = 8.3 Hz, 2H), 4.36-4.32 (m, 2H), 4.12-4.08 (m, 2H), 3.92 (s, 3H), 2.33-2.26 (m, 2H). |
| **BCD-CDK8-3-15** | 400.2 | 1H NMR (400 MHz, OMSO-$d_6$) δ 9.49 (s, 1H), 9.27 (d, *J* = 1.8 Hz, 1H), 8.71 (s, 1H), 8.46 (d, *J* = 0.9 Hz, 1H), 8.27 (s, 1H), 7.99 (s, 1H), 7.81 (d, *J* = 8.1 Hz, 2H), 7.61 (d, *J* = 8.1 Hz, 2H), 4.50-4.48 (m, 1H), 4.33-4.23 (m, 2H), 4.20-4.18 (m, 1H), 3.95-3.87 (m, 4H), 3.22 (s, 3H). |
| **BCD-CDK8-3-16** | 384.2 | 1H NMR (400 MHz, OMSO-$d_6$) δ 9.48 (s, 1H), 9.21 (s, 1H), 8.69 (s, 1H), 8.37 (s, 1H), 8.26 (s, 1H), 7.98 (s, 1H), 7.87-7.72 (m, 2H), 7.69-7.50 (m, 2H), 3.91 (s, 3H), 3.30-3.36 (m, 4H), 2.00-1.73 (m, 4H). |
| **BCD-CDK8-3-17** | 370.1 | 1H NMR (400 MHz, OMSO-$d_6$) δ 9.44 (d, *J* = 2.5 Hz, 1H), 9.35 (d, *J* = 0.8 Hz, 1H), 8.58 (dd, *J* = 2.5, 0.8 Hz, 1H), 8.56 (s, 1H), 8.27 (s, 1H), 7.99 (d, *J* = 0.8 Hz, 1H), 7.78 (d, *J* = 8.5 Hz, 2H), 7.58 (d, *J* = 8.4 Hz, 2H), 4.00 (t, *J* = 7.0 Hz, 2H), 3.91 (s, 3H), 2.55 (t, *J* = 8.1 Hz, 2H), 2.18-2.06 (m, 2H). |

(continued)

| Code | ESI-MS. $[M+H]^+$ | $^1H$ NMR, $\delta$, MD |
|---|---|---|
| BCD-CDK8-3-18 | 388.2 | $^1H$ NMR (400 MHz, OMSO-$d_g$) $\delta$ 9.51 (s, 1H), 9.46 (d, *J* = 1.7 Hz, 1H), 9.11 (br s, 1H), 8.73 (s, 1H), 8.70 (s, 1H), 8.28 (s, 1H), 8.00 (s, 1H), 7.81 (d, *J* = 8.1 Hz, 2H), 7.62 (d, *J* = 8.1 Hz, 2H), 4.84-4.25 (m, 4H), 3.92 (s, 3H), 3.27 (s, 3H). |
| BCD-CDK8-3-19 | 414.2 | $^1H$ NMR (400 MHz, DMSO) $\delta$ 9.48 (s, 1H), 9.20 (s, 1H), 8.70 (s, 1H), 8.36 (s, 1H), 8.26 (s, 1H), 7.98 (s, 1H), 7.84-7.74 (m, 2H), 7.65-7.55 (m, 2H), 4.08-3.93 (m, 1H), 3.91 (s, 3H), 3.66-3.41 (m, 4H), 3.26 (s, 1.5H), 3.16 (s, 1.5H), 2.05-1.89 (m, 2H). |
| BCD-CDK8-3-20 | 414.2 | - |
| BCD-CDK8-3-21 | 428.3 | $^1H$ NMR (400 MHz, DMSO) $\delta$ 9.48 (s, 1H), 9.10-9.04 (m, 1H), 8.68 (s, 1H), 8.34-8.20 (m, 2H), 7.98 (s, 1H), 7.84-7.76 (m, 2H), 7.63-7.54 (m, 2H), 4.05-3.92 (m, 1H), 3.91 (s, 3H), 3.55-3.15 (m, 4H), 3.31 (in $H_2O$ signal, 3H), 1.89-1.30 (s, 4H). |
| BCD-CDK8-3-22 | 358.2 | $^1H$ NMR (400 MHz, DMSO) $\delta$ 9.48 (s, 1H), 9.12 (d, *J* = 2.0 Hz, 1H), 8.69 (s, 1H), 8.30 - 8.28 (m, 1H), 8.26 (s, 1H), 7.98 (s, 1H), 7.80 (d, *J* = 8.3 Hz, 2H), 7.59 (d, *J* = 8.3 Hz, 2H), 3.91 (s, 3H), 3.02 (s, 3H), 2.97 (s, 3H). |
| BCD-CDK8-3-23 | 386.3 | $^1H$ NMR (400 MHz, DMSO) $\delta$ 9.48 (s, 1H), 9.09 (d, *J* = 2.0 Hz, 1H), 8.69 (s, 1H), 8.26 (s, 1H), 8.21 (d, *J* = 1.1 Hz, 1H), 7.99-7.97 (m, 1H), 7.79 (d, *J* = 8.3 Hz, 2H), 7.59 (d, *J* = 8.3 Hz, 2H), 3.91 (s, 3H), 3.48-3.46 (m, 2H), 3.26-3.25 (m, 2H), 1.19-1.14 (m, 3H), 1.10-1.06 (m, 3H). |
| BCD-CDK8-3-24 | 403.2 | $^1H$ NMR (400 MHz, DMSO) $\delta$ 9.24 (d, *J* = 1.7 Hz, 1H), 9.2 - 9.12 (m, 1H), 8.54 (d, *J* = 1.8 Hz, 1H), 8.23 (s, 1H), 7.93 (s, 2H), 7.71 (d, *J* = 8.1 Hz, 2H), 7.47 (d, *J* = 8.0 Hz, 2H), 7.22 - 7.11 (m, 2H), 3.90 (s, 3H), 3.50 - 3.41 (m, 4H), 3.26 (s, 3H). |
| BCD-CDK8-3-25 | 402.2 | $^1H$ NMR (400 MHz, DMSO) $\delta$ 9.46 (s, 1H), 9.13-9.01 (m, 1H), 8.68 (s, 1H), 8.35-8.26 (m, 2H), 7.98 (s, 1H), 7.80 (d, *J* = 8.2 Hz, 2H), 7.57 (d, *J* = 7.8 Hz, 2H), 3.91 (s, 3H), 3.61-3.54 (m, 4H), 3.38 (s, 3H), 3.01 (m, 4H). |
| BCD-CDK8-3-26 | 385.2 | $^1H$ NMR (400 MHz, DMSO) $\delta$ 8.99 (d, *J* = 2.0 Hz, 1H), 8.28 (d, *J* = 2.0 Hz, 1H), 8.21 (s, 1H), 7.96 (s, 1H), 7.94 (s, 1H), 7.72 (d, *J* = 8.2 Hz, 1H), 7.46 (d, *J* = 8.2 Hz, 1H), 7.17 (br s, 2H), 4.33 (t, *J* = 7.6 Hz, 2H), 4.08 (t, *J* = 7.7 Hz, 2H), 3.90 (s, 3H), 2.35-2.23 (m, 2H). |
| BCD-CDK8-3-27 | 415.2 | $^1H$ NMR (400 MHz, DMSO) $\delta$ 8.86 (d, *J* = 1.7 Hz, 1H), 8.21 (s, 1H), 8.16-8.10 (m, 1H), 7.95 (s, 1H), 7.93 (s, 1H), 7.71 (d, *J* = 8.1 Hz, 2H), 7.46 (d, *J* = 8.1 Hz, 2H), 7.20 (br s, 2H), 3.90 (s, 3H), 3.75-3.36 (m, 8H). |
| BCD-CDK8-3-28 | 389.2 | $^1H$ NMR (400 MHz, DMSO) $\delta$ 9.56 (d, *J* = 0.7 Hz, 1H), 9.49 (d, *J* = 2.0 Hz, 1H), 8.85-8.82 (m, 1H), 8.77 (s, 1H), 8.29 (s, 1H), 8.01 (d, *J* = 0.7 Hz, 1H), 7.82 (d, *J* = 8.3 Hz, 2H), 7.62 (d, *J* = 8.3 Hz, 2H), 4.51-4.46 (m, 2H), 3.92 (s, 3H), 3.77-3.66 (m, 2H), 3.30 (s, 3H). |
| BCD-CDK8-3-29 | 404.2 | $^1H$ NMR (400 MHz, DMSO) $\delta$ 9.22 (d, *J* = 1.9 Hz, 1H), 8.67 (d, *J* = 1.9 Hz, 1H), 8.22 (s, 1H), 8.00 (s, 1H), 7.95 (s, 1H), 7.72 (d, *J* = 8.2 Hz, 2H), 7.47 (d, *J* = 8.2 Hz, 2H), 7.25 (s, 2H), 4.48-4.44 (m, 2H), 3.90 (s, 3H), 3.69-3.65 (m, 2H), 3.29 (s, 3H). |
| BCD-CDK8-3-30 | 207.2$[M+2H]^+/2$, 413.3 | $^1H$ NMR (400 MHz, DMSO) $\delta$ 9.48 (s, 1H), 9.11 (d, *J* = 1.9 Hz, 1H), 8.70 (s, 1H), 8.29-8.24 (m, 2H), 7.98 (s, 1H), 7.80 (d, *J* = 8.2 Hz, 2H), 7.60 (d, *J* = 8.2 Hz, 2H), 3.91 (s, 3H), 3.75-3.6 (m, 2H), 3.5-3.3 (m, 2H), 2.45-2.20 (m, 4H), 2.20 (s, 3H). |

(continued)

| Code | ESI-MS. [M+H]$^+$ | $^1$H NMR, $\delta$, MD |
|---|---|---|
| BCD-CDK8-3-31 | 214.6[M+2H]$^+$/2, 428.2 | $^1$H NMR (400 MHz, DMSO) δ 8.83 (d, *J* = 1.5 Hz, 1H), 8.20 (s, 1H), 8.11 (s, 1H), 7.95 (s, 1H), 7.93 (s, 1H), 7.70 (d, *J* = 8.0 Hz, 2H), 7.46 (d, *J* = 8.1 Hz, 2H), 7.16 (br s, 2H), 3.90 (s, 3H), 3.75-3.55 (m, 2H), 3.51-3.40 (m, 2H), 2.43-2.25 (m, 4H), 2.12 (s, 3H). |
| BCD-CDK8-3-32 | 417.3 | $^1$H NMR (400 MHz, DMSO, 80 °C) δ 8.81 (d, *J* = 1.9 Hz, 1H), 8.11 (s, 1H), 8.10 (d, *J* = 1.8 Hz, 1H), 7.95 (s, 1H), 7.87 (s, 1H), 7.68 (d, *J* = 8.2 Hz, 2H), 7.43 (d, *J* = 8.1 Hz, 2H), 6.88 (br s, 2H), 3.91 (s, 3H), 3.61-3.39 (m, 4H), 3.18 (br s, 3H), 3.01 (s, 3H). |
| BCD-CDK8-3-33 | 400.3 | $^1$H NMR (400 MHz, DMSO) δ 9.48 (s, 1H), 9.13 (d, *J* = 1.9 Hz, 1H), 8.70 (s, 1H), 8.34-8.30 (m, 1H), 8.27 (s, 1H), 7.99 (s, 1H), 7.80 (d, *J* = 8.2 Hz, 2H), 7.60 (d, *J* = 8.2 Hz, 2H), 3.91 (s, 3H), 3.74-3.36 (m, 8H). |
| BCD-CDK8-3-34 | 415.2 | $^1$H NMR (400 MHz, DMSO) δ 8.99 (d, *J* = 1.9 Hz, 1H), 8.29 (d, *J* = 1.9 Hz, 1H), 8.21 (s, 1H), 7.96 (s, 1H), 7.94 (s, 1H), 7.72 (d, *J* = 8.2 Hz, 2H), 7.47 (d, *J* = 8.2 Hz, 2H), 7.18 (s, 2H), 4.52-4.42 (m, 1H), 4.32-4.20 (m, 2H), 4.19-4.10 (m, 1H), 3.90 (s, 3H), 3.90-3.85 (m, 1H), 3.22 (s, 3H). |
| BCD-CDK8-3-35 | 415.3 | $^1$H NMR (400 MHz, DMSO, 80 °C) δ 9.46 (s, 1H), 9.12-9.02 (m, 1H), 8.67 (s, 1H), 8.30-8.22 (m, 1H), 8.17 (s, 1H), 7.91 (s, 1H), 7.78 (d, *J* = 8.3 Hz, 2H), 7.56 (d, *J* = 8.3 Hz, 2H), 3.92 (s, 3H), 3.47-3.25 (m, 2H), 3.00 (s, 3H), 2.50-2.35 (m, 2H), 2.05 (br s, 6H). |
| BCD-CDK8-3-37 | 370.2 | $^1$H NMR (400 MHz, DMSO) δ 9.49 (s, 1H), 9.41 (d, *J* = 2.0 Hz, 1H), 8.97 (d, *J* = 3.8 Hz, 1H), 8.69 (s, 2H), 8.28 (s, 1H), 8.00 (s, 1H), 7.80 (d, *J* = 8.2 Hz, 2H), 7.63 (d, *J* = 8.2 Hz, 2H), 3.92 (s, 3H), 2.91-2.86 (m, 1H), 0.75-0.72 (m, 2H), 0.61-0.59 (m, 2H). |
| BCD-CDK8-3-38 | 385.2 | $^1$H NMR (400 MHz, DMSO) δ 9.14 (d, *J* = 1.8 Hz, 1H), 8.88 (d, *J* = 3.9 Hz, 1H), 8.51 (d, *J* = 1.9 Hz, 1H), 8.22 (s, 1H), 7.96-7.92 (m, 2H), 7.71 (d, *J* = 8.2 Hz, 2H), 7.47 (d, *J* = 8.1 Hz, 2H), 7.15 (s, 2H), 3.91 (s, 3H), 2.89-2.85 (m, 1H), 0.74-0.72 (m, 2H), 0.61-0.59 (m, 2H). |
| BCD-CDK8-3-39 | 384.2 | $^1$H NMR (400 MHz, DMSO) δ 9.50 (s, 1H), 9.46 (d, *J* = 2.0 Hz, 1H), 9.10 (t, *J* = 5.5 Hz, 1H), 8.72 (d, *J* = 1.2 Hz, 1H), 8.69 (s, 1H), 8.28 (s, 1H), 8.00 (s, 1H), 7.81 (d, *J* = 8.3 Hz, 2H), 7.62 (d, *J* = 8.3 Hz, 2H), 3.92 (s, 3H), 3.21-3.17 (m, 2H), 1.08-1.01 (m, 1H), 0.47-0.43 (m, 2H), 0.26-0.22 (m, 2H). |
| BCD-CDK8-3-40 | 399.3 | $^1$H NMR (400 MHz, DMSO) δ 9.19 (d, *J* = 1.8 Hz, 1H), 9.00 (t, *J* = 5.5 Hz, 1H), 8.55 (d, *J* = 1.9 Hz, 1H), 8.21 (s, 1H), 7.97-7.91 (m, 2H), 7.71 (d, *J* = 8.1 Hz, 2H), 7.48 (d, *J* = 8.1 Hz, 2H), 7.16 (s, 2H), 3.90 (s, 3H), 3.18 (t, *J* = 6.2 Hz, 2H), 1.04-1.02 (m, 1H), 0.46-0.43 (m, 2H), 0.25-0.23 (m, 2H). |
| BCD-CDK8-3-41 | 374.2 | $^1$H NMR (400 MHz, DMSO) δ 9.53 (s, 1H), 9.46 (d, *J* = 2.0 Hz, 1H), 9.12-9.03 (m, 1H), 8.72 (s, 1H), 8.69 (d, *J* = 1.1 Hz, 1H), 8.43 (s, 1H), 8.03-7.86 (m, 3H), 7.79 (d, *J* = 8.5 Hz, 2H), 7.18 (s, 1H), 3.52-3.42 (m, 4H), 3.27 (s, 3H). |
| BCD-CDK8-3-42 | 389.2 | $^1$H NMR (400 MHz, DMSO) δ 9.19 (d, *J* = 1.9 Hz, 2H), 8.90 (t, *J* = 5.5 Hz, 1H), 8.54 (d, *J* = 1.9 Hz, 2H), 8.21 (s, 1H), 7.94 (s, 1H), 7.93 (s, 1H), 7.70 (d, *J* = 8.2 Hz, 2H), 7.47 (d, *J* = 8.2 Hz, 2H), 7.19 (s, 2H), 4.80-4.75 (m, 1H), 3.89 (s, 3H), 3.56-3.50 (m, 2H), 3.41-3.35 (m, 2H). |
| BCD-CDK8-4-1 | 359.1 | $^1$H NMR (400 MHz, DMSO-$d_6$) $\delta$ 12.68 (s, 1H), 9.31 (s, 1H), 9.00 (s, 1H), 8.80-8.76 (m, 2H), 8.58 (d, *J* = 2.2 Hz, 1H), 8.12-8.03 (m, 2H), 7.67 (ddd, *J* = 6.8, 4.8, 2.0 Hz, 1H), 7.16 (d, *J* = 1.9 Hz, 1H), 7.13 (dd, *J* = 8.1, 2.0 Hz, 1H). 6.97 (d, *J* = 8.1 Hz, 1H), 2.75 (s, 6H). |

(continued)

| Code | ESI-MS. [M+H]$^+$ | $^1$H NMR, $\delta$, MD |
|---|---|---|
| BCD-CDK8-4-2 | 193.6 386.1 | $^1$H NMR (400 MHz, DMSO-$d_6$) $\delta$ 12.69 - 12.59 (m, 1H), 8.97 (d, *J* = 3.0 Hz, 1H), 8.80 (dt, *J* = 4.7, 1.3 Hz, 1H), 8.65 (d, *J* = 2.2 Hz, 1H), 8.33 (d, *J* = 2.2 Hz, 1H), 8.09 - 8.05 (m, 2H), 7.69 - 7.64 (m, 1H), 7.63 (s, 1H), 7.31 (d, *J* = 8.5 Hz, 1H), 7.26 (s, 1H), 6.94 (dd, *J* = 8.5, 2.6 Hz, 1H), 6.88 (d, *J* = 2.5 Hz, 1H), 3.01 (s, 6H). |
| BCD-CDK8-4-3 | 401.2 | $^1$H NMR (400 MHz, OMSO-$d_6$) $\delta$ 12.69 (s, 1H), 9.36 (s, 1H), 9.00 (s, 1H), 8.81 (s, 1H), 8.80 (s, 1H), 8.59 (d, *J*= 2.2 Hz, 1H), 8.11-8.04 (m, 2H), 7.20 (d, *J* = 2.1 Hz, 1H), 7.16 (dd, *J* = 8.1, 2.1 Hz, 1H), 7.00 (d, *J* = 8.2 Hz, 1H), 3.82-3.72 (m, 4H), 3.08-2.96 (m, 4H). |
| BCD-CDK8-4-4 | 187.1 373.1 | $^1$H NMR (400 MHz, DMSO-$d_6$) $\delta$ 12.68 (s, 1H), 8.99 (s, 1H), 8.81 (s, 1H), 8.67 (s, 1H), 8.34 (s, 1H), 8.15-8.03 (m, 2H), 7.78-7.62 (m, 2H), 7.47-7.29 (m, 2H), 7.18-7.02 (m, 2H), 3.86 (s, 3H). |
| BCD-CDK8-4-5 | 180.1 359.1 | $^1$H NMR (400 MHz, DMSO-$d_6$) $\delta$ 12.78 (s, 1H), 9.31 (s, 1H), 9.00 (dd, *J* = 2.3, 0.8 Hz, 1H), 8.82 (dd, *J* = 4.8, 1.7 Hz, 1H), 8.67 (d, *J* = 2.3 Hz, 1H), 8.60 (d, *J* = 2.3 Hz, 1H), 8.27-8.16 (m, 2H), 7.60 (ddd, *J* = 7.9, 4.9, 0.9 Hz, 1H), 7.18-7.09 (m, 2H), 6.97 (d, *J* = 8.1 Hz, 1H), 2.75 (s, 6H). |
| BCD-CDK8-4-6 | 193.6 386.1 | $^1$H NMR (400 MHz, OMSO-$d_g$) $\delta$ 12.73 (s, 1H), 8.98 (d, *J* = 1.6 Hz, 1H), 8.81 (dd, *J* = 4.8, 1.6 Hz, 1H), 8.51 (d, *J* = 2.2 Hz, 1H), 8.34 (d, *J* = 2.2 Hz, 1H), 8.22-8.17 (m, 2H), 7.65-7.57 (m, 2H), 7.28 (d, *J* = 8.5 Hz, 1H), 7.25 (s, 1H), 6.89 (dd, *J* = 8.6, 2.7 Hz, 1H), 6.83 (d, *J* = 2.7 Hz, 1H), 2.99 (s, 6H). |
| BCD-CDK8-4-7 | 201.1 401.1 | $^1$H NMR (400 MHz, OMSO-$d_g$) $\delta$ 12.85 (d, *J* = 2.4 Hz, 1H), 9.05 (d, *J* = 1.6 Hz, 1H), 8.86 (dd, *J* = 4.9, 1.6 Hz, 1H), 8.69 (d, *J* = 2.2 Hz, 1H), 8.63 (d, *J* = 2.2 Hz, 1H), 8.32 (dt, *J* = 7.9, 1.8 Hz, 1H), 8.27 (d, *J* = 3.0 Hz, 1H), 7.69 (dd, *J* = 8.0, 5.2 Hz, 1H), 7.26-7.18 (m, 2H), 7.16-7.08 (m, 1H), 3.87-3.77 (m, 4H), 3.23-3.05 (m, 4H). |
| BCD-CDK8-4-8 | 373.2 | $^1$H NMR (400 MHz, OMSO-$d_g$) $\delta$ 12.79 (s, 1H), 8.98 (d, *J* = 1.6 Hz, 1H), 8.81 (dd, *J* = 4.8, 1.5 Hz, 1H), 8.54 (d, *J* = 2.1 Hz, 1H), 8.36 (d, *J* = 2.1 Hz, 1H), 8.24-8.18 (m, 2H), 7.71 (s, 1H), 7.60 (dd, *J* = 7.7, 4.9 Hz, 1H), 7.40 (d, *J* = 8.4 Hz, 1H), 7.33 (s, 1H), 7.12 (dd, *J* = 8.5, 2.7 Hz, 1H), 7.07 (d, *J* = 2.6 Hz, 1H), 3.86 (s, 3H). |
| BCD-CDK8-4-9 | 180.2 359.3 | $^1$H NMR (400 MHz, DMSO-$d_6$) $\delta$ 13.51-11.76 (br s, 1H), 9.32 (s, 1H), 8.81 (dd, *J* = 4.3, 1.6 Hz, 2H), 8.66 (d, *J* = 2.2 Hz, 1H), 8.61 (d, *J* = 2.2 Hz, 1H), 8.20 (s, 1H), 7.74 (dd, *J* = 4.4, 1.6 Hz, 2H), 7.18-7.09 (m, 2H), 6.67 (d, *J* = 8.0 Hz, 1H), 2.75 (s, 6H). |
| BCD-CDK8-4-10 | 386.1 | $^1$H NMR (400 MHz, DMSO-*d6*) $\delta$ 12.79 (d, *J* = 2.6 Hz, 1H), 8.81 (d, *J* = 5.9 Hz, 2H), 8.51 (d, *J* = 2.1 Hz, 1H), 8.34 (d, *J* = 2.2 Hz, 1H), 8.17 (d, *J* = 3.2 Hz, 1H), 7.73 (d, *J* = 6.0 Hz, 2H), 7.62 (s, 1H), 7.28 (d, *J* = 8.5 Hz, 1H), 7.25 (s, 1H), 6.89 (dd, *J* = 8.6, 2.7 Hz, 1H), 6.83 (d, *J* = 2.7 Hz, 1H), 2.99 (s, 6H). |
| BCD-CDK8-4-11 | 201.0 401.1 | $^1$H NMR (400 MHz, DMSO-$d_6$) $\delta$ 12.84 (s, 1H), 9.37 (s, 1H), 8.83-8.77 (m, 2H), 8.68 (d, *J* = 2.2 Hz, 1H), 8.62 (d, *J* = 2.2 Hz, 1H), 8.21 (s, 1H), 7.77-7.70 (m, 2H), 7.21-7.13 (m, 2H), 6.99 (d, *J* = 8.2 Hz, 1H), 3.81-3.71 (m, 4H), 3.06-2.99 (m, 4H). |
| BCD-CDK8-4-12 | 373.1 | $^1$H NMR (400 MHz, DMSO-$d_6$) $\delta$ 8.80 (d, *J* = 5.7 Hz, 2H), 8.53 (d, *J* = 2.2 Hz, 1H), 8.36 (d, *J* = 2.2 Hz, 1H), 8.19 (s, 1H), 7.76-7.68 (m, 3H), 7.39 (d, J = 8.4 Hz, 1H), 7.33 (s, 2H), 7.12 (dd, *J* = 8.5, 2.7 Hz, 1H), 7.07 (d, *J* = 2.7 Hz, 1H), 3.86 (s, 3H). |

(continued)

| Code | ESI-MS. $[M+H]^+$ | $^1H$ NMR, $\delta$, MD |
|---|---|---|
| BCD-CDK8-4-13 | 402.1 | $^1H$ NMR (400 MHz, DMSO-$d_6$) $\delta$ 12.67 (s, 1H), 8.53 (d, *J* = 2.1 Hz, 1H), 8.34 (d, *J* = 2.0 Hz, 1H), 8.13 (d, *J* = 3.0 Hz, 1H), 7.72 (s, 1H), 7.52-7.44 (m, 1H), 7.43-7.37 (m, 2H), 7.34 (s, 1H), 7.33-7.30 (m, 1H), 7.20 (dd, *J* = 8.0, 2.3 Hz, 1H), 7.12 (dd, *J* = 8.5, 2.7 Hz, 1H), 7.06 (d, *J* = 2.7 Hz, 1H), 3.85 (2s, 6H). |
| BCD-CDK8-4-21 | 187.1 373.1 | $^1H$ NMR (400 MHz, ONSO-$d_g$) $\delta$ 12.72 (s, 1H), 9.01 (s, 1H), 8.82 (d, *J* = 2.2 Hz, 1H), 8.81 (d, *J* = 4.7 Hz, 1H), 8.66 (d, *J* = 2.1 Hz, 1H), 8.12-8.03 (m, 2H), 7.70-7.64 (m, 1H), 7.27-7.21 (m, 2H), 7.00 (d, *J* = 7.9 Hz, 1H), 3.93 (s, 3H), 2.76 (s, 6H). |
| BCD-CDK8-4-22 | 368.1 | $^1H$ NMR (400 MHz, DMSO-$d_6$) $\delta$ 12.69 (s, 1H), 9.05 (d, *J* = 3.1 Hz, 1H), 8.82-8.74 (m, 2H), 8.44 (d, *J* = 2.3 Hz, 1H), 8.09 (dt, *J* = 8.0, 1.2 Hz, 1H), 8.03 (td, *J* = 7.6, 1.7 Hz, 1H), 7.63 (ddd, *J* = 7.5, 4.7, 1.4 Hz, 1H), 7.49 (d, *J* = 8.5 Hz, 1H), 7.17-7.08 (m, 2H), 3.06 (s, 6H). |
| BCD-CDK8-4-23 | 415.2 | $^1H$ NMR (400 MHz, OMSO-$d_6$) $\delta$ 12.62 (s, 1H), 9.01 (d, *J* = 3.1 Hz, 1H), 8.83 (d, *J* = 2.2 Hz, 1H), 8.79 (d, *J* = 4.5 Hz, 1H), 8.61 (d, *J* = 2.1 Hz, 1H), 8.09 (d, *J* = 7.7 Hz, 1H), 8.04 (td, *J* = 7.6, 1.6 Hz, 1H), 7.69-7.60 (m, 1H), 7.28-7.19 (m, 2H), 7.01 (d, *J* = 8.7 Hz, 1H), 3.95 (s, 3H), 3.82-3.74 (m, 4H), 3.09-3.01 (m, 4H). |
| BCD-CDK8-4-24 | 355.0 | $^1H$ NMR (400 MHz, DMSO-$d_6$) $\delta$ 12.87 (s, 1H), 9.08 (d, *J* = 3.1 Hz, 1H), 8.85-8.78 (m, 2H), 8.52 (d, *J* = 2.1 Hz, 1H), 8.12-8.03 (m, 2H), 7.71-7.65 (m, 2H), 7.61 (d, *J* = 2.7 Hz, 1H), 7.43 (dd, *J* = 8.7, 2.7 Hz, 1H), 3.90 (s, 3H). |
| BCD-CDK8-4-25 | 187.1 373.1 | $^1H$ NMR (400 MHz, DMSO-$d_6$) $\delta$ 12.80 (s, 1H), 9.04-8.94 (m, 1H), 8.82 (dd, *J* = 4.8, 1.7 Hz, 1H), 8.73-8.65 (m, 2H), 8.27-8.19 (m, 2H), 7.61 (ddd, *J* = 7.8, 4.9, 0.9 Hz, 1H), 7.28-7.17 (m, 2H), 7.00 (d, *J* = 8.0 Hz, 1H), 3.93 (s, 3H), 2.76 (s, 6H). |
| BCD-CDK8-4-26 | 368.2 | $^1H$ NMR (400 MHz, OMSO-$d_6$) $\delta$ 12.91 (s, 1H), 9.01 (d, *J* = 1.5 Hz, 1H), 8.82 (dd, *J* = 4.8, 1.6 Hz, 1H), 8.65 (d, *J* = 2.2 Hz, 1H), 8.51 (d, *J* = 2.2 Hz, 1H), 8.30 (s, 1H), 8.26 - 8.20 (m, 1H), 7.61 (ddd, *J* = 7.9, 4.9, 0.6 Hz, 1H), 7.52 (d, *J* = 8.7 Hz, 1H), 7.22 (d, *J* = 2.7 Hz, 1H), 7.16 (dd, *J* = 8.8, 2.8 Hz, 1H), 3.02 (s, 6H). |
| BCD-CDK8-4-27 | 415.1 | $^1H$ NMR (400 MHz, DMSO-$d_6$) $\delta$ 12.83 (s, 1H), 9.00 (d, *J* = 1.7 Hz, 1H), 8.82 (dd, *J* = 4.8, 1.4 Hz, 1H), 8.70 (dd, *J* = 4.2, 2.1 Hz, 2H), 8.29-8.17 (m, 2H), 7.61 (dd, *J* = 7.8, 4.9 Hz, 1H), 7.30-7.22 (m, 2H), 7.03 (d, *J* = 8.0 Hz, 1H), 3.92 (s, 3H), 3.79-3.73 (m, 4H), 3.06 - 2.99 (m, 4H). |
| BCD-CDK8-4-28 | 355.1 | $^1H$ NMR (400 MHz, OMSO-$d_g$) $\delta$ 12.97 (d, *J* = 2.3 Hz, 1H), 9.01 (d, *J* = 1.8 Hz, 1H), 8.82 (dd, *J* = 4.8, 1.5 Hz, 1H), 8.68 (d, *J* = 2.2 Hz, 1H), 8.55 (d, *J* = 2.2 Hz, 1H), 8.34 (d, *J* = 3.1 Hz, 1H), 8.24 (dt, *J* = 7.8, 1.8 Hz, 1H), 7.67 (d, *J* = 8.7 Hz, 1H), 7.64-7.58 (m, 2H), 7.42 (dd, *J* = 8.7, 2.7 Hz, 1H), 3.90 (s, 3H). |
| BCD-CDK8-4-29 | 187.1 373.1 | $^1H$ NMR (400 MHz, DMSO-$d_6$) $\delta$ 12.85 (s, 1H), 8.81 (d, *J* = 3.3 Hz, 2H), 8.74-8.64 (m, 2H), 8.21 (s, 1H), 7.74 (d, *J* = 3.0 Hz, 2H), 7.30-7.18 (m, 2H), 7.00 (d, *J* = 7.5 Hz, 1H), 3.93 (s, 3H), 2.76 (s, 6H). |
| BCD-CDK8-4-30 | 368.2 | $^1H$ NMR (400 MHz, DMSO-$d_6$) $\delta$ 13.00 (s, 1H), 8.85 (d, *J* = 5.6 Hz, 2H), 8.64 (d, *J* = 2.1 Hz, 1H), 8.52 (d, *J* = 2.2 Hz, 1H), 8.28 (d, *J* = 3.1 Hz, 1H), 7.83 (d, *J* = 6.0 Hz, 2H), 7.52 (d, *J* = 8.7 Hz, 1H), 7.22 (d, *J* = 2.7 Hz, 1H), 7.15 (dd, *J* = 8.8, 2.8 Hz, 1H), 3.02 (s, 6H). |
| BCD-CDK8-4-31 | 415.3 | $^1H$ NMR (400 MHz, DMSO-$d_6$) $\delta$ 12.91 (s, 1H), 8.85 (d, *J* = 5.9 Hz, 2H), 8.70 (s, 2H), 8.21 (d, *J* = 2.7 Hz, 1H), 7.85-7.80 (m, 2H), 7.31-7.22 (m, 2H), 7.06 (d, *J* = 8.0 Hz, 1H), 3.94 (s, 3H), 3.82-3.74 (s, 4H), 3.11-3.03 (d, *J* = 9.8 Hz, 4H). |

(continued)

| Code | ESI-MS. $[M+H]^+$ | $^1$H NMR, $\delta$, MD |
|---|---|---|
| BCD-CDK8-4-32 | 355.2 | $^1$H NMR (400 MHz, OMSO-$d_g$) $\delta$ 13.02 (s, 1H), 8.80 (dd, J = 4.4, 1.5 Hz, 2H), 8.67 (d, J = 2.2 Hz, 1H), 8.54 (d, J = 2.3 Hz, 1H), 8.30 (s, 1H), 7.74 (dd, J = 4.5, 1.5 Hz, 2H), 7.66 (d, J = 8.7 Hz, 1H), 7.60 (d, J = 2.7 Hz, 1H), 7.42 (dd, J = 8.7, 2.7 Hz, 1H), 3.89 (s, 3H). |
| BCD-CDK8-4-33 | 330.1 | $^1$H NMR (400 MHz, OMSO-$d_g$) $\delta$ 12.87 (s, 1H), 8.83 - 8.78 (m, 2H), 8.69 (d, *J* = 2.2 Hz, 1H), 8.66 (d, *J* = 2.2 Hz, 1H), 8.22 (d, *J* = 3.1 Hz, 1H), 7.74 (dd, *J* = 4.5, 1.4 Hz, 2H), 7.68 (d, J = 8.7 Hz, 1H), 7.10 (d, J = 8.7 Hz, 1H), 3.83 (s, 3H). |
| BCD-CDK8-4-34 | 372.2 | $^1$H NMR (400 MHz, OMSO-$d_g$) $\delta$ 12.68 (s, 1H), 8.67 (s, 1H), 8.63 (s, 1H), 8.14 (s, 1H), 7.64 (d, *J* = 8.1 Hz, 2H), 7.49 (t, *J* = 7.8 Hz, 1H), 7.42 (d, *J* = 7.3 Hz, 1H), 7.33 (s, 1H), 7.21 (d, *J* = 7.6 Hz, 1H), 7.02 (d, *J* = 7.4 Hz, 2H), 3.85 (s, 3H), 3.01 (s, 6H). |
| BCD-CDK8-4-35 | 384.1 | $^1$H NMR (400 MHz, DMSO-$d_6$) $\delta$ 12.86 (s, 1H), 8.67 (s, 1H), 8.53 (s, 1H), 8.25 (d, *J* = 2.4 Hz, 1H), 7.66 (d, *J* = 8.6 Hz, 1H), $\delta$ 7.61 (d, *J* = 1.8 Hz, 1H), 7.49 (t, *J* = 7.8 Hz, 1H), 7.46-7.40 (m, *J* = 8.0 Hz, 2H), 7.34 (s, 1H), 7.22 (d, *J* = 7.8 Hz, 1H), 3.90 (s, 3H), 3.85 (s, 3H). |
| BCD-CDK8-4-36 | 385.3 | $^1$H NMR (400 MHz, DMSO) $\delta$ 12.60 (br s, 1H), 9.00 (s, 1H), 8.83-8.77 (m, 2H), 8.63 (d, *J* = 2.2 Hz, 1H), 8.12-8.05 (m, 2H), 7.71-7.62 (m, 3H), 7.11 (d, *J* = 8.8 Hz, 2H), 3.85-3.70 (m, 4H), 3.23-3.14 (m, 4H). |
| BCD-CDK8-4-37 | 385.2 | $^1$H NMR (400 MHz, DMSO) $\delta$ 12.78 (s, 1H), 9.00 (d, *J* = 1.7 Hz, 1H), 8.82 (dd, *J* = 4.8, 1.6 Hz, 1H), 8.69 (d, *J* = 2.2 Hz, 1H), 8.65 (d, *J* = 2.2 Hz, 1H), 8.25-8.19 (m, 2H), 7.67-7.57 (m, 3H), 7.10 (d, *J* = 8.8 Hz, 2H), 3.84-3.72 (m, 4H), 3.23-3.14 (m, 4H). |
| BCD-CDK8-4-38 | 385.3 | $^1$H NMR (400 MHz, DMSO) $\delta$ 12.83 (s, 1H), 8.84-8.76 (m, 2H), 8.67 (d, *J* = 9.4 Hz, 2H), 8.20 (s, 1H), 7.80-7.70 (m, 2H), 7.63 (d, *J* = 7.0 Hz, 2H), 7.10 (d, *J* = 7.2 Hz, 2H), 3.85-3.70 (m, 4H), 3.25-3.13 (m, 4H). |
| BCD-CDK8-4-39 | 343.2 | $^1$H NMR (400 MHz, DMSO) $\delta$ 12.74 (s, 1H), 8.99 (d, *J* = 1.5 Hz, 1H), 8.81 (dd, *J* = 4.8, 1.6 Hz, 1H), 8.65 (dd, *J* = 12.0, 2.3 Hz, 2H), 8.23-8.18 (m, 2H), 7.63-7.55 (m, 3H), 6.87 (d, *J* = 8.9 Hz, 2H), 2.97 (s, 6H). |
| BCD-CDK8-4-40 | 343.2 | $^1$H NMR (400 MHz, DMSO) $\delta$ 12.80 (s, 1H), 8.83-8.78 (m, 2H), 8.66 (d, *J* = 2.3 Hz, 1H), 8.64 (d, *J* = 2.3 Hz, 1H), 8.18 (s, 1H), 7.76-7.71 (m, 2H), 7.58 (d, *J* = 8.8 Hz, 2H), 6.87 (d, *J* = 8.9 Hz, 2H), 2.97 (s, 6H). |
| BCD-CDK8-5-1 | 438.2 | $^1$H NMR (400 MHz, OSMO-$d_g$) $\delta$ 12.24 (br s, 1H), 8.99 (d, *J* = 4.3 Hz, 1H), 8.26-8.20 (m, 2H), 8.12 (d, *J* = 8.4 Hz, 2H), 8.00 (s, 1H), 7.92 (d, *J* = 8.4 Hz, 2H), 7.53 (d, *J* = 4.3 Hz, 1H), 3.87-3.73 (m, 2H), 3.38 (s, 3H), 3.19-3.3.05 (m, 2H), 1.75-1.55 (m, 4H), 1.54-1.22 (m, 2H). |
| BCD-CDK8-5-1Cl | 472.1 | $^1$H NMR (400 MHz, OSMO-$d_6$) $\delta$ 12.25 (s, 1H), 8.28 (dd, *J* = 8.8, 2.0 Hz, 1H), 8.15 (d, *J* = 8.8 Hz, 1H), 8.11 (d, *J* = 8.4 Hz, 2H), 7.98 (d, *J* = 1.8 Hz, 1H), 7.90 (d, *J* = 8.4 Hz, 2H), 7.69 (s, 1H), 3.86-3.70 (m, 2H), 3.33 (s, 3H, in H2O signal), 3.22-3.11 (m, 2H), 1.73-1.58 (m, 4H), 1.56-1.24 (m, 2H). |
| BCD-CDK8-5-2i | 361.3 | $^1$H NMR (400 MHz,OSMO-$d_6$) $\delta$ 8.86 (d, *J* = 4.3 Hz, 1H), 8.08 (s, 2H), 7.89 (s, 1H), 7.63 (d, *J* = 7.4 Hz, 2H), 7.37 (d, *J* = 4.3 Hz, 1H), 6.85 (m, 3H), 5.11-4.82 (m, 1H), 4.51-4.03 (m, 1H), 3.80-3.36 (m, 2H), 3.25-2.56 (m, 8H), 2.14-1.58 (m, 2H). |
| BCD-CDK8-5-2 | 362.1 | $^1$H NMR (400 MHz, OMSO-$d_g$) $\delta$ 8.91 (dd, *J* = 4.3, 0.8 Hz, 1H), 8.15-8.07 (m, 2H), 7.90-7.85 (m, 1H), 7.69-7.60 (m, 2H), 7.53-7.44 (m, 1H), 6.91-6.80 (m, 2H), 5.41-4.55 (m, 1H), 4.45-4.36, 4.26-4.16 (2m, rotamers, 1H), 3.81-3.60 (m, 2H), 3.34-3.23 (m, 1H), 3.19-3.09, 2.99-2.90 (2m, rotamers, 1H), 2.98 (s, 6H), 2.11-1.71(m, 2H). |

(continued)

| Code | ESI-MS. [M+H]+ | $^1$H NMR, $\delta$, MD |
|---|---|---|
| BCD-CDK8-5-3i | 403.1 | $^1$H NMR (400 MHz, OSMO-$d_6$) $\delta$ $\delta$ 9.69-8.74 (br s 1H), 9.05 (d, $J$ = 4.3 Hz, 1H), 8.21 (d, $J$ = 0.7 Hz, 2H), 8.01 (s, 1H), 7.82 (d, $J$ = 4.3 Hz, 1H), 7.76 (d, $J$ = 8.8 Hz, 2H), 7.10 (d, $J$ = 8.8 Hz, 2H), 4.06-3.84 (m, 4H), 3.83-3.73 (m, 4H), 3.65-3.30 (m, 4H), 3.14-3.05 (m, 4H). |
| BCD-CDK8-5-3 | 404.2 | $^1$H NMR (400 MHz, OSMO-$d_6$) $\delta$ 8.92 (d, $J$ = 4.3 Hz, 1H), 8.13 (d, $J$ = 1.2 Hz, 2H), 7.90-7.86 (m, 1H), 7.68 (d, $J$ = 8.8 Hz, 2H), 7.50 (d, $J$ = 4.3 Hz, 1H), 7.11 (d, $J$ = 8.9 Hz, 2H), 3.98-3.67 (m, 8H), 3.65-3.34 (m, 2H), 3.27-3.07 (m, 6H). |
| BCD-CDK8-5-4i | 382.2 | $^1$H NMR (400 MHz, OSMO-$d_6$) $\delta$ 13.04 (s, 1H), 8.92 (d, $J$ = 4.3 Hz, 1H), 8.34-7.97 (m, 5H), 7.80 (d, $J$ = 8.3 Hz, 2H), 7.74 (d, $J$ = 8.4 Hz, 2H), 7.43 (d, $J$ = 4.3 Hz, 1H), 7.28, 6.67 (2 brs, 1H), 3.65-3.09 (m, 4H), 1.67-1.36 (m, 6H). |
| BCD-CDK8-5-4 | 383.2 | $^1$H NMR (400 MHz, DMSO-$d_6$) 13.02 (s, 1H), 8.95 (d, $J$ = 4.1 Hz, 1H), 8.37-7.87 (m, 5H), 7.86-7.69 (m, 4H), 7.50 (d, $J$ = 4.1 Hz, 1H), 3.96-3.63 (m, 2H), 3.23-3.00 (m, 2H), 1.78-1.53 (m, 4H), 1.52-1.21 (m, 2H). |
| BCD-CDK8-5-5 | 410.1 | $^1$H NMR (400 MHz, OMSO-$d_6$) $\delta$ 12.26 (s, 1H), 9.00 (d, $J$ = 4.3 Hz, 1H), 8.37 (s, 1H), 8.26-8.18 (m, 2H), 8.13 (d, $J$ = 8.5 Hz, 2H), 7.97 (d, $J$ = 8.5 Hz, 2H), 7.63 (d, $J$ = 4.3 Hz, 1H), 4.26-4.16 (m, 2H), 4.06-3.96 (m, 2H), 3.43 (s, 3H), 2.35-2.22 (m, 2H). |
| BCD-CDK8-5-5Cl | 444.1 | $^1$H NMR (400 MHz, DMSO-$d_6$) $\delta$ 12.27 (s, 1H), 8.31 (s, 1H), 8.28 (d, $J$ = 8.8 Hz, 1H), 8.13 (d, $J$ = 8.8 Hz, 1H), 8.12 (d, $J$ = 8.2 Hz, 2H), 7.94 (d, $J$ = 8.2 Hz, 2H), 7.75 (s, 1H), 4.29-4.13 (m, 2H), 4.13-3.97 (m, 2H), 3.35 (s, 3H), 2.37-2.18 (m, 2H). |
| BCD-CDK8-5-6 | 319.1 | $^1$H NMR (400 MHz, DMSO-$d_6$) $\delta$ 8.89 (d, $J$ = 4.6 Hz, 1H), 8.11 (d, $J$ = 8.8 Hz, 1H), 8.05 (dd, $J$ = 8.8, 1.5 Hz, 1H), 7.96 (d, $J$ = 1.4 Hz, 1H), 7.73 (d, $J$ = 8.6 Hz, 2H), 7.48 (d, $J$ = 4.7 Hz, 1H), 7.09 (d, $J$ = 8.6 Hz, 2H), 3.98 (t, $J$ = 6.8 Hz, 2H), 3.84 (s, 3H), 2.62 (t, $J$ = 7.9 Hz, 2H), 2.34-2.25 (m, 2H). |
| BCD-CDK8-5-7i | 361.2 | $^1$H NMR (400 MHz, DMSO-$d_6$) $\delta$ 8.87 (s, 1H), 8.08 (s, 2H), 7.89 (s, 1H), 7.63 (s, 2H), 7.38 (s, 1H), 7.17-6.47 (s, 1H), 6.85 (d, $J$ = 7.0 Hz, 2H), 5.21-4.79 (m, 1H), 4.52-4.04 (m, 1H), 3.81-4.45 (m, 2H), 3.17-2.65 (m, 2H), 2.97 (s, 6H), 2.05-1.61 (m, 2H). |
| BCD-CDK8-5-7 | 362.2 | $^1$H NMR (400 MHz, OMSO-$d_6$) $\delta$ 8.90 (dd, $J$ = 4.3, 0.7 Hz, 1H), 8.15-8.05 (m, 2H), 7.91-7.83 (m, 1H), 7.69-7.60 (m, 2H), 7.51-7.43 (m, 1H), 6.92-6.80 (m, 2H), 5.11, 4.98 (2d, $J$ = 3.1, 3.4 Hz, rotamers, 1H), 4.45-4.37, 4.25-4.17 (2m, rotamers, 1H), 3.81-3.60 (m, 2H), 3.34-3.23 (m, 1H), 3.18-3.09, 2.99-2.89 (2m, rotamers, 1H), 2.98 (s, 6H), 2.08-1.71 (m, 2H). |
| BCD-CDK8-5-8i | 396.2 | $^1$H NMR (400 MHz, OMSO-$d_6$) $\delta$ 8.91 (d, $J$ = 4.2 Hz, 1H), 8.22 (s, 1H), 8.15 (s, 2H), 8.07 (s, 1H), 7.95 (s, 1H), 7.79-7.69 (m, 4H), 7.41 (d, $J$ = 4.1 Hz, 1H), 7.08 (br s, 1H), 3.90 (s, 3H), 3.48-3.09 (m, 4H), 1.68-1.56 (m, 2H), 1.52-1.36 (m, 2H), 1.31-1.20 (m, 2H). |
| BCD-CDK8-5-8 | 397.1 | $^1$H NMR (400 MHz, OMSO-$d_g$) $\delta$ 8.95 (d, $J$ = 4.3 Hz, 1H), 8.23 (s, 1H), 8.21-8.13 (m, 2H), 7.98-7.89 (m, 2H), 7.81-7.70 (m, 4H), 7.50 (d, $J$ = 4.3 Hz, 1H), 3.90 (s, 3H), 3.86-3.68 (m, 2H), 3.20-3.05 (m, 2H), 1.76-1.57 (m, 4H), 1.53-1.20 (m, 2H). |
| BCD-CDK8-6-1 | 417.4 | $^1$H NMR (400 MHz, DMSO-$d_6$) 8.63 (d, $J$ = 4.4 Hz, 1H), 7.90 (d, $J$ = 9.2 Hz, 1H), 7.63 (dd, $J$ = 9.2, 1.8 Hz, 1H), 7.46 (d, $J$ = 8.5 Hz, 2H), 7.28 (s, 1H), 7.25 (d, $J$ = 4.4 Hz, 1H), 7.19 (d, $J$ = 1.9 Hz, 1H), 7.12 (d, $J$ = 8.5 Hz, 2H), 6.79 (s, 1H), 3.85-3.74 (m, 4H), 3.72 (d, $J$ = 12.4 Hz, 2H), 3.28 - 3.19 (m, 4H), 2.78-2.64 (m, 2H), 2.32-2.19 (m, 1H), 1.86-1.74 (m, 2H), 1.73-1.57 (m, 2H). |

(continued)

| Code | ESI-MS. [M+H]+ | $^1$H NMR, $\delta$, MD |
|---|---|---|
| **BCD-CDK8-6-2** | 440.1 | $^1$H NMR (400 MHz, OMSO-$d_g$) $\delta$ 12.34 (s, 1H), 9.05 (d, *J* = 4.3 Hz, 1H), 8.24-8.07 (m, 3H), 7.99-7.90 (m, 1H), 7.89 (s, 1H), 7.77-7.65 (m, 2H), 7.59 (d, *J* = 4.2 Hz, 1H), 5.11-4.87 (m, 1H), 4.40-4.17 (m, 1H), 4.20-3.99 (m, 1H), 3.69-3.42 (m, 3H), 3.17 (s, 3H), 2.04-1.68 (m, 2H). |
| **BCD-CDK8-6-3** | 229.8 [M+2H]$^{2+}$ /2 458.4 [M+H]$^+$ | $^1$H NMR (400 MHz, OMSO-$d_g$) $\delta$ 8.66 (d, *J* = 4.4 Hz, 1H), 7.90 (d, *J* = 9.3 Hz, 1H), 7.64 (dd, *J* = 9.3, 2.6 Hz, 1H), 7.50 (m, 4H), 7.29 (d, *J* = 4.4 Hz, 1H), 7.21 (s, 1H), 7.04 (d, *J* = 2.5 Hz, 1H), 6.88 (s, 1H), 3.57 (s, 2H), 3,35 (m, 2H, in $H_2O$ signal), 2.96-2.84 (m, 2H), 2.51-2.25 (m, 8H), 2.18 (s, 3H), 2.12-2.01 (m, 2H), 1.52-1.39 (m, 2H), 1.11 (s, 3H). |
| **BCD-CDK8-6-4** | 385.1 | $^1$H NMR (400 MHz, OMSO-$d_g$) $\delta$ 13.00 (br s, 1H), 9.00 (d, *J* = 4.4 Hz, 1H), 8.28-7.90 (m, 2H), 8.21-8.09 (m, 2H), 7.98 (dd, *J* = 8.8, 1.8 Hz, 1H), 7.83 (d, *J* = 8.2 Hz, 2H), 7.55 (dd, *J* = 9.4, 6.4 Hz, 3H), 4.50-4.35 (m, 1H), 4.32-4.18 (m, 2H), 4.16-4.01 (m, 1H), 3.95-3.77 (m, 1H), 3.23 (s, 3H). |
| **BCD-CDK8-6-5** | 410.1 | $^1$H NMR (400 MHz, DMSO-$d_6$) $\delta$ 12.34 (s, 1H), 9.07 (d, *J* = 4.3 Hz, 1H), 8.23-8.11 (m, 3H), 8.06-7.99 (m, 2H), 7.75 (d, *J* = 8.2 Hz, 2H), 7.61 (d, *J* = 4.4 Hz, 1H), 4.27 (t, *J* = 7.5 Hz, 2H), 4.06 (t, *J* = 7.7 Hz, 2H), 3.44 (s, 3H), 2.33-2.21 (m, 2H). |
| **BCD-CDK8-6-6** | 440.4 | $^1$H NMR (400 MHz, MQOO-$d_4$) $\delta$ 9.02 (d, *J* = 4.4 Hz, 1H), 8.24 (d, *J* = 8.6 Hz, 1H), 8.15 (d, *J* = 8.1 Hz, 2H), 7.95 (s, 1H), 7.89 (d, *J* = 8.6 Hz, 1H), 7.74 (d, *J* = 8.1 Hz, 2H), 7.62 (d, *J*= 4.4 Hz, 1H), 3.86-3.45 (m, 8H), 3.42 (s, 3H). |
| **BCD-CDK8-6-7** | 222.8 444.4 | $^1$H NMR (400 MHz, OMSO-$d_6$) $\delta$ 8.67 (d, *J* = 4.4 Hz, 1H), 7.91 (d, *J* = 9.3 Hz, 1H), 7.64 (dd, *J* = 9.4, 2.5 Hz, 1H), 7.56-7.46 (m, 4H), 7.30 (*d, J* = 4.4 Hz, 1H), 7.28 (s, 1H), 7.07 (d, *J* = 2.5 Hz, 1H), 6.79 (s, 1H), 3.76-3.65 (m, 2H), 3.57 (s, 2H), 2.77-2.63 (m, 2H), 2.50-2.30 (m, 8H), 2.30-2,19 (m, 1H), 2.18 (s, 3H), 1.83-1.72 (m, 2H), 1.71-1.56 (m, 2H). |
| **BCD-CDK8-6-8** | 431.3 | $^1$H NMR (400 MHz, OMSO-$d_6$) 8.62 (d, *J* = 4.4 Hz, 1H), 7.88 (d, *J* = 9.3 Hz, 1H), 7.62 (dd, *J* = 9.4, 2.5 Hz, 1H), 7.45 (d, *J* = 8.6 Hz, 2H), 7.24 (d, *J* = 4.4 Hz, 1H), 7.21 (s, 1H), 7.15 (d, *J* = 2.5 Hz, 1H), 7.13 (d, *J* = 8.8 Hz, 2H), 6.89 (s, 1H), 3.83-3.73 (m, 4H), 3.43-3.32 (m, 2H), 3.28-3.18 (m, 4H), 2.99-2.83 (m, 2H), 2.16-2.02 (m, 2H), 1.54-1.40 (m, 2H), 1.12 (s, 3H). |
| **BCD-CDK8-6-9** | 440.2 | $^1$H NMR (400 MHz, OMSO-$d_6$) $\delta$ 9.02 (d, *J* = 4.4 Hz, 1H), 8.20-8.09 (m, 3H), 7.99-7.88 (m, 2H), 7.61-7.51 (m, 3H), 5.03, 4.97 (2d, *J* = 3.3 Hz, rotamers, 1H), 4.36-4.28, 4.27-4.20 (2m, rotamers , 1H), 3.66-3.40 (m, 3H), 3.49-3.32, 3.26-3.19 (2m, rotamers, 1H), 2.96 (s, 3H), 2.04-1.73 (m, 2H). |
| **BCD-CDK8-6-10** | 399.1 | 1H NMR (400 MHz, OMSO-$d_g$) $\delta$ 9.14 (d, J = 4.4 Hz, 1H), 8.35-8.31 (m, 2H), 8.28 (d, J = 8.7 Hz, 1H), 8.12 (dd, J = 8.7, 1.8 Hz, 1H), 8.03 (s, 1H), 7.92 (d, J = 8.2 Hz, 2H), 7.70 (d, J = 8.2 Hz, 2H), 7.67 (d, J = 4.4 Hz, 1H), 4.63-4.54 (m, 1H), 4.44-4.37 (m, 2H), 4.27-4.19 (m, 1H), 4.08 (s, 3H), 4.06-3.99 (m, 1H), 3.39 (s, 3H). |
| **BCD-CDK8-6-11** | 369.1 | $^1$H NMR (400 MHz, DMSO-$d_6$) $\delta$ 9.02 (d, *J*= 4.4 Hz, 1H), 8.29 (s, 1H), 8.19-8.13 (m, 2H), 8.03-7.97 (m, 2H), 7.81 (d, *J* = 8.1 Hz, 2H), 7.61-7.54 (m, 3H), 4.34-4.24 (m, 2H), 4.10-4.03 (m, 2H), 3.91 (s, 3H), 2.34-2.22 (m, 2H). |
| **BCD-CDK8-6-12** | 383.1 | $^1$H NMR (400 MHz, OMSO-$d_6$) $\delta$ 8.98 (d, *J* = 4.4 Hz, 1H), 8.19 (s, 1H), 8.13 (d, *J* = 8.7 Hz, 1H), 8.04 (d, *J* = 1.4 Hz, 1H), 7.93-7.85 (m, 2H), 7.77 (d, *J* = 8.1 Hz, 2H), 7.54 (d, *J* = 8.1 Hz, 2H), 7.51 (d, *J* = 4.4 Hz, 1H), 3.92 (s, 3H), 3.56-3.34 (m, 4H), 1.96-1.78 (m, 4H). |
| **BCD-CDK8-6-13** | 369.1 | $^1$H NMR (400 MHz, DMSO-$d_6$) $\delta$ 9.00 (d, *J* = 4.8 Hz, 1H), 8.30 (m, 3H), 8.19 (d, *J* = 9.1 Hz, 1H), 8.02 (s, 1H), 7.81 (d, *J* = 8.2 Hz, 2H), 7.69-7.60 (m, 3H), 3.95-3.88 (m, 5H), 2.58-2.52 (m, 2H), 2.08 (qv, 2H). |

(continued)

| Code | ESI-MS. $[M+H]^+$ | $^1H$ NMR, $\delta$, MD |
|---|---|---|
| **BCD-CDK8-6-14** | 387.2 | $^1H$ NMR (400 MHz, OMSO-$d_g$) $\delta$ 9.08 (d, $J$ = 4.6 Hz, 1H), 8.83-8.76 (m, 1H), 8.51 (d, $J$ = 1.6 Hz, 1H), 8.29 (s, 1H), 8.26 (dd, $J$ = 8.8, 1.8 Hz, 1H), 8.20 (d, $J$ = 8.8 Hz, 1H), 8.01 (s, 1H), 7.82 (d, $J$ = 8.3 Hz, 2H), 7.65 (d, $J$ = 4.6 Hz, 1H), 7.62 (d, $J$ = 8.3 Hz, 2H), 3.92 (s, 3H), 3.51-3.39 (m, 4H), 3.26 (s, 3H). |

**Example 131.** Determination of chemical stability.

Chemical stability of the compounds described herein was determined in simulated gastric fluid and human blood plasma.

SGF concentrate without enzymes, pH=1,4 (Sigma Ireland, cat#01651) was used as simulated gastric fluid. Initial candidate solution (10 mM in DMSO) was diluted with an SGF working solution to a concentration of 10 $\mu$m (test solution). The test solution was incubated in a dry block thermostat for 2 hours at 37°C. HPLC with Agilent 1200 liquid chromatography system (Agilent, USA) was employed to determine peak areas of the compounds in test samples, said peak areas corresponding to the initial test time (prior to incubation) and the final test time (after incubation in a dry block thermostat for 2 hours at 37°C). Chromatographic analysis was performed in a gradient elution regime with a flow rate of 1 mL/min. Substance amount in % in a sample after thermostatting was determined.

Determination of stability in human blood plasma was performed using pooled human blood plasma taken from ten healthy donors. The initial candidate solution (10 mM in DMSO) was diluted with pooled blood plasma to a concentration of 10 $\mu$m (test solution). The test solution was incubated in a dry block thermostat for 4 hours at 37°C. HPLC with Agilent 1200 liquid chromatography system (Agilent, USA) was employed to determine peak areas of the compounds in test samples, said peak areas corresponding to the initial test time (prior to incubation) and the final test time (after incubation in a dry block thermostat for 4 hours at 37°C), proteins were preliminarily precipitated with acetonitrile. Chromatographic analysis was performed in a gradient elution regime with a flow rate of 1 mL/min. Substance amount in % in a sample after thermostatting was determined.

The stability of the compounds was estimated. The compounds described herein show at least 75% chemical stability, i.e. they are chemically stable in acidic medium of simulated gastric fluide and in human blood plasma (table 2).

**Table 2.** Results of determination of chemical stability of compounds.

| Compound No. | Stability in SGF, % | Stability in blood plasma, % |
|---|---|---|
| **CDK8-1-3** | 100 | 97.7 |
| **CDK8-1-4** | 98.2 | 100 |
| **CDK8-1-5** | 99.5 | 98.1 |
| **CDK8-1-6** | 98.9 | 100 |
| **CDK8-1-8** | 100 | 97.8 |
| **CDK8-1-12** | 98.1 | 98 |
| **CDK8-1-14** | 92.5 | 100 |
| **CDK8-1-14** | 100 | 100 |
| **CDK8-1-17a** | 99.6 | 93.2 |
| **CDK8-1-18** | 100 | 100 |
| **CDK8-1-20** | 99.4 | 100 |
| **CDK8-3-4** | 100 | 100 |
| **CDK8-3-8** | 95.4 | 98 |
| **CDK8-3-9** | 100 | 100 |
| **CDK8-3-14** | 100 | 100 |
| **CDK8-3-15** | 99.3 | 75.1 |
| **CDK8-3-16** | 100 | 100 |

(continued)

| Compound No. | Stability in SGF, % | Stability in blood plasma, % |
|---|---|---|
| **CDK8-3-17** | 100 | 100 |
| **CDK8-3-18** | 100 | 100 |
| **CDK8-4-3** | 100 | 100 |
| **CDK8-4-5** | 99.2 | 100 |
| **CDK8-4-7** | 99.1 | 93.8 |
| **CDK8-4-9** | 99.5 | 100 |
| **CDK8-4-11** | 98.1 | 88.5 |
| **CDK8-4-21** | 100 | 100 |
| **CDK8-4-23** | 100 | 87.4 |
| **CDK8-4-25** | 100 | 100 |
| **CDK8-4-26** | 100 | 100 |
| **CDK8-4-27** | 100 | 100 |
| **CDK8-4-28** | 100 | 100 |
| **CDK8-4-31** | 99.9 | - |
| **CDK8-4-33** | 100 | 100 |
| **CDK8-4-34** | 99.5 | 100 |
| **CDK8-4-36** | 100 | 100 |
| **CDK8-4-37** | 100 | 100 |
| **CDK8-4-38** | 100 | 100 |
| **CDK8-4-39** | 100 | 100 |
| **CDK8-4-40** | 100 | 100 |
| **CDK8-5-4** | 99.9 | 100 |
| **CDK8-5-4i** | 98.8 | 100 |
| **CDK8-5-8** | 100 | 98.2 |
| **CDK8-6-3** | 100 | - |
| **CDK8-6-4** | 100 | 100 |
| **CDK8-6-13** | 99.7 | 98.9 |
| **CDK8-6-14** | 100 | 100 |

| Compound No. | Stability in blood plasma, % |
|---|---|
| **CDK8-3-24** | 89.7 |
| **CDK8-3-26** | 95.8 |
| **CDK8-3-27** | 94.2 |
| **CDK8-3-32** | 100 |
| **CDK8-3-37** | 100 |
| **CDK8-3-42** | 100 |

**Example 132.** Measuring of enzyme stability in human liver microsomes.

Measuring of candidates' enzyme stability enabled estimation of the stability of the present compounds towards the action of enzymes in liver microsomes.

Enzyme degradation rate was measured by incubating the reaction mixture in a dry block thermostat at 37°C, said reaction mixture comprising 0,5 mg/mL of pooled human liver microsomes (XenoTech, USA, cat#H2620), 10 μM compound, 2 mM β-nicotinamide adenine dinucleotide (Carbosynth, UK, cat#NN10871) and 4 mM magnesium chloride in 0,1 M sodium-phosphate buffer, pH=7,4. The reaction was quenched with acetonitrile (100 μL of acetonitrile / 100 μL of the reaction mixture). After quenching, the samples were centrifuged at 10000 rpm for 10 minutes. Supernatant fluid was tested by chromatographic technique using Agilent1200 (Agilent, USA). Chromatographic analysis was performed in a gradient elution regime with a flow rate of 1 mL/min. A graph of the logarithm of substance's peak area as a function of time was made. The dependent factor of the line corresponded to the elimination rate constant K based on which the drug's half-life $t_{1/2}$ and degradation rate CLint were calculated:

$$\text{Elimination rate constant (k)} = (-\text{gradient})$$

$$\text{Half life } (t_{1/2}) \text{ (min)} = \frac{0.693}{k}$$

$$V\ (\mu L/mg) = \frac{\text{volume of incubation } (\mu L)}{\text{protein in the incubation (mg)}}$$

$$\text{Intrinsic Clearance } (CL_{int})\ (\mu L/min/mg\ protein) = \frac{V \times 0.693}{t_{1/2}}$$

Based on the data obtained, candidates' enzyme stability in human liver microsomes was determined. The compounds according to the present invention showed sufficient stability towards the action of human liver microsomes and enzyme degradation rate $Cl_{int}$ of less than 47 μL/min/mg. The results are shown in table 3.

**Table 3.** Results of measurement of enzyme stability of compounds

| Compound No. | Stability in microsomes, Clint, μL/min/mg | Compound No. | Stability in microsomes, Clint, μL/min/mg |
|---|---|---|---|
| **CDK8-1-3** | 24.8 | **CDK8-4-9** | 21.6 |
| **CDK8-1-4** | 18.8 | **CDK8-4-11** | 15.0 |
| **CDK8-1-6** | 23.4 | **CDK8-4-23** | 12.8 |
| **CDK8-1-8** | 23.4 | **CDK8-4-25** | 22.2 |
| **CDK8-1-12** | 3.0 | **CDK8-4-26** | 17.6 |
| **CDK8-1-14** | 12.4 | **CDK8-4-27** | 7.2 |
| **CDK8-1-17a** | 14.4 | **CDK8-4-28** | 6.8 |
| **CDK8-1-18** | 14.6 | **CDK8-4-33** | 21.2 |
| **CDK8-1-20** | 12.4 | **CDK8-4-34** | 24.2 |
| **CDK8-3-8** | 18.0 | **CDK8-4-38** | 20.0 |
| **CDK8-3-9** | 8.8 | **CDK8-4-39** | 23.6 |
| **CDK8-3-14** | 13.6 | **CDK8-4-40** | 35.0 |
| **CDK8-3-15** | 15.2 | **CDK8-5-4** | 37.0 |

(continued)

| Compound No. | Stability in microsomes, Clint, μL/min/mg | Compound No. | Stability in microsomes, Clint, μL/min/mg |
|---|---|---|---|
| **CDK8-3-16** | 27.8 | **CDK8-5-4i** | 19.0 |
| **CDK8-3-17** | 10.7 | **CDK8-6-3** | 18.6 |
| **CDK8-3-18** | 10.4 | **CDK8-6-4** | 16.2 |
| **CDK8-4-3** | 22.0 | **CDK8-6-13** | 22.6 |
| **CDK8-4-5** | 26.6 | **CDK8-6-14** | 11.8 |
| **CDK8-4-7** | 23.2 | - | - |

**Example 133.** Measuring of enzyme stability in human liver S9 fractions.

Measuring of candidates' enzyme stability enabled estimation of the stability of the present compounds towards the action of enzymes in human liver S9 fractions.

Enzyme degradation rate was measured by incubating the reaction mixture in a dry block thermostat at 37°C, said reaction mixture comprising 0,5 mg/mL of pooled human liver S9 fractions (XenoTech, USA, cat#H0610), 10 μM compound, 2 mM β-nicotinamide adenine dinucleotide (Carbosynth, UK, cat#NN10871) and 4 mM magnesium chloride in 0,1 M sodium-phosphate buffer, pH=7,4. The reaction was quenched with acetonitrile (100 μL of acetonitrile / 100 μL of the reaction mixture). After quenching, the samples were centrifuged at 10000 rpm for 10 minutes. Supernatant fluid was tested by chromatographic technique using Agilent1200 (Agilent, USA). Chromatographic analysis was performed in a gradient elution regime with a flow rate of 1 mL/min. A graph of the logarithm of substance's peak area as a function of time was made. The dependent factor of the line corresponded to the elimination rate constant K based on which the drug's half-life $t_{1/2}$ and degradation rate $CL_{int}$ were calculated:

$$\text{Elimination rate constant (k)} = (-\text{gradient})$$

$$\text{Half life } (t_{1/2})\ (\text{min}) = \frac{0.693}{k}$$

$$V\ (\mu L/mg) = \frac{\text{volume of incubation } (\mu L)}{\text{protein in the incubation (mg)}}$$

$$\text{Intrinsic Clearance } (CL_{int})\ (\mu L/min/mg\ \text{protein}) = \frac{V \times 0.693}{t_{1/2}}$$

Based on the data obtained, candidates' enzyme stability in human liver S9 fractions was determined. The compounds according to the present invention showed sufficient stability towards the action of liver S9 fractions and enzyme degradation rate $Cl_{int}$ of less than 13 μL/min/mg. The results are shown in table 4.

**Table 4.** Results of measurement of enzyme stability of compounds

| Compound No. | Stability in S9, $Cl_{int}$, μL/min/mg |
|---|---|
| **CDK8-3-19** | 2.9 |
| **CDK8-3-20** | 6.5 |
| **CDK8-3-21** | 12.9 |
| **CDK8-3-22** | 3.0 |
| **CDK8-3-23** | 9.2 |

(continued)

| Compound No. | Stability in S9, $Cl_{int}$, $\mu$L/min/mg |
|---|---|
| **CDK8-3-24** | 4.6 |
| **CDK8-3-25** | 3.3 |
| **CDK8_3_26** | 3.25 |
| **CDK8_3_27** | 1.55 |
| **CDK8_3_32** | 3.8 |
| **CDK8_3_37** | 10.05 |
| **CDK8_3_39** | 10.3 |
| **CDK8_3_40** | 2.95 |
| **CDK8_3_42** | 0.5 |

**Example 134.** Estimation of permeability through Caco-2 cell monolayer.

Estimation of permeability through Caco-2 cell monolayer enables assessment of the ability of the substances to penetrate through biological membranes by means of both active and passive transport.

Intestinal epithelial cells Caco-2 were grown on filter inserts (pore size 0.4 $\mu$m, BD Falcon with High Density) for 21 days; monolayer integrity was then checked using Lucifer Yellow (Sigma-Aldrich, USA) according to the standard protocol. In A->B transfer ("intestinal lumen" - "blood stream" transfer), solutions of the test substances in buffer, pH 6.5 (HBSS, 10 mM HEPES, 15 mM glucose) at a concentration of 10 $\mu$M were added to the upper chamber; whereas the lower chamber was filled with buffer, pH 7.4 (HBSS, 10 mM HEPES, 15 mM Glucose, 1% BSA). In B->A transfer ("blood stream" - "intestinal lumen" transfer), the upper chamber was filled with buffer, pH 6.5, whereas the solutions of the test substances in buffer, pH 7.4, at a concentration of 10 $\mu$M were added to the lower chamber. A highly permeable substance propranolol was used as a control.

After 2 hours incubation at 37 ° C under an atmosphere containing 5% $CO_2$, the amounts of the test substances in the upper and lower chambers were measured by HPLC using an Agilent1200 HPLC system (Agilent, USA), proteins were preliminarily precipitated with acetonitrile. Chromatographic analysis was performed in a gradient elution regime with a flow rate of 1 mL/min. The peak areas corresponding to the compounds were detected on chromatograms. Based on the peak areas of a compound in the calibration standards, concentration of the compound in the initial solution and in the samples from upper chamber wells and lower chamber wells was measured.

Cellular permeability coefficient *Papp* was calculated by the formula:

$$\boldsymbol{P_{app}} = (\boldsymbol{C_{(t)}} * \mathrm{V})/(\boldsymbol{C_{(0)}} * \mathrm{t} * \mathrm{Area}),$$

where

$P_{app}$ - effective permeability constant, m/s
V - solution volume (in A-> B test - 0.8 ml, in B-> A test - 0.2 ml), ml
Area - membrane surface area (0.33 $cm^2$), $cm^2$
*t* - retention time (7200 sec), sec
$C_{(o)}$ - initial solution concentration, $\mu$M
$C_{(t)}$ solution concentration after 2 hours (in A->B test - concentration in a sample from a lower chamber well, in B->A test - concentration in a sample from an upper chamber well), $\mu$M

The efflux coefficient showed the ability of cells to eliminate the substance from the bloodstream. The value was calculated using the formula:

$$\boldsymbol{efflux} = \boldsymbol{P_{app}}_{\,\mathrm{B-A}} / \boldsymbol{P_{app}}_{\,\mathrm{A-B}},$$

where

$P_{app\ A\text{-}E}$ - the value of permeability direct analysis A→ B;
$P_{app\ B\text{-}A}$ - the value of the permeability of the reverse analysis B→ A.

The compounds of the present invention showed high rate of direct "intestinal lumen" - "blood stream" transport, the efflux coefficient did not exceed 2, which indicates that Pgp transporter does not impose restrictions on substance bioavailability. The results are shown in table 5.

**Table 5**. Results of measurement of permeability through Caco-2 cell monolayer.

| Compound No. | A-B, Papp cm/s | efflux |
|---|---|---|
| **CDK8-1-3** | 64.1 | 0.44 |
| **CDK8-1-8** | 19.7 | 2.9 |
| **CDK8-1-14** | 39.9 | 0.79 |
| **CDK8-3-14** | 39.8 | 1.35 |
| **CDK8-3-18** | 46.9 | 0.9 |
| **CDK8-3-24** | 17.0 | 1.1 |
| **CDK8-3-25** | 29.3 | 0.6 |
| **CDK8_3_26** | 53.7 | 0.3 |
| **CDK8_3_27** | 22.6 | 0.9 |
| **CDK8_3_32** | 24.9 | 0.7 |
| **CDK8_3_37** | 18.9 | 1.0 |
| **CDK8_3_39** | 22.3 | 0.8 |
| **CDK8_3_42** | 11.8 | 1.6 |
| **CDK8-4-5** | 87.6 | 0.12 |
| **CDK8-4-7** | 64.5 | 0.34 |
| **CDK8-4-11** | 13.9 | 1.54 |
| **CDK8-4-21** | 106.1 | 0.09 |
| **CDK8-4-23** | 36.9 | 0.14 |
| **CDK8-4-27** | 84.6 | 0.25 |
| **CDK8-4-28** | 68.9 | 0.03 |
| **CDK8-4-31** | 36.1 | 0.35 |
| **CDK8-4-33** | 13.4 | 1.5 |
| **CDK8-4-37** | 8.63 | 1.08 |
| **CDK8-4-38** | 13.9 | 0.28 |
| **CDK8-6-14** | 64.1 | 0.44 |

**Example 135.** Antiproliferative activity towards CDK8-sensitive cell lines *in vitro*.

Antiproliferative activity of CDK8 inhibitors according to the present invention was measured in a cell-based test on continuous cell cultures MV4-11 (*biphenotypic myelomonocytic leukemia*, ATCC® CRL-9591™), KG-1 (*acute myeloid leukemia*, ATCC® CCL-246™), HL-60 (*acute promyelocytic leukemia*, ATCC® CCL-240™) using a vital stain AlamarBlue (ThermoFisher, #DAL1100). The cells were grown in RPMI-1640 (PanEco, #C330p) medium with addition of 10% FBS (Gibco, #16140-071); washed and resuspended in culture medium with 10% FBS (Gibco, #16140-071) in 96-well culture plates (Corning, #3599) with ≈ 10 $\times 10^3$ cells in 100 μm of medium per well. The compounds in question were dissolved in DMSO and diluted with a medium with 10% FBS (Gibco, #16140-071) to a final concentration ranging form 0 to 100 μM. 50 μM of diluted compounds were then added to each well (final DMSO concentration was less than 1%) and incubated under 5% $CO_2$ atmosphere at 37°C for 120 h. After incubating, 15 μL of AlamarBlue (ThermoFisher,

#DAL1100) reagent was added per well, contents of plates were mixed in an orbital shaker (Biosan, Latvia) and further incubated for 3-5 h at 37°C under 5% $CO_2$ atmosphere. Number of living cells was estimated using a microplate spectrophotometer (Tecan Infinite M200Pro, Switzerland) measuring fluorescent signal at the excitation wavelength (λEx) of 540 nm and emission wavelength (λEm) of 590 nm.

$IC_{50}$ was calculated using Magellan 7.2 software (Tecan, Switzerland) approximating experimental points by four-parameter logistic model with the optimization by Levenberg-Marquardt (table 7, table 7).

The $CC_{50}$ values were determined in the test for General cytotoxicity on HepG2 cells (*hepatocellular carcinoma*, ATCC® HB-8065™). Cells per well were seeded in 96-well plates (Corning, #3599) at a concentration ≈ 20 × $10^3$ cells in 100 μL of medium and incubated for 72 h with added compounds within the range of concentrations of 200 to 0.78 μM. Cell viability was assessed using the above method. The results are given in table 6.

**Table 6.** The results of the assessment of the specific activity of the compounds in the cell-based antiproliferative test using the cell line panel (MV-4-11) and the results of the assessment of the general toxicity using HepG2 cell line. The results are presented as average values of activity obtained in several tests.

| Compound No. | MV-4-11 | Hep-G2 | Compound No. | MV-4-11 | Hep-G2 |
|---|---|---|---|---|---|
| | $IC_{50}$, mkM | $CC_{50}$, mkM | | $IC_{50}$, mkM | $CC_{50}$, mkM |
| **CDK8-1-3** | 2.08 | 59.1 | **CDK8-4-9** | 2.10 | >200 |
| **CDK8-1-4** | 0.70 | >100 | **CDK8-4-11** | 1.33 | >200 |
| **CDK8-1-5** | 1.24 | 81.1 | **CDK8-4-21** | 0.74 | 4.7 |
| **CDK8-1-6** | 1.64 | >100 | **CDK8-4-23** | 0.10 | >100 |
| **CDK8-1-7E** | >2.5 | >100 | **CDK8-4-25** | 0.05 | 4.7 |
| **CDK8-1-8** | 3.92 | >200 | **CDK8-4-26** | 1.28 | >100 |
| **CDK8-1-12** | 3.38 | >100 | **CDK8-4-27** | 0.05 | >100 |
| **CDK8-1-14** | 0.24 | >100 | **CDK8-4-28** | >1 | >200 |
| **CDK8-1-14** | >2.5 | >50 | **CDK8-4-31** | 0.61 | >100 |
| **CDK8-1-17a** | 12.44 | >100 | **CDK8-4-33** | 19.68 | >100 |
| **CDK8-1-18** | 4.80 | >100 | **CDK8-4-34** | 0.14 | 2.6 |
| **CDK8-1-20** | >1 | >200 | **CDK8-4-36** | 0.46 | 17.0 |
| **CDK8-3-4** | 7.51 | 76.4 | **CDK8-4-37** | 0.13 | 117.5 |
| **CDK8-3-8** | 7.52 | >200 | **CDK8-4-38** | 0.39 | >100 |
| **CDK8-3-9** | 10.04 | >20 | **CDK8-4-39** | 0.19 | >100 |
| **CDK8-3-14** | 0.20 | >100 | **CDK8-4-40** | 5.56 | >100 |
| **CDK8-3-15** | 2.45 | >100 | **CDK8-5-4** | 9.56 | 66.1 |
| **CDK8-3-16** | 2.87 | >100 | **CDK8-5-4i** | 4.32 | >100 |
| **CDK8-3-17** | 8.85 | >20 | **CDK8-5-8** | 3.73 | >50 |
| **CDK8-3-18** | 0.19 | >200 | **CDK8-6-3** | 3.18 | >60 |
| **CDK8-4-3** | 1.49 | >200 | **CDK8-6-4** | 7.79 | 66.6 |
| **CDK8-4-5** | 0.62 | 7.7 | **CDK8-6-13** | 19.74 | >200 |
| **CDK8-4-7** | 0.39 | >200 | **CDK8-6-14** | 11.72 | >50 |
| **CDK8-3-19** | 0.912 | >100 | **CDK8_3_28** | 0.871 | 48.4 |
| **CDK8-3-20** | 5.044 | >100 | **CDK8-3-30** | 0.412 | 100.0 |
| **CDK8-3-21** | 0.764 | >100 | **CDK8-3-32** | 0.010 | 20.7 |
| **CDK8-3-22** | 0.432 | >100 | **CDK8-3-33** | 2.059 | 100.0 |
| **CDK8-3-23** | 0.413 | >100 | **CDK8-3-35** | 1.844 | 77.4 |

(continued)

| Compound No. | MV-4-11 | Hep-G2 | Compound No. | MV-4-11 | Hep-G2 |
|---|---|---|---|---|---|
| | $IC_{50}$, mkM | $CC_{50}$, mkM | | $IC_{50}$, mkM | $CC_{50}$, mkM |
| **CDK8-3-24** | 0.099 | >200 | **CDK8-3-37** | 0.022 | 98.2 |
| **CDK8-3-25** | 0.134 | >100 | **CDK8-3-39** | 0.055 | 50.2 |
| **CDK8-3-26** | 0.011 | 4.9 | **CDK8-3-40** | 0.009 | 200.0 |
| **CDK8-3-27** | 0.066 | 19.4 | **CDK8-3-41** | 0.572 | 200.0 |
| - | - | - | **CDK8-3-42** | 0.028 | 15.9 |

**Table 7.** The results of the assessment of the specific activity of the compounds in the cell-based antiproliferative test using the cell line panel (KG-1, HL-60). The results are presented as average values of activity obtained in several tests.

| Compound No. | KG-1 | HL-60 | Compound No. | KG-1 | HL-60 |
|---|---|---|---|---|---|
| | $IC_{50}$, mkM | $IC_{50}$, mkM | | $IC_{50}$, mkM | $IC_{50}$, mkM |
| **CDK8-1-3** | 27.60 | 81.44 | **CDK8-4-27** | 5.21 | >100 |
| **CDK8-1-5** | 4.71 | >60 | **CDK8-4-31** | 21.66 | - |
| **CDK8-1-6** | >10 | 8.18 | **CDK8-4-34** | 0.20 | 0.081 |
| **CDK8-1-14** | 23.25 | 33.94 | **CDK8-4-36** | 1.59 | - |
| **CDK8-1-14** | - | 60.68 | **CDK8-4-37** | 5.00 | - |
| **CDK8-1-18** | 2.13 | >100 | **CDK8-4-38** | 60.00 | - |
| **CDK8-3-14** | 0.05 | 69.76 | **CDK8-4-39** | 27.72 | - |
| **CDK8-3-15** | 1.13 | >60 | **CDK8-4-40** | 25.00 | - |
| **CDK8-3-16** | 0.94 | >60 | **CDK8-5-8** | 17.52 | 24.79 |
| **CDK8-3-17** | 3.93 | 46.48 | **CDK8-6-13** | 12.32 | >60 |
| **CDK8-3-18** | 0.08 | 34.98 | **CDK8-6-14** | 9.60 | 66.45 |
| | | | | | |
| **Compound No.** | **KG-1** | | | | |
| | $IC_{50}$, mkM | | | | |
| **CDK8-3-26** | 0.003 | | | | |
| **CDK8-3-31** | 0.002 | | | | |
| **CDK8-3-32** | <0.01 | | | | |
| **CDK8-3-34** | 0.005 | | | | |
| **CDK8-3-37** | 0.004 | | | | |
| **CDK8-3-38** | <0.01 | | | | |
| **CDK8-3-39** | 0.005 | | | | |
| **CDK8-3-42** | 0.009 | | | | |

## Claims

1. A compound of formula I:

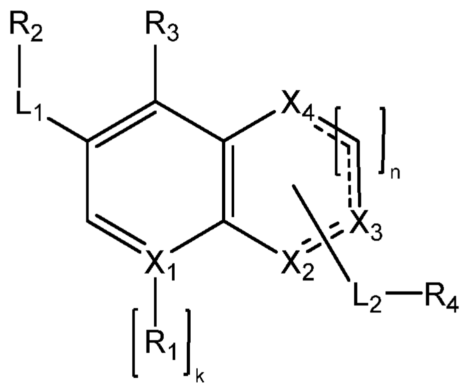

I,

or pharmaceutically acceptable salt or stereoisomer thereof,
wherein $X_1$ is N, C, CH;

each $X_2$, $X_3$, $X_4$ is independently $C(H)_m$, NH, N, $CR_{13}$, $CHR_{13}$;
each $L_1$, $L_2$ is independently a chemical bond, $-C(R_{6b})_2-$, -O-, -C(O)-, -C(O)-O-, -NH-, $-C(=NR_{19})-$;
each n, k independently is selected from 0, 1;
m is 0, 1, 2;
each $R_1$, $R_3$, $R_{13}$ is independently H, Hal, cyano, $C_1$-$C_6$ alkyl, $NH_2$;
each $R_2$, $R_4$ independently is selected from group, consisting of:

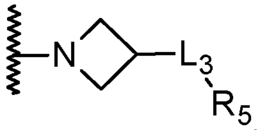
,
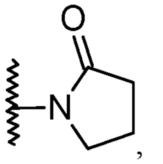
,
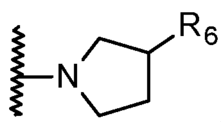
,
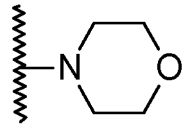
,

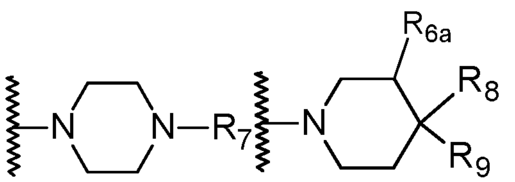
,
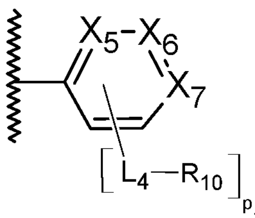
,

$-NR_{11}R_{12}$; $C_1$-$C_6$ alkyl, unsubstituted or substituted by one or several substitutes $R_{14}$;
each $X_5$, $X_6$, $X_7$ is independently C, CH, N;
each $L_3$, $L_4$ is independently chemical bond, -C(O)-, -O-, $-CH_2-$, -NH-, $-C(O)-NR_{7a}-$, -C(=NH)-;
p = 0, 1, 2, 3, 4;
$R_5$ is H; Hal; cyano; $C_1$-$C_6$ alkyl; $C_1$-$C_6$ alkyloxy; $C_1$-$C_6$ alkyloxy $C_1$-$C_6$ alkyl; $NR_{15}R_{16}$; aryl, unsubstituted or substituted by one or several substituents, selected from group, consisting of Hal, $C_1$-$C_6$ alkyl, $C_1$-$C_6$ alkoxy, $NR_{15}R_{16}$; 5-6 membered heterocyclyl with 1-2 heteroatoms, selected from N and/or O, unsubstituted or substituted by one or several substituents, selected from group, consisting of, Hal, $C_1$-$C_6$ alkyl, $C_1$-$C_6$ alkyloxy, $NR_{15}R_{16}$;
each $R_6$, $R_{6a}$, $R_{6b}$ is independently H, Hal, hydroxy, $C_1$-$C_6$ alkyl, $C_1$-$C_6$ alkyloxy; each $R_7$, $R_{7a}$ is independently H, $C_1$-$C_6$ alkyl;
each $R_8$, $R_9$ is independently H, $C_1$-$C_6$ alkyl, $-C(O)-NR_{21}R_{22}$, -CN, $-C(O)-OR_{20}$; or $R_8$ and $R_9$ together with the carbon atom they are attached to, form 5-6-membered heterocyclic ring with 1-2 heteroatoms, selected from nitrogen and/or oxygen, wherein heterocyclic ring, formed by $R_8$ and $R_9$, could be unsubstituted or substituted by 1 or 2 substituents, selected from oxo group, $C_1$-$C_6$ alkyl;
each $R_{10}$ independently is selected from group, consisting of H, Hal, $C_1$-$C_6$ alkyl, hydroxy, cyano, $C_1$-$C_6$ alkyloxy, $C_1$-$C_6$ alkyloxy $C_1$-$C_6$ alkyl, $-NR_{23}R_{24}$; 5-6 membered heterocyclyl with 1-2 heteroatoms, selected from N, O and/or S, unsubstituted or substituted by one or several $C_1$-$C_6$ alkyl; 5-6 membered heteroaryl with 1-2 heteroatoms, selected from N, O and/or S, unsubstituted or substituted by one or several $C_1$-$C_6$ alkyl; -$S(O)_2$-$C_1$-$C_6$ alkyl;

each $R_{11}$, $R_{12}$ is independently H; $C_1$-$C_6$ alkyl unsubstituted or substituted by hydroxy, $C_3$-$C_6$ cycloalkyl, -$NR_{23a}R_{24a}$; $C_1$-$C_6$ alkoxy $C_1$-$C_6$ alkyl; $C_3$-$C_6$ cycloalkyl;

each $R_{14}$ is independently Hal, -C(O)$NR_{17}R_{18}$, $C_1$-$C_6$ alkoxy;

each $R_{15}$, $R_{16}$, $R_{19}$, $R_{20}$, $R_{21}$, $R_{22}$, $R_{23}$, $R_{24}$, $R_{23a}$, $R_{24a}$ is independently H, $C_1$-$C_6$ alkyl;

each $R_{17}$, $R_{18}$ is independently H, $C_1$-$C_6$ alkyl; aryl, unsubstituted or substituted by one or several substituents selected from group consisting of Hal, $C_1$-$C_6$ alkyl, $C_1$-$C_6$ alkoxy;

each ------ independently is a single bond or a double bond;

Hal is chloro, bromo, iodo, fluoro;

2. The compound according to claim 1, wherein the compound of formula I is a compound of formula **I.1**:

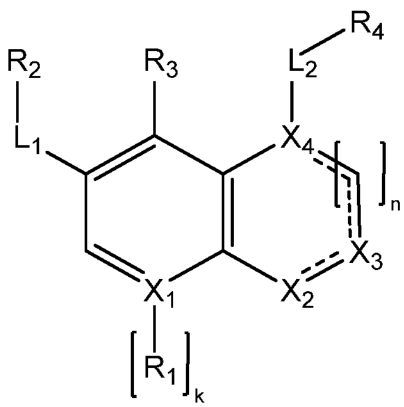

**I.1**,

wherein $X_4$ is $C(H)_m$, N;

m is 0, 1;

$X_1$ is N, C, CH;

each $X_2$, $X_3$ is independently $C(H)_m$, NH, N, $CR_{13}$, $CHR_{13}$;

each $L_1$, $L_2$ is independently chemical bond, -$C(R_{6b})_2$-, -O-, -C(O)-, -C(O)-O-, -NH-, -C(=$NR_{19}$)-;

each n, k independently is selected from 0, 1;

each $R_1$, $R_3$, $R_{13}$ is independently H, Hal, cyano, $C_1$-$C_6$ alkyl, $NH_2$;

each $R_2$, $R_4$ independently is selected from group, consisting of:

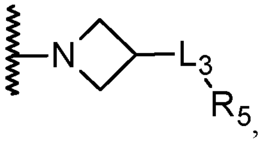, 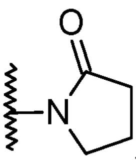, 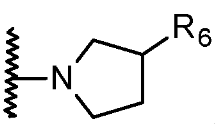, 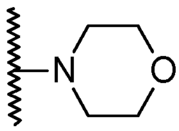,

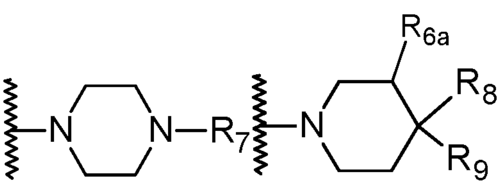, 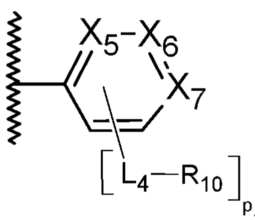,

-$NR_{11}R_{12}$; $C_1$-$C_6$ alkyl, unsubstituted or substituted by one or several substituents $R_{14}$;

each $X_5$, $X_6$, $X_7$ is independently C, CH, N;

each $L_3$, $L_4$ is independently chemical bond, -C(O)-, -O-, -$CH_2$-, -NH-, -C(O)-$NR_{7a}$ -, -C(=NH)-;

p = 0, 1, 2, 3, 4;

$R_5$ is H; Hal; cyano; $C_1$-$C_6$ alkyl; $C_1$-$C_6$ alkyloxy; $C_1$-$C_6$ alkyloxi $C_1$-$C_6$ alkyl; $NR_{15}R_{16}$; aryl, unsubstituted or substituted by one or several substituents, selected from group, consisting of Hal, $C_1$-$C_6$ alkyl, $C_1$-$C_6$ alkyloxy, $NR_{15}R_{16}$; 5-6 membered heterocyclyl c 1-2 heteroatoms, selected from N and/or O, unsubstituted or substituted by one or several substituents, selected from group, consisting of Hal, $C_1$-$C_6$ alkyl, $C_1$-$C_6$ alkyloxy, $NR_{15}R_{16}$;
each $R_6$, $R_{6a}$, $R_{6b}$ is independently H, Hal, hydroxy, $C_1$-$C_6$ alkyl, $C_1$-$C_6$ alkyloxy; each $R_7$, $R_{7a}$ is independently H, $C_1$-$C_6$ alkyl;
each $R_8$, $R_9$ is independently H, $C_1$-$C_6$ alkyl, -C(O)-$NR_{21}R_{22}$, -CN, -C(O)-$OR_{20}$; or
$R_8$ and $R_9$ together with the carbon atom they are attached to, form 5-6-membered heterocyclic ring with 1-2 heteroatoms, selected from nitrogen and/or oxygen, wherein heterocyclic ring, formed by $R_8$ and $R_9$, could be unsubstituted or substituted by 1 or 2 substituents, selected from oxo group, $C_1$-$C_6$ alkyl;
each $R_{10}$ independently is selected from group, consisting of H, Hal, $C_1$-$C_6$ alkyl, hydroxy, cyano, $C_1$-$C_6$ alkyloxy, $C_1$-$C_6$ alkyloxy $C_1$-$C_6$ alkyl, -$NR_{23}R_{24}$; 5-6 membered heterocyclyl with 1-2 heteroatoms, selected from N, O and/or S, unsubstituted or substituted by one or several $C_1$-$C_6$ alkyl; 5-6 membered heteroaryl with 1-2 heteroatoms, selected from N, O and/or S, unsubstituted or substituted by one or several $C_1$-$C_6$ alkyl; -$S(O)_2$-$C_1$-$C_6$ alkyl;
each $R_{11}$, $R_{12}$ is independently H; $C_1$-$C_6$ alkyl unsubstituted or substituted by hydroxy, $C_3$-$C_6$ cycloalkyl, -$NR_{23a}R_{24a}$; $C_1$-$C_6$ alkoxy $C_1$-$C_6$ alkyl; $C_3$-$C_6$ cycloalkyl;
each $R_{14}$ is independently Hal, -C(O)$NR_{17}R_{18}$, $C_1$-$C_6$ alkoxy;
each $R_{15}$, $R_{16}$, $R_{19}$, $R_{20}$, $R_{21}$, $R_{22}$, $R_{23}$, $R_{24}$, $R_{23a}$, $R_{24a}$ is independently H, $C_1$-$C_6$ alkyl;
each $R_{17}$, $R_{18}$ is independently H, $C_1$-$C_6$ alkyl; aryl, unsubstituted or substituted by one or several substituents selected from group consisting of Hal, $C_1$-$C_6$ alkyl, $C_1$-$C_6$ alkoxy;
each ------ independently is a single bond or a double bond;
Hal is chloro, bromo, iodo, fluoro.

**3.** The compound according to any one of claims 1-2, wherein each $L_1$, $L_2$ is independently a chemical bond, -C(O)-, -C(O)-O-, -NH-, -C(=NH)-.

**4.** The compound according to any one of claims 1-2, wherein $R_5$ is H; Hal; cyano; $C_1$-$C_6$ alkyl; $C_1$-$C_6$ alkyloxy; $C_1$-$C_6$ alkyloxy $C_1$-$C_6$ alkyl; $NR_{15}R_{16}$; aryl, wherein aryl is phenyl, unsubstituted or substituted by one or several substituents, selected from group, consisting of Hal, $C_1$-$C_6$ alkyl, $C_1$-$C_6$ alkyloxy, $NR_{15}R_{16}$; 5-6 membered heterocyclyl with 1-2 heteroatoms, selected from N and/or O, wherein heterocyclyl is 4-morpholinyl, 1-piperidinyl, 1-pyrrolidinyl, 1-piperazinyl, unsubstituted or substituted by one or several substituents, selected from group, consisting of Hal, $C_1$-$C_6$ alkyl, $C_1$-$C_6$ alkyloxy, $NR_{15}R_{16}$;
each $R_{15}$, $R_{16}$ is independently H, $C_1$-$C_6$ alkyl.

**5.** The compound according to any one of claims 1-2, wherein $R_{10}$ is independently selected from group, consisting of H, Hal, $C_1$-$C_6$ alkyl, hydroxy, cyano, $C_1$-$C_6$ alkyloxy, $C_1$-$C_6$ alkyloxy $C_1$-$C_6$ alkyl, -$NR_{23}R_{24}$; 5-6 membered heterocyclyl with 1-2 heteroatoms, selected from N, O and/or S, wherein heterocyclyl is 4-morpholinyl, 1-piperidinyl, 1-pyrrolidinyl, 1-piperazinyl, unsubstituted or substituted by one or several $C_1$-$C_6$ alkyl; 5-6 membered heteroaryl with 1-2 heteroatoms, selected from N, O and/or S, wherein heteroaryl is thienyl, imidazolyl, pyrazolyl, unsubstituted or substituted by one or several $C_1$-$C_6$ alkyl; -$S(O)_2$-$C_1$-$C_6$ alkyl;
each $R_{23}$, $R_{24}$ is independently H, $C_1$-$C_6$ alkyl.

**6.** The compound of claim 1, wherein the compound of formula I is a compound of formula **Ia**:

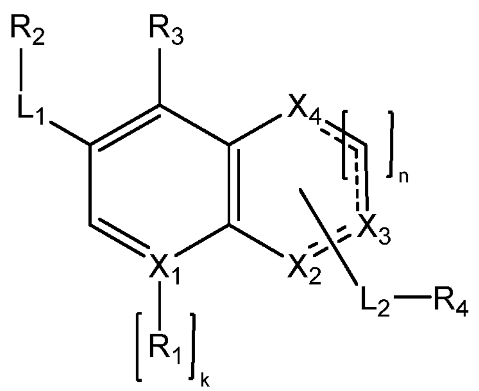

**Ia**,

or pharmaceutically acceptable salt or stereoisomer thereof,
wherein $X_1$ is N, C, CH;

each $X_2$, $X_3$, $X_4$ is independently $C(H)_m$, NH, N, $CR_{13}$;
each $L_1$, $L_2$ is independently chemical bond, $-C(R_6)_2-$, -O-, -C(O)-, -NH-, $-C(=NR_{19})-$;
each n, k independently is selected form 0, 1;
m is 0, 1, 2;
each $R_1$, $R_3$, $R_{13}$ is independently H, Hal, cyano, $C_1$-$C_6$ alkyl;
each $R_2$, $R_4$ independently is selected from group, consisting of:

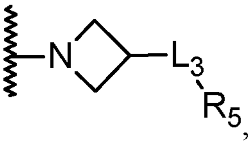, 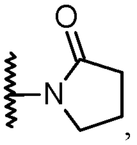, 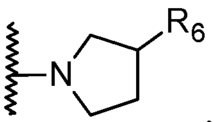, 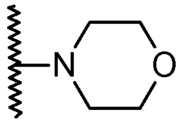,

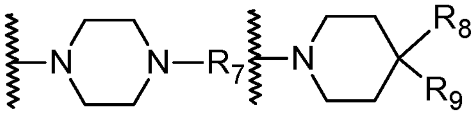, 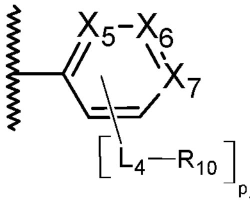,

$-NR_{11}R_{12}$; $C_1$-$C_6$ alkyl, unsubstituted or substituted by one or several substituents $R_{14}$;
each $X_5$, $X_6$, $X_7$ is independently C, CH, N;
each $L_3$, $L_4$ is independently chemical bond, -C(O)-, -O-, $-CH_2-$, -NH-, $-C(O)-NR_7-$, -C(=NH)-;
p = 0, 1, 2, 3, 4;
$R_5$ is H; Hal; cyano; $C_1$-$C_6$ alkyl; $C_1$-$C_6$ alkyloxy; $C_1$-$C_6$ alkyloxy $C_1$-$C_6$ alkyl; $NR_{15}R_{16}$; aryl, unsubstituted or substituted by one or several substituents, selected from group, consisting of Hal, $C_1$-$C_6$ alkyl, $C_1$-$C_6$ alkyloxy, $NR_{15}R_{16}$; 5-6 membered heterocyclyl with 1-2 heteroatoms, selected from N and/or O, unsubstituted or substituted by one or several substituents, selected from group, consisting of Hal, $C_1$-$C_6$ alkyl, $C_1$-$C_6$ alkyloxy, $NR_{15}R_{16}$
each $R_6$ is independently H, Hal, hydroxy, $C_1$-$C_6$ alkyloxy;
each $R_7$ is independently H, $C_1$-$C_6$ alkyl;
each $R_8$, $R_9$ is independently H, $C_1$-$C_6$ alkyl, $-C(O)-NR_{21}R_{22}$, -CN, $-C(O)-OR_{20}$; or $R_8$ and $R_9$ together with the carbon atom they are attached to, form 5-6-membered heterocyclic ring with 1-2 heteroatoms, selected from nitrogen and/or oxygen, wherein heterocyclic ring, formed by $R_8$ and $R_9$, could be unsubstituted or substituted by 1 or 2 substituents, selected from oxo group, $C_1$-$C_6$ alkyl;
each $R_{10}$ independently is selected from group, consisting of H, Hal, $C_1$-$C_6$ alkyl, hydroxy, cyano, $C_1$-$C_6$ alkyloxy, $C_1$-$C_6$ alkyloxy $C_1$-$C_6$ alkyl, $-NR_{23}R_{24}$; 5-6 membered heterocyclyl with 1-2 heteroatoms, selected from N, O and/or S, unsubstituted or substituted by one or several $C_1$-$C_6$ alkyl; 5-6 membered heteroaryl with 1-2 heteroatoms, selected from N, O and/or S, unsubstituted or substituted by one or several $C_1$-$C_6$ alkyl; $-S(O)_2-C_1-C_6$ alkyl;
each $R_{11}$, $R_{12}$ is independently H, $C_1$-$C_6$ alkyl, $C_1$-$C_6$ alkoxy, $C_1$-$C_6$ alkyloxy $C_1$-$C_6$ alkyl;
each $R_{14}$ is independently Hal, $-C(O)NR_{17}R_{18}$;
each $R_{15}$, $R_{16}$, $R_{21}$, $R_{22}$, $R_{23}$, $R_{24}$ is independently H, $C_1$-$C_6$ alkyl;
each $R_{17}$, $R_{18}$ is independently H, $C_1$-$C_6$ alkyl; aryl, unsubstituted or substituted by one or several substituents selected from group consisting of Hal, $C_1$-$C_6$ alkyl, $C_1$-$C_6$ alkyloxy;
each $R_{19}$, $R_{20}$ is independently H, $C_1$-$C_6$ alkyl;
each ------ independently is a single bond or a double bond;
Hal is chloro, bromo, iodo, fluoro;

**7.** The compound of claim 6, which is a compound of formula Ia.1:

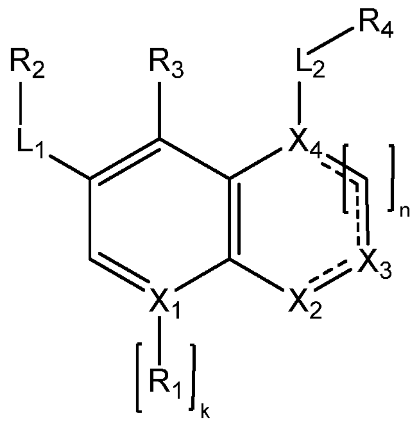

**Ia.1**,

wherein $X_4$ is $C(H)_m$, NH, N;

m is 0, 1;
$X_1$, $X_2$, $X_3$, $L_1$, $L_2$, $R_1$, $R_2$, $R_3$, $R_4$, $R_{13}$, n, k, ------ have the above meanings.

**8.** The compound according to any one of claims 1-7, wherein each $R_3$ is independently H, Hal.

**9.** The compound according to any one of claims 1-2, wherein the compound is:

1-(1-(4-((Methylsulfonyl)carbamoyl)phenyl)-1*H*-benzo[*d*]imidazol-6-yl)-*N*-phenylazetidine-3-carboxamide (BCD-CDK8-1-1)
4-(6-(Azetidine-1-carbonyl)-1*H*-benzo[d]imidazol-1-yl)-*N*-(methylsulfonyl) benzamide (BCD-CDK8-1-2)
(1-(4-(1*H*-Pyrazol-4-yl)phenyl)-1*H*-benzo[*d*]imidazol-6-yl)(piperidin-1-yl) methanone (BCD-CDK8-1-3)
(1-(1-(4-(1*H*-pyrazol-4-yl)phenyl)-1*H*-benzo[*d*]imidazol-6-yl)azetidin-3-yl) (morpholino)methanone (BCD-CDK8-1-4)
(1-(4-(1*H*-Pyrazol-4-yl)phenyl)-1*H*-benzo[*d*]imidazol-6-yl)(azetidin-1-yl) methanone (BCD-CDK8-1-5)
Methyl 4-(6-(3-(phenylcarbamoyl)azetidin-1-yl)-1*H*-benzo[*d*]imidazol-1-yl) benzoate (BCD-CDK8-1-6E)
4-(6-(3-(Phenylcarbamoyl)azetidin-1-yl)-1*H*-benzo[*d*]imidazol-1-yl)benzoic acid (BCD-CDK8-1-6A)
1-(1-(4-Carbamoylphenyl)-1*H*-benzo[*d*]imidazol-6-yl)-*N*-phenylazetidine-3-carboxamide (BCD-CDK8-1-6)
Methyl-4-(6-(3-(phenylcarbamoyl)azetidin-1-yl)-4-fluoro-1*H*-benzo[*d*]imidazol-1-yl)benzoate (BCD-CDK8-1-7E)
4-(-6-(3-(phenylcarbamoyl)azetidin-1-yl)-4-Fluoro-1*H*-benzo[*d*]imidazol-1-yl) benzoic acid (BCD-CDK8-1-7A)
1-(1-(4-Carbamoylphenyl)-4-fluoro-1*H*-benzo[*d*]imidazol-6-yl)-*N*-phenylazetidine-3-carboxamide (BCD-CDK8-1-7)
(1-(1-(4-(1*H*-Pyrazol-4-yl)phenyl)-4-fluoro-1*H*-benzo[*d*]imidazol-6-yl)azetidin-3-yl)(morpholino)methanone (BCD-CDK8-1-8)
(1-(1-(4-(Dimethylamino)phenyl)-1*H*-benzo[*d*]imidazol-6-yl)azetidin-3-yl) (morpholino)methanone (BCD-CDK8-1-9)
Azetidin-1-yl(1-(4-(dimethylamino)phenyl)-1*H*-benzo[*d*]imidazol-6-yl)methanone (BCD-CDK8-1-10)
(3-Methoxyazetidin-1-yl)(1-(4-methoxyphenyl)-1*H*-benzo[*d*]imidazol-6-yl) methanone (BCD-CDK8-1-11)
(1-(1-(4-Methoxyphenyl)-1*H*-benzo[*d*]imidazol-6-yl)azetidin-3-yl)(morpholino) methanone (BCD-CDK8-1-12)
(1-(1-(4-(Dimethylamino)phenyl)-4-fluoro-1*H*-benzo[*d*]imidazol-6-yl)azetidin-3-yl)(morpholino)methanone (BCD-CDK8-1-13)
(1-(1-(4-(1*H*-Pyrazol-4-yl)phenyl)-7-fluoro-1*H*-benzo[*d*]imidazol-6-yl)azetidin-3-yl)(morpholino)methanone (BCD-CDK8-1-14)
Methyl-4-(6-(azetidine-1-carbonyl)-7-fluoro-1*H*-benzo[*d*]imidazol-1-yl)benzoate (BCD-CDK8-1-15E)
4-(6-(Azetidine-1-carbonyl)-7-fluoro-1*H*-benzo[*d*]imidazol-1-yl)benzoic acid (BCD-CDK8-1-15A)
4-(6-(Azetidine-1-carbonyl)-7-fluoro-1*H*-benzo[*d*]imidazol-1-yl)benzamide (BCD-CDK8-1-15)
(1-(4-(1-Methyl-1*H*-pyrazol-4-yl)phenyl)-1*H*-benzo[*d*]imidazol-6-yl)(piperidin-1-yl)methanone (BCD-CDK8-1-16)
(1-(1-(4-(1-Methyl-1*H*-pyrazol-4-yl)phenyl)-1*H*-benzo[*d*]imidazol-6-yl)azetidin-3-yl)(morpholino)methanone (BCD-CDK8-1-17)
Azetidin-1-yl(1-(4-(1-methyl-1*H*-pyrazol-4-yl)phenyl)-1*H*-benzo[*d*]imidazol-6-yl) methanone (BCD-CDK8-1-18)

(1-(1-(4-(1-methyl-1*H*-pyrazol-4-yl)phenyl)-4-Fluoro-1*H*-benzo[*d*]imidazol-6-yl) azetidin-3-yl)(morpholino)methanone (BCD-CDK8-1-19)

(1-(1-(4-(1-methyl-1*H*-pyrazol-4-yl)phenyl)-7-Fluoro-1*H*-benzo[*d*]imidazol-6-yl)azetidin-3 yl)(morpholino) ь methanone (BCD-CDK8-1-20)

4-(6-((2-(Chloromethyl)-3-oxo-3-(phenylamino)propyl)amino)-4-fluoro-1*H*-benzo[*d*]imidazol-1-yl)benzamide (BCD-CDK8-1-21A)

4-(6-((2-(Chloromethyl)-3-oxo-3-(phenylamino)propyl)amino)-4-fluoro-1*H*-benzo[*d*]imidazol-1-yl)benzoic acid (BCD-CDK8-1-21)

(S)-4-(3-(3-Hydroxypyrrolidine-1-carbonyl)-1,7-naphthyridin-5-yl)-1*H*-(methylsulfonyl)benzamide (BCD-CDK8-3-1)

(*R*)-(5-(4-(1*H*-Pyrazol-4-yl)phenyl)-1,7-naphthyridin-3-yl)(3-hydroxypyrrolidin-1-yl)methanone (BCD-CDK8-3-2)

(*R*)-(5-(4-(dimethylamino)phenyl)-1,7-naphthyridin-3-yl)(3-hydroxypyrrolidin-1-yl)methanone (BCD-CDK8-3-3)

(*R*)-(3-Hydroxypyrrolidin-1-yl)(5-(4-(1-methyl-1*H*-pyrazol-4-yl)phenyl)-1,7-naphthyridin-3-yl)methanone (BCD-CDK8-3-4)

(*R*)-(3-Hydroxypyrrolidin-1-yl)(5-(4-morpholinophenyl)-1,7-naphthyridin-3-yl) methanone (BCD-CDK8-3-5)

(*R*)-(3-hydroxypyrrolidin-1-yl)(5-(4-methoxyphenyl)-1,7-naphthyridin-3-yl) methanone (BCD-CDK8-3-6)

(5-(4-(Dimethylamino)phenyl)-1,7-naphthyridin-3-yl)(4-methylpiperazin-1-yl) methanone (BCD-CDK8-3-8)

(1-(5-(4-(1-Methyl-1*H*-pyrazol-4-yl)phenyl)-1,7-naphthyridin-3-yl)azetidin-3-yl) (morpholino)methanone (BCD-CDK8-3-9)

*N*-(Methylsulfonyl)-4-(3-(piperidine-1-carbonyl)-1,7-naphthyridin-5-yl)benzamide (BCD-CDK8-3-11)

*N*,*N*-Dimethyl-1-(5-(4-((methylsulfonyl)carbamoyl)phenyl)-1,7-naphthyridin-3-yl) azetidine-3-carboxamide (BCD-CDK8-3-12)

(*R*)-4-(3-(3-Hydroxypyrrolidine-1-carbonyl)-1,7-naphthyridin-5-yl)-*N*-(methylsulfonyl)benzamide (BCD-CDK8-3-13)

Azetidin-1-yl(5-(4-(1-methyl-1*H*-pyrazol-4-yl)phenyl)-1,7-naphthyridin-3-yl) methanone (BCD-CDK8-3-14)

(3-Methoxyazetidin-1-yl)(5-(4-(1-methyl-1*H*-pyrazol-4-yl)phenyl)-1,7-naphthyridin-3-yl)methanone (BCD-CDK8-3-15)

(5-(4-(1-Methyl-1*H*-pyrazol-4-yl)phenyl)-1,7-naphthyridin-3-yl)(pyrrolidin-1-yl) methanone (BCD-CDK8-3-16)

1-(5-(4-(1-Methyl-1*H*-pyrazol-4-yl)phenyl)-1,7-naphthyridin-3-yl)pyrrolidin-2-one (BCD-CDK8-3-17)

*N*-(2-Methoxyethyl)-5-(4-(1-methyl-1*H*-pyrazol-4-yl)phenyl)-1,7-naphthyridine-3-carboxamide (BCD-CDK8-3-18)

(*R*)-(3-Methoxypyrrolidin-1-yl)(5-(4-(1-methyl-1*H*-pyrazol-4-yl)phenyl)-1,7-naphthyridin-3-yl)methanone (BCD-CDK8-3-19)

(*R*)-3-(Hydroxypiperidin-1-yl)(5-(4-(1-methyl-1*H*-pyrazol-4-yl)pheny)-1,7-naphthyridin-3-yl)methanone (BCD-CDK8-3-20)

(*R*)-(3-Methoxypiperidin-1-yl)(5-(4-(1-methyl-1*H*-pyrazol-4-yl)phenyl)-1,7-naphthyridin-3-yl)methanone (BCD-CDK8-3-21)

*N*,*N*-Methyl-5-(4-(1-methyl-1*H*-pyrazol-4-yl)phenyl)-1,7-naphthyridine-3-carboxamide (BCD-CDK8-3-22)

*N*,*N*-Diethyl-5-(4-(1-methyl-1*H*-pyrazol-4-yl)phenyl)-1,7-naphthyridine-3-carboxamide (BCD-CDK8-3-23)

8-Amino-*N*-(2-methoxyethyl)-5-(4-(1-methyl-1*H*-pyrazol-4-yl)phenyl)-1,7-naphthyridine-3-carboxamide (BCD-CDK8-3-24)

*N*-(2-Methoxyethyl)-*N*-methyl-5-(4-(1-methyl-1*H*-pyrazol-4-yl)phenyl)-1,7-naphthyridine-3-carboxamide (BCD-CDK8-3-25)

(8-Amino-5-(4-(1-methyl-1*H*-pyrazol-4-yl)phenyl)-1,7-naphthyridin-3-yl) (azetidin-1-yl)methanone (BCD-CDK8-3-26)

(8-Amino-5-(4-(1-methyl-1*H*-pyrazol-4-yl)phenyl)-1,7-naphthyridin-3-yl) (morpholino)methanone (BCD-CDK8-3-27)

2-Methoxyethyl-5-[4-(1-methyl-1*H*-pyrazol-4-yl)phenyl]-1,7-naphthyridine-3-carboxylate (BCD-CDK8-3-28)

2-Methoxyethyl-8-amino-5-(4-(1-methyl-1*H*-pyrazol-4-yl)phenyl)-1,7-naphthyridine-3 -carboxylate (BCD-CDK8-3 -29)

(4-Methylpiperazin-1-yl)(5-(4-(1-methyl-1*H*-pyrazol-4-yl)phenyl)-1,7-naphthyridin-3-yl)methanone (BCD-CDK8-3-30)

(8-Amino-5-(4-(1-methyl-1*H*-pyrazol-4-yl)phenyl)-1,7-naphthyridin-3-yl)(4-methylpiperazin-1-yl)methanone (BCD-CDK8-3-31)

8-Amino-*N*-(2-methoxyethyl)-*N*-methyl-5-(4-(1-methyl-1*H*-pyrazol-4-yl)phenyl)-1,7-naphthyridine-3-carboxamide (BCD-CDK8-3-32)

(5-(4-(1-Methyl-1*H*-pyrazol-4-yl)phenyl)-1,7-naphthyridin-3-yl) (morpholino)methanone (BCD-CDK8-3-33)

(8-Amino-5-(4-(1-methyl-1*H*-pyrazol-4-yl)phenyl)-1,7-naphthyridin-3-yl)(3-methoxyazetidin-1-yl)methanone (BCD-CDK8-3-34)
*N*-(2-(Dimethylamino)ethyl)-*N*-methyl-5-(4-(1-methyl-1*H*-pyrazol-4-yl)phenyl)-1,7-naphthyridine-3-carboxamide (BCD-CDK8-3-35)
*N*-Cyclopropyl-5-(4-(1-methyl-1*H*-pyrazol-4-yl)phenyl)-1,7-naphthyridine-3-carboxamide (BCD-CDK8-3-37)
8-Amino-*N*-cyclopropyl-5-(4-(1-methyl-1*H*-pyrazol-4-yl)phenyl)-1,7-naphthyridine-3-carboxamide (BCD-CDK8-3-38)
*N*-(Cyclopropylmethyl)-5-(4-(1-methyl-1*H*-pyrazol-4-yl)phenyl)-1,7-naphthyridine-3-carboxamide (BCD-CDK8-3-39)
8-Amino-*N*-(cyclopropylmethyl)-5-(4-(1-methyl-1*H*-pyrazol-4-yl)phenyl)-1,7-naphthyridine-3-carboxamide (BCD-CDK8-3-40)
5-(4-(1*H*-Imidazol-1-yl)phenyl)-*N*-(2-methoxyethyl)-1,7-naphthyridine-3-yl) carboxamide (BCD-CDK8-3-41)
8-Amino-*N*-(2-hydroxyethyl)-5-(4-(1-methyl-1*H*-pyrazol-4-yl)phenyl)-1,7-naphthyridine-3-carboxamide (BCD-CDK8-3-42)
(5-(4-(Dimethylamino)-3-hydroxyphenyl)-1*H*-pyrrolo[2,3-*b*]pyridin-3-yl)(pyridin-2-yl)methanone (BCD-CDK8-4-1)
5-(Dimethylamino)-2-(3-picolinoyl-1*H*-pyrrolo[2,3-*b*]pyridin-5-yl)benzamide (BCD-CDK8-4-2)
(5-(3-Hydroxy-4-morpholinophenyl)-1*H*-pyrrolo[2,3-*b*]pyridin-3-yl)(pyridin-2-yl) methanone (BCD-CDK8-4-3)
5-Methoxy-2-(3-picolinoyl-1*H*-pyrrolo[2,3-*b*]pyridin-5-yl)benzamide (BCD-CDK8-4-4)
5-(4-(Dimethylamino)-3-hydroxyphenyl)-1*H*-pyrrolo[2,3-*b*]pyridin-3-yl)(pyridin-3-yl)methanone (BCD-CDK8-4-5)
5-(Dimethylamino)-2-(3-nicotinoyl-1*H*-pyrrolo[2,3-*b*]pyridin-5-yl)benzamide (BCD-CDK8-4-6)
(5-(3-Hydroxy-4-morpholinophenyl)-1*H*-pyrrolo[2,3-*b*]pyridin-3-yl)(pyridin-3-yl) methanone (BCD-CDK8-4-7)
5-Methoxy-2-(3-nicotinoyl-1*H*-pyrrolo[2,3-*b*]pyridin-5-yl)benzamide (BCD-CDK8-4-8)
(5-(4-(Dimethylamino)-3-hydroxyphenyl)-1*H*-pyrrolo[2,3-*b*]pyridin-3-yl)(pyridin-4-yl)methanone (BCD-CDK8-4-9)
5-(Dimethylamino)-2-(3-isonicotinoyl-1*H*-pyrrolo[2,3-*b*]pyridin-5-yl)benzamide (BCD-CDK8-4-10)
(5-(3-Hydroxy-4-morpholinophenyl)-1*H*-pyrrolo[2,3-*b*]pyridin-3-yl)(pyridin-4-yl) methanone (BCD-CDK8-4-11)
2-(3-Isonicotinoyl-1*H*-pyrrolo[2,3-*b*]pyridin-5-yl)-5-methoxybenzamide (BCD-CDK8-4-12)
5-Methoxy-2-(3-(3-methoxybenzoyl)-1*H*-pyrrolo[2,3-*b*]pyridin-5-yl)benzamide (BCD-CDK8-4-13)
(5-(4-(Dimethylamino)-3-methoxyphenyl)-1*H*-pyrrolo[2,3-*b*]pyridin-3-yl)(pyridin-2-yl)methanone (BCD-CDK8-4-21)
5-(Dimethylamino)-2-(3-picolinoyl-1*H*-pyrrolo[2,3-*b*]pyridin-5-yl)benzonitrile (BCD-CDK8-4-22)
(5-(3-Methoxy-4-morpholinophenyl)-1*H*-pyrrolo[2,3-*b*]pyridin-3-yl)(pyridin-2-yl) methanone (BCD-CDK8-4-23)
5-Methoxy-2-(3-picolinoyl-1*H*-pyrrolo[2,3-*b*]pyridin-5-yl)benzonitrile (BCD-CDK8-4-24)
(5-(4-(Dimethylamino)-3-methoxyphenyl)-1*H*-pyrrolo[2,3-*b*]pyridin-3-yl)(pyridin-3-yl)methanone (BCD-CDK8-4-25)
5-(Dimethylamino)-2-(3-nicotinoyl-1*H*-pyrrolo[2,3-*b*]pyridin-5-yl)benzonitrile (BCD-CDK8-4-26)
(5-(3-Methoxy-4-morpholinophenyl)-1*H*-pyrrolo[2,3-*b*]pyridin-3-yl)(pyridin-3-yl) methanone (BCD-CDK8-4-27)
5-Methoxy-2-(3-nicotinoyl-1*H*-pyrrolo[2,3-*b*]pyridin-5-yl)benzonitrile (BCD-CDK8-4-28)
(5-(4-(Dimethylamino)-3-methoxyphenyl)-1*H*-pyrrolo[2,3-*b*]pyridin-3-yl)(pyridin-2-yl)methanone (BCD-CDK8-4-29)
5-(Dimethylamino)-2-(3-isonicotinoyl-1*H*-pyrrolo[2,3-*b*]pyridin-5-yl)benzonitrile (BCD-CDK8-4-30)
(5-(3-Methoxy-4-morpholinophenyl)-1*H*-pyrrolo[2,3-*b*]pyridin-3-yl)(pyridin-4-yl) methanone (BCD-CDK8-4-31)
2-(3-Isonicotinoyl-1*H*-pyrrolo[2,3-*b*]pyridin-5-yl)-5-methoxybenzonitrile (BCD-CDK8-4-32)
(5-(4-Methoxyphenyl)-1*H*-pyrrolo[2,3-*b*]pyridin-3-yl)(pyridin-4-yl)methanone (BCD-CDK8-4-33)
(5-(4-(Dimethylamino)phenyl)-1*H*-pyrrolo[2,3-*b*]pyridin-3-yl)(3-methoxyphenyl) methanone (BCD-CDK8-4-34)
5-Methoxy-2-(3-(3-methoxybenzoyl)-1*H*-pyrrolo[2,3-*b*]pyridin-5-yl)benzonitrile (BCD-CDK8-4-35)
(5-(4-Morpholinophenyl)-1*H*-pyrrolo[2,3-*b*]pyridin-3-yl)(pyridin-2-yl)methanone (BCD-CDK8-4-36)
(5-(4-Morpholinophenyl)-1*H*-pyrrolo[2,3-*b*]pyridin-3-yl)(pyridin-3-yl)methanone (BCD-CDK8-4-37)
(5-(4-Morpholinophenyl)-1*H*-pyrrolo[2,3-*b*]pyridin-3-yl)(pyridin-4-yl)methanone (BCD-CDK8-4-38)
(5-(4-(Dimethylamino)phenyl)-1*H*-pyrrolo[2,3-*b*]pyridin-3-yl)(pyridin-3-yl) methanone (BCD-CDK8-4-39)
(5-(4-(Dimethylamino)phenyl)-1*H*-pyrrolo[2,3-*b*]pyridin-3-yl)(pyridin-4-yl) methanone (BCD-CDK8-4-40)
*N*-(Methylsulfonyl)-4-(4-(piperidine-1-carbonyl)quinolin-6-yl)benzamide (BCD-CDK8-5-1)
4-(4-(Piperidine-1-carbonyl)-2-Chloroquinolin-6-yl)-*N*-(methylsulfonyl)benzamide (BCD-CDK8-5-1Cl)
(R)-1 -((6-(4-(dimethylamino)phenyl)quinolin-4-yl)(imino)methyl)pyrrolidin-3 -ol (BCD-CDK8-5-2i)
(R)-(6-(4-(Dimethylamino)phenyl)quinolin-4-yl)(3-hydroxypyrrolidin-1-yl) methanone (BCD-CDK8-5-2)
Morpholino(6-(4-morpholinophenyl)quinolin-4-yl)methanimine (BCD-CDK8-5-3i) Morpholino(6-(4-morpholinophenyl)quinolin-4-yl)methanone (BCD-CDK8-5-3) (6-(4-(1*H*-Pyrazol-4-yl)phenyl)quinolin-4-yl)(piperidin-1-yl)methanimine (BCD-CDK8-5-4i)
(6-(4-(1*H*-Pyrazol-4-yl)phenyl)quinolin-4-yl)(piperidin-1-yl)methanone (BCD-CDK8-5-4)
4-(4-(Azetidine-1-carbonyl)quinolin-6-yl)-*N*-(methylsulfonyl)benzamide (BCD-CDK8-5-5)
4-(4-(Azetidine-1-carbonyl)-2-chloroquinolin-6-yl)-*N*-(methylsulfonyl)benzamide (BCD-CDK8-5-5Cl)
1-(6-(4-Methoxyphenyl)quinolin-4-yl)pyrrolidin-2-one (BCD-CDK8-5-6) (*S*)-1-((6-(4-(dimethylamino)phenyl)quinolin-4-yl)(imino)methyl)pyrrolidin-3-ol (BCD-CDK8-5-7i)
(*S*)-(6-(4-(Dimethylamino)phenyl)quinolin-4-yl)(3-hydroxypyrrolidin-1-yl) methanone (BCD-CDK8-5-7)
(6-(4-(1-Methyl-1*H*-pyrazol-4-yl)phenyl)quinolin-4-yl)(piperidin-1-yl) methanimine (BCD-CDK8-5-8i)
(6-(4-(1-Methyl-1*H*-pyrazol-4-yl)phenyl)quinolin-4-yl)(piperidin-1-yl)methanone (BCD-CDK8-5-8)
1-(4-(4-Morpholinophenyl)quinolin-6-yl)piperidine-4-carboxamide (BCD-CDK8-6-1)
(*S*)-4-(6-(3-Hydroxypyrrolidine-1-carbonyl)quinolin-4-yl)-*N*-(methylsulfonyl) benzamide (BCD-CDK8-6-2)
4-Methyl-1-(4-(4-((4-methylpiperazin-1-yl)methyl)phenyl)quinolin-6-yl)piperidine-4-carboxamide (BCD-CDK8-6-3)
(4-(4-(1*H*-Pyrazol-4-yl)phenyl)quinolin-6-yl)(3-methoxyazetidin-1-yl)methanone (BCD-CDK8-6-4)
4-(6-(Azetidine-1-carbonyl)quinolin-4-yl)-*N*-(methylsulfonyl)benzamide (BCD-CDK8-6-5)
*N*-(Methylsulfonyl)-4-(6-(morpholine-4-carbonyl)quinolin-4-yl)benzamide (BCD-CDK8-6-6)
1-(4-(4-((4-Methylpiperazin-1-yl)methyl)phenyl)quinolin-6-yl)piperidine-4-carboxamide (BCD-CDK8-6-7)
4-Methyl-1-(4-(4-morpholinophenyl)quinolin-6-yl)piperidine-4-carboxamide (BCD-CDK8-6-8)
(*R*)-4-(6-(3-Hydroxypyrrolidine-1-carbonyl)quinolin-4-yl)-*N*-(methylsulfonyl) benzamide (BCD-CDK8-6-9)
(3-Methoxyazetidin-1-yl)(4-(4-(1-methyl-1*H*-pyrazol-4-yl)phenyl)quinolin-6-yl) methanone (BCD-CDK8-6-10)
Azetidin-1-yl(4-(4-(1-methyl-1H-pyrazol-4-yl)phenyl)quinolin-6-yl)methanone (BCD-CDK8-6-11)
(4-(4-(1-Methyl-1*H*-pyrazol-4-yl)phenyl)quinolin-6-yl)(pyrrolidin-1-yl)methanone (BCD-CDK8-6-12)
1-(4-(4-(1-Methyl-1*H*-pyrazol-4-yl)phenyl)quinolin-6-yl)pyrrolidin-2-one (BCD-CDK8-6-13)
*N*-(2-Methoxyethyl)-4-(4-(1-methyl-1*H*-pyrazol-4-yl)phenyl)quinoline-6-carboxamide (BCD-CDK8-6-14)

**10.** A method for inhibiting biological activity of cyclin-dependent protein kinases CDK8/19 in a subject, comprising contacting the cyclin-dependent protein kinases CDK8/19 with the compound according to any of claims 1 to 9.

**11.** A pharmaceutical composition, comprising a therapeutically effective amount of the compound according to any of claims 1 to 9, or pharmaceutically acceptable salt thereof, and one or more pharmaceutically acceptable excipients, wherein said pharmaceutical composition being intended for preventing or treating a disease or disorder mediated by the activation of cyclin-dependent protein kinases CDK8/19.

**12.** The pharmaceutical composition according to claim 11 for the prevention or treatment of a disease or disorder mediated by the activation of cyclin-dependent protein kinases CDK8/19, wherein the disease or disorder mediated by the activation of cyclin-dependent protein kinases CDK8/19 is oncological or haemato-oncological.

**13.** The pharmaceutical composition according to claim 12, wherein oncological or haemato-oncological disease is selected from the group comprising colorectal cancer, melanoma, metastatic melanoma, breast cancer, triple-negative breast cancer (TNBC), prostate cancer, metastatic ovarian cancer, metastatic stomach cancer, leucosis, acute myeloid leukemia, pancreatic cancer.

**14.** A method for treating a disease or disorder mediated by the activation of cyclin-dependent protein kinases CDK8/19 comprising administering a therapeutically effective amount of the compound according to any of claims 1 to 9, or pharmaceutically acceptable salt thereof, or the pharmaceutical composition according to claim 11 in a subject in need thereof.

**15.** The method for treating according to claim 14, wherein the disease or disorder mediated by the activation of cyclin-dependent protein kinases CDK8/19 is oncological or haemato-oncological disease.

**16.** The method for treating according to claim 15, wherein oncological or haemato-oncological disease is selected from the group comprising colorectal cancer, melanoma, metastatic melanoma, breast cancer, triple-negative breast cancer (TNBC), prostate cancer, metastatic ovarian cancer, metastatic stomach cancer, leucosis, acute myeloid leukemia, pancreatic cancer.

**17.** Use of the compound according to any of claims 1 to 9 or pharmaceutically acceptable salt thereof, or a pharmaceutical composition according to claim 11 for the treatment of a disease or disorder mediated by the activation of cyclin-dependent protein kinases CDK8/19 in a subject in need thereof.

**18.** The use according to claim 17, wherein the disease or disorder mediated by the activation of cyclin-dependent protein kinases CDK8/19, comprising an oncological or haemato-oncological disease.

**19.** The use according to claim 18, wherein oncological and haemato-oncological disease is selected from the group comprising colorectal cancer, melanoma, metastatic melanoma, breast cancer, triple-negative breast cancer (TNBC), prostate cancer, metastatic ovarian cancer, metastatic stomach cancer, leucosis, acute myeloid leukemia, pancreatic cancer.

## INTERNATIONAL SEARCH REPORT

International application No.
PCT/RU 2018/050089

A. CLASSIFICATION OF SUBJECT MATTER
please see extra sheet

According to International Patent Classification (IPC) or to both national classification and IPC

B. FIELDS SEARCHED

Minimum documentation searched (classification system followed by classification symbols)
C07D, A61K,A61P

Documentation searched other than minimum documentation to the extent that such documents are included in the fields searched

Electronic data base consulted during the international search (name of data base and, where practicable, search terms used)
PatSearch (RUPTO internal), Espacenet, DWPI, PAJ, USPTO, CIPO, DEPATIS, EAPO

C. DOCUMENTS CONSIDERED TO BE RELEVANT

| Category* | Citation of document, with indication, where appropriate, of the relevant passages | Relevant to claim No. |
|---|---|---|
| X | WO 2017/094026 A1 (COUNCIL OF SCIENTIFIC & INDUSTRIAL RESEARCH) 08.06.2017, compounds 1S2, 1S5, 1S6, 1S7, 1S10, 1S11, 1S12, 1S15 p.45-49, [0001], [0002], the claims, the abstract | 1-19 |
| X | WO 2017/100201 A1 (PLEXXICON INC.) 15.06.2017, compounds R-0002, R-0003, R-0045, R-0050, R-0064, R-0065, R-0066, R-0076 table 1, p.3-4 of the description | 1-19 |
| X | WO 2015/014768 A1 (F.HOFFMANN-LA ROCHE INC.) 05.02.2015, examples 1-7,9,11-12,16, 20,-22, 24, 27-28 | 1-3,5-8 |
| X | US 2007/0049615 A1 (PLEXXICON INC.) 01.03.2007, compounds 293, 294 p.69; 300 p.71; 26, 28 p.73; 336, 339 p.78; p.84 1-2 compounds from above; p. 87 1 compound from above, 2 compound from below; p.88 2 compound from above; p.92 2-4 and the last compounds from above; p.93 1,3,5 compounds from above; p.95 1,2,4 compounds from above; p.96 1-2 compounds from above; p.102 the last compound; p.103 2-4 compounds from above; p.104 3, 5-6 compounds from above; p.105 the last compound; p.106 3 compound from above | 1-3,5-8 |
| X | US 2010/0069381 A1 (TAKEDA PHARMACEUTICAL CO LTD) 18.03.2010, examples 37-38 | 1-3,5-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 November 2018 (19.11.2018) | 22 November 2018 (22.11.2018) |
| Name and mailing address of the ISA/ RU | Authorized officer |
| Facsimile No. | Telephone No. |

Form PCT/ISA/210 (second sheet) (April 2005)

**INTERNATIONAL SEARCH REPORT**

International application No.
PCT/RU 2018/050089

C (Continuation). DOCUMENTS CONSIDERED TO BE RELEVANT

| Category* | Citation of document, with indication, where appropriate, of the relevant passages | Relevant to claim No. |
|---|---|---|
| X | WO 2012/174312 A2 (GLAXOSMITHKLINE LLC) 20.12.2012, intermediate compound p.288 paragraph [00404] | 1-3,5-8 |
| X | WO 2017/040993 A1 (THE ARIZONA BOARD OF REGENTS ON BEHALF OF THE UNIVERSITY OF ARIZONA) 09.03.2017, compounds 1-5, 7, 14-17, 20-21, 23, 27, 32, 35, 49-51, 55-58; compounds 1-505 i 1-506 p.34-35; the claims | 1-19 |

Form PCT/ISA/210 (continuation of second sheet) (April 2005)

INTERNATIONAL SEARCH REPORT
CLASSIFICATION OF SUBJECT MATTER

International application No.
PCT/RU 2018/050089

***C07D 401/14*** *(2006.01)*
***C07D 403/04*** *(2006.01)*
***C07D 471/04*** *(2006.01)*
***C07D 403/12*** *(2006.01)*
***A61K 31/4184*** *(2006.01)*
***A61K 31/437*** *(2006.01)*
***A61K 31/4709*** *(2006.01)*
***A61K 31/4375*** *(2006.01)*
***A61P 35/00*** *(2006.01)*

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2000071518 A
- WO 2011130146 A
- WO 2003045315 A
- WO 2014029983 A
- WO 2015014768 A
- WO 2008079277 A


### Non-patent literature cited in the description


- **XU, W. ; JI, J. Y.** Dysregulation of CDK8 and Cyclin C in tumorigenesis. *J. Genet. Genomics,* 2011, vol. 38, 439-452
- **GALBRAITH, M. D. et al.** CDK8: a positive regulator of transcription. *Transcription,* 2010, vol. 1, 4-12
- **FIRESTEIN, R. ; HAHN, W. C.** Revving the Throttle on an oncogene: CDK8 takes the driver seat. *Cancer Res,* 2009, vol. 69, 7899-7901
- **ADLER, A. S. et al.** CDK8 maintains tumor de-differentiation and embryonic stem cell pluripotency. *Cancer Res.,* 2012, vol. 72, 2129-2139
- **FIRESTEIN, R. et al.** CDK8 is a colorectal cancer oncogene that regulates beta-catenin activity. *Nature,* 2008, vol. 455, 547-551
- **KAPOOR, A. et al.** The histone variant macroH2A suppresses melanoma progression through regulation of CDK8. *Nature,* 2010, vol. 468, 1105-1109
- **FIRESTEIN, R. et al.** CDK8 expression in 470 colorectal cancers in relation to beta-catenin activation, other molecular alterations and patient survival. *Int. J. Cancer,* 2010, vol. 126, 2863-2873
- **BROUDE E. et al.** Expression of CDK8 and CDK8-interacting genes as potential biomarkers in breast cancer. *Curr. Cancer Drug Targets,* 2015, vol. 15 (8), 739-749
- **GYORFFY, B. et al.** An online survival analysis tool to rapidly assess the effect of 22,277 genes on breast cancer prognosis using microarray data of 1,809 patients. *Breast Cancer Res. Treat.,* 2010, vol. 123, 725-731
- **ALARCON, C et al.** Nuclear CDKs drive Smad transcriptional activation and turnover in BMP and TGF-beta pathways. *Cell,* 2009, vol. 139, 757-769
- **DIDONATO, J. A. et al.** NF-kappaB and the link between inflammation and cancer. *Immunol. Rev.,* 2012, vol. 246, 379-400
- **ACHARYYA, S. et al.** A CXCL1 paracrine network links cancer chemoresistance and metastasis. *Cell,* 2012, vol. 150, 165-178
- **FABIAN et al.** A small molecule-kinase interaction map for clinical kinase inhibitors. *Nat. Biotechnol.,* 2005, vol. 23, 329-336
- **HUANG et al.** MED12 Controls the response to multiple cancer drugs through regulation of TGF-$\beta$ receptor signaling. *Cell,* 2012, vol. 151, 937-950
- **BERGE S.M. et al.** Pharmaceutical Salts. *J. Pharm. Sci.,* 1977, vol. 66, 1-19
- *Journal of Medicinal Chemistry,* 2010, vol. 53 (9), 3645-3674
- *Bioorganic and Medicinal Chemistry Letters,* 2009, vol. 19 (16), 4639-4642
- *Bioorganic & Medicinal Chemistry Letters,* 2009, vol. 19 (16), 4639-4642
- *Bioorganic & Medicinal Chemistry Letters,* 2014, vol. 24 (3), 790-793
- *Angewandte Chemie International Edition,* 2016, vol. 55 (23), 6776-6779